# EUROPEAN PATENT APPLICATION

(11) **EP 3 318 567 A1**
(43) Date of publication of application: **09.05.2018**
(21) Application number: 16818011.5
(22) Date of filing: 30.06.2016
(51) Int. Cl.: C07D 501/14, A61K 31/546, A61P 31/04

(54) **TRICYCLIC COMPOUND HAVING SULFINYL OR SULFONYL**

(30) Priority: 30.06.2015 JP 2015130623
(71) Applicant: Shionogi & Co., Ltd, Osaka-shi, Osaka 541-0045 (JP)
(72) Inventor: YAMAWAKI, Kenji, Toyonaka-shi, Osaka 561-0825 (JP); KUSANO, Hiroki, Toyonaka-shi, Osaka 561-0825 (JP)
(74) Representative: Vossius & Partner Patentanwälte Rechtsanwälte mbB
(86) International application number: PCT/JP2016/069404
(87) International publication number: WO 2017/002903

(57) **Abstract**

It is intended to provide a compound that exhibits a strong antibacterial spectrum against various bacteria including gram-negative bacteria, or a pharmaceutical composition having an antibacterial activity against carbapenem-resistant bacteria.

The present invention provides a compound represented by formula (I): 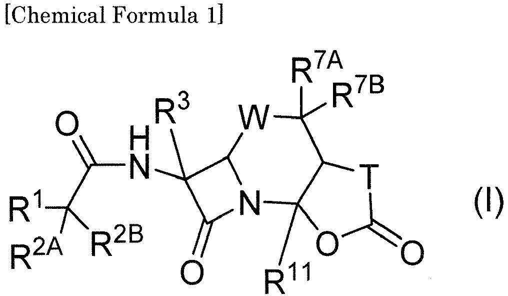 wherein -W- is -S(=O)- or -S(=O)₂-, -T- is -CR^{4A}R^{4B}- or -CR^{5A}R^{5B}-CR^{6A}R^{6B}-, R^{4A}, R^{4B}, R^{5A}, R^{5B}, R^{6A} and R^{6B} are each independently a hydrogen atom or the like, R¹ is substituted or unsubstituted heterocyclyl or the like, R^{2A} and R^{2B} are each independently a hydrogen atom or the like, or R^{2A} and R^{2B} are taken together to form substituted or unsubstituted methylidene or the like, and R³ is a hydrogen atom or the like, R¹¹ is carboxy or the like, R^{7A} and R^{7b} are each independently a hydrogen atom or the like, its ester form or a pharmaceutically acceptable salt thereof, or a hydrate thereof.

## Description

### [TECHNICAL FIELD]

The present invention relates to a novel tricyclic compound having an antibacterial activity, its ester form or a pharmaceutically acceptable salt thereof, or a hydrate thereof.

### [BACKGROUND ART]

Various β-lactam agents have been developed so far. Such β-lactam agents serve as clinically very important antibacterial agents. However, there are an increasing number of bacterial species that have acquired resistance to the β-lactam agents by producing β-lactamases, which degrade the β-lactam agents.

According to the molecular classification system of Ambler, the β-lactamases are broadly divided into four classes: class A (TEM type, SHV type, CTX-M type, KPC type etc.), class B (NDM type, IMP type, VIM type, L-1 type etc.), class C (AmpC type, ADC type etc.), and class D (OXA type etc.). Among them, the β-lactamases of classes A, C, and D are broadly divided into serine-β-lactamases, while the β-lactamases of class B are broadly divided into metallo-β-lactamases. These β-lactamases are known to hydrolyze the β-lactam agents by their respective different mechanisms.

In recent years, the presence of gram-negative bacteria highly resistant to many β-lactam agents including cephem and carbapenem by the production of serine-β-lactamases including extended-spectrum β-lactamases of class A (ESBLs) or class D as well as *Klebsiella pneumoniae* carbapenemase (KPC), and metallo-β-lactamases of class B has been a clinical issue. Particularly, bacteria of the family *Enterobacteriaceae* producing KPC or metallo-β-lactamases are known to be highly resistant to carbapenem antibacterial agents, which are regarded as being important for the treatment of infection by gram-negative bacteria.

Patent Documents 2 and 3 report β-lactam compounds having a novel skeleton, but do not describe an antibacterial activity against the above carbapenem-resistant bacteria and the like, which have been in question in recent years. From the antibacterial activity described therein, it cannot be predictable that a compound group having the skeleton of the present invention has an antibacterial activity against carbapenem-resistant bacteria.

### [PRIOR ART REFERENCES]

### [Patent Document]

[Patent Document 1] International Publication WO 2007/119511A
[Patent Document 2] European Patent Application Publication No. 0253337
[Patent Document 3] European Patent Application Publication No. 0249909

### [SUMMARY OF THE INVENTION]

### [PROBLEMS TO BE SOLVED BY THE INVENTION]

The present invention provides a tricyclic compound, which contains a 5-oxotetrahydrofuran ring or 6-oxotetrahydropyran ring and has sulfinyl or sulfonyl, exhibiting a strong antibacterial spectrum against various bacteria including gram-negative bacteria. The present invention further provides a pharmaceutical composition having an antibacterial activity against carbapenem-resistant bacteria, comprising a tricyclic compound that contains a 5-oxotetrahydrofuran ring or a 6-oxotetrahydropyran ring and has sulfinyl or sulfonyl, its ester form or a pharmaceutically acceptable salt thereof, or a hydrate thereof. Preferably, the present invention provides a compound exhibiting a strong antibacterial activity against β-lactamase-producing gram-negative bacteria, and a pharmaceutical composition comprising the compound. More preferably, the present invention provides a compound exhibiting a strong antibacterial activity against *Klebsiella pneumoniae* carbapenemase (KPC)-producing bacteria and/or class B metallo-β-lactamase (MBL)-producing gram-negative bacteria, and a pharmaceutical composition comprising the compound. Further preferably, the present invention provides a compound exhibiting an antibacterial activity effective even for gram-negative bacteria producing the above β-lactamases as well as extended-spectrum β-lactamases (ESBLs) and/or class C β-lactamases, and a compound comprising the compound.

### [MEANS FOR SOLVING THE PROBLEM]

The present invention provides a compound that solves the above problems by having at least the following structural features or a pharmaceutical composition having an antibacterial activity against carbapenem-resistant bacteria:
1) having a tricyclic core containing a 6-membered ring having sulfinyl or sulfonyl,
2) having a tricyclic core containing a 5-oxotetrahydrofuran ring or a 6-oxotetrahydropyran ring, and
3) having an amide substituent (carbonylamino group having a substituent) on a lactam ring in the tricyclic core.

### (Item 1)

A compound represented by formula (I): 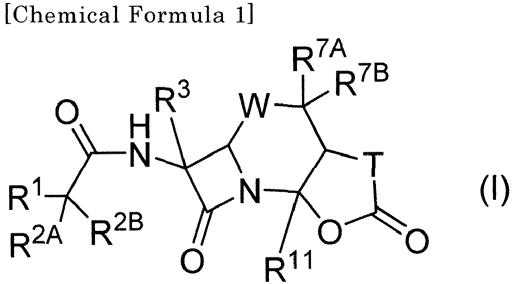 wherein
-W- is -S(=O)- or -S(=O)₂-;
-T- is -CR^{4A}R^{4B}- or -CR^{5A}R^{5B}-CR^{6A}R^{6B}-;
R^{4A}, R^{4B}, R^{5A}, R^{5B}, R^{6A} and R^{6B} are each independently a hydrogen atom, halogen, hydroxy, carboxy, acyl, acyloxy, sulfanyl, sulfo, phospho, cyano, nitro, ureido, amidino, guanidino, substituted or unsubstituted amino, substituted or unsubstituted carbamoyl, substituted or unsubstituted sulfamoyl, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylsulfonyl, substituted or unsubstituted alkenylsulfonyl, substituted or unsubstituted alkynylsulfonyl, substituted or unsubstituted alkyloxycarbonyl, substituted or unsubstituted alkenyloxycarbonyl, substituted or unsubstituted alkynyloxycarbonyl, substituted or unsubstituted alkylsulfanyl, substituted or unsubstituted alkenylsulfanyl, substituted or unsubstituted alkynylsulfanyl, substituted or unsubstituted alkylsulfinyl, substituted or unsubstituted alkenylsulfinyl, substituted or unsubstituted alkynylsulfinyl, substituted or unsubstituted non-aromatic carbocyclyl, substituted or unsubstituted non-aromatic heterocyclyl, substituted or unsubstituted aromatic carbocyclyl, substituted or unsubstituted aromatic heterocyclyl, substituted or unsubstituted non-aromatic carbocyclyloxy, substituted or unsubstituted non-aromatic heterocyclyloxy, substituted or unsubstituted aromatic carbocyclyloxy, substituted or unsubstituted aromatic heterocyclyloxy, substituted or unsubstituted non-aromatic carbocyclyloxycarbonyl, substituted or unsubstituted non-aromatic heterocyclyloxycarbonyl, substituted or unsubstituted aromatic carbocyclyloxycarbonyl, substituted or unsubstituted aromatic heterocyclyloxycarbonyl, substituted or unsubstituted non-aromatic carbocyclylsulfanyl, substituted or unsubstituted non-aromatic heterocyclylsulfanyl, substituted or unsubstituted aromatic carbocyclylsulfanyl, substituted or unsubstituted aromatic heterocyclylsulfanyl, substituted or unsubstituted non-aromatic carbocyclylsulfinyl, substituted or unsubstituted non-aromatic heterocyclylsulfinyl, substituted or unsubstituted aromatic carbocyclylsulfinyl, substituted or unsubstituted aromatic heterocyclylsulfinyl, substituted or unsubstituted non-aromatic carbocyclylsulfonyl, substituted or unsubstituted non-aromatic heterocyclylsulfonyl, substituted or unsubstituted aromatic carbocyclylsulfonyl, or substituted or unsubstituted aromatic heterocyclylsulfonyl; R¹ is substituted or unsubstituted carbocyclyl or substituted or unsubstituted heterocyclyl;
as for R^{2A} and R^{2B},
   a) R^{2A} and R^{2B} are each independently a hydrogen atom, substituted or unsubstituted amino, sulfo, substituted or unsubstituted sulfamoyl, carboxy, substituted or unsubstituted alkyloxycarbonyl, substituted or unsubstituted carbamoyl, hydroxy, or carbonyloxy having a substituent, or
   b) R^{2A} and R^{2B} are taken together to form substituted or unsubstituted methylidene, or substituted or unsubstituted hydroxyimino;
   R³ is a hydrogen atom, -OCH₃ or -NH-CH(=O);
R¹¹ is carboxy or tetrazolyl; and
R^{7A} and R^{7B} are each independently a hydrogen atom or substituted or unsubstituted alkyl,
its ester form or a pharmaceutically acceptable salt thereof, or a hydrate thereof.

### (Item 2)

The compound according to item 1, wherein R¹ is substituted or unsubstituted aromatic heterocyclyl, its ester form or a pharmaceutically acceptable salt thereof, or a hydrate thereof.

### (Item 3)

The compound according to item 1, wherein R¹ is a group represented by the following formula: 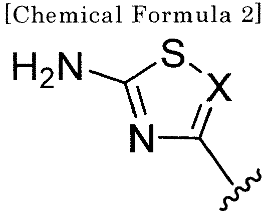 wherein X is CH, CCl, CF, or N,
its ester form or a pharmaceutically acceptable salt thereof, or a hydrate thereof.

### (Item 4)

The compound according to any one of items 1 to 3, wherein R^{2A} and R^{2B} are taken together to be methylidene having a substituent of the formulas of: 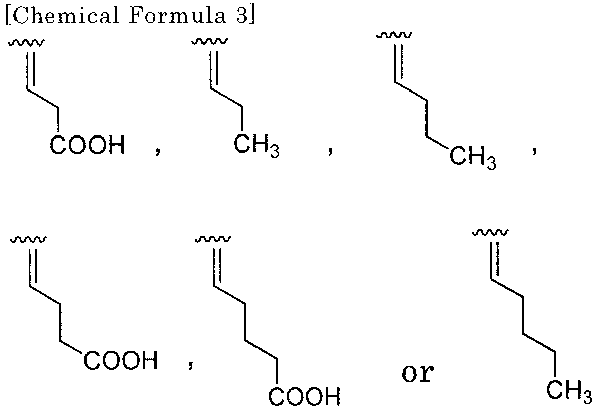 or substituted or unsubstituted hydroxyimino of the formula of: 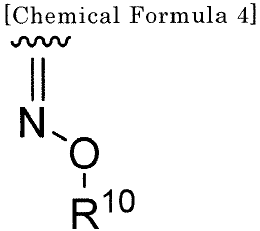 wherein R¹⁰ is a hydrogen atom or substituted or unsubstituted alkyl, its ester form or a pharmaceutically acceptable salt thereof, or a hydrate thereof.

### (Item 5)

The compound according to any one of items 1 to 3, wherein R^{2A} and R^{2B} are taken together to be a group represented by the following formula: 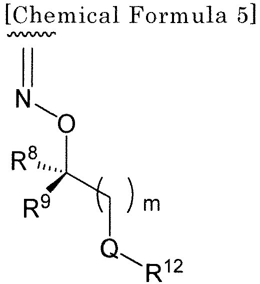 wherein R⁸ and R⁹ are each independently a hydrogen atom, halogen, hydroxy, carboxy, substituted or unsubstituted alkyl, substituted or unsubstituted carbocyclyl, or substituted or unsubstituted heterocyclyl, R⁸ and R⁹ are taken together to form substituted or unsubstituted methylidene, or R⁸ and R⁹ are taken together with the adjacent atoms to form substituted or unsubstituted non-aromatic carbocycle or substituted or unsubstituted non-aromatic heterocycle; Q is a single bond, substituted or unsubstituted carbocyclediyl or substituted or unsubstituted heterocyclediyl; m is an integer from 0 to 3; and R¹² is a hydrogen atom, halogen, alkyl, haloalkyl, alkyloxy, carboxy, hydroxy, amino, sulfo, phospho, borono, cyano, hydroxyiminomethyl, carbamoyl, alkyloxycarbonylamino, alkyloxycarbamoyl, hydroxycarbamoyl, ureido, alkylsulfonylamino, sulfamoyl, sulfamoylamino, alkylsulfonylcarbamoylamino, alkylsulfamoylcarbamoyl, substituted or unsubstituted carbocyclyl, substituted or unsubstituted heterocyclyl, or a substituted or unsubstituted quaternary ammonium group,
its ester form or a pharmaceutically acceptable salt thereof, or a hydrate thereof.

### (Item 6)

The compound according to any one of items 1 to 5, wherein R³ is a hydrogen atom, its ester form or a pharmaceutically acceptable salt thereof, or a hydrate thereof.

### (Item 7)

The compound according to any one of items 1 to 6, wherein -T- is -CR^{4A}R^{4B}-, and R^{4A} and R^{4B} are each independently a hydrogen atom or substituted or unsubstituted alkyl, its ester form or a pharmaceutically acceptable salt thereof, or a hydrate thereof.

### (Item 8)

The compound according to any one of items 1 to 7, wherein -W- is -S(=O)-, its ester form or a pharmaceutically acceptable salt thereof, or a hydrate thereof.

### (Item 9)

The compound according to any one of items 1 to 7, wherein -W- is represented by the following formula: 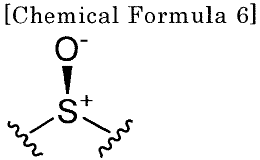 its ester form or a pharmaceutically acceptable salt thereof, or a hydrate thereof.

### (Item 10)

The compound according to any one of items 1 to 7, wherein -W- is represented by the following formula: 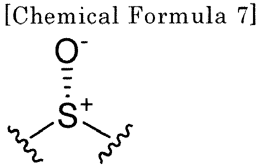 its ester form or a pharmaceutically acceptable salt thereof, or a hydrate thereof.

### (Item 11)

The compound according to any one of items 1 to 7, wherein -W- is -S(=O)₂-, its ester form or a pharmaceutically acceptable salt thereof, or a hydrate thereof.

### (Item 12)

The compound according to any one of items 1 to 11, wherein R¹¹ is carboxy, its ester form or a pharmaceutically acceptable salt thereof, or a hydrate thereof.

### (Item 13)

The compound according to any one of items 1 to 12, wherein both of R^{7A} and R^{7B} are hydrogen atoms, or R^{7A} is alkyl, and R^{7B} is a hydrogen atom, its ester form or a pharmaceutically acceptable salt thereof, or a hydrate thereof.

### (Item 14)

The compound according to item 1, wherein the formula (I) is the following formula: 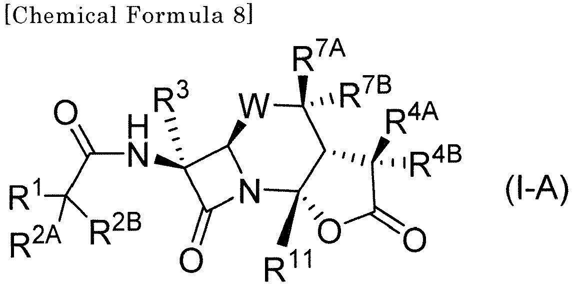 wherein -W- is represented by the following formula: 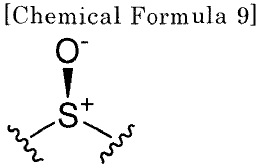 R^{4A} is a hydrogen atom; R^{4B} is a hydrogen atom or methyl;
R¹ is represented by the following formula: 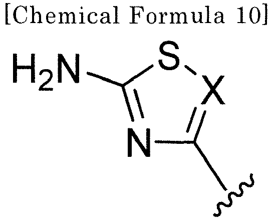 wherein X is CH;
R^{7A} is a hydrogen atom or alkyl; R^{7B} is a hydrogen atom;
R^{2A} and R^{2B} are taken together to be hydroxyimino or a group of the following formula: 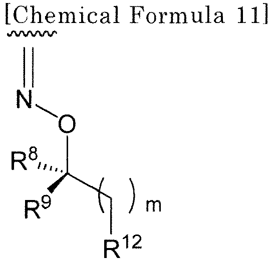 wherein R⁸ and R⁹ are each independently a hydrogen atom, halogen, hydroxy, carboxy, alkyl, or hydroxyalkyl, or R⁸ and R⁹ are taken together with the adjacent carbon atom to form substituted or unsubstituted non-aromatic carbocycle or substituted or unsubstituted non-aromatic heterocycle; m is an integer from 0 to 3; and R¹² is a hydrogen atom, carboxy, sulfo, carbamoyl, hydroxycarbamoyl, alkyloxycarbamoyl, or hydroxy;
R³ is a hydrogen atom; and
R¹¹ is carboxy,
its ester form or a pharmaceutically acceptable salt thereof, or a hydrate thereof.

### (Item 15)

The compound according to item 1, which is any one of the compounds 1-001, I-005, 1-007, 1-009, 1-015, I-023, I-024, 1-026, I-027, I-047, I-048, I-049, I-052, I-053, I-059, I-060, and 1-061, its ester form or a pharmaceutically acceptable salt thereof, or a hydrate thereof.

### (Item 16)

A pharmaceutical composition comprising the compound according to any one of items 1 to 15, its ester form or a pharmaceutically acceptable salt thereof, or a hydrate thereof.

### (Item 17)

The pharmaceutical composition according to item 16, which has an antibacterial activity.

### (Item 18)

A method for treating or preventing diseases related to bacterial infections, which comprises administering the compound according to any one of items 1 to 15, its ester form or a pharmaceutically acceptable salt thereof, or a hydrate thereof.

### (Item 19)

The compound according to any one of items 1 to 15, its ester form or a pharmaceutically acceptable salt thereof, or a hydrate thereof for treating or preventing diseases related to bacterial infections.

### (Item 1A)

A compound represented by formula (I'): 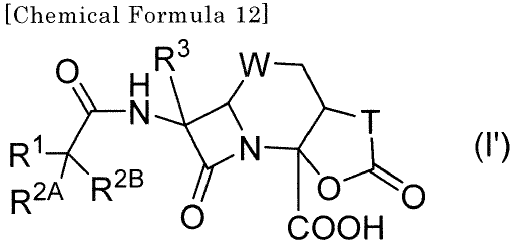 wherein
-W- is -S(=O)- or -S(=O)₂-;
-T- is -CR^{4A}R^{4B}- or -CR^{5A}R^{5B}-CR^{6A}R^{6B}-;
R^{4A}, R^{4B}, R^{5A}, R^{5B}, R^{6A} and R^{6B} are each independently a hydrogen atom, halogen, hydroxy, carboxy, acyl, acyloxy, sulfanyl, sulfo, phospho, cyano, nitro, ureido, amidino, guanidino, substituted or unsubstituted amino, substituted or unsubstituted carbamoyl, substituted or unsubstituted sulfamoyl, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylsulfonyl, substituted or unsubstituted alkenylsulfonyl, substituted or unsubstituted alkynylsulfonyl, substituted or unsubstituted alkyloxycarbonyl, substituted or unsubstituted alkenyloxycarbonyl, substituted or unsubstituted alkynyloxycarbonyl, substituted or unsubstituted alkylsulfanyl, substituted or unsubstituted alkenylsulfanyl, substituted or unsubstituted alkynylsulfanyl, substituted or unsubstituted alkylsulfinyl, substituted or unsubstituted alkenylsulfinyl, substituted or unsubstituted alkynylsulfinyl, substituted or unsubstituted non-aromatic carbocyclyl, substituted or unsubstituted non-aromatic heterocyclyl, substituted or unsubstituted aromatic carbocyclyl, substituted or unsubstituted aromatic heterocyclyl, substituted or unsubstituted non-aromatic carbocyclyloxy, substituted or unsubstituted non-aromatic heterocyclyloxy, substituted or unsubstituted aromatic carbocyclyloxy, substituted or unsubstituted aromatic heterocyclyloxy, substituted or unsubstituted non-aromatic carbocyclyloxycarbonyl, substituted or unsubstituted non-aromatic heterocyclyloxycarbonyl, substituted or unsubstituted aromatic carbocyclyloxycarbonyl, substituted or unsubstituted aromatic heterocyclyloxycarbonyl, substituted or unsubstituted non-aromatic carbocyclylsulfanyl, substituted or unsubstituted non-aromatic heterocyclylsulfanyl, substituted or unsubstituted aromatic carbocyclylsulfanyl, substituted or unsubstituted aromatic heterocyclylsulfanyl, substituted or unsubstituted non-aromatic carbocyclylsulfinyl, substituted or unsubstituted non-aromatic heterocyclylsulfinyl, substituted or unsubstituted aromatic carbocyclylsulfinyl, substituted or unsubstituted aromatic heterocyclylsulfinyl, substituted or unsubstituted non-aromatic carbocyclylsulfonyl, substituted or unsubstituted non-aromatic heterocyclylsulfonyl, substituted or unsubstituted aromatic carbocyclylsulfonyl, or substituted or unsubstituted aromatic heterocyclylsulfonyl; R¹ is substituted or unsubstituted carbocyclyl or substituted or unsubstituted heterocyclyl;
as for R^{2A} and R^{2B},
   a) R^{2A} and R^{2B} are each independently a hydrogen atom, substituted or unsubstituted amino, sulfo, substituted or unsubstituted sulfamoyl, carboxy, substituted or unsubstituted alkyloxycarbonyl, substituted or unsubstituted carbamoyl, hydroxy, or carbonyloxy having a substituent, or
   b) R^{2A} and R^{2B} are taken together to form substituted or unsubstituted methylidene, substituted or unsubstituted hydroxyimino; and
R³ is a hydrogen atom, -OCH₃ or -NH-CH(=O);
its ester form or a pharmaceutically acceptable salt thereof, or a hydrate thereof.

### (Item 2A)

The compound according to item 1A, wherein R¹ is substituted or unsubstituted aromatic carbocyclyl or substituted or unsubstituted aromatic heterocyclyl, its ester form or a pharmaceutically acceptable salt thereof, or a hydrate thereof.

### (Item 3A)

The compound according to item 1A, wherein R¹ is a group represented by the following formulas: 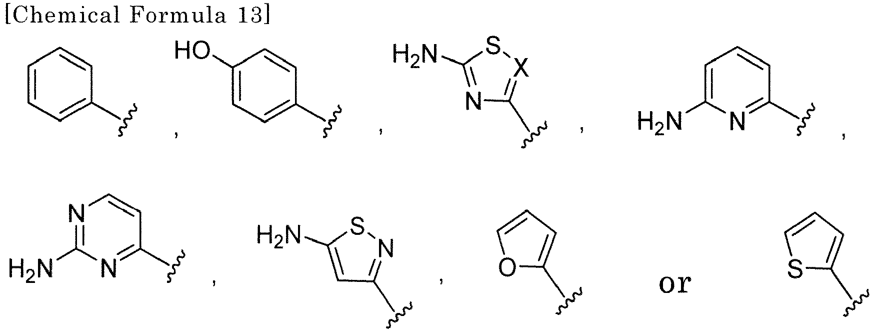 wherein X is CH, CCl, CF, CBr, or N, its ester form or a pharmaceutically acceptable salt thereof, or a hydrate thereof.

### (Item 4A)

The compound according to any one of items 1A to 3A, wherein R^{2A} and R^{2B} are taken together to be methylidene having a substituent of the formulas of: 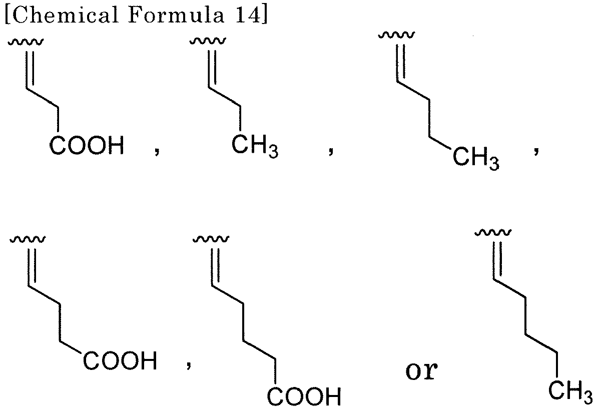 or substituted or unsubstituted hydroxyimino of the formula of: 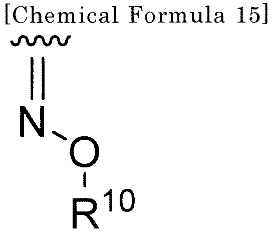 wherein R¹⁰ is a hydrogen atom, substituted or unsubstituted carbocyclyl, substituted or unsubstituted heterocyclyl or substituted or unsubstituted alkyl, its ester form or a pharmaceutically acceptable salt thereof, or a hydrate thereof.

### (Item 5A)

The compound according to any one of items 1A to 3A, wherein R^{2A} and R^{2B} are taken together to be a group represented by the following formula: 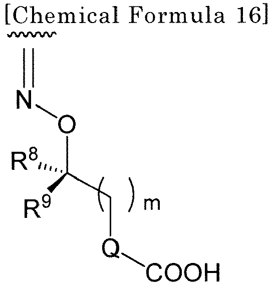 wherein R⁸ and R⁹ are each independently a hydrogen atom, halogen, hydroxy, carboxy, substituted or unsubstituted alkyl, substituted or unsubstituted carbocyclyl, or substituted or unsubstituted heterocyclyl, R⁸ and R⁹ are taken together to form substituted or unsubstituted methylidene, or R⁸ and R⁹ are taken together with the adjacent atoms to form substituted or unsubstituted non-aromatic carbocycle or substituted or unsubstituted non-aromatic heterocycle; Q is a single bond, substituted or unsubstituted carbocyclediyl or substituted or unsubstituted heterocyclediyl; and m is an integer from 0 to 3,
its ester form or a pharmaceutically acceptable salt thereof, or a hydrate thereof.

### (Item 6A)

The compound according to any one of items 1A to 5A, wherein R³ is a hydrogen atom or -OCH₃, its ester form or a pharmaceutically acceptable salt thereof, or a hydrate thereof.

### (Item 7A)

The compound according to any one of items 1A to 6A, wherein -T- is -CR^{4A}R^{4B}-, its ester form or a pharmaceutically acceptable salt thereof, or a hydrate thereof.

### (Item 8A)

The compound according to any one of items 1A to 7A, wherein -W- is -S(=O)-, its ester form or a pharmaceutically acceptable salt thereof, or a hydrate thereof.

### (Item 9A)

The compound according to any one of items 1A to 7A, wherein -W- is represented by the following formula: 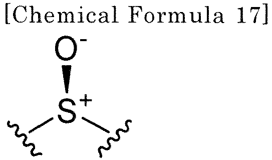 its ester form or a pharmaceutically acceptable salt thereof, or a hydrate thereof.

### (Item 10A)

The compound according to any one of items 1A to 7A, wherein -W- is represented by the following formula: 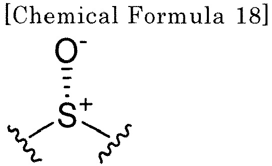 its ester form or a pharmaceutically acceptable salt thereof, or a hydrate thereof.

### (Item 11A)

The compound according to any one of items 1A to 7A, wherein -W- is -S(=O)₂-, its ester form or a pharmaceutically acceptable salt thereof, or a hydrate thereof.

### (Item 12A)

A pharmaceutical composition comprising the compound according to any one of items 1A to 11A. its ester form or a pharmaceutically acceptable salt thereof, or a hydrate thereof.

### (Item 13A)

The pharmaceutical composition according to item 12A, which has an antibacterial activity.

### (Item 14A)

A method for treating or preventing infectious diseases, which comprises administering the compound according to any one of items 1A to 11A, its ester form or a pharmaceutically acceptable salt thereof, or a hydrate thereof.

### (Item 15A)

The compound according to any one of items 1A to 11A, its ester form or a pharmaceutically acceptable salt thereof, or a hydrate thereof for treating or preventing infectious diseases.

### (Item 16A)

Use of the compound according to any one of items 1A to 11A, its ester form or a pharmaceutically acceptable salt thereof, or a hydrate thereof for manufacturing an infectious therapeautic agent or infectious preventive agent.

### (Item 17A)

A pharmaceutical composition comprising the compound according to any one of items 1A to 11A, its ester form or a pharmaceutically acceptable salt thereof, or a hydrate thereof, for oral administration.

### (Item 18A)

The pharmaceutical composition according to item 17A, which is a tablet, powder, granule, capsule, pill, film, suspension, emulsion, elixir, syrup, lemonade, spirit, aromatic water, extract, decoction or tincture.

### (Item 19A)

The pharmaceutical composition according to item 18A, which is a sugar-coated tablet, film-coated tablet, enteric-coated tablet, sustained-release tablet, troche tablet, sublingual tablet, buccal tablet, chewable tablet, orally dispersing tablet, dry syrup, soft capsule, micro capsule or sustained-release capsule.

### (Item 20A)

A pharmaceutical composition comprising the compound according to any one of items 1A to 11A, its ester form or a pharmaceutically acceptable salt thereof, or a hydrate thereof, for parenteral administration.

### (Item 21A)

The pharmaceutical composition according to item 20A, for dermal, subcutaneous, intravenous, intraarterial, intramuscular, intraperitoneal, transmucosal, inhalation, transnasal, ophthalmic, inner ear or vaginal administration.

### (Item 22A)

The pharmaceutical composition according to item 20A or 21A, which is injection, infusion, eye drop, nose drop, ear drop, aerosol, inhalation, lotion, impregnation, liniment, mouthwash, enema, ointment, plaster, jelly, cream, patch, cataplasm, external powder or suppository.

### (Item 23A)

A pharmaceutical composition comprising the compound according to any one of items 1A to 11A, its ester form or a pharmaceutically acceptable salt thereof, or a hydrate thereof, for a pediatric or geriatric patient.

### (Item 24A)

A pharmaceutical composition comprising a combination of the compound according to any one of items 1A to 11A, its ester form or a pharmaceutically acceptable salt thereof, or a hydrate thereof and a β-lactamase inhibitor, an antibacterial agent having an anti-gram-positive bacterium activity and/or an antibacterial agent having an anti-anaerobic bacterium activity.

### (Item 25A)

A pharmaceutical composition for combination therapy of the compound according to any one of items 1A to 11A, its ester form or a pharmaceutically acceptable salt thereof, or a hydrate thereof and a β-lactamase inhibitor, an antibacterial agent having an anti-gram-positive bacterium activity and/or an antibacterial agent having an anti-anaerobic bacterium activity.

### [EFFECT OF THE INVENTION]

The compounds of the present invention are useful as medicines because of having at least any of the following features:
A) exhibiting a strong antibacterial spectrum against various gram-negative bacteria,
B) exhibiting a good antibacterial spectrum against various gram-positive bacteria,
C) exhibiting a strong antibacterial activity against β-lactamase-producing gram-negative bacteria,
D) exhibiting a strong antibacterial activity against multidrug-resistant bacteria, particularly, class B metallo-β-lactamase-producing gram-negative bacteria,
E) exhibiting a strong antibacterial activity against extended-spectrum β-lactamase (ESBL)-producing bacteria,
F) exhibiting a strong antibacterial activity against gram-negative bacteria producing class C β-lactamases,
G) exhibiting a strong antibacterial activity against carbapenem-resistant bacteria,
H) exhibiting a strong antibacterial activity against bacteria of the family *Enterobacteriaceae* resistant to commercially available drugs,
I) exhibiting a strong antibacterial activity against carbapenem-resistant bacterial of the family *Enterobacteriaceae* (CRE) producing carbapenemases such as *Klebsiella pneumoniae* carbapenemase (KPC) or New Delhi metallo-beta-lactamase (NDM),
J) not exhibiting cross resistance to existing cephem agents and/or carbapenem agents,
K) not exhibiting adverse reactions such as fever after administration into an organism,
L) having high stability (e.g., solution stability against various liquids, light stability etc.) and/or solubility in water of the compounds,
M) having a feature superior in pharmacokinetics, such as high concentrations in blood, high oral absorbability, high membrane permeability, a long duration of effects, or high tissue distribution,
N) having a weak inhibitory activity against CYP enzymes (e.g., CYP1A2, CYP2C9, CYP2C19, CYP2D6, CYP3A4 etc.),
O) having high metabolic stability,
P) not causing gastrointestinal disorders (e.g., diarrhea, hemorrhagic enteritis, gastrointestinal ulcer, gastrointestinal hemorrhage etc.), and
Q) not causing kidney toxicity, hepatotoxicity, cardiac toxicity (e.g., QTc prolongation etc.), convulsion etc.

### [MODE FOR CARRYING OUT THE INVENTION]

The mode for carrying out the invention is explained below as to the present invention. It should be understood that throughout this description, the expression of a singular form (e.g., "a", "an", "the" etc. in English and corresponding articles, adjectives etc. in other languages), unless otherwise indicated, conceptually includes even a plural form thereof. It should also be understood that terms used in this description, unless otherwise indicated, are used in meanings usually used in the art. Thus, all technical terms and chemical terms used in this description, unless otherwise defined, have the same meanings as those generally understood by those skilled in the art to which the present invention belongs. If there is any contradiction, this description (including definitions) has a priority. Terms specifically used in this description are specifically defined below.

The term "consist of" means having only a constituent element.

The term "comprise" means that an unmentioned factor is not excluded with no limitation to a constituent.

The phrase "optionally substituted with the substituent group A" means optionally substituted at any position(s) with one or two or more and the same or different substituent(s) selected from the substituent group A.

The above-mentioned holds true for the phrases "optionally substituted with the substituent group B", "optionally substituted with the substituent group C", "optionally substituted with the substituent group D", "optionally substituted with the substituent group E", "optionally substituted with the substituent group F", "optionally substituted with the substituent group G", "optionally substituted with the substituent group H", "optionally substituted with the substituent group I", and "optionally substituted with the substituent group Z".

Each term used in this description, unless otherwise indicated, is as defined below when it is used alone or together with other terms.

"Halogen" means fluorine, chlorine, bromine or iodine. Fluorine or chlorine is preferable.

"Alkyl" includes a C1 to C15, preferably C1 to C10, more preferably C1 to C6 and further preferably C1 to C4 linear or branched hydrocarbon group. For example, it includes methyl, ethyl, n-propyl, isopropyl, n-butyl, isobutyl, sec-butyl, tert-butyl, n-pentyl, isopentyl, neopentyl, n-hexyl, isohexyl, n-heptyl, isoheptyl, n-octyl, isooctyl, n-nonyl, n-decyl and the like.

A preferred embodiment of "alkyl" is methyl, ethyl, n-propyl, isopropyl, n-butyl, isobutyl, sec-butyl, tert-butyl or n-pentyl. A more preferred embodiment is methyl, ethyl, n-propyl, isopropyl or tert-butyl.

"Alkenyl" includes a C2 to C15, preferably C2 to C10, more preferably C2 to C6 and further preferably C2 to C4 linear or branched hydrocarbon group having one or two or more double bond(s) at any position(s). For example, it includes vinyl, allyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl, undecenyl, dodecenyl, tridecenyl, tetradecenyl, pentadecenyl and the like.

A preferred embodiment of "alkenyl" is vinyl, allyl, propenyl, isopropenyl or butenyl.

"Alkynyl" includes a C2 to C10, preferably C2 to C8, more preferably C2 to C6 and further preferably C2 to C4 linear or branched hydrocarbon group having one or two or more triple bond(s) at any position(s). Furthermore, it may have double bond(s) at any position(s). For example, it includes ethynyl, propynyl, butynyl, pentynyl, hexynyl, heptynyl, octynyl, nonynyl, decynyl and the like.

A preferred embodiment of "alkynyl" is ethynyl, propynyl, butynyl or pentynyl.

"Acyl" means formyl or carbonyl having a substituent.

"Carbonyl having a substituent" includes substituted or unsubstituted alkylcarbonyl, substituted or unsubstituted alkenylcarbonyl, substituted or unsubstituted alkynylcarbonyl, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, and the like.

"Alkylcarbonyl" means a group wherein the above "alkyl" is bonded to a carbonyl group. For example, it includes methylcarbonyl, ethylcarbonyl, propylcarbonyl, isopropylcarbonyl, tert-butylcarbonyl, isobutylcarbonyl, sec-butylcarbonyl, penthylcarbonyl, isopenthylcarbonyl, hexylcarbonyl and the like.

A preferred embodiment of "alkylcarbonyl" is methylcarbonyl, ethylcarbonyl or n-propylcarbonyl.

"Alkenylcarbonyl" means a group wherein the above "alkenyl" is bonded to a carbonyl group. For example, it includes ethylenylcarbonyl, propenylcarbonyl and the like.

"Alkynylcarbonyl" means a group wherein the above "alkynyl" is bonded to a carbonyl group. For example, it includes ethynylcarbonyl, propynylcarbonyl and the like.

"Acyloxy" means formyloxy or carbonyloxy having a substituent. "Carbonyloxy having a substituent" means a group wherein the above "carbonyl having a substituent" is bonded to an oxygen atom. For example, it includes substituted or unsubstituted alkylcarbonyloxy, substituted or unsubstituted alkenylcarbonyloxy, substituted or unsubstituted alkynylcarbonyloxy, substituted or unsubstituted aromatic carbocyclylcarbonyloxy, substituted or unsubstituted non-aromatic carbocyclylcarbonyloxy, substituted or unsubstituted aromatic heterocyclylcarbonyloxy, substituted or unsubstituted non-aromatic heterocyclylcarbonyloxy and the like.

"Alkylcarbonyloxy" means a group wherein the above "alkylcarbonyl" is bonded to an oxygen atom. For example, it includes methylcarbonyloxy, ethylcarbonyloxy, propylcarbonyloxy, isopropylcarbonyloxy, tert-butylcarbonyloxy, isobutylcarbonyloxy, sec-butylcarbonyloxy and the like.

A preferred embodiment of "alkylcarbonyloxy" is methylcarbonyloxy or ethylcarbonyloxy.

"Alkenylcarbonyloxy" means a group wherein the above "alkenylcarbonyl" is bonded to an oxygen atom. For example, it includes ethylenylcarbonyloxy, propenylcarbonyloxy and the like.

"Alkynylcarbonyloxy" means a group wherein the above "alkynylcarbonyl" is bonded to an oxygen atom. For example, it includes ethynylcarbonyloxy, propynylcarbonyloxy and the like.

"Alkyloxy" means a group wherein the above "alkyl" is bonded to an oxygen atom. For example, it includes methoxy, ethoxy, n-propyloxy, isopropyloxy, n-butyloxy, tert-butyloxy, isobutyloxy, sec-butyloxy, pentyloxy, isopentyloxy, hexyloxy and the like.

A preferred embodiment of "alkyloxy" is methoxy, ethoxy, n-propyloxy, isopropyloxy, hexyloxy or the like.

"Alkenyloxy" means a group wherein the above "alkenyl" is bonded to an oxygen atom. For example, it includes vinyloxy, allyloxy, 1-propenyloxy, 2-butenyloxy, 2-pentenyloxy, 2-hexenyloxy, 2-heptenyloxy, 2-octenyloxy and the like.

"Alkynyloxy" means a group wherein the above "alkynyl" is bonded to an oxygen atom. For example, it includes ethynyloxy, 1-propynyloxy, 2-propynyloxy, 2-butynyloxy, 2-pentynyloxy, 2-hexynyloxy, 2-heptynyloxy, 2-octynyloxy and the like.

"Alkylsulfonyl" means a group wherein the above "alkyl" is bonded to a sulfonyl group. For example, it includes methylsulfonyl, ethylsulfonyl, propylsulfonyl, isopropylsulfonyl, tert-butylsulfonyl, isobutylsulfonyl, sec-butylsulfonyl and the like.

A preferred embodiment of "alkylsulfonyl" is methylsulfonyl or ethylsulfonyl.

"Alkenylsulfonyl" means a group wherein the above "alkenyl" is bonded to a sulfonyl group. For example, it includes ethylenylsulfonyl, propenylsulfonyl and the like.

"Alkynylsulfonyl" means a group wherein the above "alkynyl is bonded to a sulfonyl group. For example, it includes ethynylsulfonyl, propynylsulfonyl and the like.

"Alkyloxycarbonyl" means a group wherein the above "alkyloxy" is bonded to a carbonyl group. For example, it includes methyloxycarbonyl, ethyloxycarbonyl, propyloxycarbonyl, isopropyloxycarbonyl, tert-butyloxycarbonyl, isobutyloxycarbonyl, sec-butyloxycarbonyl, penthyloxycarbonyl, isopenthyloxycarbonyl, hexyloxycarbonyl and the like.

A preferred embodiment of "alkyloxycarbonyl" is methyloxycarbonyl, ethyloxycarbonyl or propyloxycarbonyl.

"Alkenyloxycarbonyl" means a group wherein the above "alkenyloxy" is bonded to a carbonyl group. For example, it includes ethylenyloxycarbonyl, propenyloxycarbonyl and the like.

"Alkynyloxycarbonyl" means a group wherein the above "alkynyloxy" is bonded to a carbonyl group. For example, it includes ethynyloxycarbonyl, propynyloxycarbonyl and the like.

"Alkylsulfanyl" means a group wherein a hydrogen atom bonded to a sulfur atom of a sulfanyl group is replaced with the above "alkyl". For example, it includes methylsulfanyl, ethylsulfanyl, n-propylsulfanyl, isopropylsulfanyl and the like. A preferred embodiment of "alkylsulfanyl" is methylsulfanyl, ethylsulfanyl, n-propylsulfanyl, isopropylsulfanyl, hexylsulfanyl or the like.

"Alkenylsulfanyl" means a group wherein a hydrogen atom bonded to a sulfur atom of a sulfanyl group is replaced with the above "alkenyl". For example, it includes ethylenylsulfanyl, propenylsulfanyl and the like.

"Alkynylsulfanyl" means a group wherein a hydrogen atom bonded to a sulfur atom of a sulfanyl group is replaced with the above "alkynyl". For example, it includes ethynylsulfanyl, propynylsulfanyl and the like.

"Alkylsulfinyl" means a group wherein the above "alkyl" is bonded to a sulfinyl group. For example, it includes methylsulfinyl, ethylsulfinyl, n-propylsulfinyl, isopropylsulfinyl and the like.

"Alkenylsulfinyl" means a group wherein the above "alkenyl" is bonded to a sulfinyl group. For example, it includes ethylenylsulfinyl, propenylsulfinyl and the like.

"Alkynylsulfinyl" means a group wherein the above "alkynyl" is bonded to a sulfinyl group. For example, it includes ethynylsulfinyl, propynylsulfinyl and the like.

"Aromatic carbocyclyl" means a cyclic aromatic hydrocarbon group which is monocyclic or polycyclic having two or more rings. For example, it includes phenyl, naphthyl, anthryl, phenanthryl and the like.

A preferred embodiment of "aromatic carbocyclyl" is phenyl.

"Non-aromatic carbocyclyl" means a cyclic saturated hydrocarbon group or a cyclic unsaturated non-aromatic hydrocarbon group, which is monocyclic or polycyclic having two or more rings. "Non-aromatic carbocyclyl", which is polycyclic having two or more rings, includes a fused ring group wherein a non-aromatic carbocycle, which is monocyclic or polycyclic having two or more rings, is fused with a ring of the above "aromatic carbocyclyl". A bond may be derived from any ring.

In addition, the "non-aromatic carbocyclyl" also includes a group having a bridge or a group to form a spiro ring as follows:

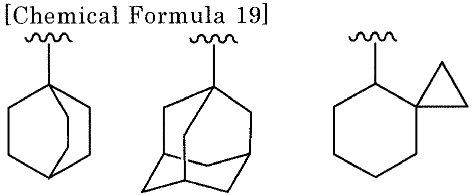

A non-aromatic carbocyclyl which is monocyclic is preferably C3 to C16, more preferably C3 to C12 and further preferably C3 to C8 carbocyclyl. For example, it includes cyclopropyl, cyclobutyl, cyclopentyl, cyclohexyl, cycloheptyl, cyclooctyl, cyclononyl, cyclodecyl, cyclopropenyl, cyclobutenyl, cyclopentenyl, cyclohexenyl, cycloheptenyl, cyclohexadienyl and the like.

A non-aromatic carbocyclyl which is polycyclic having two or more rings includes, for example, indanyl, indenyl, acenaphthyl, tetrahydronaphthyl, fluorenyl and the like.

"Aromatic carbocycle" means a ring derived from the above "aromatic carbocyclyl". "Non-aromatic carbocycle" means a ring derived from the above "non-aromatic carbocyclyl".

One embodiment of "non-aromatic carbocyclyl" is "cycloalkyl". "Cycloalkyl" means a cyclic saturated hydrocarbon group, which is monocyclic or polycyclic having two or more rings, and also includes a group having a bridge or a group to form a spiro ring. C3 to C16 cycloalkyl is preferable, C3 to C12 cycloalkyl is more preferable, and C3 to C8 cycloalkyl is further preferable. It is preferably monocyclic. For example, it includes cyclopropyl, cyclobutyl, cyclopentyl, cyclohexyl, cycloheptyl, cyclooctyl, cyclononyl, cyclodecyl, bicyclooctane, decahydronaphthalene, norbornyl, adamantyl, spirobicyclopentane and the like.

A preferred embodiment of "cycloalkyl" is cyclopropyl, cyclobutyl, cyclopentyl, cyclohexyl, cycloheptyl, or cyclooctyl.

"Carbocyclyl" includes the above "aromatic carbocyclyl" and "non-aromatic carbocyclyl". "Carbocycle" means a ring derived from the above "carbocyclyl". "Carbocyclediyl" means a divalent group derived from the above "carbocyclyl".

"Aromatic heterocyclyl" means an aromatic cyclyl, which is monocyclic or polycyclic having two or more rings, containing one or two or more and the same or different heteroatom(s) selected independently from O, S and N. An aromatic heterocyclyl, which is polycyclic having two or more rings, includes a fused ring group wherein an aromatic heterocyclyl, which is monocyclic or polycyclic having two or more rings, is fused with a ring of the above "aromatic carbocyclyl".

An aromatic heterocyclyl which is monocyclic is preferably a 5- to 8-membered and more preferably 5- or 6- membered ring. For example, a 5-membered aromatic heterocyclyl which is monocyclic includes pyrrolyl, imidazolyl, pyrazolyl, furyl, thienyl, isoxazolyl, oxazolyl, oxadiazolyl, isothiazolyl, thiazolyl, thiadiazolyl and the like, and a 6-membered aromatic heterocyclyl which is monocyclic includes pyridyl, pyridazinyl, pyrimidinyl, pyrazinyl, triazolyl, triazinyl, tetrazolyl and the like.

An aromatic heterocyclyl which is bicyclic includes, for example, indolyl, isoindolyl, indazolyl, indolizinyl, quinolinyl, isoquinolinyl, cinnolinyl, phthalazinyl, quinazolinyl, naphthyridinyl, quinoxalinyl, purinyl, pteridinyl, benzimidazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, imidazopyridyl, triazolopyridyl, imidazothiazolyl, pyrazinopyridazinyl, oxazolopyridyl, thiazolopyridyl and the like.

An aromatic heterocyclyl which is polycyclic having three or more rings includes, for example, carbazolyl, acridinyl, xanthenyl, phenothiazinyl, phenoxathiinyl, phenoxazinyl, dibenzofuryl and the like.

"Non-aromatic heterocyclyl" means a non-aromatic cyclyl, which is monocyclic or polycyclic having two or more rings, containing one or two or more and the same or different heteroatom(s) selected independently from O, S and N. "Non-aromatic heterocyclyl", which is polycyclic having two or more rings, includes a fused ring group wherein a non-aromatic heterocycle, which is monocyclic or polycyclic having two or more ring(s), is fused with a ring of the above "aromatic carbocyclyl", "non-aromatic carbocyclyl" and/or "aromatic heterocyclyl". A non-aromatic heterocyclyl, which is polycyclic having two or more rings, includes a fused ring group wherein a ring of the above "aromatic heterocyclyl" is fused with the above "non-aromatic carbocyclyl". A bond may be derived from any ring.

In addition, the "non-aromatic heterocyclyl" also includes a group having a bridge or a group to form a spiro ring as follows:

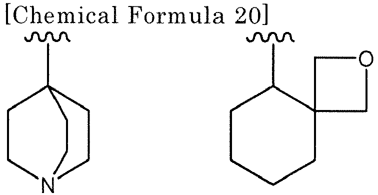

A non-aromatic heterocyclyl which is monocyclic is preferably a 3- to 8-membered and more preferably 5- or 6- membered ring. For example, it includes dioxanyl, thiiranyl, oxiranyl, oxetanyl, oxathiolanyl, azetidinyl, thianyl, thiazolidinyl, pyrrolidinyl, pyrrolinyl, imidazolidinyl, imidazolinyl, pyrazolidinyl, pyrazolinyl, piperidinyl, piperazinyl, tetrahydropyridyl, tetrahydrofuryl, tetrahydropyranyl, dihydrothiazolyl, tetrahydrothiazolyl, tetrahydroisothiazolyl, dihydrooxazinyl, hexahydroazepinyl, tetrahydrodiazepinyl, tetrahydropyridazinyl, hexahydropyrimidinyl, dioxolanyl, dioxazinyl, aziridinyl, dioxolinyl, oxepanyl, thiolanyl, thiinyl, thiazinyl, azepan-1-yl and the like.

A non-aromatic heterocyclyl which is polycyclic having two or more rings includes, for example, indolinyl, isoindolinyl, chromanyl, isochromanyl, octahydro-7H-pyrano[2,3-c]pyridin-7-yl, hexahydro-2H-pyrano[3,2-c]pyridin-6(5H)-yl, 7,8-dihydropyrido[4,3-d]pyrimidin-6(5H)-yl and the like.

"Aromatic heterocycle" means a ring derived from the above "aromatic heterocyclyl". "Non-aromatic heterocycle" means a ring derived from the above "non-aromatic heterocyclyl".

"Heterocyclyl" includes the above "aromatic heterocyclyl" and "non-aromatic heterocyclyl". "Heterocycle" means a ring derived from the above "heterocyclyl". "Heterocyclediyl" means a divalent group derived from the above "heterocyclyl".

The "aromatic carbocycle" part of "aromatic carbocyclyloxy", "aromatic carbocyclylcarbonyl", "aromatic carbocyclyloxycarbonyl", "aromatic carbocyclylcarbonyloxy", "aromatic carbocyclylsulfanyl", "aromatic carbocyclylsulfinyl" or "aromatic carbocyclylsulfonyl" is the same as the above "aromatic carbocyclyl".

"Aromatic carbocyclyloxy" means a group wherein the above "aromatic carbocycle" is bonded to an oxygen atom. For example, it includes phenyloxy, naphthyloxy and the like.

"Aromatic carbocyclylcarbonyl" means a group wherein the above "aromatic carbocycle" is bonded to a carbonyl group. For example, it includes benzoyl, naphthylcarbonyl and the like.

"Aromatic carbocyclyloxycarbonyl" means a group wherein "aromatic carbocyclyloxy" is bonded to a carbonyl group. For example, it includes phenyloxycarbonyl, naphthyloxycarbonyl and the like.

"Aromatic carbocyclylcarbonyloxy" means a group wherein "aromatic carbocyclylcarbonyl" is bonded to an oxygen atom. For example, it includes phenylcarbonyloxy, naphthylcarbonyloxy and the like.

"Aromatic carbocyclylsulfanyl" means a group wherein a hydrogen atom bonded to a sulfur atom of a sulfanyl group is replaced with "aromatic carbocycle". For example, it includes phenylsulfanyl, naphthylsulfanyl and the like.

"Aromatic carbocyclylsulfinyl" means a group wherein "aromatic carbocycle" is bonded to a sulfinyl group. For example, it includes phenylsulfinyl, naphthylsulfinyl and the like.

"Aromatic carbocyclylsulfonyl" means a group wherein "aromatic carbocycle" is bonded to a sulfonyl group. For example, it includes phenylsulfonyl, naphthylsulfonyl and the like.

The "non-aromatic carbocycle" part of "non-aromatic carbocyclyloxy", "non-aromatic carbocyclylcarbonyl", "non-aromatic carbocyclyloxycarbonyl", "non-aromatic carbocyclylcarbonyloxy", "non-aromatic carbocyclylsulfanyl", "non-aromatic carbocyclylsulfinyl", or "non-aromatic carbocyclylsulfonyl" is the same as the above "non-aromatic carbocyclyl".

"Non-aromatic carbocyclyloxy" means a group wherein the above "non-aromatic carbocycle" is bonded to an oxygen atom. For example, it includes cyclopropyloxy, cyclobutyloxy, cyclopentyloxy, cyclohexyloxy, cycloheptyloxy, cyclooctyloxy, cyclopropenyloxy, cyclobutenyloxy, cyclopentenyloxy, cyclohexenyloxy, cycloheptenyloxy, cyclohexadienyloxy, indanyloxy, tetrahydronaphthyloxy, fluorenyloxy, adamantyloxy and the like.

"Non-aromatic carbocyclylcarbonyl" means a group wherein the above "non-aromatic carbocycle" is bonded to a carbonyl group. For example, it includes cyclopropylcarbonyl, cyclohexylcarbonyl, cyclopropenylcarbonyl, indanylcarbonyl and the like.

Non-aromatic carbocyclyloxycarbonyl" means a group wherein the above "non-aromatic carbocyclyloxy" is bonded to a carbonyl group. For example, it includes cyclopropyloxycarbonyl, cyclohexyloxycarbonyl, cyclohexenyloxycarbonyl and the like.

"Non-aromatic carbocyclylcarbonyloxy" means a group wherein the above "non-aromatic carbocyclylcarbonyl" is bonded to an oxygen atom. For example, it includes cyclopropylcarbonyloxy, cyclohexylcarbonyloxy, cyclohexenylcarbonyloxy and the like.

"Non-aromatic carbocyclylsulfanyl" means a group wherein a hydrogen atom bonded to a sulfur atom of a sulfanyl group is replaced with the above "non-aromatic carbocycle". For example, it includes cyclopropylsulfanyl, cyclobutylsulfanyl, cyclopentylsulfanyl, cyclohexylsulfanyl, cycloheptylsulfanyl, cyclooctylsulfanyl, cyclopropenylsulfanyl, cyclobutenylsulfanyl, cyclopentenylsulfanyl, cyclohexenylsulfanyl, cycloheptenylsulfanyl, cyclohexadienylsulfanyl, indanylsulfanyl, tetrahydronaphthylsulfanyl, fluorenylsulfanyl, adamantylsulfanyl and the like.

"Non-aromatic carbocyclylsulfinyl" means a group wherein the above "non-aromatic carbocycle" is bonded to a sulfinyl group. For example, it includes cyclopropylsulfinyl, cyclobutylsulfinyl, cyclopentylsulfinyl, cyclohexylsulfinyl, cycloheptylsulfinyl, cyclohexenylsulfinyl, tetrahydronaphthylsulfinyl, adamantylsulfinyl and the like.

"Non-aromatic carbocyclylsulfonyl" means a group wherein the above "non-aromatic carbocycle" is bonded to a sulfonyl group. For example, it includes cyclopropylsulfonyl, cyclohexylsulfonyl, cyclohexenylsulfonyl and the like.

The "aromatic heterocycle" part of "aromatic heterocyclyloxy", "aromatic heterocyclylcarbonyl", "aromatic heterocyclyloxycarbonyl", "aromatic heterocyclylcarbonyloxy", "aromatic heterocyclylsulfanyl", "aromatic heterocyclylsulfinyl", or "aromatic heterocyclylsulfonyl" is the same as the above "aromatic heterocyclyl".

"Aromatic heterocyclyloxy" means a group wherein the above "aromatic heterocycle" is bonded to an oxygen atom. For example, it includes pyridyloxy, oxazolyloxy and the like.

"Aromatic heterocyclylcarbonyl" means a group wherein the above "aromatic heterocycle" is bonded to a carbonyl group. For example, it includes pyrrolylcarbonyl, pyrazolylcarbonyl, pyridylcarbonyl, oxazolylcarbonyl, indolylcarbonyl and the like.

"Aromatic heterocyclyloxycarbonyl" means a group wherein the above "aromatic heterocyclyloxy" is bonded to a carbonyl group. For example, it includes pyridyloxycarbonyl, oxazolyloxycarbonyl and the like.

"Aromatic heterocyclylcarbonyloxy" means a group wherein the above "aromatic heterocyclylcarbonyl" is bonded to an oxygen atom. For example, it includes pyridylcarbonyloxy, oxazolylcarbonyloxy and the like.

"Aromatic heterocyclylsulfanyl" means a group wherein a hydrogen atom bonded to a sulfur atom of a sulfanyl group is replaced with the above "aromatic heterocycle". For example, it includes pyridylsulfanyl, oxazolylsulfanyl and the like.

"Aromatic heterocyclylsulfinyl" means a group wherein the above "aromatic heterocycle" is bonded to a sulfinyl group. For example, it includes pyridylsulfinyl, oxazolylsulfinyl and the like.

"Aromatic heterocyclylsulfonyl" means a group wherein the above "aromatic heterocycle" is bonded to a sulfonyl group. For example, it includes pyridylsulfonyl, oxazolylsulfonyl and the like.

The "non-aromatic heterocycle" part of "non-aromatic heterocyclyloxy", "non-aromatic heterocyclylcarbonyl", "non-aromatic heterocyclyloxycarbonyl", "non-aromatic heterocyclylcarbonyloxy", "non-aromatic heterocyclylsulfanyl", "non-aromatic heterocyclylsulfinyl", or "non-aromatic heterocyclylsulfonyl" is the same as the above "non-aromatic heterocyclyl".

"Non-aromatic heterocyclyloxy" means a group wherein the above "non-aromatic heterocycle" is bonded to an oxygen atom. For example, it includes dioxanyloxy, thiiranyloxy, oxiranyloxy, oxetanyloxy, oxathiolanyloxy, azetidinyloxy, thianyloxy, thiazolidinyloxy, pyrrolidinyloxy, pyrrolinyloxy, imidazolidinyloxy, imidazolinyloxy, pyrazolidinyloxy, pyrazolinyloxy, piperidinyloxy, piperazinyloxy, morpholinyloxy, indolinyloxy, chromanyloxy and the like.

"Non-aromatic heterocyclylcarbonyl" means a group wherein the above "non-aromatic heterocycle" is bonded to a carbonyl group. For example, it includes dioxanylcarbonyl, oxetanylcarbonyl, pyrazolinylcarbonyl, morpholinocarbonyl, morpholinylcarbonyl, indolinylcarbonyl and the like.

"Non-aromatic heterocyclyloxycarbonyl" means a group wherein the above "non-aromatic heterocyclyloxy" is bonded to a carbonyl group. For example, it includes piperidinyloxycarbonyl, tetrahydrofuryloxycarbonyl and the like.

"Non-aromatic heterocyclylcarbonyloxy" means a group wherein the above "non-aromatic heterocyclylcarbonyl" is bonded to an oxygen atom. For example, it includes piperidinylcarbonyloxy, tetrahydrofurylcarbonyloxy and the like.

"Non-aromatic heterocyclylsulfanyl" means a group wherein a hydrogen atom bonded to a sulfur atom of a sulfanyl group is replaced with the above "non-aromatic heterocycle". For example, it includes dioxanylsulfanyl, thiiranylsulfanyl, oxiranylsulfanyl, oxetanylsulfanyl, oxathiolanylsulfanyl, azetidinylsulfanyl, thianylsulfanyl, thiazolidinylsulfanyl, pyrrolidinylsulfanyl, pyrrolinylsulfanyl, imidazolidinylsulfanyl, imidazolinylsulfanyl, pyrazolidinylsulfanyl, pyrazolinylsulfanyl, piperidinylsulfanyl, piperazinylsulfanyl, morpholinylsulfanyl, indolinylsulfanyl, chromanylsulfanyl and the like.

"Non-aromatic heterocyclylsulfinyl" means a group wherein the above "non-aromatic heterocycle" is bonded to a sulfinyl group. For example, it includes piperidinylsulfinyl, tetrahydrofurylsulfinyl and the like.

"Non-aromatic heterocyclylsulfonyl" means a group wherein the above "non-aromatic heterocycle" is bonded to a sulfonyl group. For example, it includes piperidinylsulfonyl, tetrahydrofurylsulfonyl and the like.

The substituents of "substituted or unsubstituted alkyl", "substituted or unsubstituted alkenyl", "substituted or unsubstituted alkynyl", "substituted or unsubstituted alkyloxy", "substituted or unsubstituted alkenyloxy", "substituted or unsubstituted alkynyloxy", "substituted or unsubstituted alkylsulfonyl", "substituted or unsubstituted alkenylsulfonyl", "substituted or unsubstituted alkynylsulfonyl", "substituted or unsubstituted alkyloxycarbonyl", "substituted or unsubstituted alkenyloxycarbonyl", "substituted or unsubstituted alkynyloxycarbonyl", "substituted or unsubstituted alkylsulfanyl", "substituted or unsubstituted alkenylsulfanyl", "substituted or unsubstituted alkynylsulfanyl", "substituted or unsubstituted alkylsulfinyl", "substituted or unsubstituted alkenylsulfinyl", "substituted or unsubstituted alkynylsulfinyl", "substituted or unsubstituted alkylcarbonyl", "substituted or unsubstituted alkenylcarbonyl", "substituted or unsubstituted alkynylcarbonyl", "substituted or unsubstituted alkylcarbonyloxy", "substituted or unsubstituted alkenylcarbonyloxy", "substituted or unsubstituted alkynylcarbonyloxy" and "substituted or unsubstituted carboxyalkyl" include groups selected from the substituent group A given below. A carbon atom at any position(s) may be bonded to one or two or more group(s) selected from the substituent group A given below. Furthermore, the substituent group A may be substituted with one or two or more substituent(s) selected from the substituent group Z. When two or more of the substituents are present, they may be the same or different.

The substituent group A: halogen, hydroxy, carboxy, amino, imino, hydroxyamino, hydroxyiminomethyl, formyl, formyloxy, carbamoyl, sulfamoyl, sulfanyl, sulfino, sulfo, thioformyl, thiocarboxy, dithiocarboxy, thiocarbamoyl, cyano, nitro, nitroso, azide, hydrazino, ureido, amidino, guanidino, trialkylsilyl, alkyloxy, alkenyloxy, alkynyloxy, haloalkyloxy, alkylcarbonyl, alkenylcarbonyl, alkynylcarbonyl, monoalkylamino, dialkylamino, alkylsulfonyl, alkenylsulfonyl, alkynylsulfonyl, monoalkylcarbonylamino, dialkylcarbonylamino, monoalkylsulfonylamino, dialkylsulfonylamino, alkylimino, alkenylimino, alkynylimino, alkylcarbonylimino, alkenylcarbonylimino, alkynylcarbonylimino, alkyloxyimino, alkenyloxyimino, alkynyloxyimino, alkylcarbonyloxy, alkenylcarbonyloxy, alkynylcarbonyloxy, alkyloxycarbonyl, alkenyloxycarbonyl, alkynyloxycarbonyl, alkylsulfanyl, alkenylsulfanyl, alkynylsulfanyl, alkylsulfinyl, alkenylsulfinyl, alkynylsulfinyl, monoalkylcarbamoyl, dialkylcarbamoyl, monoalkylsulfamoyl, dialkylsulfamoyl, aromatic carbocyclyl, non-aromatic carbocyclyl, aromatic heterocyclyl, non-aromatic heterocyclyl, aromatic carbocyclyloxy, non-aromatic carbocyclyloxy, aromatic heterocyclyloxy, non-aromatic heterocyclyloxy, aromatic carbocyclylcarbonyl, non-aromatic carbocyclylcarbonyl, aromatic heterocyclylcarbonyl, non-aromatic heterocyclylcarbonyl, aromatic carbocyclyloxycarbonyl, non-aromatic carbocyclyloxycarbonyl, aromatic heterocyclyloxycarbonyl, non-aromatic heterocyclyloxycarbonyl, aromatic carbocyclylalkyloxy, non-aromatic carbocyclylalkyloxy, aromatic heterocyclylalkyloxy, non-aromatic heterocyclylalkyloxy, aromatic carbocyclylalkyloxycarbonyl, non-aromatic carbocyclylalkyloxycarbonyl, aromatic heterocyclylalkyloxycarbonyl, non-aromatic heterocyclylalkyloxycarbonyl, aromatic carbocyclylalkylamino, non-aromatic carbocyclylalkylamino, aromatic heterocyclylalkylamino, non-aromatic heterocyclylalkylamino, aromatic carbocyclylsulfanyl, non-aromatic carbocyclylsulfanyl, aromatic heterocyclylsulfanyl, non-aromatic heterocyclylsulfanyl, non-aromatic carbocyclylsulfonyl, aromatic carbocyclylsulfonyl, aromatic heterocyclylsulfonyl, and non-aromatic heterocyclylsulfonyl.

The substituents on the ring of "aromatic carbocycle", "non-aromatic carbocycle", "aromatic heterocycle", "non-aromatic heterocycle", "carbocycle" or "heterocycle" of "substituted or unsubstituted aromatic carbocyclyl", "substituted or unsubstituted non-aromatic carbocyclyl", "substituted or unsubstituted aromatic heterocyclyl", "substituted or unsubstituted non-aromatic heterocyclyl", "substituted or unsubstituted aromatic carbocyclyloxy", "substituted or unsubstituted non-aromatic carbocyclyloxy", "substituted or unsubstituted aromatic heterocyclyloxy", "substituted or unsubstituted non-aromatic heterocyclyloxy", "substituted or unsubstituted aromatic carbocyclylcarbonyl", "substituted or unsubstituted non-aromatic carbocyclylcarbonyl", "substituted or unsubstituted aromatic heterocyclylcarbonyl", "substituted or unsubstituted non-aromatic heterocyclylcarbonyl", "substituted or unsubstituted aromatic carbocyclyloxycarbonyl", "substituted or unsubstituted non-aromatic carbocyclyloxycarbonyl", "substituted or unsubstituted aromatic heterocyclyloxycarbonyl", "substituted or unsubstituted non-aromatic heterocyclyloxycarbonyl", "substituted or unsubstituted aromatic carbocyclylsulfanyl", "substituted or unsubstituted non-aromatic carbocyclylsulfanyl", "substituted or unsubstituted aromatic heterocyclylsulfanyl", "substituted or unsubstituted non-aromatic heterocyclylsulfanyl", "substituted or unsubstituted aromatic carbocyclylsulfinyl", "substituted or unsubstituted non-aromatic carbocyclylsulfinyl", "substituted or unsubstituted aromatic heterocyclylsulfinyl", "substituted or unsubstituted non-aromatic heterocyclylsulfinyl", "substituted or unsubstituted aromatic carbocyclylsulfonyl", "substituted or unsubstituted non-aromatic carbocyclylsulfonyl", "substituted or unsubstituted aromatic heterocyclylsulfonyl", "substituted or unsubstituted non-aromatic heterocyclylsulfonyl", "substituted or unsubstituted carbocyclyl", "substituted or unsubstituted heterocyclyl", "substituted or unsubstituted non-aromatic carbocycle", "substituted or unsubstituted non-aromatic heterocycle", "substituted or unsubstituted carbocyclediyl", "substituted or unsubstituted heterocyclediyl", "substituted or unsubstituted aromatic carbocyclylcarbonyloxy", "substituted or unsubstituted non-aromatic carbocyclylcarbonyloxy", "substituted or unsubstituted aromatic heterocyclylcarbonyloxy" and "substituted or unsubstituted non-aromatic heterocyclylcarbonyloxy" include the substituent group B given below. An atom at any position(s) on the ring may be bonded to one or two or more group(s) selected from the substituent group B given below. Furthermore, the substituent group B may be substituted with one or two or more substituent(s) selected from the substituent group Z. When two or more of the substituents are present, they may be the same or different.

The substituent group B: oxo, halogen, hydroxy, carboxy, amino, imino, hydroxyamino, hydroxyiminomethyl, formyl, formyloxy, carbamoyl, sulfamoyl, sulfanyl, sulfino, sulfo, thioformyl, thiocarboxy, dithiocarboxy, thiocarbamoyl, cyano, cyanoalkyl, nitro, nitroso, azide, hydrazino, ureido, amidino, guanidino, trialkylsilyl, alkyl, alkenyl, alkynyl, haloalkyl, alkyloxy, alkenyloxy, alkynyloxy, haloalkyloxy, hydroxyalkyloxy, alkyloxyalkyl, alkyloxyalkyloxy, hydroxyalkyl, hydroxyalkenyl, hydroxyalkynyl, cyanoalkyl, alkyloxyalkyl, alkyloxyalkyloxyalkyloxy, alkylcarbonyl, alkenylcarbonyl, alkynylcarbonyl, monoalkylamino, dialkylamino, alkylsulfonyl, alkenylsulfonyl, alkynylsulfonyl, monoalkylcarbonylamino, dialkylcarbonylamino, monoalkylsulfonylamino, dialkylsulfonylamino, alkylimino, alkenylimino, alkynylimino, alkylcarbonylimino, alkenylcarbonylimino, alkynylcarbonylimino, alkyloxyimino, haloalkyloxyimino, alkenyloxyimino, alkynyloxyimino, alkyloxyalkyloxyimino, methylidene, alkylmethylidene, alkyloxycarbonylmethylidene, alkylcarbonyloxy, alkenylcarbonyloxy, alkynylcarbonyloxy, alkyloxycarbonyl, alkenyloxycarbonyl, alkynyloxycarbonyl, alkyloxycarbonylalkyloxy, alkyloxycarbonylalkyl, alkylsulfanyl, alkenylsulfanyl, alkynylsulfanyl, alkylsulfinyl, alkenylsulfinyl, alkynylsulfinyl, monoalkylcarbamoyl, dialkylcarbamoyl, monoalkylsulfamoyl, dialkylsulfamoyl, aromatic carbocyclyl, non-aromatic carbocyclyl, aromatic heterocyclyl, non-aromatic heterocyclyl, aromatic carbocyclyloxy, non-aromatic carbocyclyloxy, aromatic heterocyclyloxy, non-aromatic heterocyclyloxy, aromatic carbocyclylcarbonyl, non-aromatic carbocyclylcarbonyl, aromatic heterocyclylcarbonyl, non-aromatic heterocyclylcarbonyl, aromatic carbocyclyloxycarbonyl, non-aromatic carbocyclyloxycarbonyl, aromatic heterocyclyloxycarbonyl, non-aromatic heterocyclyloxycarbonyl, aromatic carbocyclylalkyl, non-aromatic carbocyclylalkyl, aromatic heterocyclylalkyl, non-aromatic heterocyclylalkyl, aromatic carbocyclylalkenyl, non-aromatic carbocyclylalkenyl, aromatic carbocyclylalkyloxy, non-aromatic carbocyclylalkyloxy, aromatic heterocyclylalkyloxy, non-aromatic heterocyclylalkyloxy, aromatic carbocyclylalkyloxyalkyloxy, aromatic carbocyclyloxyalkyloxy, aromatic carbocyclylalkyloxycarbonyl, non-aromatic carbocyclylalkyloxycarbonyl, aromatic heterocyclylalkyloxycarbonyl, non-aromatic heterocyclylalkyloxycarbonyl, aromatic carbocyclyloxyalkyl, non-aromatic carbocyclyloxyalkyl, aromatic heterocyclyloxyalkyl, non-aromatic heterocyclyloxyalkyl, aromatic carbocyclylalkyloxyalkyl, non-aromatic carbocyclylalkyloxyalkyl, aromatic heterocyclylalkyloxyalkyl, non-aromatic heterocyclylalkyloxyalkyl, aromatic carbocyclyloxyimino, non-aromatic carbocyclyloxyimino, alkyloxy-aromatic carbocyclylalkyloxy, aromatic carbocyclylsulfamoyl, non-aromatic carbocyclylsulfamoyl, aromatic heterocyclylsulfamoyl, non-aromatic heterocyclylsulfamoyl, aromatic carbocyclylalkylsulfamoyl, aromatic carbocyclylalkylamino, non-aromatic carbocyclylalkylamino, aromatic heterocyclylalkylamino, non-aromatic heterocyclylalkylamino, aromatic carbocyclylsulfanyl, non-aromatic carbocyclylsulfanyl, aromatic heterocyclylsulfanyl, non-aromatic heterocyclylsulfanyl, non-aromatic carbocyclylsulfonyl, aromatic carbocyclylsulfonyl, aromatic heterocyclylsulfonyl, and non-aromatic heterocyclylsulfonyl.

"Substituted or unsubstituted non-aromatic carbocyclyl" and "substituted or unsubstituted non-aromatic heterocyclyl" may be substituted with "oxo". This case means, for example, a group wherein two hydrogen atoms on a carbon atom are replaced with oxo as below.

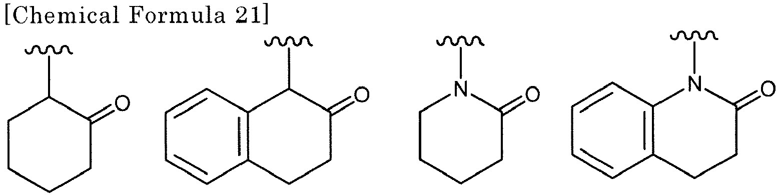

The non-aromatic carbocycle or non-aromatic heterocycle parts of the above "substituted or unsubstituted non-aromatic carbocycle", "substituted or unsubstituted non-aromatic heterocycle", "substituted or unsubstituted non-aromatic carbocyclylalkyl", "substituted or unsubstituted aromatic heterocyclylalkyl", "substituted or unsubstituted non-aromatic carbocyclyloxy", "substituted or unsubstituted non-aromatic heterocyclyloxy", "substituted or unsubstituted non-aromatic carbocyclylcarbonyl", "substituted or unsubstituted non-aromatic heterocyclylcarbonyl", "substituted or unsubstituted non-aromatic carbocyclyloxycarbonyl", "substituted or unsubstituted non-aromatic heterocyclyloxycarbonyl", "substituted or unsubstituted non-aromatic carbocyclylcarbonyloxy", "substituted or unsubstituted non-aromatic heterocyclylcarbonyloxy", "substituted or unsubstituted non-aromatic carbocyclylsulfanyl", "substituted or unsubstituted non-aromatic heterocyclylsulfanyl", "substituted or unsubstituted non-aromatic carbocyclylsulfinyl", "substituted or unsubstituted non-aromatic heterocyclylsulfinyl", "substituted or unsubstituted non-aromatic carbocyclylsulfonyl", and "substituted or unsubstituted non-aromatic heterocyclylsulfonyl" may be substituted with "oxo" as above.

The substituents of "substituted or unsubstituted amino", "substituted or unsubstituted carbamoyl" and "substituted or unsubstituted sulfamoyl" include the substituent group C given below. When two of the substituents are present, they may be the same or different. Furthermore, the substituent group C may be substituted with one or two or more substituent(s) selected from the substituent group Z. When two or more of the substituents are present, they may be the same or different.

The substituent group C: hydroxy, amino, trialkylsilyl, alkyl, alkenyl, alkynyl, haloalkyl, alkyloxyalkyl, alkylcarbonyl, alkenylcarbonyl, alkynylcarbonyl, monoalkylamino, dialkylamino, alkylsulfonyl, alkenylsulfonyl, alkynylsulfonyl, alkyloxy, alkenyloxy, alkynyloxy, alkyloxycarbonyl, alkenyloxycarbonyl, alkynyloxycarbonyl, carbamoyl, sulfamoyl, aromatic carbocyclyl, non-aromatic carbocyclyl, aromatic heterocyclyl, non-aromatic heterocyclyl, aromatic carbocyclyloxy, non-aromatic carbocyclyloxy, aromatic heterocyclyloxy, non-aromatic heterocyclyloxy, aromatic carbocyclylcarbonyl, non-aromatic carbocyclylcarbonyl, aromatic heterocyclylcarbonyl, non-aromatic heterocyclylcarbonyl, aromatic carbocyclyloxycarbonyl, non-aromatic carbocyclyloxycarbonyl, aromatic heterocyclyloxycarbonyl, non-aromatic heterocyclyloxycarbonyl, aromatic carbocyclylalkyl, non-aromatic carbocyclylalkyl, aromatic heterocyclylalkyl, non-aromatic heterocyclylalkyl, aromatic carbocyclylalkyloxy, non-aromatic carbocyclylalkyloxy, aromatic heterocyclylalkyloxy, non-aromatic heterocyclylalkyloxy, aromatic carbocyclylalkyloxycarbonyl, non-aromatic carbocyclylalkyloxycarbonyl, aromatic heterocyclylalkyloxycarbonyl, non-aromatic heterocyclylalkyloxycarbonyl, aromatic carbocyclylalkyloxyalkyl, non-aromatic carbocyclylalkyloxyalkyl, aromatic heterocyclylalkyloxyalkyl, non-aromatic heterocyclylalkyloxyalkyl, non-aromatic carbocyclylsulfonyl, aromatic carbocyclylsulfonyl, aromatic heterocyclylsulfonyl, and non-aromatic heterocyclylsulfonyl.

The substituent group Z: oxo, halogen, hydroxy, carboxy, amino, imino, hydroxyamino, hydroxyiminomethyl, formyl, formyloxy, carbamoyl, sulfamoyl, sulfanyl, sulfino, sulfo, thioformyl, thiocarboxy, dithiocarboxy, thiocarbamoyl, cyano, cyanoalkyl, nitro, nitroso, azide, hydrazino, ureido, amidino, guanidino, trialkylsilyl, alkyl, alkenyl, alkynyl, haloalkyl, alkyloxy, alkenyloxy, alkynyloxy, haloalkyloxy, hydroxyalkyloxy, alkyloxyalkyl, alkyloxyalkyloxy, hydroxyalkyl, hydroxyalkenyl, hydroxyalkynyl, cyanoalkyl, alkyloxyalkyl, alkyloxyalkyloxyalkyloxy, alkylcarbonyl, alkenylcarbonyl, alkynylcarbonyl, monoalkylamino, dialkylamino, alkylsulfonyl, alkenylsulfonyl, alkynylsulfonyl, monoalkylcarbonylamino, dialkylcarbonylamino, monoalkylsulfonylamino, dialkylsulfonylamino, alkylimino, alkenylimino, alkynylimino, alkylcarbonylimino, alkenylcarbonylimino, alkynylcarbonylimino, alkyloxyimino, haloalkyloxyimino, alkenyloxyimino, alkynyloxyimino, alkyloxyalkyloxyimino, methylidene, alkylmethylidene, alkyloxycarbonylmethylidene, alkylcarbonyloxy, alkenylcarbonyloxy, alkynylcarbonyloxy, alkyloxycarbonyl, alkenyloxycarbonyl, alkynyloxycarbonyl, alkyloxycarbonylalkyloxy, alkyloxycarbonylalkyl, alkylsulfanyl, alkenylsulfanyl, alkynylsulfanyl, alkylsulfinyl, alkenylsulfinyl, alkynylsulfinyl, monoalkylcarbamoyl, dialkylcarbamoyl, monoalkylsulfamoyl, dialkylsulfamoyl, aromatic carbocyclyl, non-aromatic carbocyclyl, aromatic heterocyclyl, non-aromatic heterocyclyl, aromatic carbocyclyloxy, non-aromatic carbocyclyloxy, aromatic heterocyclyloxy, non-aromatic heterocyclyloxy, aromatic carbocyclylcarbonyl, non-aromatic carbocyclylcarbonyl, aromatic heterocyclylcarbonyl, non-aromatic heterocyclylcarbonyl, aromatic carbocyclyloxycarbonyl, non-aromatic carbocyclyloxycarbonyl, aromatic heterocyclyloxycarbonyl, non-aromatic heterocyclyloxycarbonyl, aromatic carbocyclylalkyl, non-aromatic carbocyclylalkyl, aromatic heterocyclylalkyl, non-aromatic heterocyclylalkyl, aromatic carbocyclylalkenyl, non-aromatic carbocyclylalkenyl, aromatic carbocyclylalkyloxy, non-aromatic carbocyclylalkyloxy, aromatic heterocyclylalkyloxy, non-aromatic heterocyclylalkyloxy, aromatic carbocyclylalkyloxyalkyloxy, aromatic carbocyclyloxyalkyloxy, aromatic carbocyclylalkyloxycarbonyl, non-aromatic carbocyclylalkyloxycarbonyl, aromatic heterocyclylalkyloxycarbonyl, non-aromatic heterocyclylalkyloxycarbonyl, aromatic carbocyclyloxyalkyl, non-aromatic carbocyclyloxyalkyl, aromatic heterocyclyloxyalkyl, non-aromatic heterocyclyloxyalkyl, aromatic carbocyclylalkyloxyalkyl, non-aromatic carbocyclylalkyloxyalkyl, aromatic heterocyclylalkyloxyalkyl, non-aromatic heterocyclylalkyloxyalkyl, aromatic carbocyclyloxyimino, non-aromatic carbocyclyloxyimino, alkyloxy-aromatic carbocyclylalkyloxy, aromatic carbocyclylsulfamoyl, non-aromatic carbocyclylsulfamoyl, aromatic heterocyclylsulfamoyl, non-aromatic heterocyclylsulfamoyl, aromatic carbocyclylalkylsulfamoyl, aromatic carbocyclylalkylamino, non-aromatic carbocyclylalkylamino, aromatic heterocyclylalkylamino, non-aromatic heterocyclylalkylamino, aromatic carbocyclylsulfanyl, non-aromatic carbocyclylsulfanyl, aromatic heterocyclylsulfanyl, non-aromatic heterocyclylsulfanyl, non-aromatic carbocyclylsulfonyl, aromatic carbocyclylsulfonyl, aromatic heterocyclylsulfonyl, and non-aromatic heterocyclylsulfonyl.

The substituents of "substituted or unsubstituted methylidene" and "substituted or unsubstituted hydroxyimino" include substituted or unsubstituted alkyl, substituted or unsubstituted alkenyl, substituted or unsubstituted alkynyl, substituted or unsubstituted carbocyclyl, substituted or unsubstituted heterocyclyl and the like. When a plurality of substituents are used in substitution, the substituents may be the same or different.

"Haloalkyl" means a group wherein one or two or more "halogen" described above is bonded to the above "alkyl". For example, it includes monofluoromethyl, monofluoroethyl, monofluoropropyl, 2,2,3,3,3-pentafluoropropyl, monochloromethyl, trifluoromethyl, trichloromethyl, 2,2,2-trifluoroethyl, 2,2,2-trichloroethyl, 1,2-dibromoethyl, 1,1,1-trifluoropropane-2-yl and the like.

A preferred embodiment of "haloalkyl" is trifluoromethyl or trichloromethyl.

"Haloalkyloxy" means a group wherein the above "haloalkyl" is bonded to an oxygen atom. For example, it includes monofluoromethoxy, monofluoroethoxy, trifluoromethoxy, trichloromethoxy, trifluoroethoxy, trichloroethoxy and the like.

A preferred embodiment of "haloalkyloxy" is trifluoromethoxy, trichloromethoxy or the like.

"Alkyloxyalkyl" means a group wherein the above "alkyloxy" is bonded to the above "alkyl". For example, it includes methoxymethyl, methoxyethyl, ethoxymethyl and the like.

"Alkyloxyalkyloxy" means a group wherein the above "alkyloxy" is bonded to the above "alkyloxy". For example, it includes methoxymethoxy, methoxyethoxy, ethoxymethoxy, ethoxyethoxy and the like.

"Alkylimino" means a group wherein a hydrogen atom bonded to a nitrogen atom of an imino group is replaced with the above "alkyl". For example, it includes methylimino, ethylimino, n-propylimino, isopropylimino and the like.

"Alkenylimino" means a group wherein a hydrogen atom bonded to a nitrogen atom of an imino group is replaced with the above "alkenyl". For example, it includes ethylenylimino, propenylimino and the like.

"Alkynylimino" means a group wherein a hydrogen atom bonded to a nitrogen atom of an imino group is replaced with the above "alkynyl". For example, it includes ethynylimino, propynylimino and the like.

"Alkylcarbonylimino" means a group wherein a hydrogen atom bonded to a nitrogen atom of an imino group is replaced with the above "alkylcarbonyl". For example, it includes methylcarbonylimino, ethylcarbonylimino, n-propylcarbonylimino, isopropylcarbonylimino and the like.

"Alkenylcarbonylimino" means a group wherein a hydrogen atom bonded to a nitrogen atom of an imino group is replaced with the above "alkenylcarbonyl". For example, it includes ethylenylcarbonylimino, propenylcarbonylimino and the like.

"Alkynylcarbonylimino" means a group wherein a hydrogen atom bonded to a nitrogen atom of an imino group is replaced with the above "alkynylcarbonyl". For example, it includes ethynylcarbonylimino, propynylcarbonylimino and the like.

"Alkyloxyimino" means a group wherein a hydrogen atom bonded to a nitrogen atom of an imino group is replaced with the above "alkyloxy". For example, it includes methyloxyimino, ethyloxyimino, n-propyloxyimino, isopropyloxyimino and the like.

"Alkenyloxyimino" means a group wherein a hydrogen atom bonded to a nitrogen atom of an imino group is replaced with the above "alkenyloxy". For example, it includes ethylenyloxyimino, propenyloxyimino and the like.

"Alkynyloxyimino" means a group wherein a hydrogen atom bonded to a nitrogen atom of an imino group is replaced with the above "alkynyloxy". For example, it includes ethynyloxyimino, propynyloxyimino and the like.

"Trialkylsilyl" means a group wherein three "alkyl" described above are bonded to a silicon atom. The three alkyl groups may be the same or different. For example, it includes trimethylsilyl, triethylsilyl, tert-butyldimethylsilyl and the like.

The alkyl part of "carbocyclylalkyl", "aromatic carbocyclylalkyl", "non-aromatic carbocyclylalkyl", "heterocyclylalkyl", "aromatic heterocyclylalkyl", "non-aromatic heterocyclylalkyl", "carbocyclylalkyloxy", "aromatic carbocyclylalkyloxy", "non-aromatic carbocyclylalkyloxy", "heterocyclylalkyloxy", "aromatic heterocyclylalkyloxy", "non-aromatic heterocyclylalkyloxy", "carbocyclylalkyloxycarbonyl", "aromatic carbocyclylalkyloxycarbonyl", "non-aromatic carbocyclylalkyloxycarbonyl", "heterocyclylalkyloxycarbonyl", "aromatic heterocyclyloxycarbonyl", "non-aromatic heterocyclyloxycarbonyl", "carbocyclylalkyloxyalkyl", "aromatic carbocyclylalkyloxyalkyl", "non-aromatic carbocyclylalkyloxyalkyl", "heterocyclylalkyloxyalkyl", "aromatic heterocyclylalkyloxy alkyl", "non-aromatic heterocyclylalkyloxyalkyl", "carbocyclylalkylamino", "aromatic carbocyclylalkylamino", "non-aromatic carbocyclylalkylamino", "heterocyclylalkylamino", "aromatic heterocyclylalkylamino", "non-aromatic heterocyclylalkylamino" or "carboxyalkyl" is the same as the above "alkyl".

"Aromatic carbocyclylalkyl" means an alkyl substituted with one or two or more "aromatic carbocyclyl" described above. For example, it includes benzyl, phenethyl, phenylpropyl, benzhydryl, trityl, naphthylmethyl, a group of the formula of: 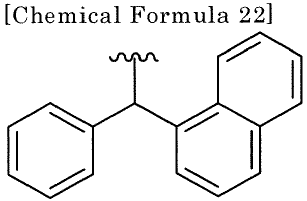 and the like.

A preferred embodiment of "aromatic carbocyclylalkyl" is benzyl, phenethyl, benzhydryl or the like.

"Non-aromatic carbocyclylalkyl" means an alkyl substituted with one or two or more "non-aromatic carbocyclyl" described above. "Non-aromatic carbocyclylalkyl" also includes "non-aromatic carbocyclylalkyl" wherein the alkyl part is substituted with the above "aromatic carbocyclyl". For example, it includes cyclopropylmethyl, cyclobutylmethyl, cyclopentylmethyl, cyclohexylmethyl, a group of the formula of: 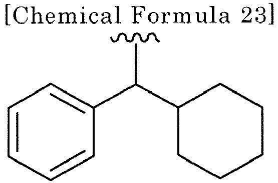 and the like.

"Carbocyclylalkyl" includes "aromatic carbocyclylalkyl" and "non-aromatic carbocyclylalkyl". A preferred embodiment of "carbocyclylalkyl" is benzyl, phenethyl, benzhydryl, cyclopropylmethyl, cyclobutylmethyl, cyclopentylmethyl, cyclohexylmethyl, a group of the formula of: 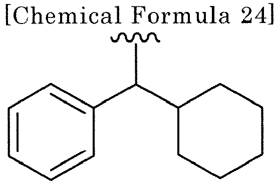 or the like.

"Aromatic heterocyclylalkyl" means an alkyl substituted with one or two or more "aromatic heterocyclyl" described above. "Aromatic heterocyclylalkyl" also includes "aromatic heterocyclylalkyl" wherein the alkyl part is substituted with the above "aromatic carbocyclyl" and/or "non-aromatic carbocyclyl". For example, it includes pyridylmethyl, furanylmethyl, imidazolylmethyl, indolylmethyl, benzothiophenylmethyl, oxazolylmethyl, isoxazolylmethyl, thiazolylmethyl, isothiazolylmethyl, pyrazolylmethyl, isopyrazolylmethyl, pyrrolidinylmethyl, benzoxazolylmethyl, groups of the formula of 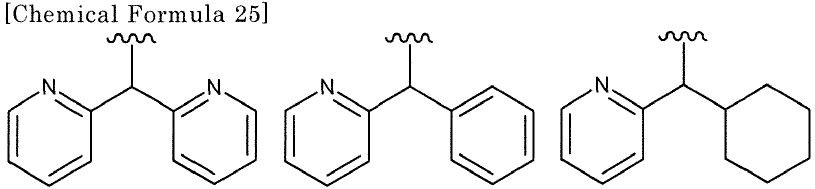 and the like.

"Non-aromatic heterocyclylalkyl" means an alkyl substituted with one or more "non-aromatic heterocyclyl" described above. "Non-aromatic heterocyclylalkyl" also includes "non-aromatic heterocyclylalkyl" wherein the alkyl part is substituted with the above "aromatic carbocyclyl", "non-aromatic carbocyclyl" and/or "aromatic heterocyclyl". For example, it includes tetrahydropyranylmethyl, morpholinylethyl, piperidinylmethyl, piperazinylmethyl, groups of the formula of 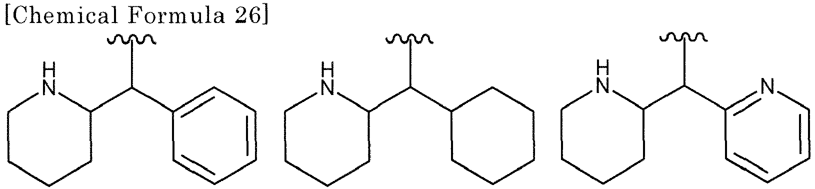 and the like.

"Heterocyclylalkyl" includes "aromatic heterocyclylalkyl" and "non-aromatic heterocyclylalkyl". A preferred embodiment of "heterocyclylalkyl" is pyridylmethyl, furanylmethyl, imidazolylmethyl, indolylmethyl, benzothiophenylmethyl, oxazolylmethyl, isoxazolylmethyl, thiazolylmethyl, isothiazolylmethyl, pyrazolylmethyl, isopyrazolylmethyl, pyrrolidinylmethyl, benzoxazolylmethyl, tetrahydropyranylmethyl, morpholinylethyl, piperidinylmethyl, piperazinylmethyl, a group of any of the formulas of: 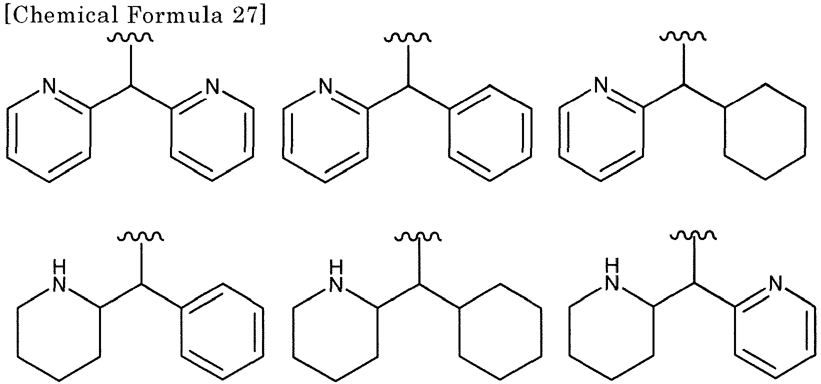 or the like.

"Aromatic carbocyclylalkyloxy" means an alkyloxy substituted with one or two or more "aromatic carbocyclyl" described above. For example, it includes benzyloxy, phenethyloxy, phenylpropyloxy, benzhydryloxy, trityloxy, naphthylmethyloxy, a group of the formula of: 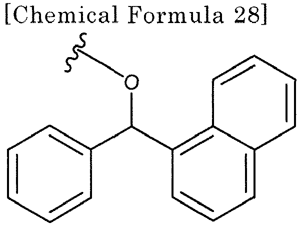 and the like.

"Non-aromatic carbocyclylalkyloxy" means an alkyloxy substituted with one or two or more "non-aromatic carbocyclyl" described above. "Non-aromatic carbocyclylalkyloxy" also includes "non-aromatic carbocyclylalkyloxy" wherein the alkyl part is substituted with the above "aromatic carbocyclyl". For example, it includes cyclopropylmethyloxy, cyclobutylmethyloxy, cyclopenthylmethyloxy, cyclohexylmethyloxy, a group of the formula of 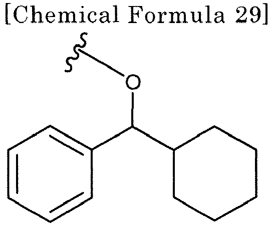 and the like.

"Aromatic heterocyclylalkyloxy" means an alkyloxy substituted with one or two or more "aromatic heterocyclyl" described above. "Aromatic heterocyclylalkyloxy" also includes "aromatic heterocyclylalkyloxy" wherein the alkyl part is substituted with the above "aromatic carbocyclyl" and/or "non-aromatic carbocyclyl". For example, it includes pyridylmethyloxy, furanylmethyloxy, imidazolylmethyloxy, indolylmethyloxy, benzothiophenylmethyloxy, isothiazolylmethyloxy, pyrazolylmethyloxy, isopyrazolylmethyloxy, pyrrolidinylmethyloxy, benzoxazolylmethyloxy, groups of the formula of 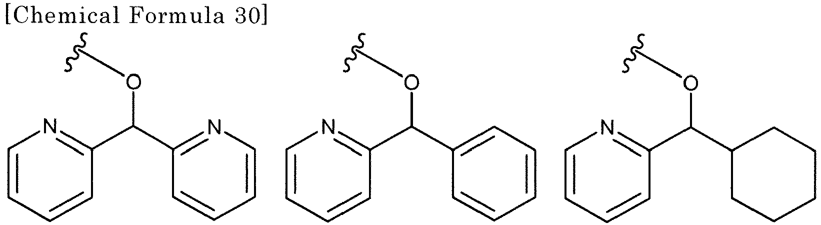 and the like.

"Non-aromatic heterocyclylalkyloxy" means an alkyloxy substituted with one or two or more "non-aromatic heterocyclyl" described above. "Non-aromatic heterocyclylalkyloxy" also includes "non-aromatic heterocyclylalkyloxy" wherein the alkyl part is substituted with the above "aromatic carbocyclyl", "non-aromatic carbocyclyl" and/or "aromatic heterocyclyl". For example, it includes tetrahydropyranylmethyloxy, morpholinylethyloxy, piperidinylmethyloxy, piperazinylmethyloxy, groups of the formula of 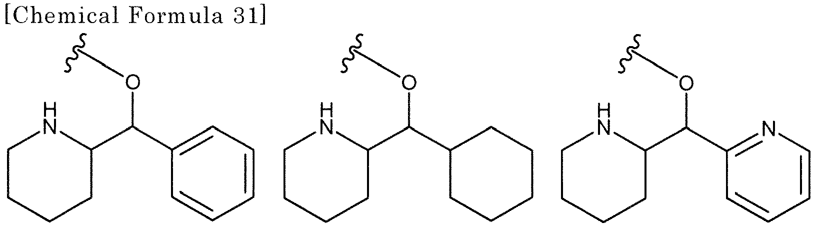 and the like.

"Aromatic carbocyclylalkyloxycarbonyl" means an alkyloxycarbonyl substituted with one or two or more "aromatic carbocyclyl" described above. For example, it includes benzyloxycarbonyl, phenethyloxycarbonyl, phenylpropyloxycarbonyl, benzhydryloxycarbonyl, trityloxycarbonyl, naphthylmethyloxycarbonyl, a group of the formula of: 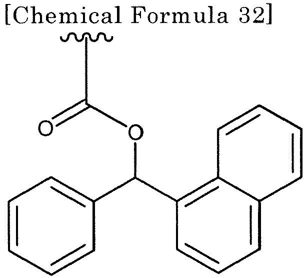 and the like.

"Non-aromatic carbocyclylalkyloxycarbonyl" means an alkyloxycarbonyl substituted with one or two or more "non-aromatic carbocyclyl" described above. "Non-aromatic carbocyclylalkyloxycarbonyl" also includes "non-aromatic carbocyclylalkyloxycarbonyl" wherein the alkyl part is substituted with the above "aromatic carbocyclyl". For example, it includes cyclopropylmethyloxycarbonyl, cyclobutylmethyloxycarbonyl, cyclopenthylmethyloxycarbonyl, cyclohexylmethyloxycarbonyl, a group of the formula of 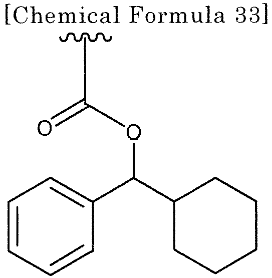 and the like.

"Aromatic heterocyclylalkyloxycarbonyl" means an alkyloxycarbonyl substituted with one or two or more "aromatic heterocyclyl" described above. "Aromatic heterocyclylalkyloxycarbonyl" also include "aromatic heterocyclylalkyloxycarbonyl" wherein the alkyl part is substituted with the above "aromatic carbocyclyl" and/or "non-aromatic carbocyclyl". For example, it includes pyridylmethyloxycarbonyl, furanylmethyloxycarbonyl, imidazolylmethyloxycarbonyl, indolylmethyloxycarbonyl, benzothiophenylmethyloxycarbonyl, oxazolylmethyloxycarbonyl, isoxazolylmethyloxycarbonyl, thiazolylmethyloxycarbonyl, isothiazolylmethyloxycarbonyl, pyrazolylmethyloxycarbonyl, isopyrazolylmethyloxycarbonyl, pyrrolidinylmethyloxycarbonyl, benzoxazolylmethyloxycarbonyl, groups of the formula of 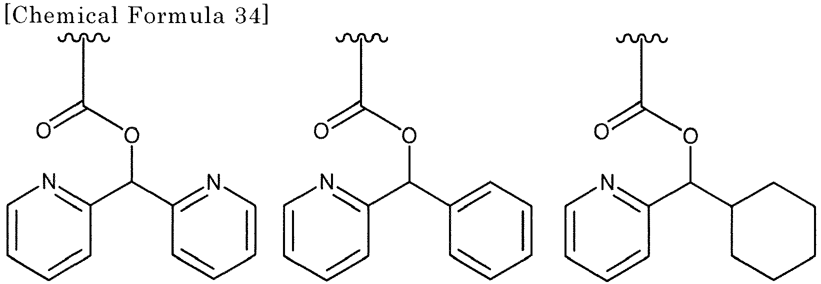 and the like.

"Non-aromatic heterocyclylalkyloxycarbonyl" means an alkyloxycarbonyl substituted with one or two or more "non-aromatic heterocyclyl" described above. "Non-aromatic heterocyclylalkyloxycarbonyl" also includes "non-aromatic heterocyclylalkyloxycarbonyl" wherein the alkyl part is substituted with the above "aromatic carbocyclyl", "non-aromatic carbocyclyl" and/or "aromatic heterocyclyl". For example, it includes tetrahydropyranylmethyloxy, morpholinylethyloxy, piperidinylmethyloxy, piperazinylmethyloxy, groups of the formula of 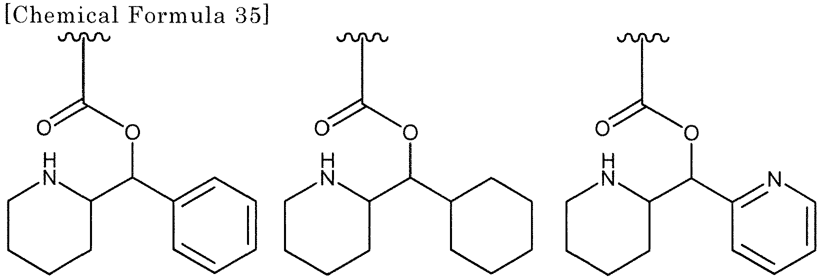 and the like.

"Aromatic carbocyclylalkyloxyalkyl" means an alkyloxyalkyl substituted with one or two or more "aromatic carbocyclyl" described above. For example, it includes benzyloxymethyl, phenethyloxymethyl, phenylpropyloxymethyl, benzhydryloxymethyl, trityloxymethyl, naphthylmethyloxymethyl, a group of the formula of: 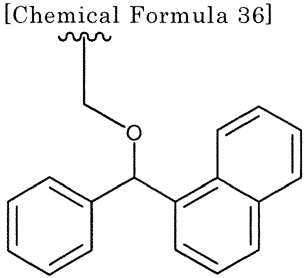 and the like.

"Non-aromatic carbocyclylalkyloxyalkyl" means an alkyloxyalkyl substituted with one or two or more "non-aromatic carbocyclyl" described above. "Non-aromatic carbocyclylalkyloxyalkyl" also includes "non-aromatic carbocyclylalkyloxyalkyl" wherein the alkyl part bonded to the non-aromatic carbocycle is substituted with the above "aromatic carbocyclyl". For example, it includes cyclopropylmethyloxymethyl, cyclobutylmethyloxymethyl, cyclopenthylmethyloxymethyl, cyclohexylmethyloxymethyl, a group of the formula of 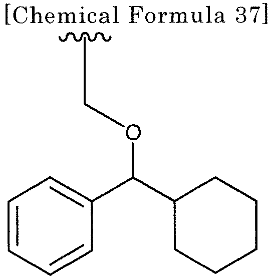 and the like.

"Aromatic heterocyclylalkyloxyalkyl" means an alkyloxyalkyl substituted with one or two or more "aromatic heterocyclyl" described above. "Aromatic heterocyclylalkyloxyalkyl" also includes "aromatic heterocyclylalkyloxyalkyl" wherein the alkyl part bonded to the aromatic heterocycle is substituted with the above "aromatic carbocyclyl" and/or "non-aromatic carbocyclyl". For example, it includes pyridylmethyloxymethyl, furanylmethyloxymethyl, imidazolylmethyloxymethyl, indolylmethyloxymethyl, benzothiophenylmethyloxymethyl, oxazolylmethyloxymethyl, isoxazolylmethyloxymethyl, thiazolylmethyloxymethyl, isothiazolylmethyloxymethyl, pyrazolylmethyloxymethyl, isopyrazolylmethyloxymethyl, pyrrolidinylmethyloxymethyl, benzoxazolylmethyloxymethyl, groups of the formula of 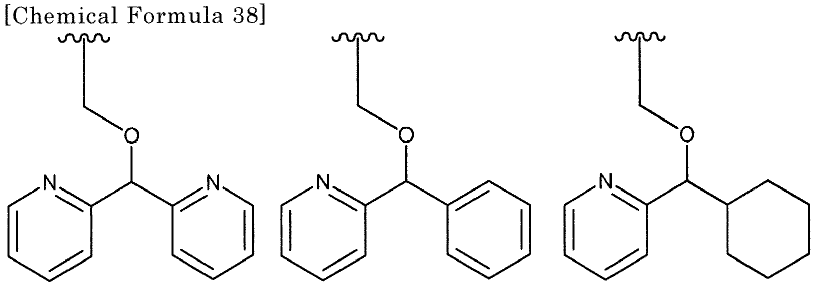 and the like.

"Non-aromatic heterocyclylalkyloxyalkyl" means an alkyloxyalkyl substituted with one or two or more "non-aromatic heterocyclyl" described above. "Non-aromatic heterocyclylalkyloxyalkyl" also includes "non-aromatic heterocyclylalkyloxyalkyl" wherein the alkyl part bonded to the non-aromatic heterocycle is substituted with the above "aromatic carbocyclyl", "non-aromatic carbocyclyl" and/or "aromatic heterocyclyl". For example, it includes tetrahydropyranylmethyloxymethyl, morpholinylethyloxymethyl, piperidinylmethyloxymethyl, piperazinylmethyloxymethyl, groups of the formula of 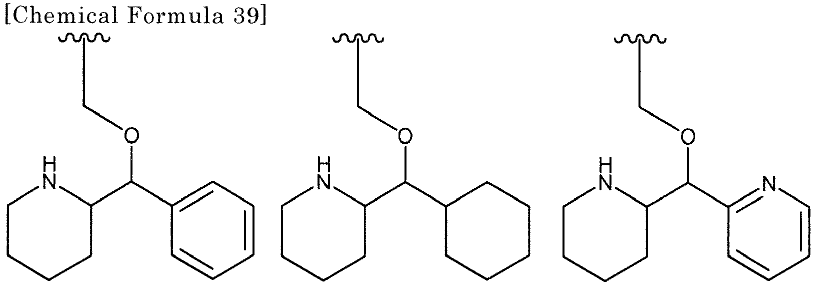 and the like.

"Carboxyalkyl" means a group wherein a hydrogen atom bonded to a carbon atom of the above "alkyl" is replaced with one or two or more carboxy group(s). For example, it includes carboxymethyl, 1-carboxyethyl, 2-carboxyethyl, 1-carboxypropyl, 2-carboxypropyl, 1,2-dicarboxyethyl, 2-carboxy-(1-methyl)ethyl, 2-carboxy-(1-carboxymethy])ethyl and the like.

An alkyl substituted with one or two carboxy group(s) is preferable.

The alkyl is preferably C1 to C8, more preferably C1 to C6, and further preferably C1 to C3 alkyl.

"Monoalkylamino" means a group wherein one hydrogen atom bonded to a nitrogen atom of an amino group is replaced with the above "alkyl". For example, it includes methylamino, ethylamino, isopropylamino and the like.

"Dialkylamino" means a group wherein two hydrogen atoms bonded to a nitrogen atom of an amino group are replaced with the above "alkyl". The two "alkyl" may be the same or different. For example, it includes dimethylamino, methylethylamino, methylisopropylamino, tert-butylmethylamino and the like.

"Hydroxyimino" means a N-hydroxyimino group.

"Hydroxyiminomethyl" means a N-hydroxyiminomethyl group and refers to a group represented by the following formula: 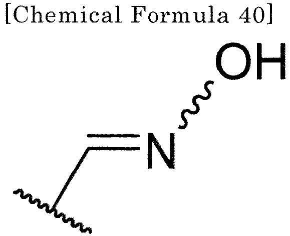 wherein the bond of the wavy line to the hydroxy group means that the bond is cis, trans, or a mixture thereof.

"Hydroxyiminoethyl" means a N-hydroxyiminoethyl group and refers to a group represented by the following formula: 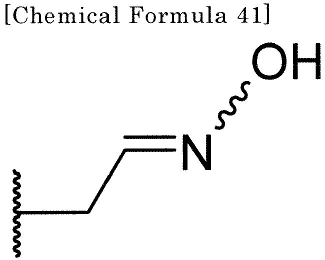 wherein the bond of the wavy line to the hydroxy group means that the bond is cis, trans, or a mixture thereof.

"Hydroxyiminopropyl" means a N-hydroxyiminopropyl group and refers to a group represented by the following formula: 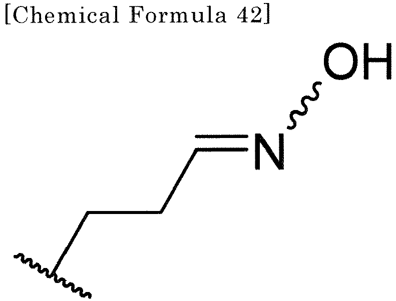 wherein the bond of the wavy line to the hydroxy group means that the bond is cis, trans, or a mixture thereof.

"Hydroxyalkyl" means a group wherein a hydrogen atom bonded to a carbon atom of the above "alkyl" is replaced with one or two or more hydroxy group(s). For example, it includes hydroxymethyl, 1-hydroxyethyl, 2-hydroxyethyl, 1-hydroxypropyl, 1,2-hydroxyethyl, 2-hydroxy-(1-hydroxymethyl)ethyl and the like.

An alkyl substituted one or two or more hydroxy group(s) is preferable.

The alkyl is preferably C1 to C8, more preferably C1 to C6, and further preferably C1 to C3 alkyl.

The "alkyloxy" part of "alkyloxycarbonylamino" or "alkyloxyaminocarbonyl" is the same as the above "alkyloxy".

The "alkylsulfonyl" part of "alkylsulfonylamino" or "alkylsulfonylaminocarbonylamino" is the same as the above "alkylsulfonyl".

The "alkylamino" part of "alkylaminosulfonylaminocarbonyl" includes the above "monoalkylamino" and "dialkylamino".

"Quaternary ammonium group" is a cyclic or non-cyclic saturated or unsaturated monovalent group containing a quaternary ammonium cation (N⁺). In the case of a non-cyclic group, a bond may be added to the quaternary ammonium cation, and in the case of a cyclic group, a bond may be added to any ring-constituting atom containing the quaternary ammonium cation. The group may have one or two or more heteroatom(s) selected from O, S and N, in addition to the quaternary ammonium cation. For example, it includes a trimethyl ammonium group, a pyridinium group, a pyrimidinium group, a pyrrolidinium group, a piperidinium group and the like.

A cyclic saturated or unsaturated monovalent group containing a quaternary ammonium cation is preferable.

The substituents of "substituted or unsubstituted quaternary ammonium group" include one or two or more group(s) selected from the substituent group B. The substituents may be bonded to an atom at any position(s) containing the quaternary ammonium cation. Furthermore, the substituent group B may be substituted with one or two or more group(s) selected from the substituent group Z. When two or more of the substituents are present, they may be the same or different. A group selected from the substituent group E is preferable.

The substituent group E: halogen, hydroxy, carboxy, alkyl, haloalkyl, alkyloxy, acyl, carbamoyl, sulfamoyl, alkylsulfonyl, alkyloxycarbonyl, carbocyclyl, heterocyclyl, carbocyclylalkyl, and heterocyclylalkyl.

The substituents of "substituted or unsubstituted hydroxyiminomethyl" include groups selected from the substituent group B. The substituents may be bonded to an atom at any position(s). Furthermore, the substituent group B may be substituted with one or two or more group(s) selected from the substituent group Z. When two or more of the substituents are present, they may be the same or different. A group selected from the substituent group H is preferable.

The substituent group H: alkyl, haloalkyl, hydroxyalkyl, carboxyalkyl, alkyloxy, carbocyclyl, heterocyclyl, carbocyclylalkyl, and heterocyclylalkyl.

"Aminoalkyl" means a group wherein a hydrogen atom bonded to a carbon atom of the above "alkyl" is replaced with one or two or more amino group(s). For example, it includes aminomethyl, aminoethyl, aminopropyl, 1,2-diaminoethyl and the like.

An alkyl substituted with one amino group is preferable. The alkyl is preferably C1 to C8, more preferably C1 to C6, and further preferably C1 to C3 alkyl.

"Cyanoalkyl" means a group wherein a hydrogen atom bonded to a carbon atom of the above "alkyl" is replaced with one or two or more cyano group(s). For example, it includes cyanomethyl, cyanoethyl, cyanopropyl, 1,3-dicyanopropyl and the like.

An alkyl substituted with one cyano group is preferable. The alkyl is preferably C1 to C8, more preferably C1 to C6, and further preferably C1 to C3 alkyl.

"Carbamoylalkyl" means a group wherein a hydrogen atom bonded to a carbon atom of the above "alkyl" is replaced with one or two or more carbamoyl group(s). For example, it includes carbamoylmethyl, carbamoylethyl, carbamoylpropyl, 1,3-carbamoylpropyl and the like.

An alkyl substituted one carbamoyl group is preferable. The alkyl is preferably C1 to C8, more preferably C1 to C6, and further preferably C1 to C3 alkyl.

"Sulfoalkyl" means a group wherein a hydrogen atom bonded to a carbon atom of the above "alkyl" is replaced with one or two or more sulfo group(s). For example, it includes sulfomethyl, sulfoethyl, sulfopropyl, 1,3-disulfopropyl and the like.

An alkyl substituted with one sulfo group is preferable. The alkyl is preferably C1 to C8, more preferably C1 to C6, and further preferably C1 to C3 alkyl.

"Hydroxyiminoalkyl" means a group wherein a hydrogen atom bonded to a carbon atom of the above "alkyl" is replaced with one hydroxyimino. For example, it includes hydroxyiminomethyl, hydroxyiminoethyl, hydroxyiminopropyl, hydroxyiminobutyl, hydroxyiminopentyl, hydroxyiminohexyl and the like.

The alkyl is preferably C1 to C8, more preferably C1 to C6, and further preferably C1 to C3 alkyl.

Examples or preferred embodiments of R¹, R^{2A}, R^{2B}, R³, W, T, R^{4A}, R^{4B}, R^{5A}, R^{5B}, R^{6A}, R^{6B}, R^{7A}, R^{7B}, X, R⁸, R⁹, R¹¹, R¹², Q, m and R¹⁰ in the compound represented by formula (I) or (I') are described. However, the scope of the present invention is not limited by those described above. A compound having a possible combination of those described above is preferable.

A preferred embodiment of the ring of "substituted or unsubstituted carbocyclyl or substituted or unsubstituted heterocyclyl" of R¹ is a 5- or 6-membered ring, and a more preferred embodiment is substituted or unsubstituted aromatic carbocyclyl or substituted or unsubstituted aromatic heterocyclyl. Preferred examples of the substituents include halogen, hydroxy, amino and the like. The substituents may be added to any one or several possible position(s). The number of substituents is preferably 1 to 3, more preferably 1 or 2.

Preferred examples of R¹ include phenyl, hydroxyphenyl, dihydroxyphenyl, phenyl substituted with any number of halogen, phenyl substituted with any number of halogen and hydroxy, aminothiazolyl, aminothiazolyl substituted with any number of halogen, aminothiadiazolyl, isothiazolyl, aminoisothiazolyl, aminoisothiazolyl substituted with any number of halogen, thienyl, furyl, benzothiazolyl, pyridyl, aminopyridyl, pyrimidinyl, aminopyrimidinyl, pyridazinyl, pyridyl substituted with any number of halogen and amino, pyrimidinyl substituted with any number of halogen and amino and the like. A more preferred example is phenyl, hydroxyphenyl, dihydroxyphenyl, chlorodihydroxyphenyl, aminothiazolyl, aminothiazolyl substituted with any number of halogen, aminothiadiazolyl, aminopyridyl, aminopyrimidinyl, aminoisothiazolyl or the like. A further preferred example is aminothiazolyl, aminochlorothiazolyl, aminofluorothiazolyl, aminobromothiazolyl or aminothiadiazolyl.

Preferred specific examples of R¹ include a group of the formulas of: 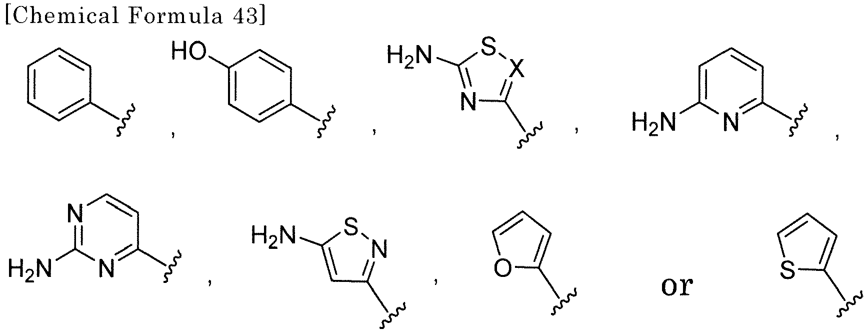 wherein X is CH, CCl, CF, CBr or N.

More preferred specific examples of R¹ include a group of the formula of: 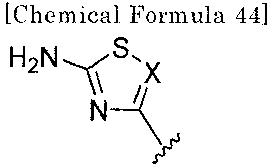 wherein X is CH, CCl, CF, CBr or N.

A more preferred example of X is CH, CF or N.

Particularly preferred examples of the carbocyclyl of R¹ include groups of the formula of:

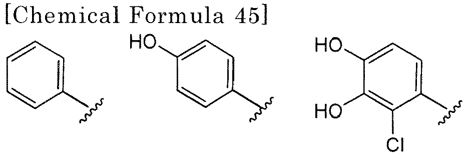

Particularly preferred examples of the heterocyclyl of R¹ include groups of the formula of:

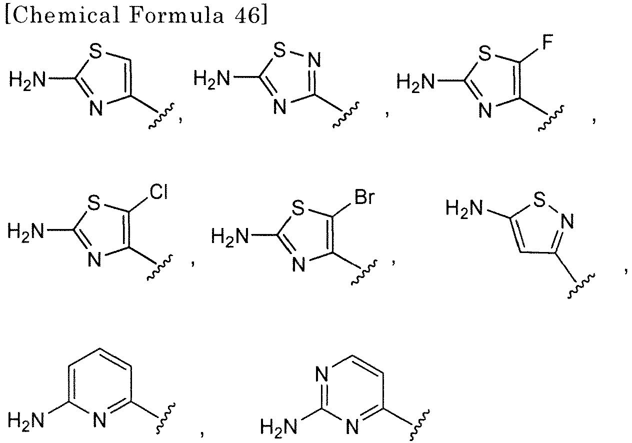

Further preferred examples of the heterocyclyl of R¹ include groups of the formula of:

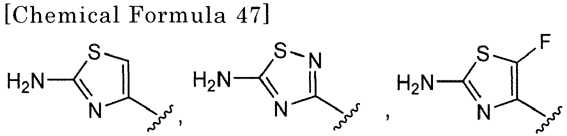

As for R^{2A} and R^{2B}, a) when R^{2A} and R^{2B} are each independently a hydrogen atom, substituted or unsubstituted amino, sulfo, substituted or unsubstituted sulfamoyl, carboxy, substituted or unsubstituted alkyloxycarbonyl, substituted or unsubstituted carbamoyl, hydroxy, or carbonyloxy having a substituent, preferred examples of the following formula: 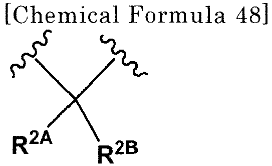 include the following formula: 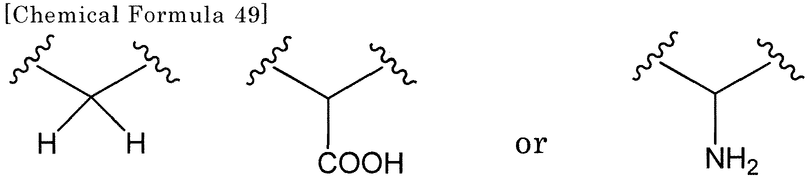 substituted amino of the formulas of: 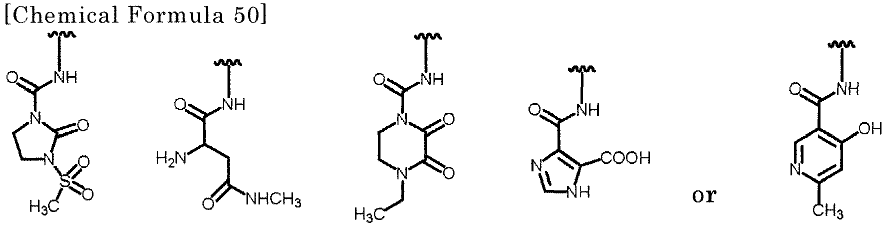 substituted sulfamoyl of the formulas of: 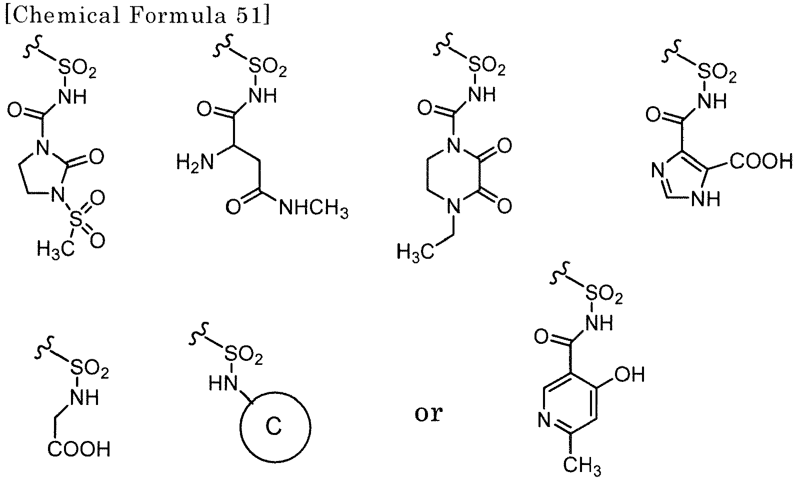 wherein Ring C represents substituted or unsubstituted heterocyclyl; substituted carbamoyl of the formulas of: 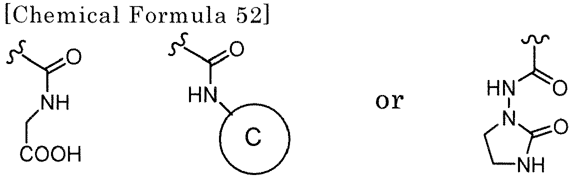 wherein Ring B represents substituted or unsubstituted heterocyclyl; or substituted carbonyloxy of the formulas of: 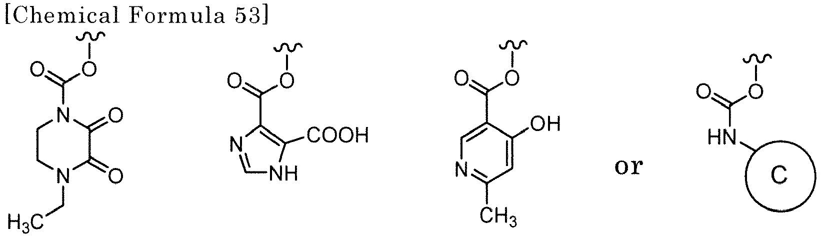 wherein Ring C represents substituted or unsubstituted heterocyclyl and the like.

Alternatively, when R^{2A} and R^{2B} are taken together to form substituted or unsubstituted methylidene, a group represented by the formula of: 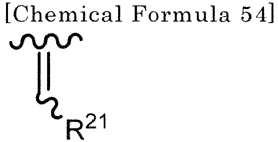 wherein R²¹ is substituted or unsubstituted alkyl, and the wavy line between the carbon atom to form a double bond and R²¹ represents cis, trans or a mixture thereof, is preferable. A preferred substituent of the substituted or unsubstituted alkyl of R²¹ is halogen, hydroxy, or carboxy, and carboxy is more preferable. R²¹ is preferably substituted or unsubstituted C1 to C3 alkyl.
The formula: 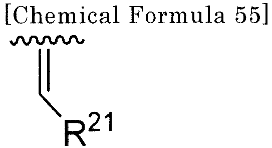 wherein R²¹ is the same as above
is preferable.

A preferred embodiment of the formula of: 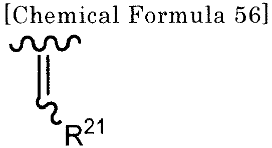 is 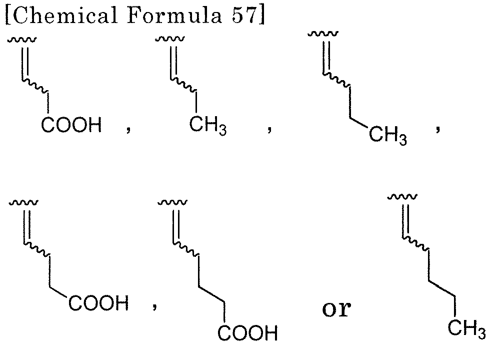 wherein the wavy line between the carbon atom to form a double bond and the carboxy group or the alkyl group means cis, trans or a mixture thereof, more preferably 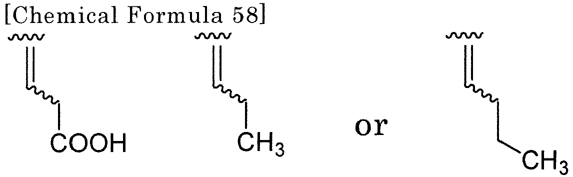 wherein the wavy line between the carbon atom to form a double bond and the carboxy group or the alkyl group means cis, trans or a mixture thereof, and further preferably

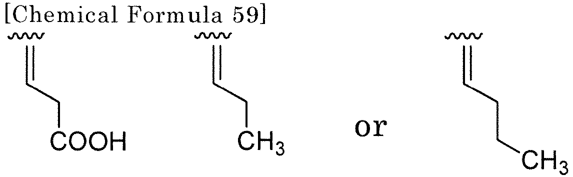

Alternatively, R^{2A} and R^{2B} may be taken together to form substituted or unsubstituted hydroxyimino and are taken together to be preferably a group represented by the formula of: 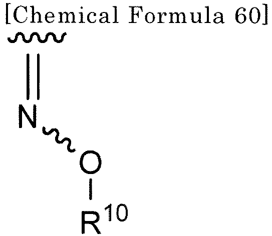 wherein R¹⁰ is a hydrogen atom, substituted or unsubstituted carbocyclyl, substituted or unsubstituted heterocyclyl, or substituted or unsubstituted alkyl, and the wavy line between the nitrogen atom of the imino group and the oxygen atom represents cis, trans, or a mixture thereof,
and more preferably a group represented by the formula of: 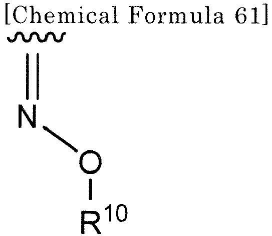 wherein R¹⁰ is the same as above.

When R¹⁰ is substituted or unsubstituted alkyl, a preferred embodiment of the substituents is halogen, hydroxy, carboxy, cyano, substituted or unsubstituted amino, substituted or unsubstituted carbamoyl, sulfo, phospho, ureido, substituted or unsubstituted sulfamoyl, substituted or unsubstituted alkyloxy, substituted or unsubstituted alkenyloxy, substituted or unsubstituted alkynyloxy, acyl, acyloxy, substituted or unsubstituted alkyloxycarbonyl, substituted or unsubstituted alkenyloxycarbonyl, substituted or unsubstituted alkynyloxycarbonyl, substituted or unsubstituted carbocyclyl, substituted or unsubstituted heterocyclyl, substituted or unsubstituted heterocyclyloxy, substituted or unsubstituted carbocyclyloxy, substituted or unsubstituted carbocyclylcarbonyl, substituted or unsubstituted heterocyclylcarbonyl, substituted or unsubstituted carbocyclyloxycarbonyl, substituted or unsubstituted heterocyclyloxycarbonyl or the like, and a group selected from halogen, hydroxy, carboxy, substituted or unsubstituted amino, substituted or unsubstituted carbamoyl, substituted or unsubstituted alkyloxy, substituted or unsubstituted hydroxyiminomethyl, and a substituted or unsubstituted quaternary ammonium group and the like is more preferable. The substituents may be added to any one or two or more possible position(s). When two or more of the substituents are present, they may be the same or different.

A more preferred embodiment of the substituents is a group selected from halogen, hydroxy, carboxy, cyano, substituted or unsubstituted amino, substituted or unsubstituted carbamoyl, sulfo, substituted or unsubstituted heterocyclyl, substituted or unsubstituted hydroxyiminomethyl, and a substituted or unsubstituted quaternary ammonium group.

A further preferred embodiment of the substituents is a group selected from halogen, hydroxy, carboxy, cyano, amino optionally substituted with the substituent group E, carbamoyl optionally substituted with the substituent group E, sulfo, heterocyclyl optionally substituted with the substituent group E, hydroxyiminomethyl optionally substituted with the substituent group H, and a quaternary ammonium group optionally substituted with the substituent group E.

When R¹⁰ is substituted or unsubstituted carbocyclyl or substituted or unsubstituted heterocyclyl, a preferred embodiment of the substituents is halogen, hydroxy, carboxy, cyano, sulfo, substituted or unsubstituted alkyl, substituted or unsubstituted amino, substituted or unsubstituted carbamoyl, phospho, ureido, substituted or unsubstituted hydroxyiminomethyl, a substituted or unsubstituted quaternary ammonium group or the like.

A more preferred embodiment of the substituents is halogen, hydroxy, carboxy, carbamoyl, sulfo, cyano, hydroxyalkyl, carboxyalkyl, carbamoylalkyl, sulfoalkyl, cyanoalkyl or the like.

A further preferred embodiment of the substituents is hydroxy, carboxy, hydroxymethyl, or carboxymethyl.

R¹⁰ is preferably a hydrogen atom, alkyl, substituted or unsubstituted non-aromatic carbocyclyl, substituted or unsubstituted aromatic heterocyclylalkyl, substituted or unsubstituted haloalkyl, substituted or unsubstituted aminoalkyl, substituted or unsubstituted cyanoalkyl, substituted or unsubstituted carboxyalkyl, substituted or unsubstituted carbamoylalkyl, substituted or unsubstituted sulfoalkyl, substituted or unsubstituted hydroxyalkyl, substituted or unsubstituted hydroxyiminoalkyl, substituted or unsubstituted pyridiniumalkyl, and more preferably non-aromatic carbocyclyl optionally substituted with the substituent group F, aromatic heterocyclylalkyl optionally substituted with the substituent group F, haloalkyl optionally substituted with the substituent group G, carboxyalkyl optionally substituted with the substituent group G, cyanoalkyl optionally substituted with the substituent group G, aminoalkyl optionally substituted with the substituent group E, carbamoylalkyl optionally substituted with the substituent group G, sulfoalkyl optionally substituted with the substituent group G, hydroxyalkyl optionally substituted with the substituent group G, or pyridiniumalkyl optionally substituted with the substituent group E.

The substituent group G: halogen, hydroxy, and carboxy.

When R^{2A} and R^{2B} are taken together to form substituted or unsubstituted hydroxyimino, preferred examples include the following group: 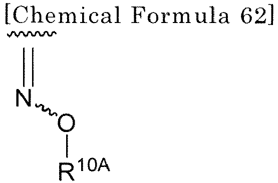 wherein R^{10A} is a hydrogen atom, alkyl optionally substituted with the substituent group D, or carbocyclyl optionally substituted with the substituent group D, and the wavy line between the nitrogen atom of the imino group and the oxygen atom represents cis, trans, or a mixture thereof.

The substituent group D: halogen, hydroxy, carboxy, cyano, amino optionally substituted with the substituent group C, carbamoyl optionally substituted with the substituent group C, sulfo, phospho, borono, sulfamoyl optionally substituted with the substituent group C, alkyloxy, alkenyloxy, alkynyloxy, acyl, acyloxy, alkyloxycarbonyl, alkenyloxycarbonyl, alkynyloxycarbonyl, hydroxyiminomethyl optionally substituted with the substituent group H, heterocyclyl optionally substituted with the substituent group F, carbocyclyl optionally substituted with the substituent group F, heterocyclyloxy, carbocyclyloxy, carbocyclylcarbonyl, heterocyclylcarbonyl, oxycarbonyl, heterocyclyloxycarbonyl, and a quaternary ammonium group optionally substituted with the substituent group E.

The substituent group F: halogen, hydroxy, carboxy, oxo, alkyl, and haloalkyl.

More preferred examples include the following group: 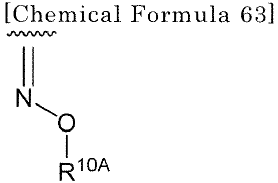 wherein R^{10A} is the same as above.

A preferred example of R^{10A} is a hydrogen atom, alkyl optionally substituted with the substituent group I, non-aromatic carbocyclyl optionally substituted with the substituent group F.

The substituent group I: halogen, hydroxy, carboxy, cyano, amino optionally substituted with the substituent group C, carbamoyl optionally substituted with the substituent group C, sulfo, sulfamoyl optionally substituted with the substituent group C, heterocyclyl optionally substituted with the substituent group F, and a quaternary ammonium group optionally substituted with the substituent group E.

When R^{2A} and R^{2B} are taken together to form substituted or unsubstituted hydroxyimino, another preferred example includes the following group: 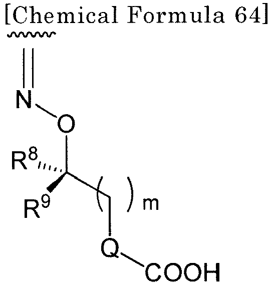 wherein R⁸ and R⁹ are each independently a hydrogen atom, halogen, hydroxy, carboxy, substituted or unsubstituted amino, substituted or unsubstituted carbamoyl, substituted or unsubstituted alkyl, substituted or unsubstituted carbocyclyl, or substituted or unsubstituted heterocyclyl, R⁸ and R⁹ are taken together to form substituted or unsubstituted methylidene, or R⁸ and R⁹ are taken together with the adjacent atoms to form substituted or unsubstituted carbocyclyl or substituted or unsubstituted heterocyclyl; Q is a single bond, substituted or unsubstituted carbocyclyl or substituted or unsubstituted heterocyclyl; and m is an integer from 0 to 3. 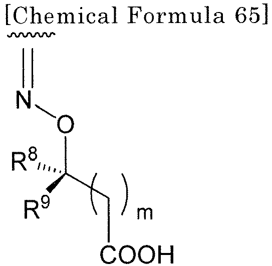 wherein R⁸ and R⁹ are each independently a hydrogen atom, halogen, hydroxy, carboxy, alkyl, hydroxyalkyl, or carboxyalkyl, or R⁸ and R⁹ are taken together with the adjacent atoms to form carbocyclyl optionally substituted with the substituent group F or heterocyclyl optionally substituted with the substituent group F; and m is 0 or 1
is more preferable.

When R^{2A} and R^{2B} are taken together to form substituted or unsubstituted hydroxyimino, a further alternative preferred embodiment is the following group: 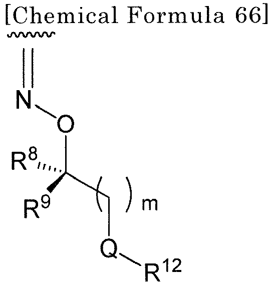 wherein R⁸ and R⁹ are each independently a hydrogen atom, halogen, hydroxy, carboxy, substituted or unsubstituted alkyl, substituted or unsubstituted carbocyclyl, or substituted or unsubstituted heterocyclyl, R⁸ and R⁹ are taken together to form substituted or unsubstituted methylidene, or R⁸ and R⁹ are taken together with the adjacent atoms to form substituted or unsubstituted non-aromatic carbocycle or substituted or unsubstituted non-aromatic heterocycle; Q is a single bond, substituted or unsubstituted carbocyclediyl, or substituted or unsubstituted heterocyclediyl; m is an integer from 0 to 3; and R¹² is a hydrogen atom, haloalkyl, or a group selected from the substituent group D. The formula: 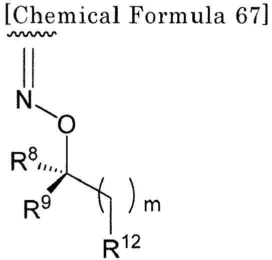 wherein R⁸ and R⁹ are each independently a hydrogen atom, halogen, hydroxy, carboxy, alkyl, hydroxyalkyl, or carboxyalkyl, or R⁸ and R⁹ are taken together with the adjacent atoms to form carbocyclyl optionally substituted with the substituent group F or heterocyclyl optionally substituted with the substituent group F; m is 0 or 1; and R¹² is a hydrogen atom, haloalkyl, or a group selected from the substituent group D
is more preferable.

R¹² is preferably a hydrogen atom, halogen, hydroxy, carboxy, cyano, amino optionally substituted with the substituent group C, carbamoyl optionally substituted with the substituent group C, sulfo, sulfamoyl optionally substituted with the substituent group C, hydroxyiminomethyl optionally substituted with the substituent group H, heterocyclyl optionally substituted with the substituent group F, or a quaternary ammonium group optionally substituted with the substituent group E.

R¹² is more preferably a hydrogen atom, halogen, hydroxy, carboxy, cyano, amino, sulfo, carbamoyl, sulfamoyl, hydroxyiminomethyl, trifluoromethyl, alkyloxycarbonylamino, sulfamoylamino, acylamino, hydroxycarbamoyl, or alkyloxycarbamoyl.

Q is preferably a single bond.

m is preferably an integer from 0 to 2, and more preferably 0 or 1.

Examples of the case where "R⁸ and R⁹ are each independently a hydrogen atom, halogen, hydroxy, carboxy, substituted or unsubstituted amino, substituted or unsubstituted carbamoyl, substituted or unsubstituted alkyl, substituted or unsubstituted carbocyclyl, or substituted or unsubstituted heterocyclyl" include a hydrogen atom, a fluorine atom, a chlorine atom, hydroxy, carboxy, methyl, ethyl, isopropyl, tert-butyl, monofluoromethyl, difluoromethyl, trifluoromethyl, carboxymethyl, carboxyethyl, carbamoylmethyl, carbamoylethyl, hydroxymethyl, hydroxyethyl, methoxymethyl, ethoxymethyl, methoxyethyl, ethoxyethyl, methylthiomethyl, ethylthiomethyl, benzyl, 4-hydroxybenzyl, 4-methoxybenzyl, 4-carboxybenzyl, 3,4-dihydroxyphenyl, naphthyl, cyclopropyl, cyclobutyl, cyclopentyl, cyclohexyl, pyrrolyl, imidazolyl, pyrazolyl, pyridyl, pyridazinyl, pyrimidinyl, pyrazinyl, triazolyl, triazinyl, tetrazolyl, isoxazolyl, oxazolyl, oxadiazolyl, isothiazolyl, thiazolyl, thiadiazolyl, furyl, and thienyl and the like.

Preferably, R⁸ and R⁹ are each independently a hydrogen atom, a fluorine atom, methyl, ethyl, isopropyl, carboxymethyl, hydroxymethyl, or hydroxyethyl.

A preferred combination of R⁸ and R⁹ is (R⁸, R⁹) = (a hydrogen atom, a hydrogen atom), (a hydrogen atom, a fluorine atom), (a fluorine atom, a hydrogen atom), (a hydrogen atom, methyl), (methyl, a hydrogen atom), (methyl, methyl), (a hydrogen atom, ethyl), (ethyl, a hydrogen atom), (ethyl, ethyl), (a hydrogen atom, isopropyl), (isopropyl, a hydrogen atom), (isopropyl, isopropyl), (a hydrogen atom, carboxymethyl), (carboxymethyl, a hydrogen atom), (a hydrogen atom, hydroxymethyl), (hydroxymethyl, a hydrogen atom), (a hydrogen atom, hydroxyethyl), or (hydroxyethyl, a hydrogen atom).

A more preferred combination of R⁸ and R⁹ is (R⁸, R⁹) = (a hydrogen atom, a hydrogen atom), (a hydrogen atom, a fluorine atom), (a fluorine atom, a hydrogen atom), (a hydrogen atom, methyl), (methyl, a hydrogen atom), (methyl, methyl), (a hydrogen atom, ethyl), (ethyl, a hydrogen atom), (a hydrogen atom, isopropyl), (isopropyl, a hydrogen atom), (a hydrogen atom, hydroxymethyl), (hydroxymethyl, a hydrogen atom), (a hydrogen atom, hydroxyethyl), or (hydroxyethyl, a hydrogen atom).

An alternative preferred combination of R⁸ and R⁹ is (R⁸, R⁹) = (a hydrogen atom, a hydrogen atom), (methyl, a hydrogen atom), (methyl, methyl), (ethyl, a hydrogen atom), (isopropyl, a hydrogen atom), (hydroxymethyl, a hydrogen atom), or (hydroxyethyl, a hydrogen atom).

A further preferred embodiment is the following group: 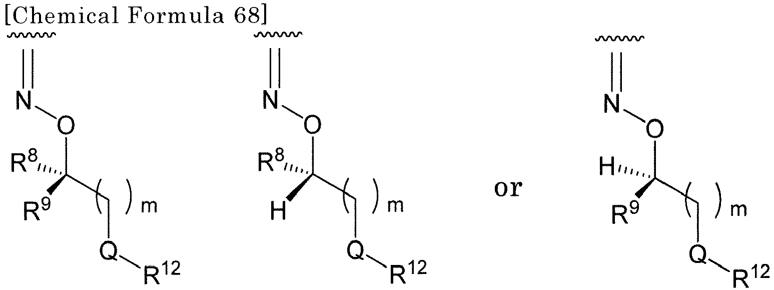 wherein each symbol is the same as above.

A more preferred embodiment is a group of the formulas of:

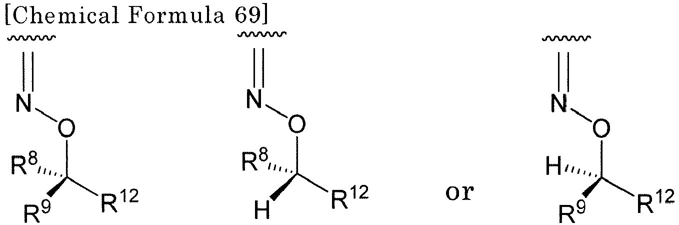

Examples of the case where "R⁸ and R⁹ are taken together to form substituted or unsubstituted methylidene" include the following formula: 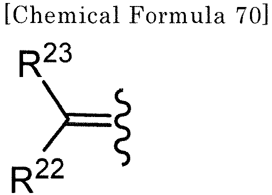 wherein R²² and R²³ are each independently a hydrogen atom or substituted or unsubstituted alkyl. As preferred examples, R²² and R²³ are each independently a hydrogen atom, methyl, fluoromethyl, trifluoromethyl, ethyl or the like. More preferably, both of them are hydrogen atoms.

When "R⁸ and R⁹ are taken together with the adjacent atoms to form substituted or unsubstituted non-aromatic carbocycle or substituted or unsubstituted non-aromatic heterocycle", a 3- to 6-membered ring which is monocyclic is preferable, and a 3- or 4-membered ring is more preferable. Preferred examples include cyclopropane, cyclobutane, cycloheptane, cyclohexane, aziridine, oxirane, thiirane, aziridine, thiirene, azetidine, oxetane, thietane, pyrrolidine, tetrahydrofuran, tetrahydrothiophene, piperidine, tetrahydropyran, tetrahydrothiopyran and the like. More preferred examples include cyclopropane and cyclobutane.

When "R⁸ and R⁹ are taken together with the adjacent atoms to form substituted or unsubstituted non-aromatic carbocycle or substituted or unsubstituted non-aromatic heterocycle", the substituents are one to five group(s) selected from the substituent group B. When a plurality of the substituents are present, they may be the same or different. One to three group(s) selected from the substituent group F are more preferable. When a plurality of the substituents are present, they may be the same or different.

Preferred embodiments of R⁸ and R⁹ are each independently a hydrogen atom, a fluorine atom, methyl, ethyl, isopropyl, carboxy, hydroxy, carboxymethyl, hydroxymethyl, or hydroxyethyl, or R⁸ and R⁹ are taken together with the adjacent atoms to be non-aromatic carbocycle optionally substituted with the substituent group F.

More preferred embodiments of R⁸ and R⁹ are each independently a hydrogen atom, a fluorine atom, methyl, hydroxy, carboxy, carboxymethyl, hydroxymethyl, or hydroxyethyl, or R⁸ and R⁹ are taken together with the adjacent atoms to be cyclopropyl optionally substituted with the substituent group F.

R³ is a hydrogen atom, OCH₃ or NH-CH(=O) and is preferably a hydrogen atom.

R¹¹ is carboxy or tetrazolyl. When R¹¹ is tetrazolyl, a group of any of the following formulas: 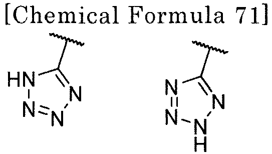 is preferable.

R¹¹ is preferably carboxy.

-W- is -S(=O)- or -S(=O)₂-. When -W- is -S(=O)-, it includes a group of the following formula: 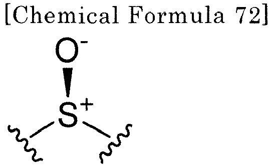 or 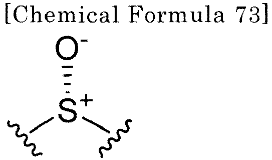 or a mixture thereof, and is preferably a group of the following formula: 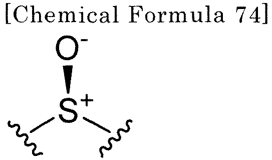 or 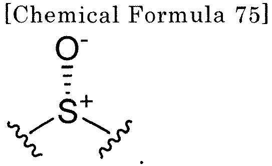
-W- is preferably a group of the following formula: 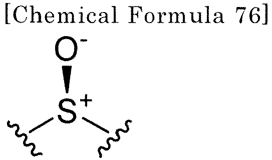 or 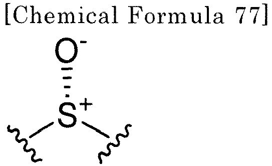
or -S(=O)₂-.
-T- is -CR^{4A}R^{4B}- or -CR^{5A}R^{5B}-CR^{6A}R^{6B}-. Preferred examples of R^{4A}, R^{4B}, R^{5A}, R^{5B}, R^{6A} and R^{6B} each independently include a hydrogen atom, halogen, hydroxy, amino, monoalkylamino, dialkylamino, carboxy, carboxy, sulfamoyl, monoalkylsulfamoyl, dialkylsulfamoyl, alkyl, haloalkyl, haloalkyloxy, alkyloxy, acyl, acyloxy, cyano, amidino, guanidino, and carbamoyloxy. Preferred embodiments of R^{4A} and R^{4B} are each independently a hydrogen atom, hydroxy, amino, carboxy, methyl, methoxy, trifluoromethyl, trifluoromethoxy, or cyano. More preferably, R^{4A} is a hydrogen atom, and R^{4B} is a hydrogen atom or hydroxy. Preferred embodiments of R^{5A}, R^{5B}, R^{6A} and R^{6B} are each independently a hydrogen atom, hydroxy, amino, carboxy, methyl, methoxy, trifluoromethyl, trifluoromethoxy, or cyano. As more preferred embodiments, each of R^{5A} and R^{6A} is a hydrogen atom, and R^{5B} and R^{6B} are each independently a hydrogen atom or hydroxy. Further preferably, each of R^{5A}, R^{5B}, R^{6A} and R^{6B} is a hydrogen atom. -T- is preferably -CR^{4A}R^{4B}-, and more preferably -CH₂-, -C(CH₃)H-, -C(CH₃)₂- or -C(OH)H-.

Another preferred embodiment of -T- is a group of the following formula: 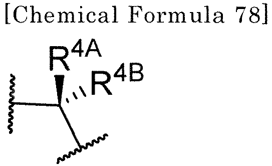 wherein R^{4A} and R^{4B} are the same as above.

R^{4A} is preferably a hydrogen atom, and R^{4B} is preferably a hydrogen atom, methyl, or hydroxy. More preferably, each of R^{4A} and R^{4B} is a hydrogen atom, or R^{4A} is a hydrogen atom, and R^{4B} is methyl.

R^{7A} and R^{7B} are each independently a hydrogen atom or substituted or unsubstituted alkyl. A preferred substituent of "substituted or unsubstituted alkyl" is a group selected from halogen, hydroxy, cyano, alkyloxy, and haloalkyloxy. A more preferred substituent is halogen. The alkyl of "substituted or unsubstituted alkyl" is C1 to C8 alkyl and is preferably C1 to C6, more preferably C1 to C3, and further preferably C1 alkyl.

Preferred embodiments of R^{7A} and R^{7B} are each independently a hydrogen atom or alkyl. More preferably, they are each independently a hydrogen atom or methyl. As further preferred embodiments, both of R^{7A} and R^{7B} are hydrogen atoms, or any one of R^{7A} and R^{7B} is a hydrogen atom, and the other is methyl. As particularly preferred embodiments, both of R^{7A} and R^{7B} are hydrogen atoms, or R^{7A} is methyl, and R^{7B} is a hydrogen atom.

Another preferred embodiment of R^{7A} and R^{7B} is a group of the following formula: 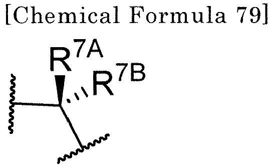 wherein R^{7A} and R^{7B} are the same as above.

In formula (I), a preferred combination of -W- and -T- is described below. i) -W- is represented by the following formula: 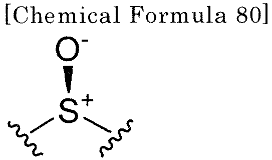 or 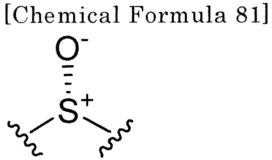 and -T- is -CR^{4A}R^{4B}- or -CR^{5A}R^{5B}-CR^{6A}R^{6B}-.
ii) -W- is -S(=O)₂, and -T- is -CR^{4A}R^{4B}- or -CR^{5A}R^{5B}-CR^{6A}R^{6B}-.

A preferred embodiment of the following formula: 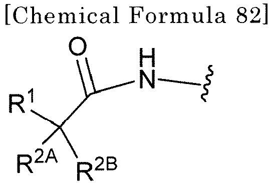 is the following formulas: 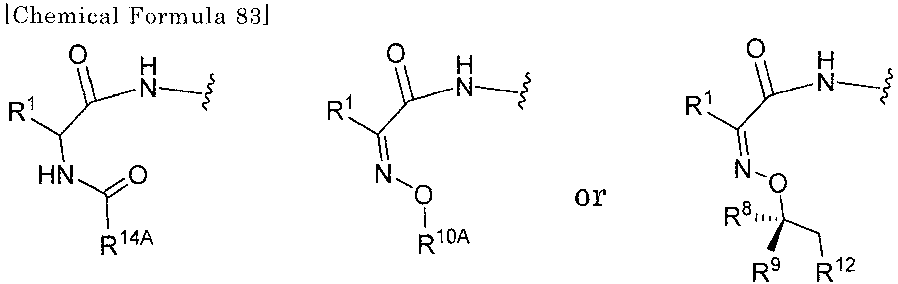 wherein R¹ is represented by the following formulas: 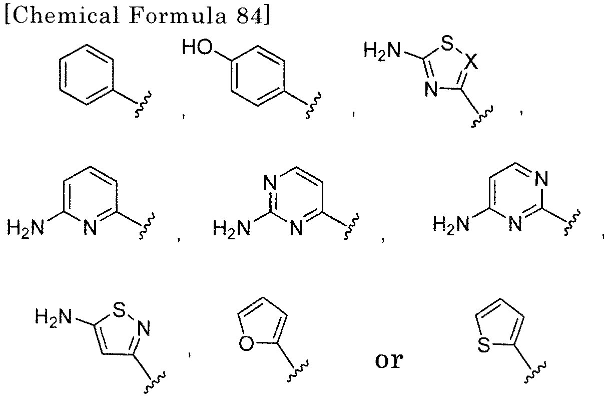 wherein X is CH, CCl, CF, CBr or N; and
the other symbols are the same as the above.

Preferred embodiments of the compounds of the present invention are illustrated below. All combinations of specific examples are illustrated as the compounds described in the following embodiments.

### (Embodiment 1)

-W- is represented by the following formula: 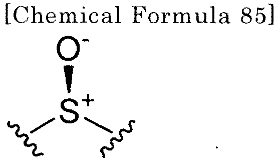 or 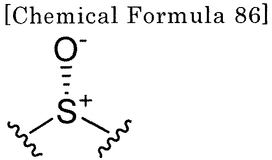
-T- is -CR^{4A}R^{4B}- or -CR^{5A}R^{5B}-CR^{6A}R^{6B}-;
R^{4A} and R^{4B} are each independently a hydrogen atom or hydroxy;
each of R^{5A}, R^{5B}, R^{6A} and R^{6B} is a hydrogen atom;
R¹ is represented by the following formulas: 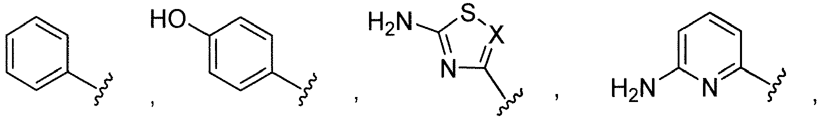 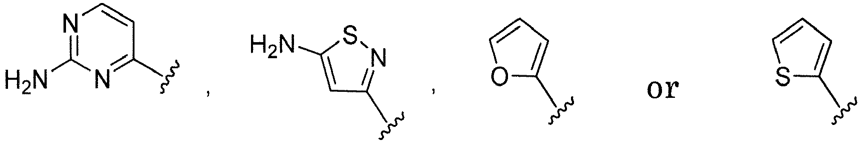 wherein each symbol is the same as above;
   R^{2A} and R^{2B} are taken together to be methylidene having a substituent of the formulas of: 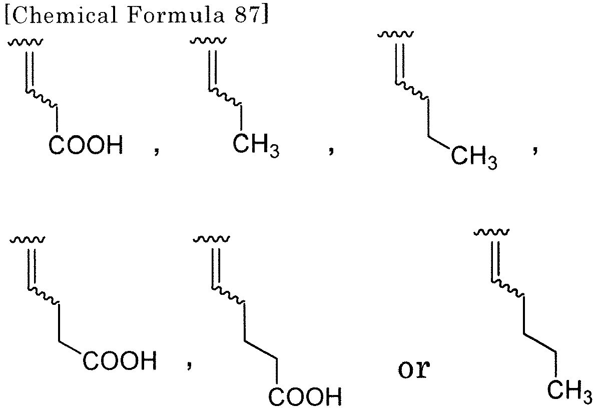 wherein the wavy line between the carbon atom to form a double bond and the carboxy group or the alkyl group means cis, trans or a mixture thereof,
   or a group of the following formula: 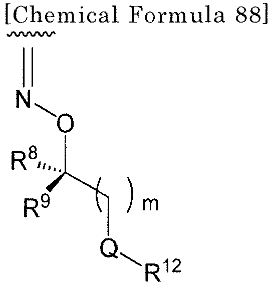 wherein R⁸ and R⁹ are each independently a hydrogen atom, halogen, hydroxy, carboxy, alkyl, or hydroxyalkyl, or R⁸ and R⁹ are taken together with the adjacent carbon atom to form substituted or unsubstituted non-aromatic carbocycle or substituted or unsubstituted non-aromatic heterocycle; m is an integer from 0 to 3; and R¹² is a hydrogen atom, carboxy, sulfo, carbamoyl, hydroxycarbamoyl, alkyloxycarbamoyl, or hydroxy;
   R^{7A} is a hydrogen atom or alkyl; R^{7B} is a hydrogen atom;
   R¹¹ is carboxy; and
   R³ is a hydrogen atom or OCH₃.

### (Embodiment 2)

-W- is -S(=O)₂;
-T- is -CR^{4A}R^{4B}- or -CR^{5A}R^{5B}-CR^{6A}R^{6B}-;
R^{4A} and R^{4B} are each independently a hydrogen atom or hydroxy;
each of R^{5A}, R^{5B}, R^{6A} and R^{6B} is a hydrogen atom;
R¹ is represented by the following formulas: 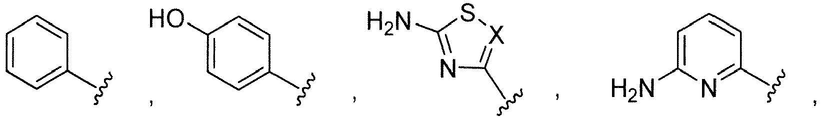 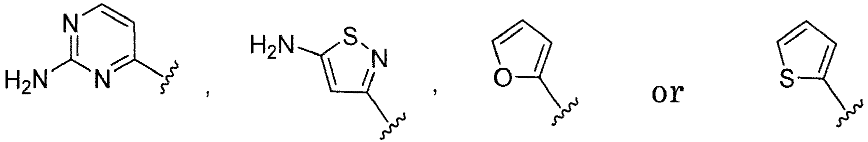 wherein each symbol is the same as above;
   R^{2A} and R^{2B} are taken together to be methylidene having a substituent of the formulas of: 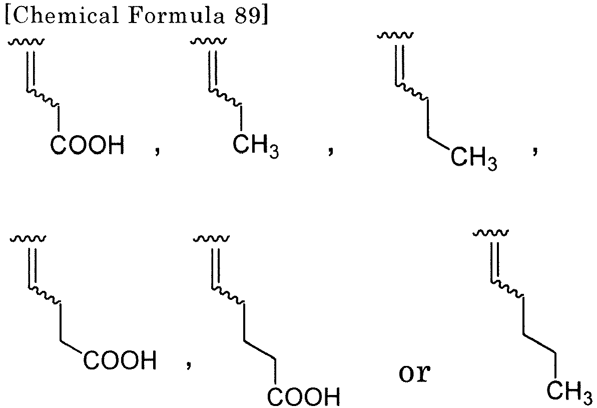 wherein the wavy line between the carbon atom to form a double bond and the carboxy group or the alkyl group means cis, trans or a mixture thereof,
   or a group of the following formula: 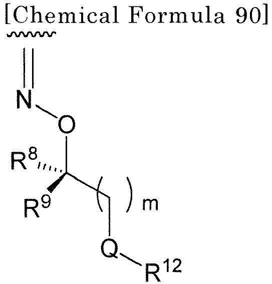 wherein each symbol is the same as above; and
   R³ is a hydrogen atom or OCH₃.

### (Embodiment 3)

Formula (I) is the following formula (I-1): 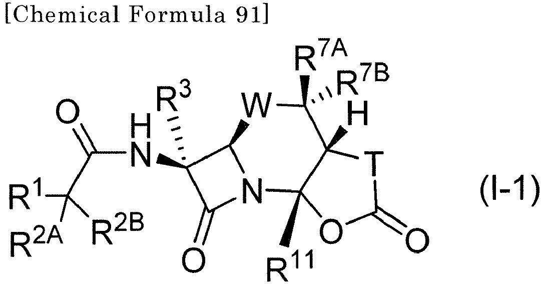 wherein W is a group of the following formula: 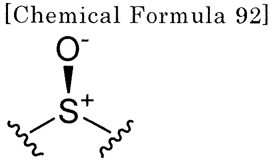 or -S(=O)₂-;
-T- is -CR^{4A}R^{4B}-;
R^{4A} and R^{4B} are each independently a hydrogen atom or substituted or unsubstituted alkyl;
R¹ is substituted or unsubstituted aromatic heterocyclyl;
R^{2A} and R^{2B} are taken together to be methylidene having a substituent of the formulas of: 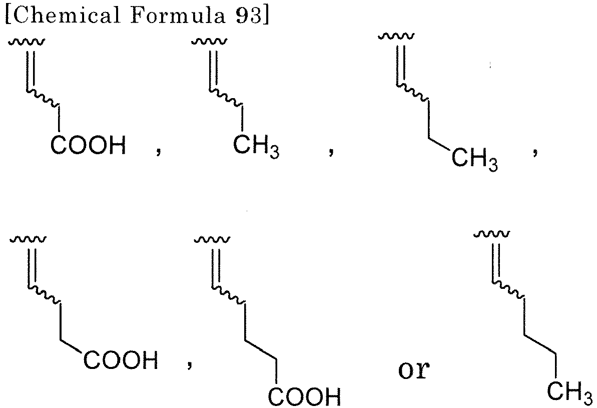 wherein the wavy line between the carbon atom to form a double bond and the carboxy group or the alkyl group means cis, trans or a mixture thereof,
or substituted or unsubstituted hydroxyimino of the formula of: 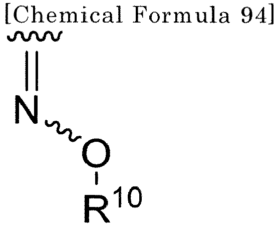 wherein R¹⁰ is a hydrogen atom or substituted or unsubstituted alkyl, and the wavy line between the nitrogen atom of the imino group and the oxygen atom represents cis, trans, or a mixture thereof;
R¹¹ is carboxy or tetrazolyl;
R^{7A} is a hydrogen atom or substituted or unsubstituted alkyl; R^{7B} is a hydrogen atom; and R³ is a hydrogen atom.

### (Embodiment 4)

Formula (I) is the following formula (I-1): 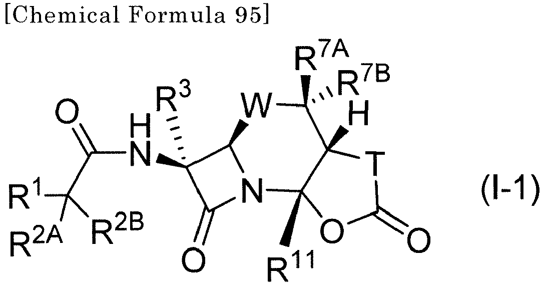 wherein W is a group of the following formula: 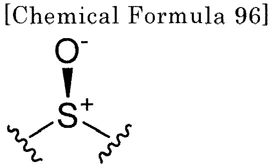 or -S(=O)₂-;
-T- is -CR^{4A}R^{4B}-;
R^{4A} and R^{4B} are each independently a hydrogen atom or substituted or unsubstituted alkyl;
R¹ is a group represented by the following formula: 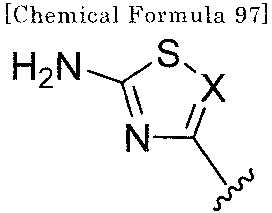 wherein X is CH, CCl, CF, or N;
R^{2A} and R^{2B} are taken together to be methylidene having a substituent of the formulas of: 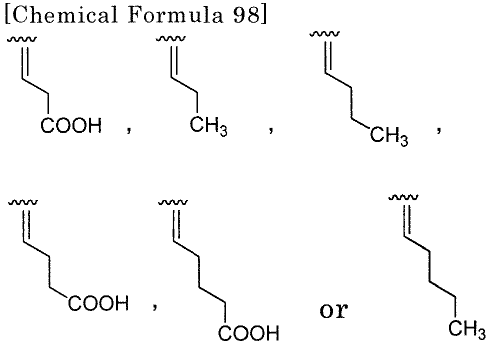 or substituted or unsubstituted hydroxyimino of the formula of: 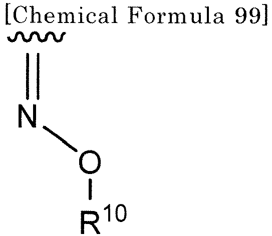 wherein R¹⁰ is a hydrogen atom or substituted or unsubstituted alkyl, and the wavy line between the nitrogen atom of the imino group and the oxygen atom represents cis, trans, or a mixture thereof;
R¹¹ is carboxy or tetrazolyl;
R^{7A} is a hydrogen atom or substituted or unsubstituted alkyl; R^{7B} is a hydrogen atom; and R3 is a hydrogen atom.

### (Embodiment 5)

Formula (I) is the following formula (I-A): 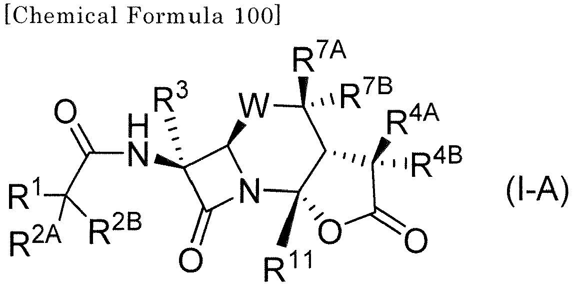 wherein W is a group of the following formula: 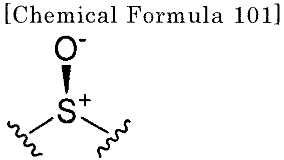 or -S(=O)₂-;
R^{4A} is a hydrogen atom; R^{4B} is a hydrogen atom or methyl;
R¹ is a group represented by the following formula: 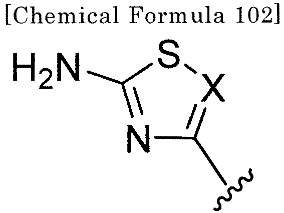 wherein X is CH, CCl, CF, or N;
R^{7A} is a hydrogen atom or alkyl; R^{7B} is a hydrogen atom;
R^{2A} and R^{2B} are taken together to be N-hydroxyimino or a group of the following formula: 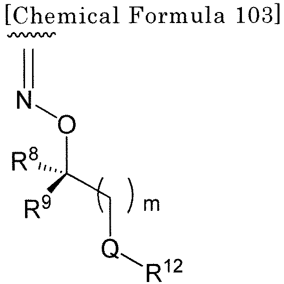 wherein R⁸ and R⁹ are each independently a hydrogen atom, halogen, hydroxy, carboxy, substituted or unsubstituted alkyl, substituted or unsubstituted carbocyclyl, or substituted or unsubstituted heterocyclyl, R⁸ and R⁹ are taken together to form substituted or unsubstituted methylidene, or R⁸ and R⁹ are taken together with the adjacent atoms to form substituted or unsubstituted non-aromatic carbocycle or substituted or unsubstituted non-aromatic heterocycle; Q is a single bond, substituted or unsubstituted carbocyclediyl, or substituted or unsubstituted heterocyclediyl; m is an integer from 0 to 3; and R¹² is a hydrogen atom, haloalkyl, or a group selected from the substituent group D;
R³ is a hydrogen atom; and
R¹¹ is carboxy.

### (Embodiment 6)

Formula (I) is the following formula: 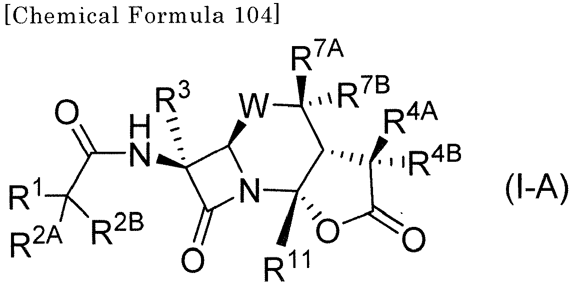 wherein -W- is represented by the following formula: 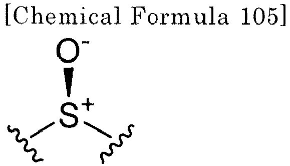 R^{4A} is a hydrogen atom; R^{4B} is a hydrogen atom or methyl;
R¹ is represented by the following formula: 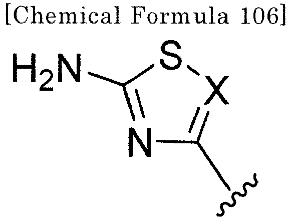 wherein X is CH;
R^{7A} is a hydrogen atom or alkyl; R^{7B} is a hydrogen atom;
R^{2A} and R^{2B} are taken together to be N-hydroxyimino or a group of the following formula: 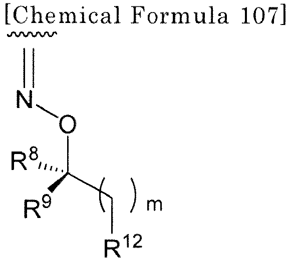 wherein R⁸ and R⁹ are each independently a hydrogen atom, halogen, hydroxy, carboxy, alkyl, or hydroxyalkyl, or R⁸ and R⁹ are taken together with the adjacent carbon atom to form substituted or unsubstituted non-aromatic carbocycle or substituted or unsubstituted non-aromatic heterocycle; m is an integer from 0 to 3; and R¹² is a hydrogen atom, carboxy, sulfo, carbamoyl, hydroxycarbamoyl, alkyloxycarbamoyl, or hydroxy;
R³ is a hydrogen atom; and
R¹¹ is carboxy.

### (Embodiment 7)

Formula (I) is the following formula: 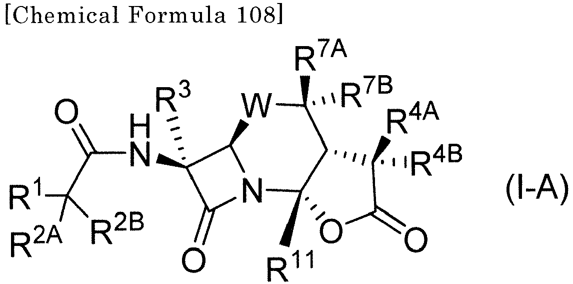 wherein -W- is represented by the following formula: 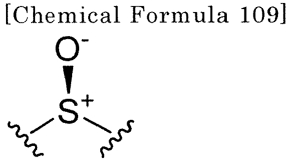 R^{4A} is a hydrogen atom; R^{4B} is a hydrogen atom or methyl;
R¹ is represented by the following formula: 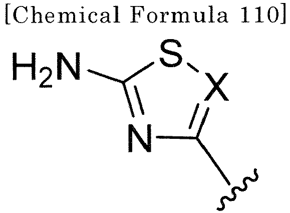 wherein X is CH;
R^{7A} is a hydrogen atom or alkyl; R^{7B} is a hydrogen atom;
R^{2A} and R^{2B} are taken together to be N-hydroxyimino or a group of the following formula: 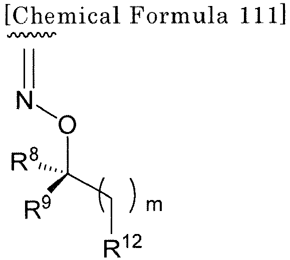 wherein R⁸ and R⁹ are each independently a hydrogen atom, halogen, hydroxy, carboxy, alkyl, or hydroxyalkyl, or R⁸ and R⁹ are taken together with the adjacent carbon atom to form substituted or unsubstituted non-aromatic carbocycle or substituted or unsubstituted non-aromatic heterocycle; m is an integer from 0 to 3; and R¹² is a hydrogen atom, halogen, hydroxy, carboxy, cyano, amino, sulfo, carbamoyl, sulfamoyl, hydroxyiminomethyl, trifluoromethyl, alkyloxycarbonylamino, sulfamoylamino, acylamino, hydroxycarbamoyl, or alkyloxycarbamoyl;
R³ is a hydrogen atom; and
R¹¹ is carboxy.

The compounds (I) of the present invention are not limited to specific isomers but include all possible isomers (e.g., keto-enol isomers, imine-enamine isomers, diastereoisomers, optical isomers, rotamers, geometric isomers or the like), racemates or mixtures thereof.

For example, 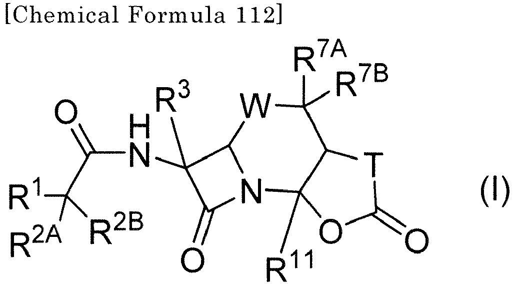 in formula (I) includes 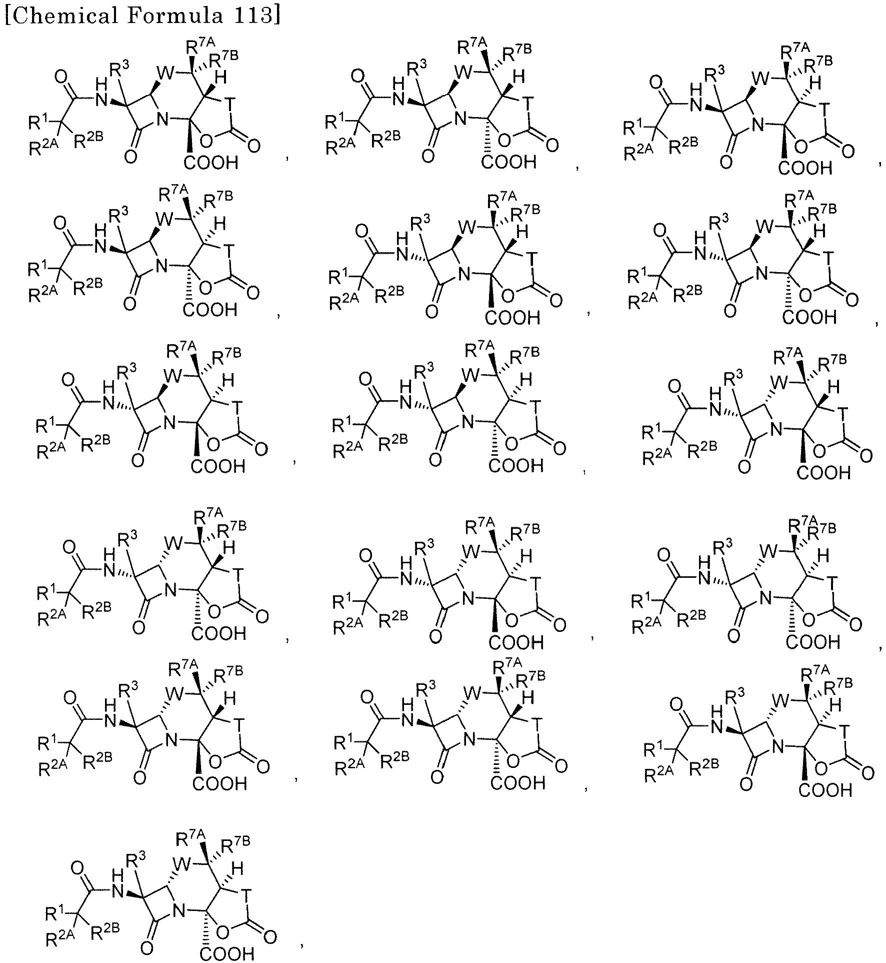 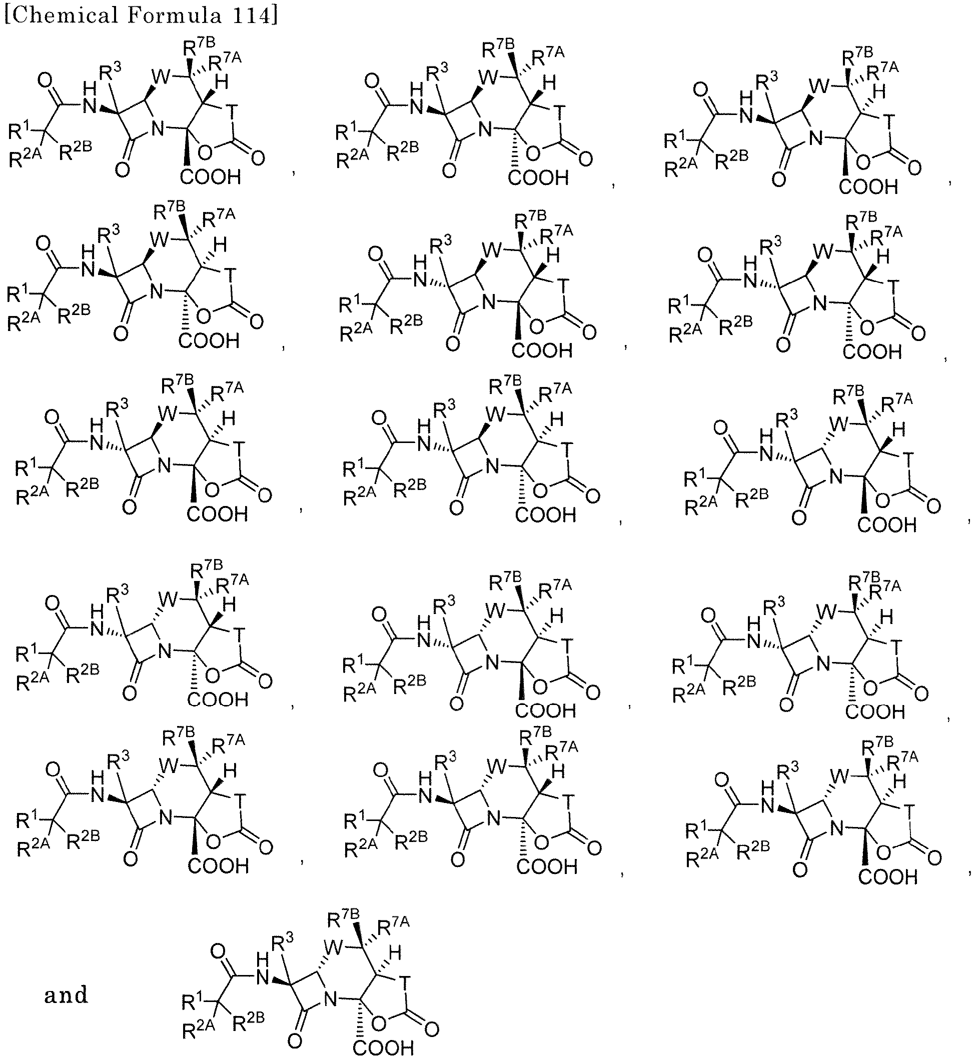 and the like.
Formula (I-1): 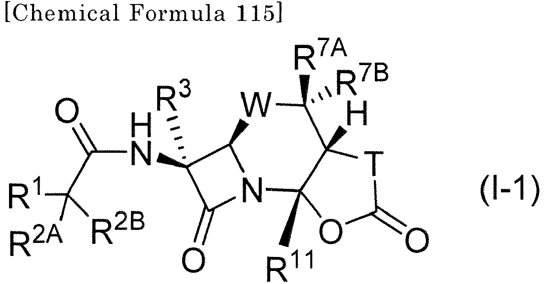 wherein each symbol is the same as above
is preferable.

The expression of the following formulas means the same configuration as that of the above formula (I-1):

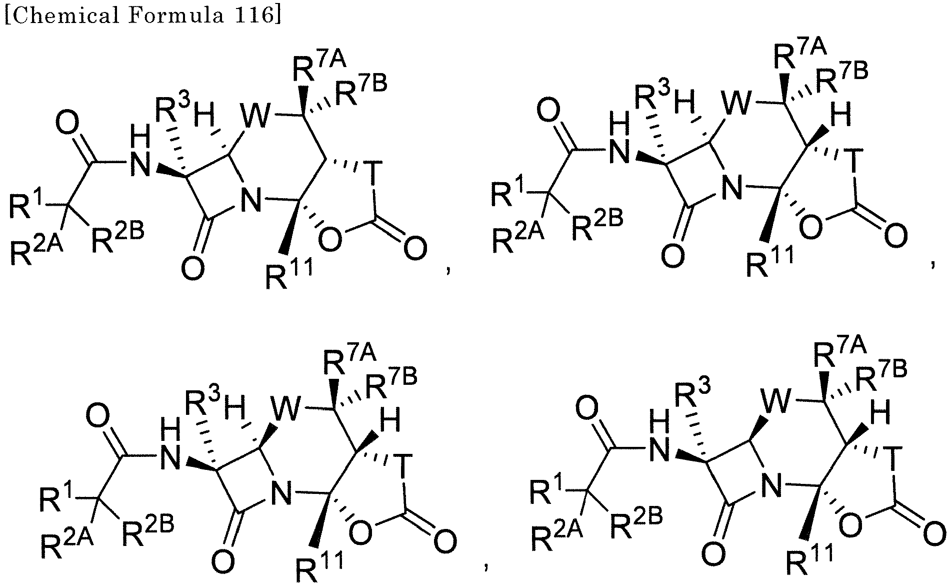

A position of substitution on the skeleton of formula (I) is designated as follows: an α-side chain and a β-side chain in this description represent groups bonded to the α-position and the β-position of the following core:

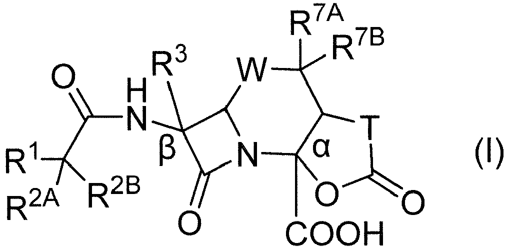

The ester forms of the compounds of formula (I) or (I') preferably include ester forms of carboxy at the α-position and/or carboxy on the β-side chain. The ester forms of carboxy on the β-side chain can include forms represented by the formula of: 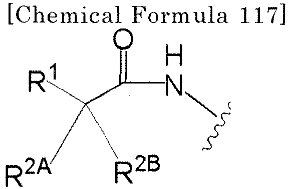 wherein each symbol is the same as above, and
wherein substituted or unsubstituted amino, substituted or unsubstituted sulfamoyl, carboxy, substituted or unsubstituted lower alkyloxycarbonyl, substituted or unsubstituted carbamoyl, or carboxy in carbonyloxy having a substituent, etc. has an ester structure at the end of R¹, or R^{2A} or R^{2B} (e.g., in the case of carboxy (-COOH), the ester forms are represented by the structure of -COOR^{P1} together with R^{P1} which represents an ester residue of a carboxy-protecting group or the like) and the like, and include ester that is easily converted to carboxy through metabolism *in vivo.*

Protecting groups for the above carboxy and the like can be groups that permit protection and/or deprotection by the method such as those described in Protective Groups in Organic Synthesis, T.W. Greene, John Wiley & Sons Inc. (1991), etc., and include, for example, lower alkyl (e.g., methyl, ethyl, t-butyl), lower alkylcarbonyloxymethyl (e.g., pivaloyl), optionally substituted arylalkyl (e.g., benzyl, benzhydryl, phenethyl, p-methoxybenzyl, p-nitrobenzyl), silyl groups (e.g., t-butyldimethylsilyl, diphenyl-t-butylsilyl) and the like.

One or more hydrogen, carbon and/or other atoms in the compounds represented by formula (I) or formula (I') may be replaced with isotopes of hydrogen, carbon and/or other atoms respectively. Example s of isotopes include hydrogen, carbon, nitrogen, oxygen, phosphorus, sulfur, fluorine, iodine and chlorine, such as ²H, ³H, ¹¹C, ¹³C, ¹⁴C, ¹⁵N, ¹⁸O, ¹⁷O, ³¹P, ³²P, ³⁵S, ¹⁸F, ¹²³I and ³⁶Cl respectively. The compounds represented by formula (I) or formula (I') include the compounds replaced with these isotopes. The compounds replaced with the above isotopes are useful as medicines and include all of radiolabeled compounds of the compound of formula (I) or formula (I'). A "method of radiolabeling" in the manufacture of the "radiolabeled compounds" is encompassed by the present invention, and is useful for studies on metabolized drug pharmacokinetics, studies on binding assay and/or diagnostic tools.

A radiolabeled compound of the compounds represented by formula (I) or formula (I') can be prepared using well-known methods in the art. For example, a tritium-labeled compound represented by formula (I) or formula (I') can be prepared by introducing a tritium to a certain compound represented by formula (I) or formula (I'), through a catalytic dehalogenation reaction using a tritium. This method comprises reacting with an appropriately-halogenated precursor of the compound represented by formula (I) or formula (I') with tritium gas in the presence of an appropriate catalyst, such as Pd/C, and in the presence or absent of a base. The other appropriate method of preparing a tritium-labeled compound can be referred to "Isotopes in the Physical and Biomedical Sciences, Vol. 1, Labeled Compounds (Part A), Chapter 6 (1987)". A ¹⁴C-labeled compound can be prepared by using a raw material having ¹⁴C.

The salts of the compounds represented by formula (I) or (I') include salts of a carboxy group at the α-position and/or a carboxy group at the β-position and/or a β-side chain amino group formed with inorganic acids or organic acids.

The pharmaceutically acceptable salts of the compounds represented by formula (I) or formula (I') include, for example, salts with alkaline metal (e.g., lithium, sodium, potassium or the like), alkaline earth metal (e.g., calcium, barium or the like), magnesium, transition metal (e.g., zinc, iron or the like), ammonia, organic bases (e.g., trimethylamine, triethylamine, dicyclohexylamine, ethanolamine, diethanolamine, triethanolamine, meglumine, ethylenediamine, pyridine, picoline, quinoline or the like) or amino acids, or salts with inorganic acids (e.g., hydrochloric acid, sulfuric acid, nitric acid, carbonic acid, hydrobromic acid, phosphoric acid, hydroiodic acid or the like) or organic acids (e.g., formic acid, acetic acid, propionic acid, trifluoroacetic acid, citric acid, lactic acid, tartaric acid, oxalic acid, maleic acid, fumaric acid, mandelic acid, glutaric acid, malic acid, benzoic acid, phthalic acid, ascorbic acid, benzenesulfonic acid, p-toluenesulfonic acid, methanesulfonic acid, ethanesulfonic acid or the like). Especially, salts with hydrochloric acid, sulfuric acid, phosphoric acid, tartaric acid, methanesulfonic acid and the like are included. These salts can be formed by the usual methods.

The compounds represented by formula (I) or formula (I') or pharmaceutically acceptable salts thereof may form solvates (e.g., hydrates or the like) and/or crystal polymorphs. The present invention encompasses those various solvates and crystal polymorphs. "Solvates" may be those wherein any numbers of solvent molecules (e.g., water molecules or the like) are coordinated with the compounds represented by formula (I) or formula (I'). When the compounds represented by formula (I) or pharmaceutically acceptable salts thereof are allowed to stand in the atmosphere, the compounds may absorb water, resulting in attachment of adsorbed water or formation of hydrates. Recrystallization of the compounds represented by formula (I) or formula (I') or pharmaceutically acceptable salts thereof may produce crystal polymorphs.

The compounds represented by formula (I) or formula (I') or pharmaceutically acceptable salts thereof may form prodrugs. The present invention also encompasses such various prodrugs. Prodrugs are derivatives of the compounds of the present invention that have chemically or metabolically degradable groups, and compounds that are converted to the pharmaceutically active compounds of the present invention through solvolysis or under physiological conditions and in vivo. Prodrugs include compounds that are converted to the compounds represented by formula (I) or formula (I') through enzymatic oxidation, reduction, hydrolysis or the like under physiological conditions and in vivo, compounds that are converted to the compounds represented by formula (I) or formula (I') through hydrolysis by gastric acid etc., and the like. Methods for selecting and preparing suitable prodrug derivatives are described in, for example, "Design of Prodrugs, Elsevier, Amsrdam, 1985". Prodrugs themselves may have some activity.

When the compounds represented by formula (I) or formula (I') or pharmaceutically acceptable salts thereof have hydroxy group(s), prodrugs include acyloxy derivatives and sulfonyloxy derivatives that are prepared by, for example, reacting compounds having hydroxy group(s) with suitable acyl halide, suitable acid anhydride, suitable sulfonyl chloride, suitable sulfonyl anhydride and mixed anhydride, or with a condensing agent. For example, they include CH₃COO-, C₂H₅COO-, t-BuCOO-, C₁₅H₃₁COO-, PhCOO-, (m-NaOOCPh)COO-, NaOOCCH₂CH₂COO-, CH₃CH(NH₂)COO-, CH₂N(CH₃)₂COO-, CH₃SO₃-, CH₃CH₂SO₃-, CF₃SO₃-, CH₂FSO₃-, CF₃CH₂SO₃-, p-CH₃O-PhSO₃-, PhSO₃- and p-CH₃PhSO₃.

As described below in the general procedures and Examples, the compounds of the present invention represented by formula (I) or (I') are synthesized by respectively bonding side chain sites to the α-positions and the β-positions of the skeletons of intermediates given below. The above protecting group P includes protecting groups described in general synthesis described below, and preferred examples include a benzhydryl group, a p-methoxybenzyl group, a trityl group, a 2,6-dimethoxybenzyl group, a methoxymethyl group, a benzyloxymethyl group, a 2-(trimethylsilyl)ethoxymethyl group and the like. As leaving groups, halogen (Cl, Br, I, and F), methanesulfonyloxy, p-toluenesulfonyloxy, trifluoromethanesulfonyloxy and the like are exemplified.

### (Production method A)

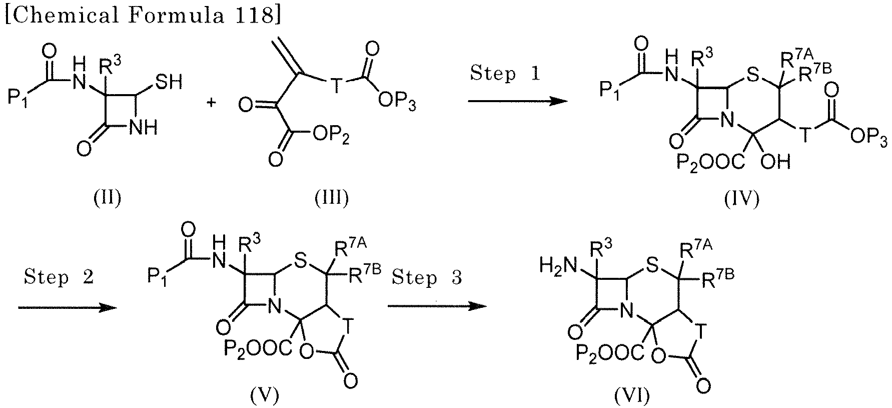

wherein W, T, R³, R^{7A} and R^{7B} are the same as above, -W¹ is -H, -SH or -CH₂-SH, P¹ represents a protecting group of an amino group, and P² and P³ each independently represent a protecting group of carboxy.

### Step 1

A compound (IV) is synthesized by subjecting a compound (II) and a compound (III) to addition reaction and intramolecular cyclization reaction. As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., acetonitrile, propionitrile), nitros (e.g., nitromethane, nitroethane, nitrobenzene), dimethyl sulfoxide, alcohols (e.g., methanol, ethanol, t-butanol etc.), water and the like are exemplified. These solvents may be used alone or may be used in combination of two or more.

Acetone and HMPA are preferable. The reaction temperature is usually approximately -100 to 100°C, preferably approximately -20 to 40°C, and more preferably approximately 10 to 30°C. The reaction time differs depending on the solvent or the reaction temperature and is usually 0.5 to 48 hours.

### Step 2

A compound (V) is synthesized by subjecting the protecting group P³ of the compound (IV) to deprotection reaction under acidic conditions, followed by intramolecular cyclization reaction in the presence of a condensing agent. As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., acetonitrile, propionitrile), nitros (e.g., nitromethane, nitroethane, nitrobenzene), dimethyl sulfoxide, alcohols (e.g., methanol, ethanol, t-butanol etc.), water and the like are exemplified. These solvents may be used alone or may be used in combination of two or more. Dichloromethane is preferable. As the acid used in the deprotection reaction, organic acids and inorganic acids are exemplified. For example, trifluoroacetic acid, tosylic acid, hydrochloric acid, sulfuric acid, phosphoric acid and the like are exemplified. Trifluoroacetic acid is preferable. As the condensing agent, 1-ethyl-3-(3-dimethylaminopropyl)carbodiimide hydrochloride, dicyclohexylcarbodiimide, carbonyldiimidazole and the like are exemplified. The reaction temperature is usually approximately -100 to 100°C, preferably approximately -20 to 40°C, and more preferably approximately 0 to 20°C. The reaction time differs depending on the solvent or the reaction temperature and is usually 0.5 to 48 hours.

### Step 3

A compound (VI) is synthesized by subjecting the carboxy-protecting group P¹ containing the acyl group of the compound (V) to alcoholysis reaction in the presence of a base or deprotection reaction under acidic conditions. As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., acetonitrile, propionitrile), nitros (e.g., nitromethane, nitroethane, nitrobenzene), dimethyl sulfoxide, alcohols (e.g., methanol, ethanol, t-butanol etc.), water and the like are exemplified. These solvents may be used alone or may be used in combination of two or more. Dichloromethane is preferable. The alcoholysis reaction can be activated with phosphorus pentachloride, phosphorus pentabromide, phosphorus oxychloride, thionyl chloride or the like. Phosphorus pentachloride is preferable. As the base, organic bases and the like are exemplified. For example, triethylamine, pyridine, diisopropylethylamine, N-methylimidazole, N-methylmorpholine, dimethylaniline and the like are exemplified. Pyridine is preferable. Then, an alcohol is added. As the alcohol, methanol, ethanol, propanol or the like can be used. Ethanol is preferable. As the acid used in the deprotection reaction under acidic conditions, organic acids and inorganic acids are exemplified. For example, trifluoroacetic acid, tosylic acid, hydrochloric acid, sulfuric acid, phosphoric acid and the like are exemplified. The reaction temperature is usually approximately -100 to 100°C, preferably approximately -70 to 20°C, and more preferably approximately -70 to -30°C, for the alcoholysis reaction. The reaction temperature is usually approximately -100 to 100°C, preferably approximately -20 to 40°C, and more preferably approximately -20 to 20°C, for the deprotection reaction under acidic conditions. The reaction time differs depending on the solvent or the reaction temperature and is usually 0.5 to 48 hours. 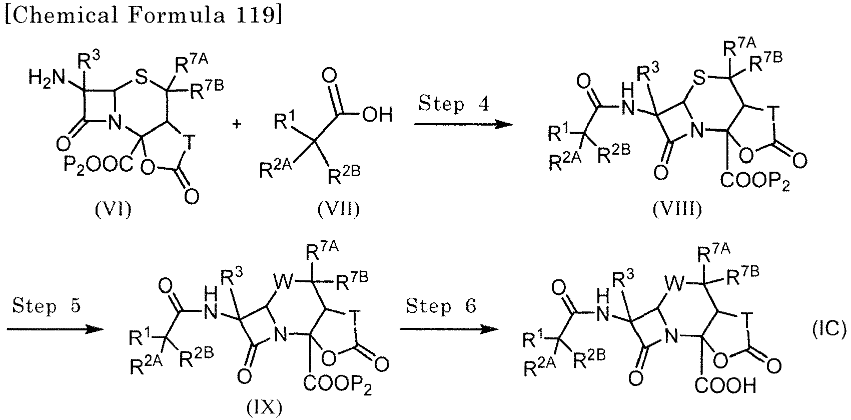 wherein each symbol is the same as above.

### Step 4

A compound (VIII) is synthesized by subjecting the compound (VI) to condensation reaction with a compound (VII) in the presence of a base. As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., MeCN, propionitrile), nitros (e.g., nitromethane, nitroethane, nitrobenzene), dimethyl sulfoxide, alcohols (e.g., methanol, ethanol, t-butanol etc.), water and the like are exemplified. These solvents may be used alone or may be used in combination of two or more. As the condensing agent, 1-ethyl-3-(3-dimethylaminopropyl)carbodiimide hydrochloride, phosphorus oxychloride, methanesulfonyl chloride, dicyclohexylcarbodiimide, carbonyldiimidazole, phenylphosphoric acid dichloride and the like are exemplified. As the base, triethylamine, pyridine, diisopropylethylamine, N-methylimidazole, N-methylmorpholine and the like are exemplified. The reaction temperature is usually approximately -100 to 100°C, preferably approximately -80 to 20°C, and more preferably approximately -20 to 20°C. The reaction time differs depending on the reagent or the solvent used or the reaction temperature and is usually 0.5 to 24 hours.

### Step 5

A compound (IX) is synthesized by oxidizing the compound (VIII). As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., MeCN, propionitrile), nitros (e.g., nitromethane, nitroethane, nitrobenzene), dimethyl sulfoxide, alcohols (e.g., methanol, ethanol, t-butanol etc.), water and the like are exemplified. These solvents may be used alone or may be used in combination of two or more. As the oxidizing agent, peracetic acid, m-chloroperbenzoic acid, hydrogen peroxide, sodium tungstate and the like are exemplified. The reaction temperature is usually approximately -100 to 100°C, preferably approximately -80 to 20°C, and more preferably approximately -20 to 20°C. The reaction time differs depending on the reagent or the solvent used or the reaction temperature and is usually 0.5 to 24 hours.

### Step 6

A compound (IC) is synthesized by subjecting all the protecting groups in the compound (IX) to deprotection reaction under acidic conditions. As the acid, an organic acid or an inorganic acid can be used. For example, trifluoroacetic acid, tosylic acid, hydrochloric acid, sulfuric acid, phosphoric acid, formic acid, aluminum chloride, titanium chloride and the like are exemplified. As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., MeCN, propionitrile), nitros (e.g., nitromethane, nitroethane, nitrobenzene), dimethyl sulfoxide, alcohols (e.g., methanol, ethanol, t-butanol etc.), water and the like are exemplified. These solvents may be used alone or may be used in combination of two or more. Dichloromethane is preferable. The reaction temperature is usually approximately - 100 to 100°C, preferably approximately -80 to 20°C, and more preferably approximately -20 to 20°C. The reaction time differs depending on the reagent or the solvent used or the reaction temperature and is usually 0.5 to 24 hours.

The obtained compound (IC) may be further chemically modified to synthesize its ester form, a pharmaceutically acceptable salt thereof, or a solvate thereof.

### (Production method B)

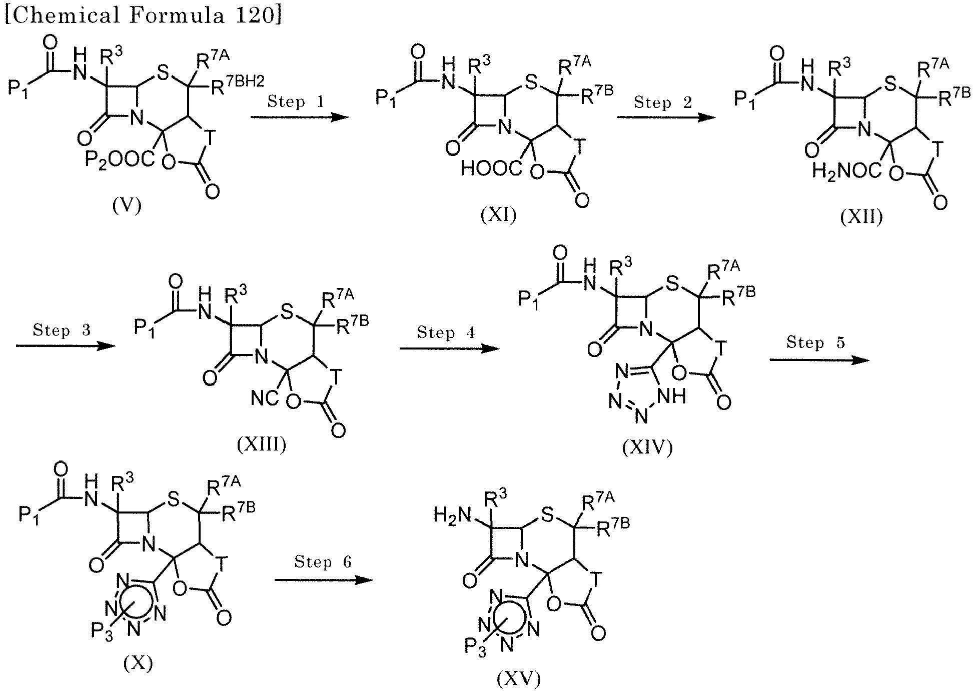

wherein P₃ represents a protecting group of tetrazolyl, and the other symbols are the same as above. As the protecting group of tetrazolyl, for example, a protecting group similar to the protecting group of a carboxy group is used.

### Step 1

A compound (XI) is synthesized by deprotecting the compound (V). The protecting group P2 of the compound (V) can be usually deprotected under acidic conditions or by catalytic hydrogenation, though the deprotection procedures differ depending on the protecting group. As the acid, an organic acid or an inorganic acid can be used. For example, trifluoroacetic acid, tosylic acid, hydrochloric acid, sulfuric acid, phosphoric acid, formic acid, aluminum chloride, titanium chloride and the like are exemplified. Aluminum chloride is preferable. As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., MeCN, propionitrile), nitros (e.g., nitromethane, nitroethane, nitrobenzene), dimethyl sulfoxide, alcohols (e.g., methanol, ethanol, t-butanol etc.), water and the like are exemplified. These solvents may be used alone or may be used in combination of two or more. Dichloromethane is preferable. The reaction temperature is usually approximately - 100 to 100°C, preferably approximately -80 to 20°C, and more preferably approximately -20 to 20°C. The reaction time differs depending on the reagent or the solvent used or the reaction temperature and is usually 0.5 to 24 hours.

### Step 2

A compound (XII) is synthesized by converting the carboxylic acid of the compound (XI) to an amide group. As the activation reagent for the carboxylic acid, thionyl chloride, oxalyl chloride, ethyl chlorocarbonate, di-tert-butyl dicarbonate, carbonyldiimidazole, dicyclohexylcarbodiimide and the like are exemplified. Di-tert-butyl dicarbonate is preferable. As the amidating agent, ammonia, ammonium chloride, ammonium formate, ammonium carbonate and the like are exemplified. Ammonium carbonate is preferable. As the base, pyridine, dimethylaminopyridine, picoline, triethylamine, diisopropylethylamine and the like are exemplified. Pyridine is preferable. As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., acetonitrile, propionitrile), nitros (e.g., nitromethane, nitroethane, nitrobenzene), dimethyl sulfoxide, alcohols (e.g., methanol, ethanol, t-butanol etc.), water and the like are exemplified. These solvents may be used alone or may be used in combination of two or more. Dioxane is preferable. The reaction temperature is usually approximately -100 to 100°C, preferably approximately -80 to 20°C, and more preferably approximately -20 to 20°C. The reaction time differs depending on the reagent or the solvent used or the reaction temperature and is usually 0.5 to 24 hours.

### Step 3

A compound (XIII) is synthesized by converting the amide group of the compound (XII) to a cyano group using a dehydration reagent in the presence of a base. As the dehydration reagent, thionyl chloride, oxalyl chloride, trifluoroacetic anhydride, acetic anhydride, phosphorus pentachloride, diphosphorus pentoxide and the like are exemplified. Trifluoroacetic anhydride is preferable. The reaction may be performed in the presence of a base. As the base, pyridine, picoline, triethylamine, lutidine, diisopropylethylamine and the like are exemplified. Pyridine is preferable. As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., acetonitrile, propionitrile), nitros (e.g., nitromethane, nitroethane, nitrobenzene), dimethyl sulfoxide, alcohols (e.g., methanol, ethanol, t-butanol etc.), water and the like are exemplified. These solvents may be used alone or may be used in combination of two or more. Tetrahydrofuran is preferable. The reaction temperature is usually approximately -100 to 100°C, preferably approximately -80 to 20°C, and more preferably approximately -20 to 20°C. The reaction time differs depending on the reagent or the solvent used or the reaction temperature and is usually 0.5 to 24 hours.

### Step 4

Subsequently, a compound (XIV) is synthesized by converting the cyano group of the compound (XIII) to a tetrazolyl group. In the case of converting the cyano group to a tetrazolyl group, the conversion can be performed using trimethylsilylazide, sodium azide, hydrazoic acid, or diphenylphosphoric azide. Trimethylsilylazide is preferable. A tin reagent or the like may be added as a reaction catalyst. As the tin reagent, dibutyltin oxide and the like are exemplified. As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., acetonitrile, propionitrile), nitros (e.g., nitromethane, nitroethane, nitrobenzene), dimethyl sulfoxide, alcohols (e.g., methanol, ethanol, t-butanol etc.), water and the like are exemplified. These solvents may be used alone or may be used in combination of two or more. Dioxane is preferable. The reaction temperature is usually approximately -50 to 150°C, preferably approximately 20 to 120°C, and more preferably approximately 60 to 100°C. The reaction time differs depending on the reagent or the solvent used or the reaction temperature and is usually 0.5 to 24 hours.

### Step 5

A compound (X) is synthesized by introducing a protecting group to the tetrazolyl group of the compound (XIV). As the protecting group, a p-methoxybenzyl group, a diphenylmethyl group, a trimethylsilylethyl group, a benzyloxymethyl group, a methoxymethyl group and the like are exemplified. These protecting groups can be introduced by reacting corresponding alkyl halides in the presence of a base or using diazo forms. As the base, pyridine, picoline, triethylamine, lutidine, diisopropylethylamine and the like are exemplified.

### Step 6

A compound (XV) is synthesized by subjecting the carboxy-protecting group P1 containing the acyl group of the compound (X) to alcoholysis reaction in the presence of a base or deprotection reaction under acidic conditions. As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., acetonitrile, propionitrile), nitros (e.g., nitromethane, nitroethane, nitrobenzene), dimethyl sulfoxide, alcohols (e.g., methanol, ethanol, t-butanol etc.), water and the like are exemplified. These solvents may be used alone or may be used in combination of two or more. Dichloromethane is preferable. The alcoholysis reaction can be activated with phosphorus pentachloride, phosphorus pentabromide, phosphorus oxychloride, thionyl chloride or the like. Phosphorus pentachloride is preferable. As the base, organic bases and the like are exemplified. For example, triethylamine, pyridine, diisopropylethylamine, N-methylimidazole, N-methylmorpholine, dimethylaniline and the like are exemplified. Pyridine is preferable. Then, an alcohol is added. As the alcohol, methanol, ethanol, propanol or the like can be used. Ethanol is preferable. As the acid used in the deprotection reaction under acidic conditions, organic acids and inorganic acids are exemplified. For example, trifluoroacetic acid, tosylic acid, hydrochloric acid, sulfuric acid, phosphoric acid and the like are exemplified. The reaction temperature is usually approximately -100 to 100°C, preferably approximately -70 to 20°C, and more preferably approximately -70 to -30°C, for the alcoholysis reaction. The reaction temperature is usually approximately -100 to 100°C, preferably approximately -20 to 40°C, and more preferably approximately -20 to 20°C, for the deprotection reaction under acidic conditions. The reaction time differs depending on the solvent or the reaction temperature and is usually 0.5 to 48 hours. 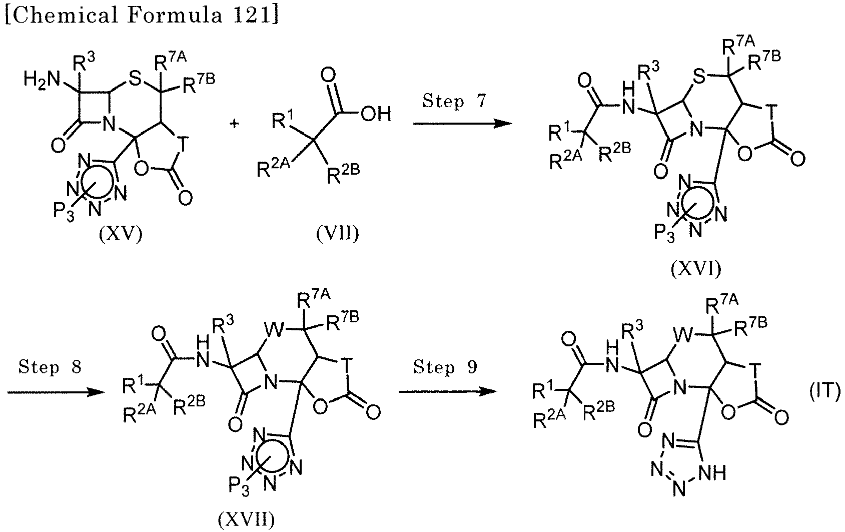 wherein each symbol is the same as above.

### Step 7

A compound (XVI) is synthesized by subjecting the compound (XV) to condensation reaction with a compound (VII) in the presence of a base. As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., MeCN, propionitrile), nitros (e.g., nitromethane, nitroethane, nitrobenzene), dimethyl sulfoxide, alcohols (e.g., methanol, ethanol, t-butanol etc.), water and the like are exemplified. These solvents may be used alone or may be used in combination of two or more. As the condensing agent, 1-ethyl-3-(3-dimethylaminopropyl)carbodiimide hydrochloride, phosphorus oxychloride, methanesulfonyl chloride, dicyclohexylcarbodiimide, carbonyldiimidazole, phenylphosphoric acid dichloride and the like are exemplified. As the base, triethylamine, pyridine, diisopropylethylamine, N-methylimidazole, N-methylmorpholine and the like are exemplified. The reaction temperature is usually approximately -100 to 100°C, preferably approximately -80 to 20°C, and more preferably approximately -20 to 20°C. The reaction time differs depending on the reagent or the solvent used or the reaction temperature and is usually 0.5 to 24 hours.

### Step 8

A compound (XVIII) is synthesized by oxidizing the compound (XVI). As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., MeCN, propionitrile), nitros (e.g., nitromethane, nitroethane, nitrobenzene), dimethyl sulfoxide, alcohols (e.g., methanol, ethanol, t-butanol etc.), water and the like are exemplified. These solvents may be used alone or may be used in combination of two or more. As the oxidizing agent, peracetic acid, m-chloroperbenzoic acid, hydrogen peroxide, sodium tungstate and the like are exemplified. The reaction temperature is usually approximately -100 to 100°C, preferably approximately -80 to 20°C, and more preferably approximately -20 to 20°C. The reaction time differs depending on the reagent or the solvent used or the reaction temperature and is usually 0.5 to 24 hours.

### Step 9

A compound (IT) is synthesized by subjecting all the protecting groups in the compound (XVIII) to deprotection reaction under acidic conditions. As the acid, an organic acid or an inorganic acid can be used. For example, trifluoroacetic acid, tosylic acid, hydrochloric acid, sulfuric acid, phosphoric acid, formic acid, aluminum chloride, titanium chloride and the like are exemplified. As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., MeCN, propionitrile), nitros (e.g., nitromethane, nitroethane, nitrobenzene), dimethyl sulfoxide, alcohols (e.g., methanol, ethanol, t-butanol etc.), water and the like are exemplified. These solvents may be used alone or may be used in combination of two or more. Dichloromethane is preferable. The reaction temperature is usually approximately - 100 to 100°C, preferably approximately -80 to 20°C, and more preferably approximately -20 to 20°C. The reaction time differs depending on the reagent or the solvent used or the reaction temperature and is usually 0.5 to 24 hours.

### (Production method C)

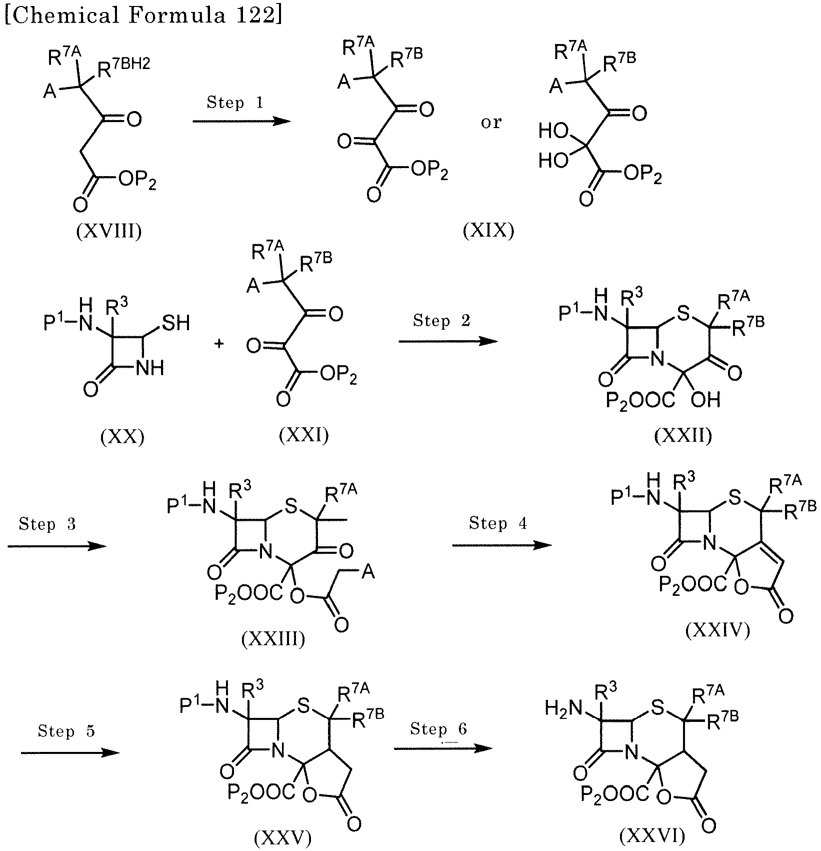

wherein each symbol is the same as above.

### Step 1

A compound (XIX) is synthesized by oxidizing the compound (XVIII). As the oxidizing agent, selenium dioxide, oxone and the like are exemplified. As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., acetonitrile, propionitrile), nitros (e.g., nitromethane, nitroethane, nitrobenzene), dimethyl sulfoxide, alcohols (e.g., methanol, ethanol, t-butanol etc.), water and the like are exemplified. These solvents may be used alone or may be used in combination of two or more. Dichloromethane is preferable. The reaction temperature is usually approximately -50 to 150°C, preferably approximately 20 to 120°C, and more preferably approximately 60 to 100°C. The reaction time differs depending on the reagent or the solvent used or the reaction temperature and is usually 0.5 to 24 hours.

### Step 2

A compound (XXI) is synthesized by the alkylation reaction and subsequent cyclization reaction of the compound (XIX) with a compound (XX). As the base, triethylamine, diisopropylethylamine, pyridine, morpholine, and lutidine are exemplified. Triethylamine is preferable. As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., acetonitrile, propionitrile), nitros (e.g., nitromethane, nitroethane, nitrobenzene), dimethyl sulfoxide, alcohols (e.g., methanol, ethanol, t-butanol etc.), water and the like are exemplified. These solvents may be used alone or may be used in combination of two or more. Acetone and HMPA are preferable. The reaction temperature is usually approximately -100 to 100°C, preferably approximately -20 to 40°C, and more preferably approximately 10 to 30°C. The reaction time differs depending on the solvent or the reaction temperature and is usually 0.5 to 48 hours.

### Step 3

A compound (XXII) is synthesized by the reaction of the compound (XXI) with α-haloacetic acid halide. As the base used, triethylamine, diisopropylethylamine, pyridine, morpholine, and lutidine are exemplified. Triethylamine is preferable. As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., acetonitrile, propionitrile), nitros (e.g., nitromethane, nitroethane, nitrobenzene), dimethyl sulfoxide, water and the like are exemplified. These solvents may be used alone or may be used in combination of two or more. Dichloromethane is preferable. The reaction temperature is usually approximately -100 to 100°C, preferably approximately -20 to 40°C, and more preferably approximately 0 to 20°C. The reaction time differs depending on the solvent or the reaction temperature and is usually 0.5 to 48 hours.

### Step 4

A compound (XXIII) is synthesized by converting the halide of the compound (XXII) to a phosphonium salt, followed by intramolecular cyclization in the presence of a base. For the phosphonium salt formation, triphenylphosphine, triethylphosphine, tributylphosphine or the like is used, and triphenylphosphine is preferable. As the base, sodium carbonate, sodium hydrogen carbonate, potassium carbonate, sodium hydroxide, potassium hydroxide, lithium hydroxide, triethylamine, diisopropylethylamine, sodium methoxide, sodium ethoxide, and potassium tert-butoxide are exemplified. Sodium hydrogen carbonate is preferable. As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., acetonitrile, propionitrile), nitros (e.g., nitromethane, nitroethane, nitrobenzene), dimethyl sulfoxide, alcohols (e.g., methanol, ethanol, t-butanol etc.), water and the like are exemplified. These solvents may be used alone or may be used in combination of two or more. Dimethylformamide is preferable. The reaction temperature is usually approximately -100 to 100°C, preferably approximately -20 to 40°C, and more preferably approximately 10 to 30°C. The reaction time differs depending on the solvent or the reaction temperature and is usually 0.5 to 48 hours.

### Step 5

A compound (XXIV) is synthesized by reducing the double bond of the compound (XXIII). The reduction is performed by catalytic hydrogenation or using a reducing agent. As the reducing agent, for example, sodium borohydride, lithium borohydride, boron hydride and the like are exemplified. As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., acetonitrile, propionitrile), nitros (e.g., nitromethane, nitroethane, nitrobenzene), dimethyl sulfoxide, alcohols (e.g., methanol, ethanol, t-butanol etc.), water and the like are exemplified. These solvents may be used alone or may be used in combination of two or more. Methanol, isopropanol and the like are preferable. The reaction temperature is usually approximately -100 to 50°C, preferably approximately -60 to 0°C, and more preferably approximately -50 to -20°C. The reaction time differs depending on the solvent or the reaction temperature and is usually 0.5 to 48 hours.

### Step 6

A compound (XXV) is synthesized by subjecting the carboxyamino-protecting group P1 containing the acyl group or the carbonyl group of the compound (XXIV) to alcoholysis reaction in the presence of a base or deprotection reaction under acidic conditions. As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., acetonitrile, propionitrile), nitros (e.g., nitromethane, nitroethane, nitrobenzene), dimethyl sulfoxide, alcohols (e.g., methanol, ethanol, t-butanol etc.), water and the like are exemplified. These solvents may be used alone or may be used in combination of two or more. Dichloromethane is preferable. The alcoholysis reaction can be activated with phosphorus pentachloride, phosphorus pentabromide, phosphorus oxychloride, thionyl chloride or the like. Phosphorus pentachloride is preferable. As the base, organic bases and the like are exemplified. For example, triethylamine, pyridine, diisopropylethylamine, N-methylimidazole, N-methylmorpholine, dimethylaniline and the like are exemplified. Pyridine is preferable. Then, an alcohol is added. As the alcohol, methanol, ethanol, propanol or the like can be used. Ethanol is preferable. As the acid used in the deprotection reaction under acidic conditions, organic acids and inorganic acids are exemplified. For example, trifluoroacetic acid, tosylic acid, hydrochloric acid, sulfuric acid, phosphoric acid and the like are exemplified. The reaction temperature is usually approximately -100 to 100°C, preferably approximately -70 to 20°C, and more preferably approximately -70 to -30°C, for the alcoholysis reaction. The reaction temperature is usually approximately -100 to 100°C, preferably approximately -20 to 40°C, and more preferably approximately -20 to 20°C, for the deprotection reaction under acidic conditions.

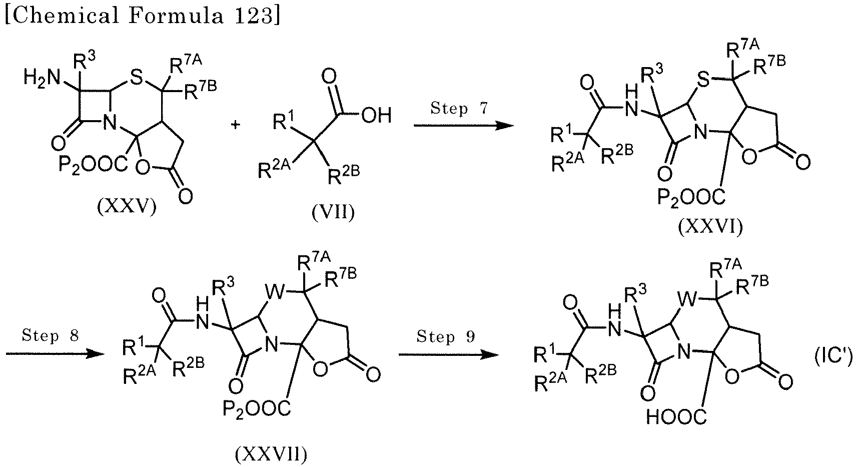

### Step 7

A compound (XXVI) is synthesized by subjecting the compound (XXV) to condensation reaction with a compound (VII) in the presence of a base. As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., MeCN, propionitrile), nitros (e.g., nitromethane, nitroethane, nitrobenzene), dimethyl sulfoxide, water and the like are exemplified. These solvents may be used alone or may be used in combination of two or more. As the condensing agent, 1-ethyl-3-(3-dimethylaminopropyl)carbodiimide hydrochloride, phosphorus oxychloride, methanesulfonyl chloride, dicyclohexylcarbodiimide, carbonyldiimidazole, phenylphosphoric acid dichloride and the like are exemplified. As the base, triethylamine, pyridine, diisopropylethylamine, N-methylimidazole, N-methylmorpholine, dimethylaniline and the like are exemplified. The reaction temperature is usually approximately -100 to 100°C, preferably approximately -80 to 20°C, and more preferably approximately -20 to 20°C. The reaction time differs depending on the reagent or the solvent used or the reaction temperature and is usually 0.5 to 24 hours.

### Step 8

A compound (XXVII) is synthesized by oxidizing the compound (XXVI). As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., MeCN, propionitrile), nitros (e.g., nitromethane, nitroethane, nitrobenzene), dimethyl sulfoxide, alcohols (e.g., methanol, ethanol, t-butanol etc.), water and the like are exemplified. These solvents may be used alone or may be used in combination of two or more. As the oxidizing agent, peracetic acid, m-chloroperbenzoic acid, hydrogen peroxide, sodium tungstate and the like are exemplified. The reaction temperature is usually approximately -100 to 100°C, preferably approximately -80 to 20°C, and more preferably approximately -20 to 20°C. The reaction time differs depending on the reagent or the solvent used or the reaction temperature and is usually 0.5 to 24 hours.

### Step 9

A compound (IC') is synthesized by subjecting all the protecting groups in the compound (XXVII) to deprotection reaction under acidic conditions. As the acid, an organic acid or an inorganic acid can be used. For example, trifluoroacetic acid, tosylic acid, hydrochloric acid, sulfuric acid, phosphoric acid, formic acid, aluminum chloride, titanium chloride and the like are exemplified. As the reaction solvent, for example, ethers (e.g., anisole, dioxane, tetrahydrofuran, diethyl ether, tert-butyl methyl ether, diisopropyl ether), esters (e.g., ethyl formate, ethyl acetate, n-butyl acetate), halogenated hydrocarbons (e.g., dichloromethane, chloroform, carbon tetrachloride), hydrocarbons (e.g., n-hexane, benzene, toluene), amides (e.g., formamide, N,N-dimethylformamide, N,N-dimethylacetamide, N-methylpyrrolidone), ketones (e.g., acetone, methyl ethyl ketone), nitriles (e.g., MeCN, propionitrile), nitros (e.g., nitromethane, nitroethane, nitrobenzene), dimethyl sulfoxide, alcohols (e.g., methanol, ethanol, t-butanol etc.), water and the like are exemplified. These solvents may be used alone or may be used in combination of two or more. Dichloromethane is preferable. The reaction temperature is usually approximately - 100 to 100°C, preferably approximately -80 to 20°C, and more preferably approximately -20 to 20°C. The reaction time differs depending on the reagent or the solvent used or the reaction temperature and is usually 0.5 to 24 hours.

The compounds of the present invention have a wide spectrum of antibacterial activity and can be used for preventing or treating various diseases, for example, airway infection, urinary tract infection, respiratory tract infection, sepsis, nephritis, cholecystitis, mouth infection, endocarditis, pneumonia, meningitis, otitis media, enterocolitis, purulent accumulation, wound infection, opportunistic infection and the like, caused by pathogenic bacteria in various mammals including humans.

The compounds of the present invention exhibit a high antibacterial activity, particularly, against gram-negative bacteria, preferably gram-negative bacteria of the family *Enterobacteriaceae* (*E. coli, Klebsiella, Serratia, Enterobacter, Citrobacter, Morganella, Providencia, Proteus* etc.), gram-negative bacteria that become colonized in the respiratory tract (*Hemophilus, Moraxella* etc.) and glucose non-fermentative gram-negative bacteria (*Pseudomonas* except for *Pseudomonas aeruginosa, Stenotrophomonas, Burkholderia, Acinetobacter* etc.). The compounds of the present invention are stable against β-lactamases belonging to classes A, B, C and D produced by these gram-negative bacteria and have a high antibacterial activity against various β-lactam agent-resistant gram-negative bacteria such as bacteria producing ESBLs typified by TEM type, SHV type, KPC type and the like. Particularly, the compounds of the present invention are very stable even against metallo-β-lactamases belonging to class B, including NDM type, IMP type, VIM type, L-1 type and the like and are therefore effective even for various gram-negative bacteria resistant to β-lactam agents including cephem and carbapenem. Furthermore, the preferred compounds also have a feature such as high concentrations in blood, a long duration of effects and/or remarkable tissue distribution, in terms of *in vivo* kinetics. Also, the preferred compounds are safe in terms of adverse reactions in such a way that they do not manifest fever and do not manifest kidney toxicity. Moreover, the preferred compounds have high water solubility and good *in vivo* kinetics and are suitable as injections and oral drugs.

The compounds of the present invention can be administered orally or parenterally. In the case of oral administration, the compounds of the present invention can be used in any forms of usual formulations, for example, solid formulations such as tablets, powders, granules, and capsules, and liquid formulations such as solutions, oil suspensions, syrups, and elixirs. In the case of parenteral administration, the compounds of the present invention can be used as aqueous or oil suspended injections or nasal drops. For preparation thereof, any of common excipients, binders, lubricants, aqueous solvents, oil solvents, emulsifiers, suspending agents, preservatives, stabilizers and the like can be used. The formulations of the present invention are manufactured by combining (e.g., mixing) therapeutically effective amounts of the compounds of the present invention with pharmaceutically acceptable carriers or diluents.

The compounds of the present invention can be administered parenterally or orally as injections, capsules, tablets, or granules and are preferably administered as injections. The dosage is usually approximately 0.1 to 100 mg/day and preferably approximately 0.5 to 50 mg/day, per kg body weight of a patient or an animal, which can be administered in two to four divided portions per day, if desired. The carriers for use in injections are, for example, distilled water, saline and the like. Also, a base or the like may be used for pH adjustment. The carriers for use in capsules, granules, or tablets are known excipients (e.g., starch, lactose, saccharide, calcium carbonate, calcium phosphate etc.), binders (e.g., starch, gum arabic, carboxymethylcellulose, hydroxypropylcellulose, crystalline cellulose etc.), lubricants (e.g., magnesium stearate, talc etc.) in the art and the like.

### [EXAMPLES]

The present invention will be described in more detail with reference to, but not limited to, the following Examples, Reference Examples, Test Examples and Formulation Examples.

In this description, meanings of each abbreviation are as follows:
Boc: tert-Butoxycarbonyl
BH or Bzh: Benzhydryl
DIAD: Diisopropyl azodicarboxylate
DMF: N,N-Dimethylformamide
DMA: N,N-Dimethylacetamide
EDC: 1-(3-Dimethylaminopropyl)-3-ethylcarbodiimide
HOBt: 1-Hydroxybenzotriazole
mCPBA: m-Chloroperbenzoic acid
Me: Methyl
ODS: Octadecylsilyl
t-Bu: tert-Butyl
TFA: Trifluoroacetic acid
TBAF: Tetrabutyl ammonium fluoride
PMB: p-Methoxybenzyl
Ph: Phenyl

NMR analysis of examples was performed by 400 MHz using DMSO-d₆, CDCl₃ or the like.

LCMS analysis of examples was performed under the conditions as described below.

### Measurement condition A:

Column: ACQUITY UPLC (registered trademark) BEH C18(1.7µm
i.d.2. 1x50mm)(Waters)
Flow rate: 0.8 mL/min
PDA detection wavelength: 254nm
Mobile phases: [A] is 0.1% formic acid solution, and [B] is 0.1% formic acid in acetonitrile solvent.
Gradient: linear gradient of 5% to 100% solvent [B] for 3.5 minutes was performed, and then 100% solvent [B] was maintained for 0.5 minute.

### Measurement condition B:

Column: Shim-pack XR-ODS (2.2µm, i.d.50x3.0mm) (Shimadzu)
Flow rate: 1.6 mL/min
PDA detection wavelength: 254nm
Mobile phases: [A] is 0.1% formic acid solution, and [B] is 0.1% formic acid in acetonitrile solvent.
Gradient: linear gradient of 10% to 100% solvent [B] for 3 minutes was performed, and 100% solvent [B] was maintained for 0.5 minute.

The X-ray powder diffractometry (XRPD) of crystalline solids obtained in examples was performed under the measurement condition 1 or 2 as described below according to the powder X-ray diffractometry described in General Tests of the Japanese Pharmacopoeia. In the case of measurement under the measurement condition 2, peaks appearing at 2-theta (2θ) values of about 38°C are the peaks of aluminum.

### (Measurement condition 1):

D-8 Discover manufactured by Bruker Corp.
Measurement method: reflection method
Type of light source: Cu tube
Wavelength used: CuKα ray
Tube current: 40 mA
Tube voltage: 40 kV
Sample plate: glass
Incident angle of X-ray: 3° and 12°

### (Measurement condition 2):

MiniFlex600 manufactured by Rigaku Corp.
Type of light source: Cu tube
Wavelength used: CuKα ray
Tube current: 10 mA
Tube voltage: 30 Kv
Sample plate: Al
Measurement range: 3° to 40°
Step width: 0.01 deg
Scan speed: 10 deg/min

### [EXAMPLE 1]

### Synthesis of compound I-001 and compound 1-002

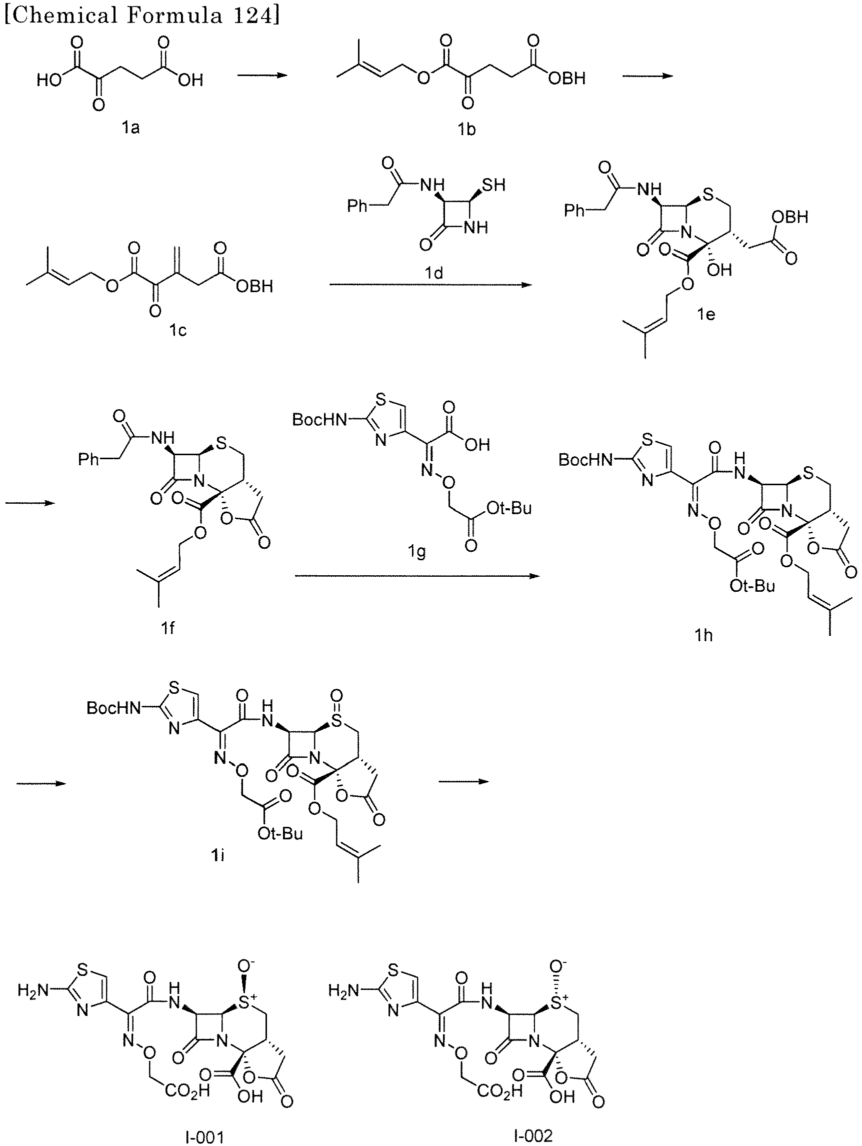

### Step 1 Synthesis of compound 1b

A solution of the compound 1a (43.8 g, 300 mmol) in DMF (307 mL) was warmed to 60°C. Dicyclohexylamine (59.6 mL, 300 mmol) and 1-bromo-3-methyl-2-butene (38.1 mL, 300 mmol) were added to the solution. The mixture was stirred at 60°C for 1 hour. Then, the precipitates were removed by filtration. Water was added to the filtrate, followed by extraction with ethyl acetate. The organic layer was washed by water and brine and then dried over anhydrous magnesium sulfate. The solvent was evaporated under reduced pressure. Under ice cooling, diphenyldiazomethane (64.1 g, 330 mmol) was added to a solution of the obtained residue in tetrahydrofuran (321 mL). The mixture was stirred at room temperature for 7 hours. The mixture was left standing at room temperature for 2 days. Then, the solvent was evaporated under reduced pressure. The obtained crude product was purified by silica gel column chromatography (hexane-ethyl acetate) to afford the compound 1b (104 g, yield 91%).
¹H-NMR (CDCl₃) δ: 1.73 (3H, s), 1.76 (3H, s), 2.79 (2H, t, J = 6.2 Hz), 3.18 (2H, t, J = 6.2 Hz), 4.74 (2H, d, J = 7.3 Hz), 5.38 (1H, t, J = 7.3 Hz), 6.86 (1H, s), 7.26-7.36 (10H, m).

### Step 2 Synthesis of compound 1c

To a solution of the compound 1b (104 g, 273 mmol) in dichloromethane (520 mL), N,N,N',N'-tetramethyldiaminomethane (149 mL, 1093 mmol) was added. Under ice cooling, acetic anhydride (129 mL, 1367 mL) and acetic acid (109 mL, 1914 mmol) were added to the mixture. The mixture was stirred at room temperature for 1 hour. Then, the solvent was evaporated under reduced pressure. Then, water was added to the residue, followed by extraction with ethyl acetate. The organic layer was washed by water and then dried over anhydrous magnesium sulfate. The solvent was evaporated under reduced pressure. The obtained residue was purified by column chromatography (hexane-ethyl acetate) to afford the compound 1c (79 g, 74%). ¹H-NMR (CDCl₃) δ: 1.74 (3H, s), 1.77 (3H, s), 3.48 (2H, s), 4.78 (2H, d, J = 7.4 Hz), 5.39 (1H, t, J = 7.4 Hz), 6.25 (1H, s), 6.36 (1H, s), 6.86 (1H, s), 7.26-7.35 (10H, m).

### Step 3 Synthesis of compound 1e

To a solution of the compound 1c (10.0 g, 25.5 mmol) in acetone (100 mL), the compound 1d (6.02 g, 25.5 mmol) and hexamethylphosphoric triamide (15.5 mL, 89 mmol) were added. The mixture was stirred at room temperature for 1 hour. Water was added to the mixture, followed by extraction with ethyl acetate. The organic layer was washed by water and brine and then dried over anhydrous magnesium sulfate. The solvent was evaporated under reduced pressure. The obtained residue was purified by column chromatography (hexane-ethyl acetate) to afford the compound 1e (2.1 g, yield 13.1%).
¹H-NMR (CDCl₃) δ: 1.65 (3H, s), 1.69 (3H, s), 2.34 (2H, t, J = 4.5 Hz), 2.69 (1H, dd, J = 13.6, 2.8 Hz), 2.81 (1H, br s), 2.91-2.98 (1H, m), 3.59 (1H, d, J = 16.1 Hz), 3.65 (1H, d, J = 16.1 Hz), 3.77 (1H, s), 4.67 (1H, dd, J = 12.0, 7.5 Hz), 4.88 (1H, dd, J = 12.0, 7.5 Hz), 5.08 (1H, d, J = 4.7 Hz), 5.33 (1H, t, J = 7.5 Hz), 5.51 (1H, dd, J = 9.5, 4.7 Hz), 6.09 (1H, d, J = 9.5 Hz), 6.86 (1H, s), 7.26-7.40 (17H, m).

### Step 4 Synthesis of compound 1f

Under nitrogen atmosphere, a solution of the compound 1e (6.80 g, 10.8 mmol) in dichloromethane (34 mL) was cooled to -10°C. A solution of TFA (34 mL, 441 mmol) in dichloromethane (34 mL) was added dropwise to the solution. The mixture was stirred at -10°C for 30 minutes. Water was added to the reaction mixture, followed by extraction with dichloromethane. The organic layer was washed by water and brine and then dried over anhydrous magnesium sulfate. The solvent was evaporated. A solution of the obtained residue in dichloromethane (50 mL) was cooled to 0°C. EDC hydrochloride (4.15 g, 21.6 mmol) was added to the solution. The mixture was stirred at room temperature for 1 hour. Water was added to the mixture, followed by extraction with dichloromethane. The organic layer was washed by dilute hydrochloric acid and brine and then dried over anhydrous magnesium sulfate. The solvent was evaporated under reduced pressure. The obtained residue was purified by column chromatography (hexane-ethyl acetate) to afford the compound 1f (4.0 g, 83%).
¹H-NMR (CDCl₃) δ: 1.71 (3H, s), 1.77 (3H, s), 2.60-2.67 (2H, m), 2.75 (1H, dd, J = 18.1, 9.0 Hz), 2.94 (1H, dd, J = 14.4, 4.3 Hz), 3.19-3.25 (1H, m), 4.73-4.83 (2H, m), 4.96-4.98 (1H, m), 5.36 (1H, t, J = 6.8 Hz), 5.53 (1H, dd, J = 8.7, 4.7 Hz), 6.16 (1H, d, J = 8.6 Hz), 7.26-7.39 (6H, m).

### Step 5 Synthesis of compound 1h

A suspension of phosphorus pentachloride (1.25 g, 6.00 mmol) in dichloromethane (13.3 mL) was cooled to -40°C. Then, pyridine (0.969 mL, 12.0 mmol) was added, and subsequently, the compound 1f (1.33 g, 3.00 mmol) was added to the suspension. Under ice cooling, the mixture was stirred for 1 hour. Then, the reaction mixture was cooled to -78°C. Ethanol (13.3 mL) was added to the reaction mixture. After stirring at -30°C for 30 minutes, an aqueous solution of sodium hydrogen carbonate was added to the reaction mixture, followed by extraction with dichloromethane. The organic layer was washed by water and brine and dried over magnesium sulfate. Inorganic matter was removed by filtration. Then, the filtrate was concentrated into approximately 10 ml under reduced pressure to afford a dichloromethane solution (solution A). A solution of the compound 1g (682 mg, 1.70 mmol) in DMA (4.9 mL) was cooled to -20°C. Then, triethylamine (0.291 mL, 2.10 mmol) and methanesulfonyl chloride (0.148 mL, 1.90 mmol) were added to the solution. The mixture was stirred at -20°C for 1 hour to afford solution B.

Dichloromethane (5 ml) was added to half the amount of the solution A (approximately 5 ml, corresponding to 1.50 mmol). Under ice cooling, pyridine (0.121 mL, 1.50 mmol) and the solution B were added to the mixture. Under ice cooling, the mixture was stirred for 1 hour. Then, an aqueous solution of dilute hydrochloric acid was added to the mixture, followed by extraction with ethyl acetate. The organic layer was washed by a saturated aqueous solution of sodium bicarbonate, water, and brine in this order and dried over anhydrous magnesium sulfate. Magnesium sulfate was filtered off. Then, the filtrate was concentrated under reduced pressure. The residue was subjected to silica gel column chromatography, followed by elution with hexane/ethyl acetate. Fractions containing the desired compound were concentrated under reduced pressure to afford the compound 1h (0.44 g, yield 41%).
¹H-NMR (CDCl₃) δ: 8.74 (1H, d, J = 8.5 Hz), 8.07 (1H, s), 7.39 (1H, s), 5.66 (1H, dd, J = 8.5, 4.6 Hz), 5.39 (1H, t, J = 7.3 Hz), 5.08 (1H, d, J = 4.6 Hz), 4.86-4.71 (4H, m), 3.33-3.27 (1H, m), 3.10 (1H, dd, J = 14.6, 4.1 Hz), 2.83-2.73 (2H, m), 2.64 (1H, dd, J = 14.6, 8.5 Hz), 1.76 (3H, s), 1.72 (3H, s), 1.54 (9H, s), 1.47 (9H, s).

### Step 6 Synthesis of compound 1i

The compound 1h (0.75 g, 1.06 mmol) was dissolved in acetonitrile (4 ml) and DMA (4 ml). The solution was cooled to -20°C. Then, 37% peracetic acid solution (0.20 ml, 1.11 mmol) was added to the solution. Under ice cooling, the mixture was stirred for 1 hour. Then, the reaction mixture was separated into aqueous and organic layers by the addition of 10% aqueous solution of sodium sulfite and ethyl acetate. The organic layer was washed by water, a saturated aqueous solution of sodium bicarbonate and brine and dried over magnesium sulfate. Magnesium sulfate was filtered off. Then, the filtrate was concentrated under reduced pressure. The residue was subjected to silica gel column chromatography, followed by elution with hexane/ethyl acetate. Fractions containing the desired compound were concentrated under reduced pressure to afford the compound 1i (0.64 g, yield 84%) as an isomeric mixture of sulfoxide.
¹H-NMR (CDCl₃) δ: 9.18 (0.2H, d, J = 7.0 Hz), 8.41 (1.0H, d, J = 9.3 Hz), 8.12 (1.0H, s), 7.34 (0.8H, s), 7.32 (0.2H, s), 5.97 (1.0H, dd, J = 9.2, 4.8 Hz), 5.39 (1.2H, t, J = 7.3 Hz), 5.23-5.20 (0.2H, m), 4.91-4.62 (6.0H, m), 3.81-3.72 (1.0H, m), 3.64 (0.2H, dd, J = 14.7, 3.6 Hz), 3.57-3.51 (0.2H, m), 3.42 (1.0H, dd, J = 14.5, 5.0 Hz), 3.20 (0.2H, dd, J = 14.7, 5.8 Hz), 2.96 (1.0H, dd, J = 17.8, 7.7 Hz), 2.86 (0.2H, dd, J = 18.5, 9.3 Hz), 2.47-2.38 (2.0H, m), 1.74 (7.2H, t, J = 10.0 Hz), 1.54 (10.8H, s), 1.48-1.45 (10.8H, m).

### Step 7 Synthesis of compound 1-001 and compound 1-002

The compound 1i (0.64 g, 0.882 mmol) was dissolved in dichloromethane (10 ml). The solution was cooled to -40°C. Then, anisole (1.16 ml, 10.6 mmol) and a 2 mol/L solution of aluminum chloride in nitromethane (5.29 ml, 10.6 mmol) were added in this order to the solution. The mixture was stirred at -30°C for 30 minutes. The reaction mixture was dissolved in water, 2 mol/L hydrochloric acid, and acetonitrile. Then, the solution was washed by diisopropyl ether. HP20-SS resin was added to the aqueous layer. Acetonitrile was evaporated under reduced pressure. The obtained mixed solution was subjected to HP20-SS column chromatography, followed by elution with water/acetonitrile. Fractions containing the desired compounds were concentrated under reduced pressure. Then, the residue was freeze-dried to afford the compound 1-001 and the compound 1-002 as white powders.
Yield amount: compound I-001: 272.8 mg (yield 57%)
compound I-002: 57.6 mg (yield 12%)
Compound 1-001
¹H-NMR (D₂O) δ: 7.22 (1H, s), 5.88 (1H, d, J = 4.8 Hz), 5.00 (1H, d, J = 4.8 Hz), 3.65-3.52 (2H, m), 3.06 (1H, dd, J = 18.3, 7.7 Hz), 2.82 (1H, dd, J = 14.6, 12.7 Hz), 2.55 (1H, d, J = 18.3 Hz).
Anal. : C16H15N5O10S2(H2O)2.3
Calc. : C, 35.40; H, 3.64; N, 12.90; S, 11.81 (%)
Found: C, 35.39; H, 3.57; N, 13.11; S, 11.79 (%)
Compound 1-002
¹H-NMR (DMSO-D₆) δ: 9.87 (1H, d, J = 7.9 Hz), 7.27 (2H, s), 6.85 (1H, s), 5.59 (1H, dd, J = 7.9, 4.6 Hz), 4.90 (1H, d, J = 4.6 Hz), 4.58 (2H, s), 3.02-2.87 (2H, m).
Anal. : C16H15N5O10S2(H2O)2.1
Calc. : C, 35.64; H, 3.59; N, 12.99; S, 11.89 (%)
Found: C, 35.72; H, 3.66; N, 13.18; S, 11.73 (%)

### [EXAMPLE 2]

### Synthesis of compound 1-003

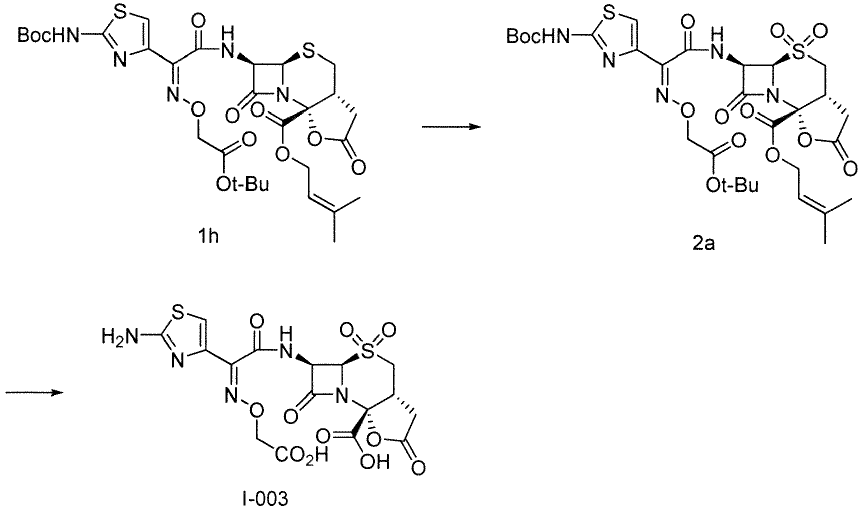

### Step 1 Synthesis of compound 2a

The compound 1h (0.75 g, 1.06 mmol) was dissolved in dichloromethane (10 ml). Under ice cooling, 70% mCPBA (0.52 g, 2.11 mmol) was added to the solution. Under ice cooling, the mixture was stirred for 1 hour. Then, the reaction mixture was separated into aqueous and organic layers by the addition of a saturated aqueous solution of sodium bicarbonate. The organic layer was washed by water, a saturated aqueous solution of sodium bicarbonate and brine and dried over magnesium sulfate. Magnesium sulfate was filtered off. Then, the filtrate was concentrated under reduced pressure. The residue was subjected to silica gel column chromatography, followed by elution with hexane/ethyl acetate. Fractions containing the desired compound were concentrated under reduced pressure to afford the compound 2a (0.09 g, yield 12%).
¹H-NMR (CDCl₃) δ: 8.70 (1H, d, J = 9.5 Hz), 7.36 (1H, s), 6.07 (1H, dd, J = 9.5, 5.0 Hz), 5.38 (1H, t, J = 7.5 Hz), 4.95 (1H, d, J = 5.0 Hz), 4.88 (1H, dd, J = 11.9, 7.7 Hz), 4.81 (1H, t, J = 6.0 Hz), 4.75 (1H, d, J = 16.8 Hz), 4.69 (1H, d, J = 16.8 Hz), 4.01-3.95 (1H, m), 3.32 (1H, dd, J = 15.1, 5.3 Hz), 3.00 (1H, dd, J = 15.1, 12.2 Hz), 2.89 (1H, dd, J = 18.0, 7.6 Hz), 2.43 (1H, d, J = 17.7 Hz), 1.78 (3H, s), 1.73 (3H, s), 1.54 (9H, s), 1.46 (9H, s).

### Step 2 Synthesis of compound I-003

The compound 1-003 was obtained by the similar synthesis of the step 7 of the compound I-001 using the compound 2a (0.09 g, 0.121 mmol).
Yield amount: 20.2 mg (yield 32%)
¹H-NMR (DMSO-D₆) δ: 9.50 (1H, d, J = 8.1 Hz), 7.18 (2H, s), 6.77 (1H, s), 5.83 (1H, dd, J = 8.1, 4.7 Hz), 5.25 (1H, d, J = 4.7 Hz), 4.48 (2H, s), 2.92 (1H, d, J = 17.3 Hz), 2.35 (1H, d, J = 18.4 Hz).
MS (m+1) = 518

### [EXAMPLE 3]

### Synthesis of compound 1-036

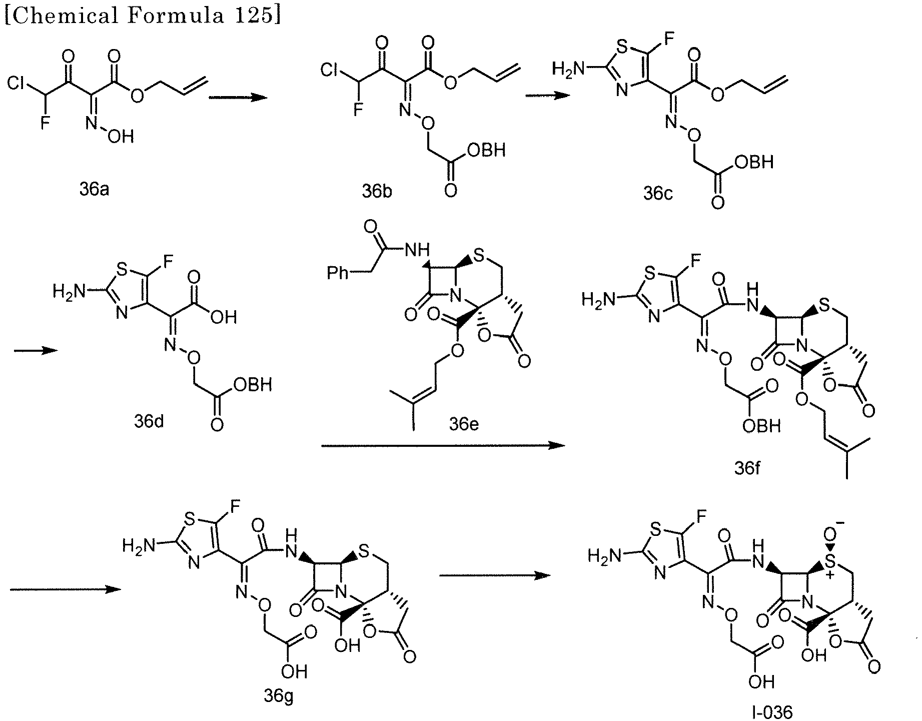

### Step 1 Synthesis of compound 36c

To a solution of the compound 36a (5.0 g, 33.5 mmol) in tetrahydrofuran, triphenylphosphine (8.80 g, 33.5 mmol) was added. The mixture was cooled to 0°C. DIAD (6.52 mL, 33.5 mmol) and benzhydryl hydroxyacetate were added to the mixture. The mixture was stirred at 0°C for 1 hour. The reaction mixture was poured into a hexane-ethyl acetate mixed solution. The resulting insoluble matter was filtered off. The solvent in the filtrate was evaporated under reduced pressure. The obtained residue was purified by silica gel column chromatography (hexane-ethyl acetate) to afford 10 g of a crude product containing the compound 36b. Thiourea (15.3 g, 201 mmol) was added to a solution of the compound 36b in DMA (60 mL). The mixture was stirred at 30°C for 24 hours. Insoluble matter was filtered off. The solvent in the filtrate was evaporated under reduced pressure. The obtained residue was purified by silica gel column chromatography (hexane-ethyl acetate) to afford the compound 36c (1.5 g, 9.5%).
¹H-NMR (CDCl₃) δ: 4.78 (2H, d, J = 5.8 Hz), 4.94 (4H, br s), 5.26 (1H, dd, J = 10.4, 1.0 Hz), 5.36 (1H, dd, J = 17.2, 1.3 Hz), 5.90-6.00 (1H, m), 6.96 (1H, s), 7.26-7.33 (10H, m).

### Step 2 Synthesis of compound 36d

Under nitrogen atmosphere, morpholine (789 mg, 9.05 mmol) and Pd(PPh₃)₄ (105 mg, 0.091 mmol) were added to a solution of the compound 36c (850 mg, 1.81 mmol) in tetrahydrofuran (13 mL). The mixture was stirred at room temperature for 1 hour. Water was added to the mixture, followed by extraction with ethyl acetate. The organic layer was washed by dilute hydrochloric acid and brine and then dried over anhydrous magnesium sulfate. The solvent was evaporated under reduced pressure to quantitatively afford the compound 36d.
MS (m-1) =428.01

### Step 3 Synthesis of compound 36g

A suspension of phosphorus pentachloride (0.754 g, 3.62 mmol) in dichloromethane (10 mL) was cooled to -40°C. Then, pyridine (0.584 mL, 7.24 mmol) was added, and subsequently, the compound 36e (0.805 g, 1.81 mmol) was added to the suspension. Under ice cooling, the mixture was stirred for 1 hour. Then, the reaction mixture was cooled to -78°C. Methanol (8.1 mL) was added to the reaction mixture. After stirring at -30°C for 2 hours, an aqueous solution of sodium hydrogen carbonate was added to the reaction mixture, followed by extraction with dichloromethane. The organic layer was dried over anhydrous magnesium sulfate. Then, inorganic matter was removed by filtration. Ethyl acetate was added to the filtrate. Dichloromethane and methanol were evaporated under reduced pressure to afford an ethyl acetate solution (solution A). HOBt (0.367 g, 2.72 mmol) was added to a solution of the compound 36d (777 mg, 1.81 mmol) in dichloromethane (10 mL). The reaction mixture was cooled to 0°C. EDC hydrochloride (0.416 g, 2.17 mmol) was added to the reaction mixture. Under ice cooling, the mixture was stirred for 1 hour. The resulting insoluble matter was filtered off. Under ice cooling, the filtrate was added to the solution A. The mixture was stirred for 1 hour. Water was added to the mixture, followed by extraction with ethyl acetate. The organic layer was washed by dilute hydrochloric acid, an aqueous solution of sodium hydrogen carbonate and brine. Then, the organic layer was dried over anhydrous magnesium sulfate. The solvent was evaporated under reduced pressure. The obtained residue was purified by silica gel column chromatography (hexane-ethyl acetate) to afford the crude product of the compound 36f (1 g). This crude product was dissolved in dichloromethane (10 mL). Anisole (1.48 mL, 13.6 mmol) was added to the solution. The mixture was cooled to -30°C. A 2 mol/L solution of aluminum chloride in nitromethane (1.48 mL, 13.6 mmol) was added to the mixture. The mixture was stirred at -30°C for 1 hour. Ice, diisopropyl ether, and acetonitrile were added in this order to the reaction mixture. The mixture was stirred to completely dissolve insoluble matter. Then, the aqueous layer was separated. The organic layer was subjected to extraction with water again. Then, all the aqueous layers were combined. HP20-SS resin was added to the mixture. Acetonitrile was evaporated under reduced pressure. The obtained mixed solution was purified by ODS column chromatography (water-acetonitrile). Fractions containing the desired compound were collected and concentrated under reduced pressure. Then, the residue was freeze-dried to afford the compound 36g (270 mg) as a white powder.
¹H-NMR (D₂O) δ: 2.62 (1H, d, J = 18.2 Hz), 2.94-2.78 (2H, m), 3.15-3.06 (2H, m), 4.67 (2H, s), 5.26 (1H, d, J = 4.5 Hz), 5.61 (1H, d, J = 4.5 Hz).
Anal. : C16H14FN5O9S2(H2O)4.2
Calc. : C, 33.19; H, 3.90; F, 3.28; N, 12.09; S 11.07 (%)
Found: C, 33.14; H, 3.60; F, 3.25; N, 12.11; S, 11.05 (%)

### Step 4 Synthesis of compound 1-036

Under nitrogen atmosphere, the compound 36g (16 mg, 0.034 mmol) was dissolved in a mixed solution of DMA (320 mL) and acetonitrile (230 mL). Under ice cooling, 38% peracetic acid (0.020 mL) was added to the solution. The mixture was stirred for 1 hour. An aqueous solution of sodium hydrogen sulfite was added to the reaction mixture. The mixture was stirred for 5 minutes. Then, ice, diisopropyl ether, and acetonitrile were added in this order to the mixture. The mixture was stirred to completely dissolve insoluble matter. Then, the aqueous layer was separated. The organic layer was subjected to extraction with water again. Then, all the aqueous layers were combined. HP20-SS resin was added to the mixture. Acetonitrile was evaporated under reduced pressure. The obtained mixed solution was purified by HP20-SS resin column chromatography (water-acetonitrile). Fractions containing the desired compound were collected and concentrated under reduced pressure. Then, the residue was freeze-dried to afford the compound 1-036 (3 mg) as a white powder.
MS (m+1) =520

### [EXAMPLE 4]

### Synthesis of compound 1-005

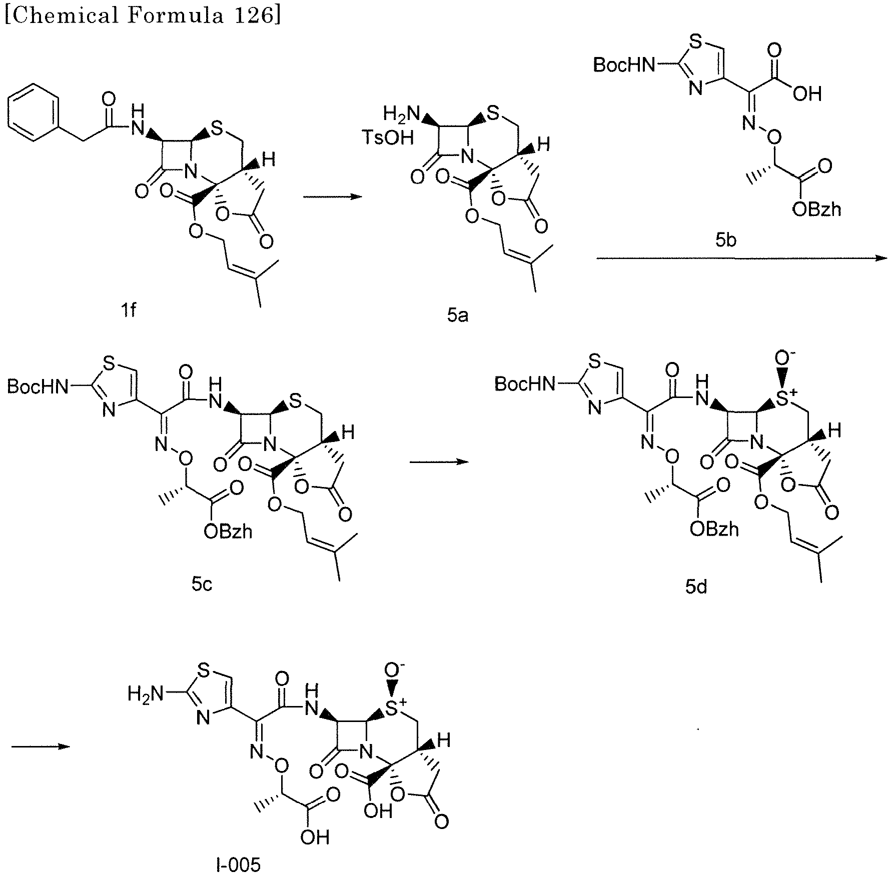

### Step 1 Synthesis of compound 5a

Under nitrogen atmosphere, phosphorus pentachloride (2.81 g, 13.5 mmol) and pyridine (1.20 ml, 14.9 mmol) were suspended in dichloromethane (15 ml) at -78°C. A solution of the compound If (3.00 g, 6.75 mmol) in dichloromethane (15 ml) was added to the suspension. Under ice cooling, the mixture was stirred for 1 hour and then cooled to -78°C. Ethanol (15 ml) was added to the mixture. The mixture was stirred at -25°C to -15°C for 1 hour. Then, a saturated aqueous solution of sodium bicarbonate was added to the reaction mixture. The organic layer was separated and dried over magnesium sulfate. Magnesium sulfate was filtered off. Then, tosylic acid monohydrate (5.14 g, 27.0 mmol) and ethyl acetate (50 ml) were added to the filtrate. Then, the mixture was concentrated under reduced pressure. Dichloromethane was evaporated. The precipitated crystals were collected by filtration to afford the compound 5a (1.64 g, yield 49%).
1H-NMR (DMSO-d6) δ: 8.64 (3.0H, br s), 7.47 (2.0H, d, J = 8.0 Hz), 7.11 (2.0H, d, J = 7.8 Hz), 5.37 (1.0H, t, J = 7.0 Hz), 5.16 (1.0H, d, J = 4.6 Hz), 4.83 (1.0H, d, J = 4.6 Hz), 4.76 (1.0H, dd, J = 12.0, 7.3 Hz), 4.66 (1.0H, dd, J = 12.3, 7.3 Hz), 3.00 (1.0H, dd, J = 18.2, 6.9 Hz), 2.87-2.77 (2.0H, m), 2.29 (3.0H, s), 1.74 (3.0H, s), 1.69 (3.0H, s). XRPD (measurement condition 1): diffraction angle 2θ (°): 8.0, 11.1, 13.1, 17.1, 21.6, 22.9, 25.0, 25.5, 27.1

### Steps 2, 3, and 4 Synthesis of compound 1-005

To a suspension of the compound 5b (578 mg, 1.1 mmol) synthesized as described in Bioorg. Med. Chem. 2007, 15, 6716-6732 and the compound 3a (499 mg, 1.0 mmol) in ethyl acetate (10 mL), phenyl dichlorophosphate (232 mg, 1.1 mmol) was added, and then, N-methylmorpholine (405 mg, 4.0 mmol) was added at 0°C. The mixture was stirred at 0°C for 1 hour. Then, water was added to the mixture, followed by extraction with ethyl acetate. The organic layer was washed by 1 mol/L hydrochloric acid, 8.4% aqueous solution of sodium hydrogen carbonate, water, and brine in this order and dried over anhydrous magnesium sulfate. Inorganic matter was removed by filtration. The filtrate was concentrated under reduced pressure to afford the compound 5c (965.1 mg) as the crude product of a yellow foam.

The compound I-005 (184.4 mg, 35.7% yield (3 steps)) was obtained as a white solid by the similar synthesis of the steps 6 and 7 of Example 1 using the compound 5c (834 mg, 1.0 mmol).
¹H-NMR (D2O) δ: 1.58 (3H, d, J = 7.2 Hz), 2.56 (1H, d, J = 18.3 Hz), 2.83 (1H, dd, J = 14.6, 12.5 Hz), 3.07 (1H, dd, J = 18.3, 7.8 Hz), 3.51-3.57 (1H, m), 3.61-3.69 (1H, m), 4.96 (1H, q, J = 7.1 Hz), 5.02 (1H, d, J = 4.8 Hz), 5.91 (1H, t, J = 7.0 Hz), 7.24 (1H, s).
MS (M+1) = 515, (measurement condition A)
Anal. : C17H17N5O10S2(H2O)2.5
Calc. C: 36.43% H: 3.96% N: 12.49% S: 11.44%
Found C: 36.28% H: 3.93% N: 12.63% S: 11.64%

### [EXAMPLE 5]

### Synthesis of compound I-027

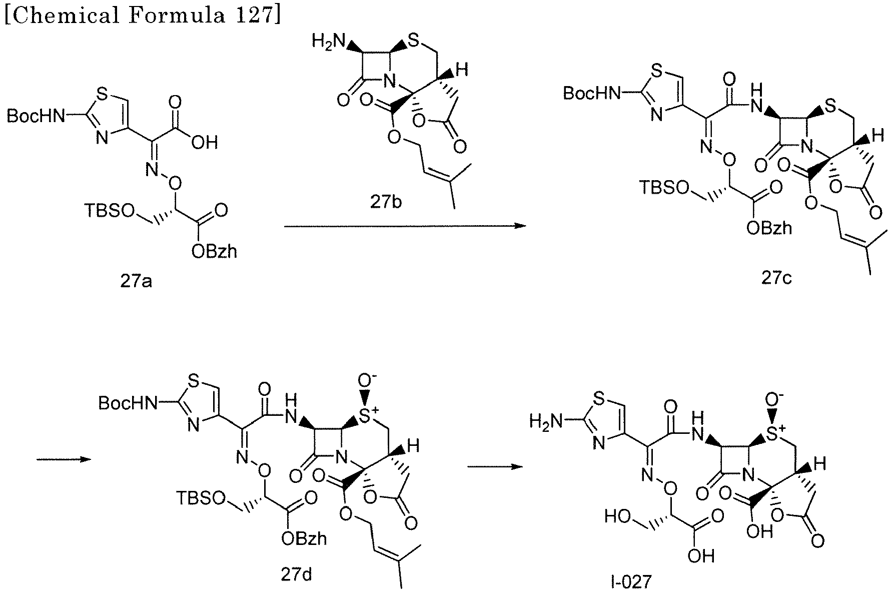

### Steps 1, 2, and 3 Synthesis of compound 1-027

The compound 27c (617.2 mg, 42.7% yield) was obtained as the crude product of a clear colorless oil by the similar synthesis of the step 5 of Example 1 using the compound 27a (1181 mg, 1.8 mmol) synthesized as described in JP5909441B2 and the compound 27b (490 mg, 1.5 mmol).

The compound 1-027 (144.7 mg, 42.5% yield (2 steps)) was obtained as a white solid by the similar synthesis of the steps 6 and 7 of Example 1 using the obtained compound 27c (617.2 mg, 0.64 mmol).
1H-NMR (D2O) δ: 2.56 (1.0H, d, J = 18.2 Hz), 2.81-2.84 (1.2H, m), 3.00-3.09 (1.2H, m), 3.22 (0.2H, dd, J = 12.0, 6.0 Hz), 3.51-3.57 (1.2H, m), 3.65 (1.0H, dd, J = 14.9, 5.1 Hz), 3.72 (0.2H, dd, J = 7.1, 3.5 Hz), 4.04-4.08 (2.4H, m), 4.94-4.95 (1.2H, m), 5.02 (1.0H, d, J = 4.5 Hz), 5.13 (0.2H, d, J = 4.3 Hz), 5.71 (0.2H, d, J = 4.3 Hz), 5.91 (1.0H, d, J = 4.8 Hz), 6.80 (0.2H, s), 7.19 (0.2H, s), 7.23 (1.0H, s).
MS (M+1) = 531, (measurement condition A)
Anal. : C17H17N5O11S2(H2O)2.6
Calc. C: 35.31% H: 3.87% N: 12.11% S: 11.09%
Found C: 35.46% H: 3.94% N: 12.09% S: 10.68%

### [EXAMPLE 6]

### Synthesis of compounds 1-037 and 1-038

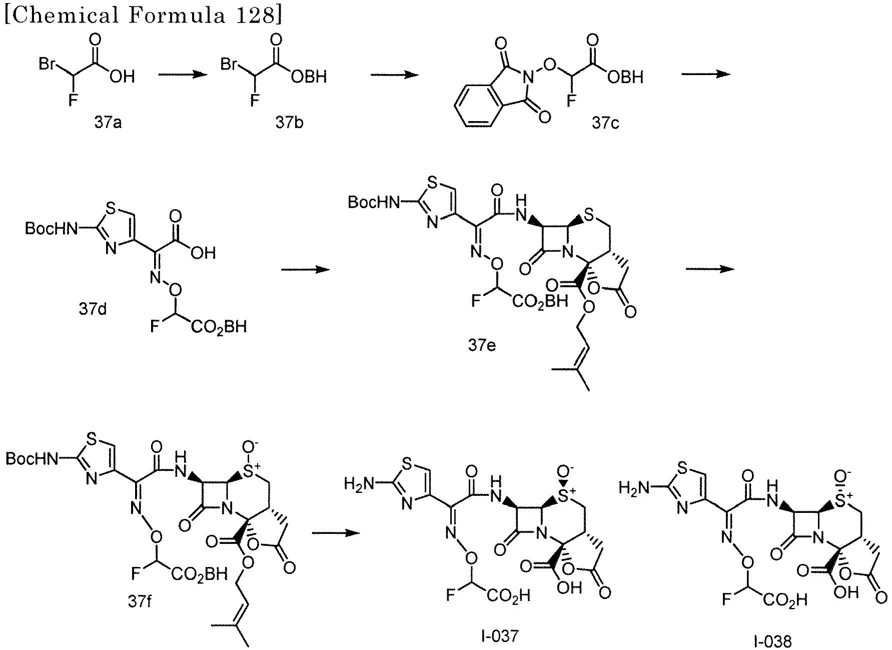

### Step 1 Synthesis of compound 37b

The compound 37a (5.00 g, 31.9 mmol) was dissolved in tetrahydrofuran (50 ml). Under ice cooling, a solution of diphenyldiazomethane (8.04 g, 41.4 mmol) in tetrahydrofuran (10 ml) was added to the solution. The mixture was stirred overnight at room temperature and then concentrated under reduced pressure. The residue was subjected to silica gel column chromatography, followed by elution with hexane/ethyl acetate. Fractions containing the desired compound were concentrated under reduced pressure to afford the compound 37b (9.86 g, 96%).
1H-NMR (CDCl3) δ: 7.39-7.31 (10H, m), 6.98 (1H, s), 6.66 (1H, d, J = 50.6 Hz).

### Step 2 Synthesis of compound 37c

The obtained compound 37b (2.00 g, 6.19 mmol) and N-hydroxyphthalimide (1.21 g, 7.43 mmol) were dissolved in dimethylformamide (20 ml). Diisopropylethylamine (1.30 ml, 7.43 mmol) was added to the solution. The mixture was stirred overnight at room temperature. Then, water was added to the mixture, followed by extraction with ethyl acetate. The organic layer was washed by water and brine in this order and dried over anhydrous magnesium sulfate. Magnesium sulfate was filtered off. Then, the filtrate was concentrated under reduced pressure. Diisopropyl ether was added to the residue to precipitate a solid. The precipitates were collected by filtration to afford the compound 37c (2.28 g, 91%).
¹H-NMR (CDCl3) δ: 7.87 (2H, dd, J = 5.6, 3.1 Hz), 7.80 (2H, dd, J = 5.5, 3.1 Hz), 7.43-7.28 (10H, m), 7.01 (1H, s), 6.02 (1H, d, J = 57.2 Hz).

### Step 3 Synthesis of compound 37d

The crude product of the compound 37d (3.45 g) was obtained by the similar synthesis of the step 2 of Example 7 using the compound 37c (2.28 g, 5.62 mmol).
1H-NMR (CDCl3) δ: 7.38-7.21 (10H, m), 7.14 (1H, s), 6.98 (1H, s), 6.18 (1H, d, J = 56.5 Hz), 1.53 (9H, s).

### Step 4 Synthesis of compound 37e

The compound 37e (2.08 g, 55%) was obtained as a 1:1 mixture of diastereomers derived from the configuration of the fluorine group by the similar synthesis of the step 5 of Example 1 using the compound 37d (2.08 g, 4.95 mmol). 1H-NMR (CDCl3) δ: 7.39-7.28 (10.0H, m), 6.99 (0.5H, s), 6.98 (0.5H, s), 6.32-6.17 (1.0H, m), 5.62-5.55 (1.0H, m), 5.37 (1.0H, t, J = 6.8 Hz), 4.90-4.73 (3.0H, m), 3.19 (1.0H, br s), 2.86-2.40 (4.0H, m), 1.75 (3.0H, s), 1.71 (3.0H, s), 1.55 (9.0H, s).

### Step 5 Synthesis of compound 37f

The compound 37f (0.91 g, 89%) was obtained as a mixture by the similar synthesis of the step 6 of Example 1 using the compound 37e (1.0 g, 1.19 mmol). LC/MS (measurement condition A), retention time: 2.66, 2.71 min, [M+H] = 854

### Step 6 Synthesis of compounds 1-037 and 1-038

The compounds 1-037 and 1-038 were obtained by the similar synthesis of the step 7 of Example 1 using the compound 37f (0.91 g, 1.07 mmol) and using titanium tetrachloride instead of aluminum trichloride.
Compound I-037: 79.3 mg (yield 12%)
1H-NMR (D2O) δ: 7.38 (1H, d, J = 3.4 Hz), 6.10 (1H, d, J = 57.2 Hz), 5.88 (1H, d, J = 4.8 Hz), 5.01 (1H, d, J = 4.8 Hz), 3.64 (1H, dd, J = 14.7, 5.2 Hz), 3.57-3.51 (1H, m), 3.06 (1H, dd, J = 18.3, 7.8 Hz), 2.82 (1H, dd, J = 14.6, 12.6 Hz), 2.56 (1H, d, J = 18.2 Hz).
C16H14FN5O10S2(H2O)3.1
Calc. C: 33.41%, H: 3.54%, F: 3.30%, N: 12.17%, S: 11.15%
Found C: 33.49%, H: 3.60%, F: 3.19%, N: 12.29%, S: 11.04%
Compound 1-038: 41.5 mg (yield 6%)
1H-NMR (D2O) δ: 7.30 (1H, s), 6.08 (1H, d, J = 56.2 Hz), 5.71 (1H, d, J = 4.4 Hz), 5.11 (1H, d, J = 4.4 Hz), 3.71 (1H, dd, J = 13.2, 4.6 Hz), 3.62-3.55 (1H, m), 3.21 (1H, dd, J = 13.2, 11.0 Hz), 3.03 (1H, dd, J = 18.5, 8.3 Hz), 2.84 (1H, dd, J = 18.6, 3.1 Hz). C16H14FN5O10S2(H2O)3.4
Calc. C: 33.09%, H: 3.61%, F: 3.27%, N: 12.06%, S: 11.04%
Found C: 33.08%, H: 3.65%, F: 3.30%, N: 12.23%, S: 10.93%

### [EXAMPLE 7]

### Synthesis of compound 1-039

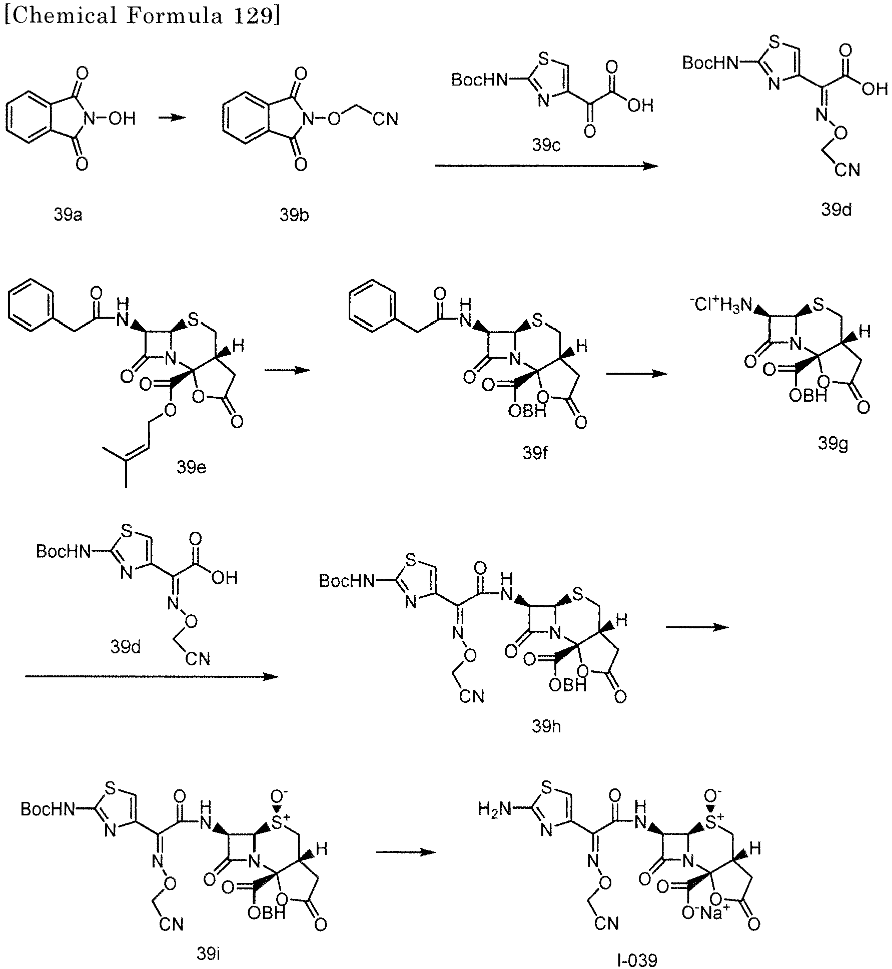

### Step 1 Synthesis of compound 39b

The compound 39a (4.89 g, 30.0 mmol) was dissolved in dimethylformamide (50.0 mL). The solution was cooled to 0°C. Triethylamine (4.57 mL, 33.0 mmol) and 2-bromoacetonitrile (2.20 mL, 33.0 mmol) were added to the solution. The mixture was stirred at room temperature. After the completion of reaction, the reaction mixture was poured into purified water (150 mL). The precipitates were collected by filtration, washed by purified water and then hexane, and dried in air to afford the compound 39b (5.32 g, yield 88%).
1H-NMR (CDCl3) δ: 7.93-7.88 (m, 2H), 7.85-7.80 (m, 2H), 4.96 (s, 2H).

### Step 2 Synthesis of compound 39d

The compound 39b (2.02 g, 10.0 mmol) was dissolved in dichloromethane (25.0 mL). The solution was cooled to -30°C. Methylhydrazine (0.583 mL, 11.0 mmol) was added to the solution. The mixture was stirred at room temperature for 30 minutes. The precipitates were removed. The reaction mixture was concentrated under reduced pressure. The residue was dissolved in dichloromethane (25.0 mL). The compound 39c (2.72 g, 10.0 mmol) was added to the solution. The mixture was stirred overnight at room temperature. The solvent was evaporated. Ethyl acetate, purified water, and a 2 mol/L aqueous solution of hydrochloric acid were added to the obtained residue, followed by extraction with ethyl acetate. The organic layer was washed by purified water and then brine, dried using anhydrous magnesium sulfate, and filtered. The obtained filtrate was concentrated under reduced pressure. The precipitates were collected by filtration and washed by ethyl acetate to afford the compound 39d (1.88 g, yield 58%).
1H-NMR (DMSO-D6) δ: 11.87 (br s, 1H), 7.55 (s, 1H), 5.11 (s, 2H), 1.48 (s, 9H).

### Step 3 Synthesis of compound 39f

The compound 39e (31.5 g, 70.9 mmol) was dissolved in tetrahydrofuran (320 mL). 5% palladium carbon (15.1 g, 7.09 mmol) was added to the solution. Under hydrogen atmosphere, the mixture was stirred at room temperature for 4 hours and 30 minutes. The catalyst was removed by filtration through Celite. 5% palladium carbon (15.1 g, 7.09 mmol) was added again to the filtrate. Under hydrogen atmosphere, the mixture was stirred at room temperature for 1 hour. The catalyst was removed by filtration through Celite. Diphenyldiazomethane (15.1 g, 78.0 mmol) was added to the filtrate. The mixture was stirred at room temperature for 1 hour. The solvent was evaporated. Ethyl acetate and diisopropyl ether were added to the residue. The resulting solid was collected by filtration and dried to afford the compound 39f (27.5 g, yield 71%).
1H-NMR (CDCl3) δ: 7.40-7.30 (m, 14H), 6.94 (s, 1H), 6.02 (d, J = 8.8 Hz, 1H), 5.55 (dd, J = 8.8, 4.7 Hz, 1H), 4.99 (d, J = 4.7 Hz, 1H), 3.67 (d, J = 16.2 Hz, 1H), 3.61 (d, J = 16.2 Hz, 1H), 3.17-3.08 (m, 1H), 2.90 (dd, J = 14.6, 4.5 Hz, 1H), 2.62 (dd, J = 14.6, 9.7 Hz, 1H), 2.54-2.50 (m, 2H).

### Step 4 Synthesis of compound 39g

Phosphorus pentachloride (15.4 g, 73.7 mmol) was suspended in dichloromethane (200 mL). The suspension was cooled to 0°C. Pyridine (6.55 mL, 81.0 mmol) and the compound 6 (20.0 g, 36.9 mmol) were added to the suspension. The mixture was stirred at 0°C for 30 minutes. This solution was cooled to -78°C. Ethanol (200 mL) was added to the solution. The mixture was warmed to -30°C and stirred for 2 hours. Purified water (33.2 mL, 1.84 mol) was added to the solution. The mixture was stirred. The reaction mixture was further diluted with purified water. Dichloromethane was evaporated. The precipitated crystals were collected by filtration. The obtained crystals were washed by purified water and ethyl acetate and dried to afford the compound 39g (14.1 g, yield 83%) as crystals.
1H-NMR (DMSO-D6) δ: 8.79 (br s, 2H), 7.47-7.27 (m, 10H), 6.91 (s, 1H), 5.18 (d, J = 4.6 Hz, 1H), 4.84 (d, J = 4.6 Hz, 1H), 3.46-3.27 (m, 2H), 3.06 (dd, J = 18.3, 7.1 Hz, 1H), 2.89 (dd, J = 14.3, 6.0 Hz, 1H), 2.74 (dd, J = 10.2, 20.0 Hz, 1H).
XRPD (measurement condition 2): diffraction angle 2θ (°): 4.2, 8.2, 10.0, 16.2, 17.7, 20.3, 21.4, 23.7, 23.9, 27.6, 28.4, 29.1, 29.5, 32.6
Anal. C22H20N2O5SHCl(H2O) 1.0
Calc. : C, 55.17; H, 4.84; N, 5.85; S, 6.69; Cl, 7.40 (%)
Found: C, 55.26; H, 4.87; N, 6.23; S, 6.68; Cl, 7.14 (%)

### Step 5 Synthesis of compound 39h

The compound 39 g (461 mg, 1.00 mmol) was suspended in ethyl acetate (5.00 mL). The compound 39d (392 mg, 1.20 mmol) was added to the suspension. The mixture was cooled to -40°C. Phenyl dichlorophosphate (0.193 mL, 1.30 mmol) and N-methylmorpholine (0.44 mL, 4.00 mmol) were added to the suspension. The mixture was stirred at -40°C for 1 hour. Purified water was added to the solution. The solvent was evaporated, followed by extraction with ethyl acetate. The organic layer was washed by purified water and then brine and dried over anhydrous magnesium sulfate. After filtration, the solvent was evaporated. The obtained residue was subjected to silica gel chromatography to afford the compound 39h (758 mg).
1H-NMR (CDCl3) δ: 8.70 (br s, 1H), 7.56-7.47 (m, 1H), 7.38-7.20 (m, 10H), 6.94 (s, 1H), 5.71 (dd, J = 8.5, 4.6 Hz, 1H), 5.15 (d, J = 4.6 Hz, 1H), 4.92-4.82 (m, 2H), 3.24-3.17 (m, 1H), 3.10-3.04 (m, 1H), 2.76-2.67 (m, 1H), 2.57-2.52 (m, 2H), 1.54 (s, 9H).

### Step 6 Synthesis of compound 39i

The compound 39h (366 mg, 0.50 mmol) was dissolved in dichloromethane (4.00 mL). The solution was cooled to -40°C. m-chloroperbenzoic acid (138 mg, 0.550 mmol) was added to the solution. The mixture was stirred at -40°C for 30 minutes. An aqueous solution of sodium thiosulfate was added to the reaction mixture. The solvent was evaporated, followed by extraction with ethyl acetate. The organic layer was washed by purified water and then brine and dried over anhydrous magnesium sulfate. After filtration, the solvent was evaporated. The obtained residue was subjected to silica gel chromatography to afford the compound 39i (257 mg, yield 69%).
1H-NMR (CDCl3) δ: 8.54 (br s, 1H), 7.78 (d, J = 9.9 Hz, 1H), 7.37-7.28 (m, 11H), 7.01 (s, 1H), 6.03 (dd, J = 9.9, 4.9 Hz, 1H), 4.91-4.82 (m, 2H), 4.68 (d, J = 4.9 Hz, 1H), 3.74-3.67 (m, 1H), 3.46 (dd, J = 14.6, 5.0 Hz, 1H), 2.63 (dd, J = 17.8, 7.8 Hz, 1H), 2.53-2.44 (m, 1H), 2.33 (d, J = 17.8 Hz, 1H), 1.60 (s, 9H).

### Step 7 Synthesis of compound I-039

The compound 39i (257 mg, 0.344 mmol) was dissolved in dichloromethane (3.0 mL). The solution was cooled to -30°C. Anisole (0.225 mL, 2.06 mmol) and a 2 mol/L solution of aluminum chloride in nitromethane (1.03 mL, 2.06 mmol) were added to the solution. The mixture was stirred at -30°C for 30 minutes. Purified water and diisopropyl ether were added to the reaction mixture. Acetonitrile was added to the reaction mixture. After dissolution of the precipitates, the aqueous layer was separated. The organic layer was subjected to extraction with purified water. HP20SS was added to combined aqueous layers. The mixture was concentrated. The concentrated suspension was subjected to column chromatography with HP20SS connected to ODS, followed by elution with water-acetonitrile. Fractions containing the compound of interest were collected and pH-adjusted to 7 with a 0.2 mol/L aqueous solution of sodium hydroxide. A piece of dry ice was added to the mixture. The mixture was concentrated under reduced pressure. The concentrate was freeze-dried to afford the compound I-039 as a powder (yield amount 121 mg, yield 70%).
1H-NMR (D2O) δ: 7.18 (s, 1H), 5.87 (d, J = 4.5 Hz, 1H), 5.05 (s, 2H), 5.01 (d, J = 4.8 Hz, 1H), 3.64 (dd, J = 14.7, 5.1 Hz, 1H), 3.58-3.52 (m, 1H), 3.08 (dd, J = 18.4, 7.8 Hz, 1H), 2.83 (dd, J = 12.1, 14.7 Hz, 1H), 2.57 (d, J = 18.4 Hz, 1H).
MS (M+1): 483, retention time: 0.48 min (measurement condition A)
Anal. C16H13N6NaO8S2(H2O)3.3
Calc. : C, 34.08; H, 3.50; N, 14.90; Na, 4.08; S, 11.37 (%)
Found: C, 34.00; H, 3.45; N, 15.05; Na, 4.45; S, 11.21 (%)

### [EXAMPLE 8]

### Synthesis of compound I-040

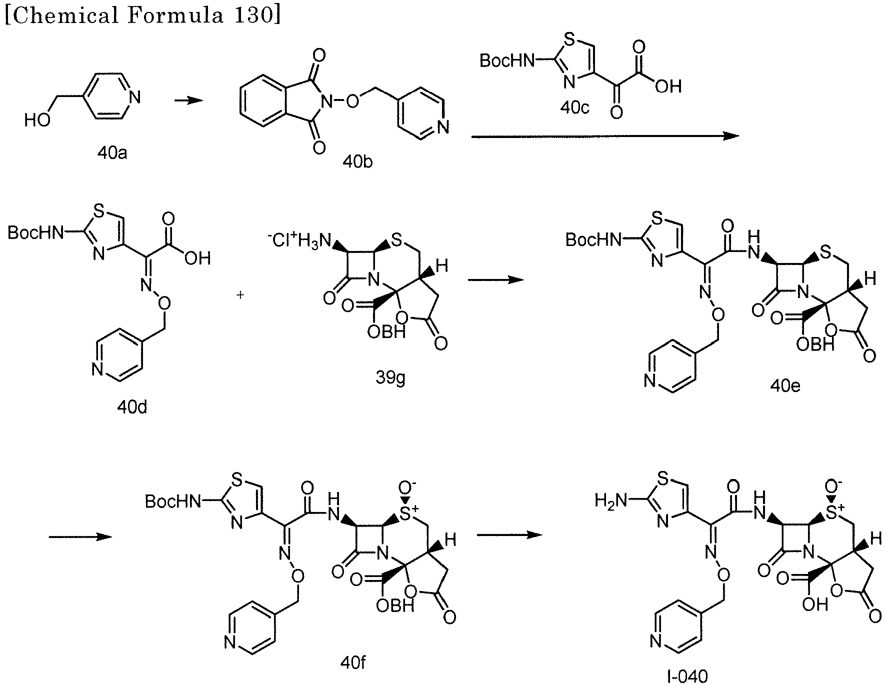

### Step 1 Synthesis of compound 40b

The compound 40a (3.27 g, 30.0 mmol) was dissolved in tetrahydrofuran (100 mL). The solution was cooled to 0°C. Triphenylphosphine (9.44 g, 36.0 mmol), N-hydroxyphthalimide (5.87 g, 36.0 mmol), and DIAD (7.00 mL, 36.0 mmol) were added to the solution. The mixture was stirred at room temperature for 1 hour. The solvent was evaporated. Methanol was added to the residue. The obtained solid was collected by filtration, washed by methanol, and dried to afford the compound 40b (6.12 g, yield 80%).
1H-NMR (CDCl3) δ: 8.66 (d, J = 6.0 Hz, 2H), 7.85-7.83 (m, 2H), 7.77-7.75 (m, 2H), 7.48 (d, J = 6.0 Hz, 2H), 5.25 (s, 2H).

### Step 2 Synthesis of compound 40d

The compound 40b (2.54 g, 10.0 mmol) was dissolved in dichloromethane (25.0 mL). The solution was cooled to -30°C. Methylhydrazine (0.583 mL, 11.0 mmol) was added to the solution. The mixture was stirred at room temperature for 30 minutes. The precipitates were removed. The reaction mixture was concentrated under reduced pressure. The residue was dissolved in dichloromethane (25.0 mL). The compound 40c (2.72 g, 10.0 mmol) was added to the solution. The mixture was stirred overnight at room temperature. The solvent was evaporated. Ethyl acetate, purified water, and a 2 mol/L aqueous solution of hydrochloric acid were added to the obtained residue, followed by extraction using ethyl acetate. The obtained organic layer was washed by purified water and then brine and dried using anhydrous magnesium sulfate. After filtration, the solvent was evaporated. The obtained solid was collected by filtration and washed by ethyl acetate to afford the compound 40d (1.84 g, yield 49%).
1H-NMR (DMSO-D6) δ: 11.77 (s, 1H), 8.56 (d, J = 6.3 Hz, 2H), 7.43 (s, 1H), 7.33 (d, J = 6.3 Hz, 2H), 5.26 (s, 2H), 1.46 (s, 9H).

### Step 3 Synthesis of compound 40e

The compound 40d (416 mg, 1.10 mmol) and the compound 39g (461 mg, 1.00 mmol) were suspended in dichloromethane (5.00 mL). The suspension was cooled to 0°C. Pyridine (0.178 mL, 2.20 mmol) and 1-(3-dimethylaminopropyl)-3-ethylcarbodiimide hydrochloride (211 mg, 1.10 mmol) were added to the suspension. The mixture was stirred at 0°C for 1 hour. Purified water was added to the solution. The solvent was evaporated, followed by extraction with ethyl acetate. The organic layer was washed by purified water and then brine. The organic layer was dried over anhydrous magnesium sulfate. After filtration, the solvent was evaporated. The obtained residue was subjected to silica gel chromatography to afford the compound 40e (417 mg, yield 53%).
1H-NMR (CDCl3) δ: 8.52-8.42 (m, 3H), 7.56 (d, J = 7.8 Hz, 1H), 7.38-7.22 (m, 12H), 6.97 (s, 1H), 5.74 (dd, J = 8.6, 4.7 Hz, 1H), 5.33-5.26 (m, 2H), 5.12 (d, J = 4.6 Hz, 1H), 3.12-3.06 (m, 1H), 2.91 (dd, J = 19.4, 6.7 Hz, 1H), 2.64-2.46 (m, 3H), 1.54 (s, 9H).

### Step 4 Synthesis of compound 40f

The compound 40f (156 mg, yield 76%) was obtained by the similar synthesis of the step 6 of Example 7 using the compound 40e (200 mg, 0.255 mmol).
1H-NMR (CDCl3) δ: 8.63 (s, 1H), 8.57 (d, J = 5.9 Hz, 2H), 7.72 (d, J = 9.9 Hz, 1H), 7.37-7.29 (m, 13H), 7.01 (s, 1H), 6.08 (dd, J = 9.9, 4.9 Hz, 1H), 5.29 (s, 2H), 4.64 (d, J = 4.9 Hz, 1H), 3.72-3.65 (m, 1H), 3.36 (dd, J = 14.5, 5.0 Hz, 1H), 2.62 (dd, J = 17.9, 7.8 Hz, 1H), 2.47-2.37 (m, 1H), 2.30 (d, J = 17.9 Hz, 1H), 1.54 (s, 9H).

### Step 5 Synthesis of compound 1-040

The compound 40f (156 mg, 0.195 mmol) was dissolved in dichloromethane (2.0 mL). The solution was cooled to -30°C. Anisole (0.128 mL, 1.17 mmol) and a 2 mol/L solution of aluminum chloride in nitromethane (0.584 mL, 1.17 mmol) were added to the solution. The mixture was stirred at -30°C for 30 minutes. Purified water and diisopropyl ether were added to the reaction mixture. Acetonitrile was added to the reaction mixture. After dissolution of the precipitates, the aqueous layer was separated. The organic layer was subjected to extraction with purified water. HP20SS was added to combined aqueous layers. The mixture was concentrated. The concentrated suspension was subjected to column chromatography with HP20SS connected to ODS, followed by elution with water-acetonitrile. Fractions containing the compound of interest were collected and concentrated under reduced pressure. The concentrate was freeze-dried to afford the compound 1-040 as a powder (yield amount 65.3 mg, yield 63%).
¹H-NMR (DMSO-D6) δ: 9.35 (d, J = 7.3 Hz, 1H), 8.53 (d, J = 6.0 Hz, 2H), 7.40 (d, J = 5.9 Hz, 2H), 7.21 (s, 2H), 6.90 (s, 1H), 5.76 (dd, J = 7.3, 4.8 Hz, 1H), 5.20 (s, 2H), 4.97 (d, J = 4.8 Hz, 1H), 3.51-3.33 (m, 2H), 3.05 (dd, J = 18.2, 8.0 Hz, 1H), 2.87 (t, J = 13.5 Hz, 1H), 2.45 (d, J = 18.8 Hz, 1H).
MS (M+1): 535, retention time: 0.32 min (measurement condition A)
Anal. C20H18N6O8S2(H2O)4.3
Calc. : C, 39.25; H, 4.38; N, 13.73; S, 10.48 (%)
Found: C, 39.15; H, 4.13; N, 13.94; S, 10.45 (%)

### [EXAMPLE 9]

### Synthesis of compound 1-041

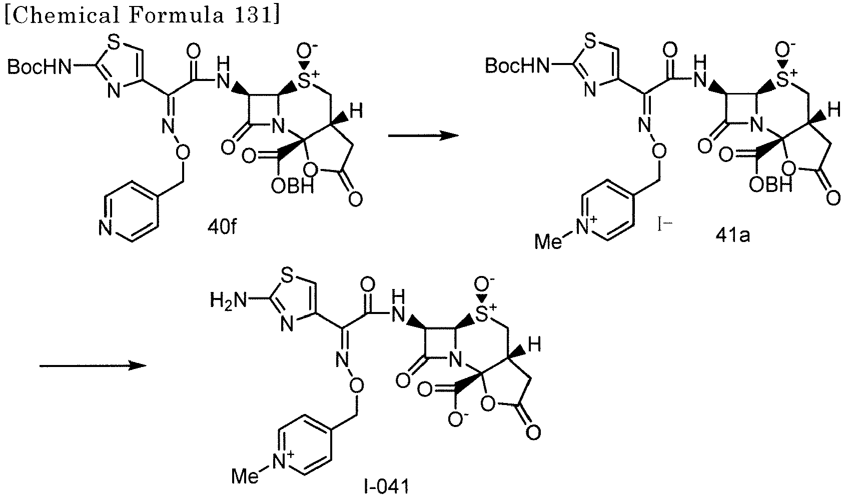

### Step 1 Synthesis of compound 41a

The compound 40f (206 mg, 0.257 mmol) was dissolved in dimethylformamide (0.618 mL). Methyl iodide (0.080 mL, 1.29 mmol) was added to the solution. The mixture was stirred overnight at room temperature. Purified water was added to the reaction mixture, followed by extraction with ethyl acetate. The organic layer was washed by purified water and then brine and dried over anhydrous magnesium sulfate. After filtration, the solvent was evaporated to afford the compound 41a. The compound 41a was used directly in the next reaction without being purified.

### Step 2 Synthesis of compound 1-041

The compound I-041 (71.0 mg, yield 50%) was obtained by the similar synthesis of the step 5 of Example 8 using the compound 41a (0.257 mmol).
1H-NMR (D2O) δ: 8.77-8.71 (m, 2H), 8.05-8.00 (m, 2H), 7.13-7.08 (m, 1H), 5.95-5.90 (m, 1H), 5.57 (s, 2H), 5.06-4.97 (m, 1H), 4.37 (s, 3H), 3.66-3.49 (m, 2H), 3.13-3.02 (m, 1H), 2.89-2.77 (m, 1H), 2.62-2.52 (m, 1H).
MS (M+1): 549, retention time: 0.29 min (measurement condition A)
Anal. C21H20N6O8S2(H2O)4.5
Calc. : C, 40.06; H, 4.64; N, 13.35; S, 10.18 (%)
Found: C, 40.10; H, 4.65; N, 13.33; S, 10.10 (%)

### [EXAMPLE 10]

### Synthesis of compound 1-042

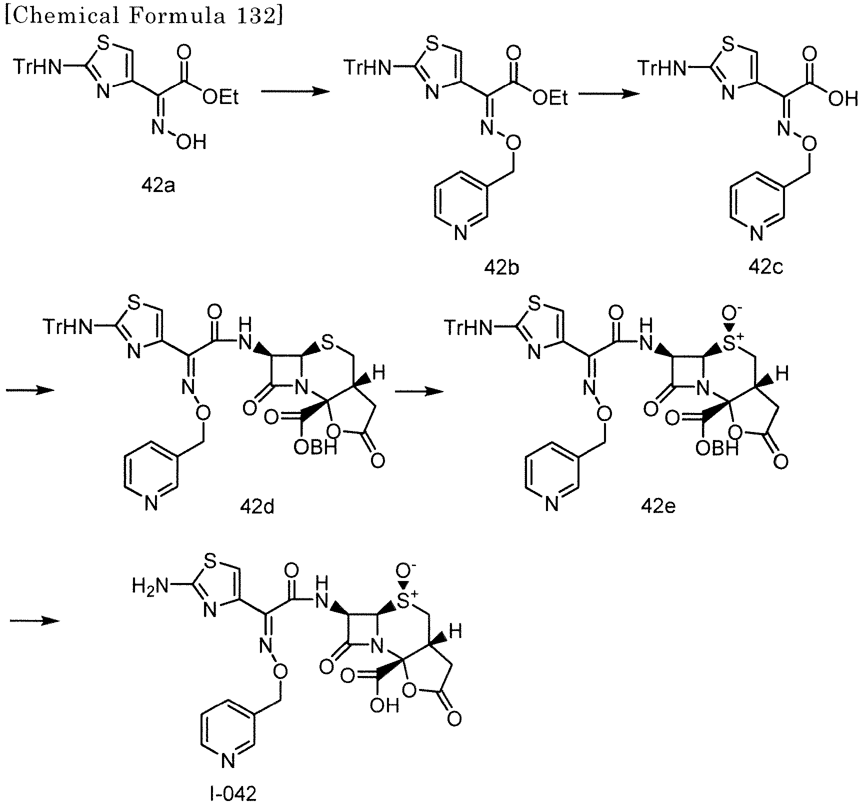

### Step 1 Synthesis of compound 42b

The compound 42a (2.29 g, 5.00 mmol) was dissolved in tetrahydrofuran (20.0 mL). The solution was cooled to 0°C. Pyridine-3-methanol (655 mg, 6.00 mmol), tributylphosphine (1.48 mL, 6.00 mmol), and 1,1'(azodicarbonyl)dipiperidine (1.51 g, 6.00 mmol) were added to the solution. The mixture was stirred overnight at room temperature. The reaction mixture was concentrated under reduced pressure. The obtained residue was subjected to silica gel chromatography to afford the compound 42b (886 mg, yield 32%).
1H-NMR (CDC13) δ: 8.62-8.58 (m, 1H), 8.54 (dd, J = 4.9, 1.5 Hz, 1H), 7.69 (d, J = 7.8 Hz, 1H), 7.32-7.28 (m, 15H), 6.96 (s, 1H), 6.51 (s, 1H), 5.31 (s, 2H), 4.35 (q, J = 7.0 Hz, 2H), 2.05 (s, 1H), 1.30 (t, J = 7.0 Hz, 6H).

### Step 2 Synthesis of compound 42c

The compound 42b (886 mg, 1.62 mmol) was dissolved in tetrahydrofuran (4.00 mL) and methanol (4.00 mL). A 1 mol/L aqueous solution of sodium hydroxide (3.23 mL, 3.23 mmol) was added to the solution. The mixture was stirred at room temperature for 30 minutes and then heated to reflux for 30 minutes. The reaction mixture was cooled to room temperature. A 2 mol/L aqueous solution of hydrochloric acid (1.62 mL, 3.23 mmol) was added to the reaction mixture. Tetrahydrofuran and methanol were evaporated. The precipitates were collected by filtration, washed by purified water and ethyl acetate, and dried to afford the compound 42c (660 mg, yield 79%).
1H-NMR (CDCl3) δ: 8.83 (s, 1H), 8.55-8.50 (m, 2H), 7.39 (dd, J = 7.7, 4.8 Hz, 1H), 7.34-7.19 (m, 16H), 6.85 (s, 1H), 5.15 (s, 2H).

### Step 3 Synthesis of compound 42d

The compound 42d (821 mg, yield 77%) was obtained by the similar synthesis of the step 3 of Example 8 using the compound 42c (531 mg, 1.27 mmol).
1H-NMR (CDCl3) δ: 8.60 (d, J = 2.0 Hz, 1H), 8.53-8.50 (m, 1H), 7.72 (dt, J = 7.8, 1.9 Hz, 1H), 7.35-7.28 (m, 25H), 7.00 (s, 1H), 6.94 (s, 1H), 6.85 (d, J = 8.8 Hz, 1H), 6.73 (s, 1H), 5.61 (dd, J = 8.8, 4.8 Hz, 1H), 5.32 (s, 2H), 5.02 (d, J = 4.8 Hz, 1H), 3.14-3.02 (m, 1H), 2.84 (dd, J = 14.4, 4.5 Hz, 1H), 2.58-2.45 (m, 3H).

### Step 4 Synthesis of compound 42e

The compound 42e (350 mg, yield 84%) was obtained by the similar synthesis of the step 6 of Example 7 using the compound 42d (410 mg, 0.442 mmol).
1H-NMR (CDCl3) δ: 8.65 (d, J = 1.6 Hz, 1H), 8.53 (dd, J = 4.8, 1.4 Hz, 1H), 7.76 (d, J = 7.8 Hz, 1H), 7.42 (d, J = 10.2 Hz, 1H), 7.35-7.28 (m, 25H), 7.06 (s, 1H), 6.99 (s, 1H), 6.66 (s, 1H), 6.00 (dd, J = 10.2, 4.9 Hz, 1H), 5.33 (s, 2H), 4.51 (d, J = 4.9 Hz, 1H), 3.69-3.63 (m, 1H), 3.27 (dd, J = 14.4, 4.9 Hz, 1H), 2.60 (dd, J = 17.9, 7.7 Hz, 1H), 2.37-2.24 (m, 2H).

### Step 5 Synthesis of compound 1-042

The compound 42e (175 mg, 0.186 mmol) was dissolved in dichloromethane (1.0 mL). The solution was cooled to 0°C. Anisole (0.122 mL, 1.13 mmol) and trifluoroacetic acid (0.572 mL, 7.42 mmol) were added to the solution. The mixture was stirred at 0°C for 1 hour. Purified water and diisopropyl ether were added to the reaction mixture. Acetonitrile was added to the reaction mixture. After dissolution of the precipitates, the aqueous layer was separated. The organic layer was subjected to extraction with purified water. HP20SS was added to combined aqueous layers. The mixture was concentrated. The concentrated suspension was subjected to column chromatography with HP20SS connected to ODS, followed by elution with water-acetonitrile. Fractions containing the compound of interest were collected and concentrated under reduced pressure. The concentrate was freeze-dried to afford the compound 1-042 as a powder (yield amount 65.3 mg, yield 63%).
1H-NMR (DMSO-D6) δ: 9.25 (d, J = 7.4 Hz, 1H), 8.60 (d, J = 1.6 Hz, 1H), 8.51 (dd, J = 4.6, 1.4 Hz, 1H), 7.83 (d, J = 7.8 Hz, 1H), 7.39 (dd, J = 7.7, 4.8 Hz, 1H), 7.21 (s, 2H), 6.88 (s, 1H), 5.73 (dd, J = 7.4, 4.9 Hz, 1H), 5.18 (s, 2H), 4.95 (d, J = 4.6 Hz, 1H), 3.48-3.45 (m, 1H), 3.43-3.32 (m, 1H), 3.04 (dd, J = 18.1, 7.9 Hz, 1H), 2.85 (t, J = 13.4 Hz, 1H), 2.45 (d, J = 18.1 Hz, 1H).
MS (M+1): 536, retention time: 0.38 min (measurement condition A)
Anal. C20H18N6O8S2(H2O)3.2
Calc. : C, 40.57; H, 4.15; N, 14.19; S, 10.83 (%)
Found: C, 40.58; H, 4.08; N, 14.16; S, 10.91 (%)

### [EXAMPLE 11]

### Synthesis of compound 1-043

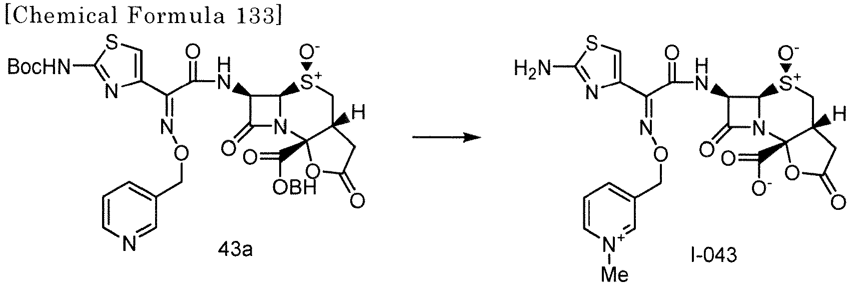

### Step 1

The compound 1-043 was obtained by the similar synthesis of Example 8 using the compound 43a (245 mg, 0.306 mmol) obtained by the similar synthesis of the steps 1 to 4 of Example 8 using pyridine-3-methanol.
1H-NMR (DMSO-D6) δ: 9.09-9.02 (m, 2H), 8.91 (d, J = 6.0 Hz, 1H), 8.54 (d, J = 8.0 Hz, 1H), 8.13 (dd, J = 7.9, 6.1 Hz, 1H), 7.23 (s, 2H), 6.94 (s, 1H), 5.59-5.53 (m, 1H), 5.37 (s, 2H), 4.78 (d, J = 4.8 Hz, 1H), 4.38 (s, 3H), 3.30-3.23 (m, 2H), 2.75-2.61 (m, 2H), 2.15 (d, J = 17.3 Hz, 1H).
MS (M+1): 548.97, retention time: 0.30 min (measurement condition A)
Anal. C21H20N6O8S2(H2O)5.2
Calc. : C, 40.41; H, 4.59; N, 13.46; S, 10.27 (%)
Found: C, 40.33; H, 4.50; N, 13.68; S, 10.22 (%)

### [EXAMPLE 12]

### Synthesis of compound I-044

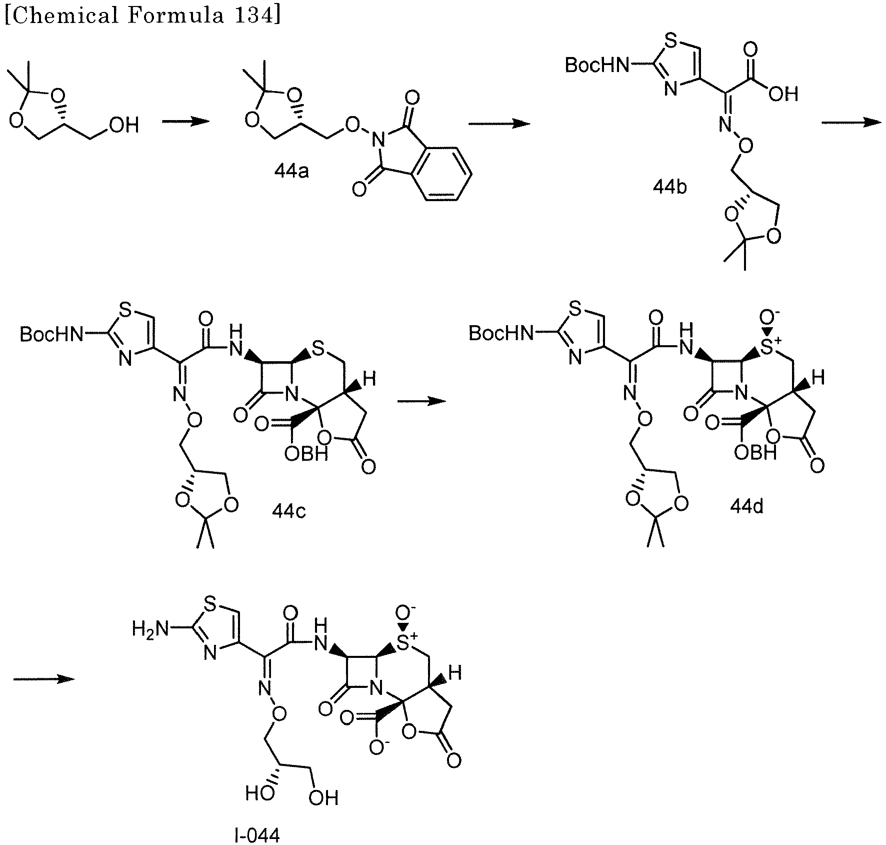

### Step 1 Synthesis of compound 44a

(R)-Isopropylidene glycerol (3.71 mL, 30.0 mmol) was dissolved in tetrahydrofuran (50.0 mL). The solution was cooled to 0°C. Triphenylphosphine (8.66 g, 33.0 mmol), N-hydroxyphthalimide (5.38 g, 33.0 mmol), and a 2.7 mol/L solution of dimethyl azodicarboxylate in toluene (12.22 mL, 33.0 mmol) were added to the solution. The mixture was stirred at room temperature for 30 minutes. The reaction mixture was concentrated under reduced pressure. Methanol was added to the residue. The mixture was stirred. The obtained solid was collected by filtration, washed by methanol, and dried to afford the compound 44a (1.78 g, yield 21%).
1H-NMR (CDCl3) δ: 7.86-7.83 (m, 2H), 7.78-7.75 (m, 2H), 4.53-4.47 (m, 1H), 4.36-4.29 (m, 1H), 4.20-4.12 (m, 2H), 4.02-3.96 (m, 1H), 1.41 (s, 3H), 1.35 (s, 3H).

### Step 2 Synthesis of compound 44b

The compound 44b (1.16 g, yield 45%) was obtained by the similar synthesis of the step 2 of Example 8 using the compound 44a (1.77 g, 6.38 mmol).
1H-NMR (CDC13) δ: 7.34 (s, 1H), 4.50-4.44 (m, 1H), 4.36-4.26 (m, 2H), 4.07 (dd, J = 6.7, 8.5 Hz, 1H), 3.84 (dd, J = 8.5, 5.8 Hz, 1H), 1.55 (s, 9H), 1.43 (s, 3H), 1.35 (s, 3H).

### Step 3 Synthesis of compound 44c

The compound 44c was obtained by the similar synthesis of the step 5 of Example 7 using the compound 44b (0.442 g, 1.10 mmol).
1H-NMR (CDCl3) δ: 7.54 (d, J = 7.8 Hz, 1H), 7.39-7.28 (m, 12H), 6.97 (s, 1H), 5.67 (dd, J = 8.3, 4.7 Hz, 1H), 5.13 (d, J = 4.7 Hz, 1H), 4.46-4.38 (m, 1H), 4.36-4.24 (m, 2H), 4.05 (dd, J = 6.4, 8.8 Hz, 1H), 3.80 (dd, J = 5.4, 8.5 Hz, 1H), 3.20-3.15 (m, 1H), 3.08-3.01 (m, 1H), 2.69 (dd, J = 14.4, 9.8 Hz, 1H), 2.55 (d, J = 5.6 Hz, 2H), 1.54 (s, 9H), 1.40 (s, 3H), 1.32 (s, 3H).

### Step 4 Synthesis of compound 44d

The compound 44d (729 mg, yield 88%) was obtained by the similar synthesis of the step 6 of Example 7 using the compound 44c (1.00 mmol).
1H-NMR (CDCl3) δ: 8.45 (s, 1H), 7.72 (d, J = 9.8 Hz, 1H), 7.35-7.29 (m, 11H), 7.00 (s, 1H), 6.05 (dd, J = 9.8, 4.9 Hz, 1H), 4.66 (d, J = 4.9 Hz, 1H), 4.47-4.41 (m, 1H), 4.38-4.32 (m, 1H), 4.26-4.19 (m, 1H), 4.15-4.05 (m, 1H), 3.83 (dd, J = 8.5, 5.6 Hz, 1H), 3.75-3.68 (m, 1H), 3.41 (dd, J = 14.5, 5.0 Hz, 1H), 2.63 (dd, J = 17.9, 7.7 Hz, 1H), 2.53-2.44 (m, 1H), 2.33 (d, J = 17.2 Hz, 1H), 1.54 (s, 9H), 1.41 (s, 3H), 1.34 (s, 3H).

### Step 5 Synthesis of compound I-044

The compound I-044 (210 mg, yield 78%) was obtained by the similar synthesis of the step 7 of Example 7 using the compound 44d (412 mg, 0.500 mmol).
1H-NMR (D2O) δ: 7.05 (s, 1H), 5.89 (d, J = 4.8 Hz, 1H), 5.02 (d, J = 4.8 Hz, 1H), 4.34 (dd, J = 11.7, 4.1 Hz, 1H), 4.25 (dd, J = 11.7, 6.7 Hz, 1H), 4.13-4.05 (m, 1H), 3.74-3.69 (m, 1H), 3.68-3.60 (m, 2H), 3.58-3.52 (m, 1H), 3.07 (dd, J = 18.3, 7.8 Hz, 1H), 2.84 (dd, J = 12.5, 14.6 Hz, 1H), 2.57 (d, J = 18.3 Hz, 1H).
MS (M+1): 518, retention time: 0.36 min (measurement condition A)
Anal. C17H18N5NaO10S2(H2O)2(H2O)0.9
Calc. : C, 34.51; H, 4.05; N, 11.84; S, 10.84; Na, 3.89 (%)
Found: C, 34.57; H, 4.12; N, 11.91; S, 10.72; Na, 3.98 (%)

### [EXAMPLE 13]

### Synthesis of compound I-045

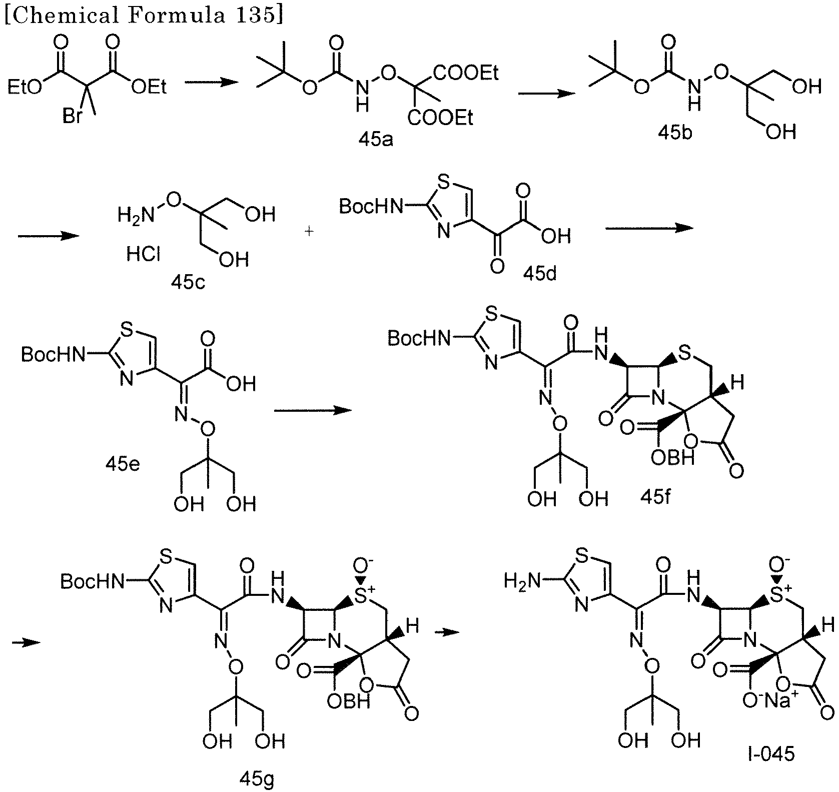

### Step 1 Synthesis of compound 45a

Potassium tert-butoxide (5.05 g, 45.0 mmol) was suspended in DMSO (80.0 mL). A solution of N-Boc-hydroxylamine (5.47 g, 30.0 mmol) in DMSO was added to the suspension. The mixture was stirred at room temperature for 1 hour and 30 minutes. Ice-cooled water was added to the reaction mixture. The compound of interest was extracted from the aqueous layer with ethyl acetate. Collected organic layers were washed by purified water and then brine and dried using anhydrous magnesium sulfate. After filtration, the solvent was evaporated. The obtained residue was subjected to silica gel chromatography to afford the compound 45a (9.20 g, yield 100%).
1H-NMR (CDCl3) δ: 7.71 (br s, 1H), 4.27 (q, J = 6.3 Hz, 4H), 1.72 (s, 3H), 1.45 (dt, J = 25.7, 8.8 Hz, 9H), 1.30 (t, J = 7.2 Hz, 6H).

### Step 2 Synthesis of compound 45b

To tetrahydrofuran (30.0 mL), lithium aluminum hydride (0.759 g, 20.0 mmol) was added. The mixture was cooled to 0°C. The compound 1 (3.05 g, 10.0 mmol) was added to the mixture. The mixture was stirred at 0°C for 1 hour and 30 minutes. Lithium aluminum hydride (0.190 g, 5.00 mmol) was added again to the mixture. The mixture was stirred at 0°C for 30 minutes. Sodium sulfate decahydrate (10.0 g) was added in small portions to the reaction mixture. Anhydrous sodium sulfate was further added to the reaction mixture. The mixture was stirred overnight at room temperature. The solid was removed. The filtrate was concentrated under reduced pressure to afford the compound 45b (1.00 g, yield 45%).
1H-NMR (CDCl3) δ: 6.96 (br s, 1H), 3.70-3.61 (m, 4H), 3.52 (br s, 2H), 1.49 (s, 9H), 1.15 (s, 3H).

### Step 3 Synthesis of compound 45c

The compound 45b (1.00 g, 4.55 mmol) was dissolved in dichloromethane. A 4 mol/L solution of hydrochloric acid in ethyl acetate (17.06 mL, 68.3 mmol) was added to the solution. The mixture was stirred at room temperature for 45 minutes. The supernatant was removed. Ethyl acetate was added again to the residue. The supernatant was removed again. The obtained residue was dried under reduced pressure to afford the compound 45c. The obtained compound 45c was used in the next reaction without being purified.
1H-NMR (D2O) δ: 3.82 (d, J = 12.5 Hz, 2H), 3.72 (d, J = 12.5 Hz, 2H), 1.35 (s, 3H).

### Step 4 Synthesis of compound 45e

The compound 45c (0.717 g, 4.55 mmol) was suspended in dichloromethane (10.0 mL). The compound 45d (0.991 g, 3.64 mmol), triethylamine (0.599 mL, 4.32 mmol), and methanol (23.0 mL) were added to the suspension. The mixture was stirred at room temperature for 1 hour and 30 minutes. Purified water was added to the reaction mixture. The solvent was evaporated. Ethyl acetate, purified water, and a 2 mol/L aqueous solution of hydrochloric acid were added to the residue, followed by extraction with ethyl acetate. The organic layer was washed by purified water and then brine and dried using anhydrous magnesium sulfate. After filtration, the solvent was evaporated to afford the compound 45e (1.31 g, yield 77%). The obtained compound 45e was used in the next reaction without being purified.
1H-NMR (DMSO-D6) δ: 11.76 (s, 1H), 7.41 (s, 1H), 3.57-3.41 (m, 4H), 1.47 (s, 9H), 1.18 (s, 3H).

### Step 5 Synthesis of compound 45f

The compound 45f (918 mg, yield 78%) was obtained by the similar synthesis of the step 3 of Example 8 using the compound 45e (732 mg, 1.95 mmol).
1H-NMR (CDCl3) δ: 8.90 (s, 1H), 8.44 (d, J = 8.7 Hz, 1H), 7.31 (tt, J = 9.7, 4.2 Hz, 11H), 6.96 (s, 1H), 5.66 (dd, J = 8.7, 4.7 Hz, 1H), 5.10 (d, J = 4.7 Hz, 1H), 3.79 (br s, 4H), 3.57 (br s, 1H), 3.41 (br s, 1H), 3.13-3.06 (m, 1H), 3.05-2.97 (m, 1H), 2.63 (dd, J = 9.7, 14.4 Hz, 1H), 2.55-2.48 (m, 2H), 1.54 (s, 9H), 1.24 (s, 3H).

### Step 6 Synthesis of compound 45g

The compound 45g (727 mg, yield 78%) was obtained by the similar synthesis of the step 6 of Example 7 using the compound 56f (917 mg, 1.17 mmol).
1H-NMR (CDCl3) δ: 8.79 (br s, 1H), 8.23 (d, J = 9.1 Hz, 1H), 7.37-7.29 (m, 11H), 7.00 (s, 1H), 5.94 (dd, J = 9.1, 4.8 Hz, 1H), 4.73 (d, J = 4.8 Hz, 1H), 3.85-3.60 (br m, 7H), 3.43 (dd, J = 14.7, 4.8 Hz, 1H), 2.60-2.49 (m, 2H), 2.29 (d, J = 17.4 Hz, 1H), 1.54 (s, 9H), 1.28 (s, 3H).

### Step 7 Synthesis of compound 1-045

The compound 1-045 (241 mg, yield 87%) was obtained by the similar synthesis of the step 7 of Example 7 using the compound 45g (399 mg, 0.500 mmol).
1H-NMR (D2O) δ: 7.03 (s, 1H), 5.91 (d, J = 4.8 Hz, 1H), 5.02 (d, J = 4.5 Hz, 1H), 3.86-3.73 (m, 4H), 3.65 (dd, J = 14.7, 5.2 Hz, 1H), 3.55 (ddd, J = 5.1, 7.8, 12.7 Hz, 1H), 3.07 (dd, J = 18.3, 7.8 Hz, 1H), 2.84 (dd, J = 14.6, 12.5 Hz, 1H), 2.57 (d, J = 18.3 Hz, 1H), 1.34 (s, 3H).
MS (M+1): 518, retention time: 0.36 min (measurement condition A)

### [EXAMPLE 14]

### Synthesis of compound I-046

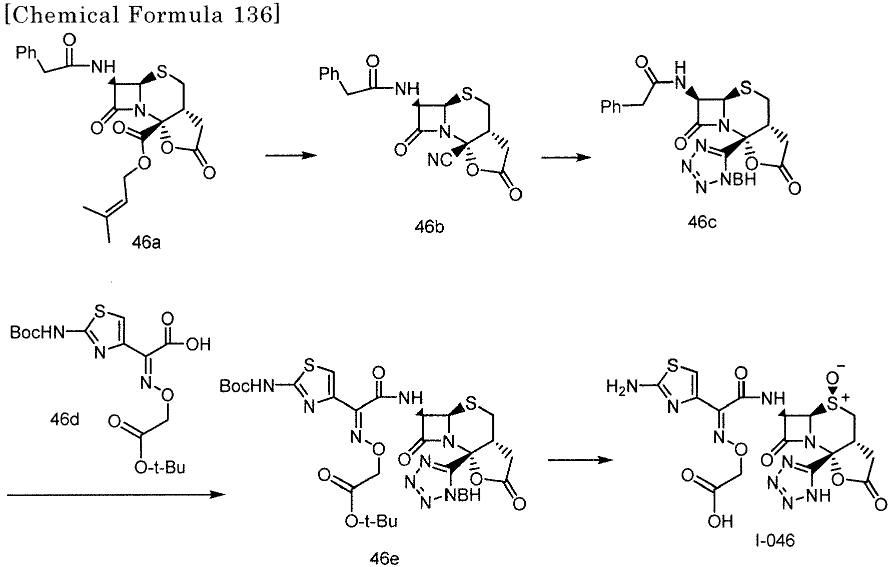

### Step 1 Synthesis of compound 46b

A solution of the compound 46a (2.67 g, 6.00 mmol) in dichloromethane (50 mL) was cooled to -30°C. Anisole (1.97 mL, 18.0 mmol) and a 2 mol/L solution of aluminum chloride in nitromethane (9.00 mL, 18.0 mmol) were added to the solution. The mixture was stirred at -30°C for 1 hour. Dilute hydrochloric acid was added to the reaction mixture, followed by extraction with ethyl acetate. The organic layer was subjected to extraction with an aqueous solution of sodium hydrogen carbonate. The extract was pH-adjusted to 2 by the addition of dilute hydrochloric acid, followed by extraction again with ethyl acetate. The organic layer was washed by brine and then dried over anhydrous magnesium sulfate. The solvent was evaporated under reduced pressure. 1,4-Dioxane (22 mL), ammonium carbonate (0.72 g, 7.50 mmol), di-tert-butyl dicarbonate (1.81 mL, 7.80 mmol), and pyridine (0.242 mL, 3.00 mmol) were added to the obtained residue. The mixture was stirred overnight at room temperature. Water was added to the reaction mixture, followed by extraction with ethyl acetate. The organic layer was washed by dilute hydrochloric acid, an aqueous solution of sodium hydrogen carbonate, and brine. Then, the solvent was evaporated under reduced pressure. Pyridine (1.45 mL, 18.0 mmol) was added to a solution of the obtained residue in tetrahydrofuran (23 mL). The reaction mixture was cooled to -30°C. Then, trifluoroacetic anhydride (1.27 mL, 9.00 mmol) was added to the reaction mixture. The mixture was stirred at -30°C for 1 hour. Water was added to the reaction mixture, followed by extraction with ethyl acetate. The organic layer was washed by dilute hydrochloric acid, an aqueous solution of sodium hydrogen carbonate, and brine and then dried over anhydrous magnesium sulfate. The solvent was evaporated under reduced pressure. The obtained residue was purified by silica gel column chromatography (hexane-ethyl acetate) to afford the compound 46b (900 mg, 42%).
MS (M+1) = 358.13 (measurement condition A)

### Step 2 Synthesis of compound 46c

To a solution of the compound 46b (900 mg) in 1,4-dioxane (9.0 mL), trimethylsilylazide (0.667 mL, 5.04 mmol) and dibutyltin oxide (62.8 mg, 0.25 mmol) were added. The reaction mixture was warmed to 90°C and then stirred for 1.5 hours. Diisopropyl ether was added to the reaction mixture, followed by extraction with an aqueous solution of sodium hydrogen carbonate. The aqueous layer was pH-adjusted to 2 by the addition of dilute hydrochloric acid, followed by extraction with ethyl acetate. The organic layer was washed by brine and then dried over anhydrous magnesium sulfate. The solvent was evaporated under reduced pressure. A solution of the obtained residue in tetrahydrofuran (10 mL) was cooled to 0°C. Diphenyldiazomethane (0.587 g, 3.0 mmol) was added to the solution. The mixture was stirred at room temperature for 2 hours. The solvent was evaporated under reduced pressure. The obtained residue was purified by silica gel chromatography (hexane-ethyl acetate) to afford a crude product containing the compound 46c (1.2 g).
MS (M+1) = 567, (measurement condition A)

### Step 3 Synthesis of compound 46e

A suspension of phosphorus pentachloride (0.656 g, 3.15 mmol) in dichloromethane (23 mL) was cooled to -78°C. Then, pyridine (0.288 mL, 3.57 mmol) was added, and subsequently, the compound 46c (1.2 g, 2.1 mmol) was added to the suspension. Under ice cooling, the mixture was stirred for 1 hour. Then, the reaction mixture was cooled to -78°C. Ethanol (12 mL) was added to the reaction mixture. After stirring at -30°C for 1 hour, an aqueous solution of sodium hydrogen carbonate was added to the reaction mixture, followed by extraction with dichloromethane. The organic layer was dried over anhydrous magnesium sulfate. Then, inorganic matter was removed by filtration. Ethyl acetate was added to the filtrate. Dichloromethane was evaporated under reduced pressure to afford an ethyl acetate solution (solution A). A solution of the compound 46d (885 mg, 2.21 mmol) in DMA (10 mL) was cooled to -10°C. Triethylamine (0.335 mL, 2.4 mmol) and methanesulfonyl chloride (0.180, 2.3 mmol) were added to the solution. The mixture was stirred at -10°C for 1 hour to afford a solution of methanesulfonic acid ester of the compound 46d in dichloromethane (solution B). The preceding solution A was cooled to 0°C. Pyridine (0.254 mL, 3.15 mmol) and the solution B were added to the solution A. The mixture was stirred at 0°C for 1 hour. Water was added to the reaction mixture, followed by extraction with ethyl acetate. The organic layer was washed by dilute hydrochloric acid, an aqueous solution of sodium hydrogen carbonate and brine. Then, the organic layer was dried over anhydrous magnesium sulfate. The solvent was evaporated under reduced pressure. The obtained residue was purified by silica gel column chromatography (hexane-ethyl acetate) to afford the crude product of the compound 46e (900 mg).
[M+1] = 832, (measurement condition A)

### Step 4 Synthesis of compound I-046

A solution of the crude product containing the compound 46e (333 mg) in DMA (6.6 mL)-acetonitrile (3.3 mL) was cooled to 0°C. An aqueous solution of peracetic acid (36%, 0.148 mL, 0.80 mmol) was added to the solution. The mixture was stirred at 0°C for 1 hour. Then, an aqueous solution of sodium hydrogen sulfite was added to the mixture, followed by extraction with ethyl acetate. The organic layer was washed by dilute hydrochloric acid, an aqueous solution of sodium hydrogen carbonate and brine. Then, the organic layer was dried over anhydrous magnesium sulfate. The solvent was evaporated under reduced pressure. A solution of the obtained residue in dichloromethane was cooled to -30°C. Anisole (0.515 mL, 4.71 mmol) and a 2 mol/L solution of aluminum chloride in nitromethane (2.36 mL, 4.71 mmol) were added to the solution. The mixture was stirred at -30°C for 1 hour. Ice, diisopropyl ether, and acetonitrile were added in this order to the reaction mixture. The mixture was stirred to completely dissolve insoluble matter. Then, the aqueous layer was separated. The organic layer was subjected to extraction with water again. Then, all the aqueous layers were combined. HP20-SS resin was added to the mixture. Acetonitrile was evaporated under reduced pressure. The obtained mixed solution was purified by ODS column chromatography (water-acetonitrile). Fractions containing the desired compound were collected, concentrated under reduced pressure, and then freeze-dried to afford the compound I-046 (60 mg) as a white powder.
1H-NMR (D2O) δ: 2.65 (1H, d, J = 18.4 Hz), 2.91 (1H, dd, J = 18.4, 7.6 Hz), 3.07 (1H, dd, J = 14.5, 13.0 Hz), 3.76 (1H, dd, J = 14.5, 5.2 Hz), 3.85-3.91 (1H, m), 4.70 (2H, s), 5.22 (1H, d, J = 4.8 Hz), 5.89 (1H, d, J = 4.8 Hz), 7.12 (1H, s)
C16H15N9O8S2(H2O)3.0 Calc. C : 33.16%, H : 3.65%, N : 21.75%, S : 11.06%, Found C : 33.15%, H : 3.61%, N : 21.69%, S : 11.02%.
[M+1] = 526, (measurement condition A)

### [EXAMPLE 15]

### Synthesis of compound 1-051

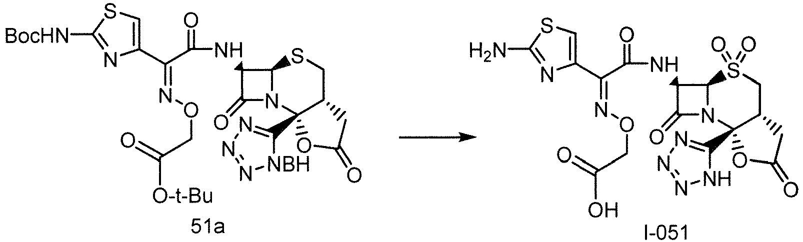

### Step 1 Synthesis of compound I-051

To the crude product containing the compound 51a (333 mg), dichloromethane (6.0 mL) and mCPBA (70%, 370 mg) were added. Under ice cooling, the mixture was stirred for 1 hour. Then, an aqueous solution of sodium hydrogen sulfite was added to the mixture, followed by extraction with ethyl acetate. The organic layer was washed by dilute hydrochloric acid, an aqueous solution of sodium hydrogen carbonate and brine. Then, the organic layer was dried over anhydrous magnesium sulfate. The solvent was evaporated under reduced pressure. A solution of the obtained residue in dichloromethane was cooled to -30°C. Anisole (0.505 mL, 4.63 mmol) and a 2 mol/L solution of aluminum chloride in nitromethane (2.31 mL, 4.63 mmol) were added to the solution. The mixture was stirred at -30°C for 1 hour. Ice, diisopropyl ether, and acetonitrile were added in this order to the reaction mixture. The mixture was stirred to completely dissolve insoluble matter. Then, the aqueous layer was separated. The organic layer was subjected to extraction with water again. Then, all the aqueous layers were combined. HP20-SS resin was added to the mixture. Acetonitrile was evaporated under reduced pressure. The obtained mixed solution was purified by ODS column chromatography (water-acetonitrile). Fractions containing the desired compound were collected, concentrated under reduced pressure and then freeze-dried to afford the compound I-051 (45 mg) as a white powder.
¹H-NMR (D2O) δ: 2.63 (1H, d, J = 18.2 Hz), 2.84 (1H, dd, J = 18.2, 7.7 Hz), 3.77-3.80 (2H, m), 4.19-4.25 (1H, m), 4.54 (2H, s), 5.57 (1H, d, J = 5.1 Hz), 5.94 (1H, d, J = 5.1 Hz), 7.03 (1H, s).
C16H15N9O9S2(H2O)2.7 Calc. C : 32.57%, H : 3.48%, N : 21.36%, S : 10.87%, Found C : 32.63%, H : 3.48%, N : 21.27%, S : 10.73%.
[M+1] = 542, (measurement condition A)

### [EXAMPLE 16]

### Synthesis of compound I-047

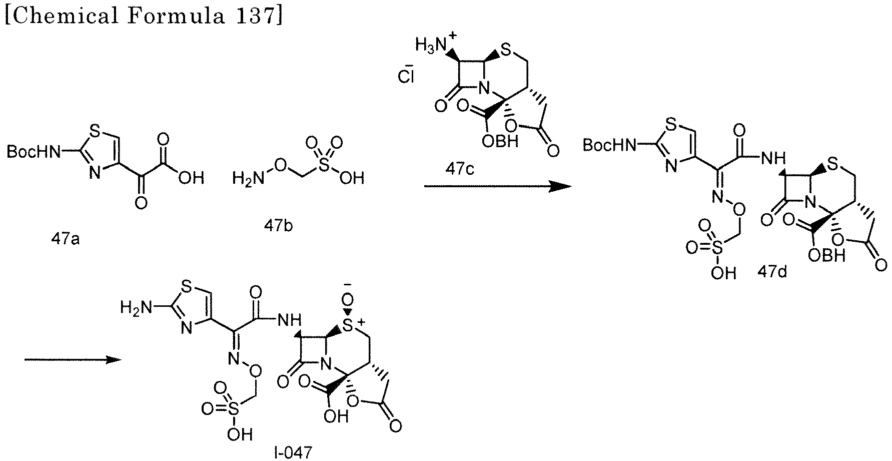

### Step 1 Synthesis of compound 47d

To the compound 47a (1.26 g, 4.63 mmol) and the compound 47b (600 mg, 4.72 mmol), dichloromethane (3.6 mL) and methanol 5 mL) were added. Sodium hydrogen carbonate (397 mg, 4.72 mmol) was added to the mixture. The mixture was stirred at room temperature for 1 hour. Then, the reaction mixture was concentrated. The compound 47c (1.38 g, 3 mmol) and ethyl acetate (13.8 mL) were added to the obtained residue. The mixture was cooled to -20°C. Then, phenyl dichlorophosphate (0.670 mL, 4.5 mmol) and N-methylmorpholine (1.98 mL, 18.0 mmol) were added to the mixture. The mixture was stirred for 30 minutes. Acetonitrile (10 m) was added to the mixture. The mixture was stirred at room temperature for 30 minutes. Then, water was added to the reaction mixture, followed by extraction with ethyl acetate. The organic layer was washed by dilute hydrochloric acid and brine and then dried over anhydrous magnesium sulfate. The solvent was evaporated under reduced pressure to afford a crude product containing the compound 47d (2.4 g).
MS (M+1) = 788.02 (measurement condition A)

### Step 2 Synthesis of compound 1-047

To the crude product containing the compound 47d (1.2 g), dichloromethane (6.0 mL) was added. The mixture was cooled to -40°C. mCPBA (70%, 370 mg) was added to the mixture. The mixture was stirred at -40°C for 1 hour. Then, an aqueous solution of sodium hydrogen sulfite was added to the mixture, followed by extraction with ethyl acetate. The organic layer was washed by dilute hydrochloric acid, an aqueous solution of sodium hydrogen carbonate and brine. Then, the organic layer was dried over anhydrous magnesium sulfate. The solvent was evaporated under reduced pressure. A solution of the obtained residue in dichloromethane was cooled to -30°C. Anisole (1.64 mL, 15.0 mmol) and a 2 mol/L solution of aluminum chloride in nitromethane (7.50 mL, 15.0 mmol) were added to the solution. The mixture was stirred at -30°C for 1 hour. Ice, diisopropyl ether, and acetonitrile were added in this order to the reaction mixture. The mixture was stirred to completely dissolve insoluble matter. Then, the aqueous layer was separated. The organic layer was subjected to extraction with water again. Then, all the aqueous layers were combined. HP20-SS resin was added to the mixture. Acetonitrile was evaporated under reduced pressure. The obtained mixed solution was purified by ODS column chromatography (water-acetonitrile). Fractions containing the desired compound were collected, concentrated under reduced pressure and then freeze-dried to afford the compound 1-047 (15 mg) as a white powder. ¹H-NMR (D₂O) δ: 2.56 (1H, d, J = 18.2 Hz), 2.84-2.77 (1H, m), 3.06 (1H, dd, J = 19.1, 7.5 Hz), 3.59 (2H, t, J = 19.1 Hz), 5.00 (1H, d, J = 4.5 Hz), 5.06 (2H, s), 5.89 (1H, d, J = 4.5 Hz), 7.13 (1H, s).
[M+1] = 538, (measurement condition A)

### [EXAMPLE 17]

### Synthesis of compound I-048

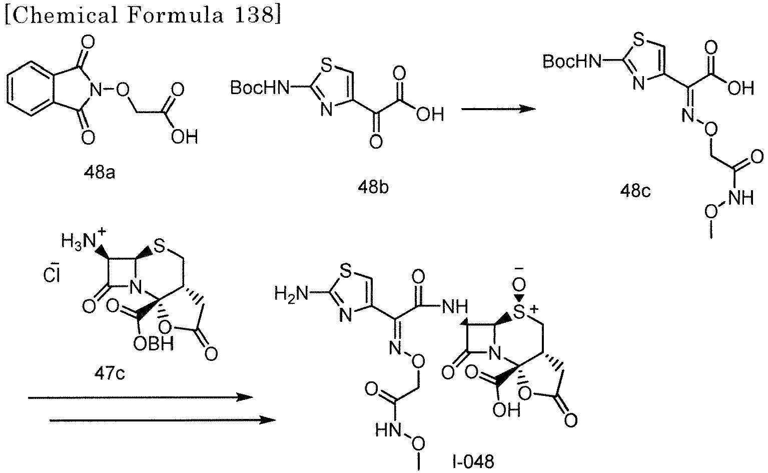

### Step 1 Synthesis of compound 48c

To the compound 48a (2.65 g, 12.0 mmol), dichloromethane (26.5 mL) was added. Oxalyl chloride (1.26 mL, 14.4 mmol) was added to the mixture. DMF (0.0093 mL, 0.12 mmol) was added to the mixture. The mixture was stirred at room temperature for 1 hour. Half the amount of this dichloromethane solution was used as solution A. Dichloromethane (1.4 mL) and pyridine (1.06 mL, 13.2 mmol) were added to O-methylhydroxyamine hydrochloride (551 mg, 6.00 mmol). The mixture was cooled to 0°C. The solution A was added to the mixture. The mixture was stirred at 0°C for 1 hour. Dilute hydrochloric acid and methanol were added to the reaction mixture. After stirring, the solid was collected by filtration. Dichloromethane (6 mL), acetonitrile (10 mL) and methylhydrazine (0.127 mL, 2.40 mmol) were added to 600 mg of the obtained solid. The mixture was stirred at room temperature for 1 hour. Insoluble matter was removed. The obtained filtrate was cooled to 0°C. The compound 48b (653 mg, 2.40 mmol) and methanol (5 mL) were added to the filtrate. The mixture was stirred at 0°C for 30 minutes. Dilute hydrochloric acid was added to the reaction mixture, followed by extraction with ethyl acetate. The organic layer was washed by brine and then dried over anhydrous magnesium sulfate. The solvent was evaporated under reduced pressure to afford a crude product containing the compound 48c (860 mg).
MS (M+1) = 376, (measurement condition A)

### Step 2 Synthesis of compound 1-048

The compound I-048 (46 mg) was obtained by the similar synthesis of the steps 1 and 2 of Example 16 using the crude product containing the compound 48c (374 mg) and the compound 47c (461 mg, 1.0 mmol).
¹H-NMR (D₂O) δ: 2.56 (1H, d, J = 18.4 Hz), 2.83 (1H, dd, J = 14.3, 13.0 Hz), 3.06 (1H, dd, J = 18.3, 7.7 Hz), 3.51-3.57 (1H, m), 3.63 (1H, dd, J = 14.8, 5.2 Hz), 3.76 (3H, s), 4.81-4.86 (2H, m), 4.82-4.84 (2H, m), 5.01 (1H, d, J = 4.5 Hz), 5.87 (1H, d, J = 4.5 Hz), 7.24 (1H, s).
C17H18N6O10S2(H2O)3.5 Calc. C : 34.40%, H : 4.25%, N : 14.16%, S : 10.80%, Found C : 34.35%, H 4.21%, N : 14.28%, S : 10.73%.
MS (M+1) = 531, (measurement condition A)

### [EXAMPLE 18]

### Synthesis of compound I-049

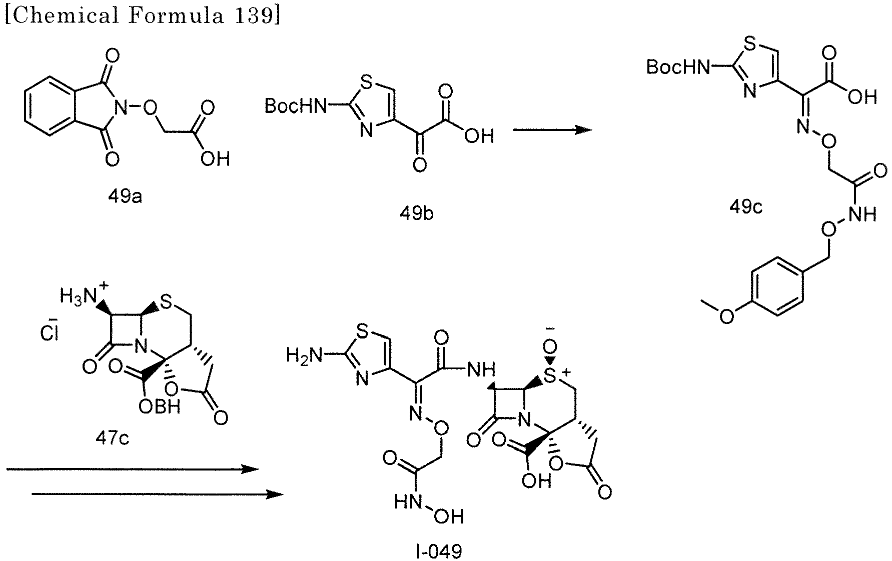

### Step 1 Synthesis of compound 49c

A crude product containing the compound 49c (1.9 g) was obtained by the similar synthesis of the step 1 of Example 17 using the compound 49a (1.35 g, 6 mmol), the compound 49b (1.07 g, 3.93 mmol) and O-(4-methoxybenzyl)hydroxyamine (1.10 g, 7.2 mmol).
MS (M+1) = 481, (measurement condition B)

### Step 2 Synthesis of compound I-049

The compound I-049 (46 mg) was obtained by the similar synthesis of the steps 1 and 2 of Example 16 using the crude product containing the compound 49c (374 mg) and the compound 47c (461 mg, 1.0 mmol).
¹H-NMR (D₂O) δ: 2.55 (1H, d, J = 18.4 Hz), 2.82 (1H, dd, J = 14.4, 12.9 Hz), 3.06 (1H, dd, J = 18.3, 7.7 Hz), 3.51-3.57 (1H, m), 3.63 (1H, dd, J = 14.8, 5.2 Hz), 4.80 (3H, d, J = 4.3 Hz), 5.00 (1H, d, J = 4.8 Hz), 5.86 (1H, d, J = 4.8 Hz), 7.19 (1H, s). C16H16N6O10S2(H2O)3.1 Calc. C : 33.58%, H : 3.91%, N : 14.68%, S : 11.20%, Found C : 33.71%, H 4.05%, N : 14.67%, S : 10.98%.
MS (M+1) = 517, (measurement condition A)

### [EXAMPLE 19]

### Synthesis of compound I-050

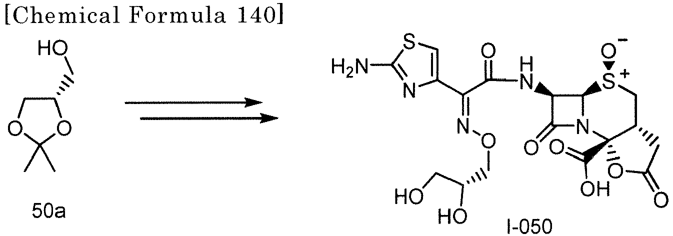

### Step 1 Synthesis of compound I-050

The compound I-050 (160 mg) was obtained by the similar synthesis of the steps 1 to 5 of Example 12 using the compound 50a.
¹H-NMR (D₂O) δ: 2.55 (1H, d, J = 18.3 Hz), 2.82 (1H, dd, J = 14.4, 12.9 Hz), 3.06 (1H, dd, J = 18.3, 8.0 Hz), 3.50-3.57 (1H, m), 3.60-3.72 (4H, m), 4.06-4.11 (1H, m), 4.30 (1H, dd, J = 11.9, 5.9 Hz), 4.37 (1H, dd, J = 11.6, 5.8 Hz), 5.00 (1H, d, J = 4.8 Hz), 5.87 (1H, d, J = 4.8 Hz), 7.15 (1H, s).
C17H19N5O10S2(H2O)2.1 Calc. C : 36.77%, H : 4.21%, N : 12.61%, S : 11.55%. Found C : 36.94%, H : 4.32%, N : 12.56%, S : 11.30%.
[M+1] = 518.01 (measurement condition A)

### [EXAMPLE 20]

### Synthesis of compound I-015

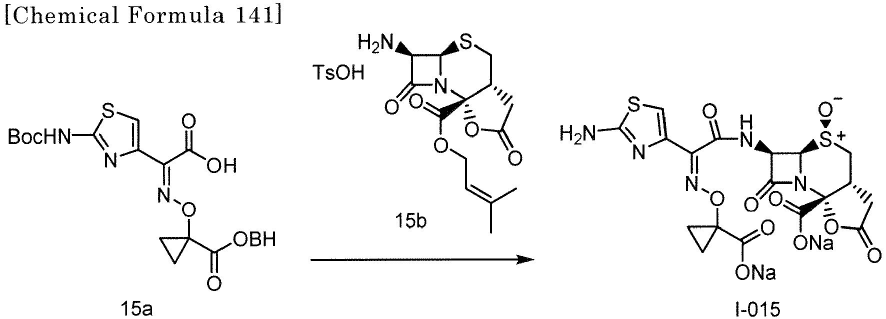

### Step 1 Synthesis of compound I-015

An eluate of a free form of the compound I-015 was obtained by the similar approach of the step 2 of Example 17 using the compound 15a (538 mg, 1.00 mmol) synthesized according to the synthesis of the compound II-2 of WO2010050468, and the compound 15b (836 mg, 1.68 mmol). An aqueous solution of 2 equivalents of sodium hydroxide was added to the eluate. The mixture was concentrated under reduced pressure and freeze-dried to afford the compound I-015 (156 mg).
1H-NMR (D2O) δ: 1.26-1.42 (4H, m), 2.57 (1H, d, J = 18.4 Hz), 2.79-2.85 (1H, m), 3.07 (1H, dd, J = 18.4, 7.6 Hz), 3.66-3.52 (2H, m), 5.00 (1H, d, J = 4.8 Hz), 5.86 (1H, d, J = 4.8 Hz), 7.08 (1H, s).
[M+1] = 528, (measurement condition A)
C18H15N5Na2O10S2(H2O)5.1 Calc. : C : 32.59% H : 3.83% N : 10.56% Na : 6.93% S : 9.67% Found: C : 32.49% H : 3.80% N : 10.74% Na : 7.08% S : 9.58%

### [EXAMPLE 21]

### Synthesis of compound I-052

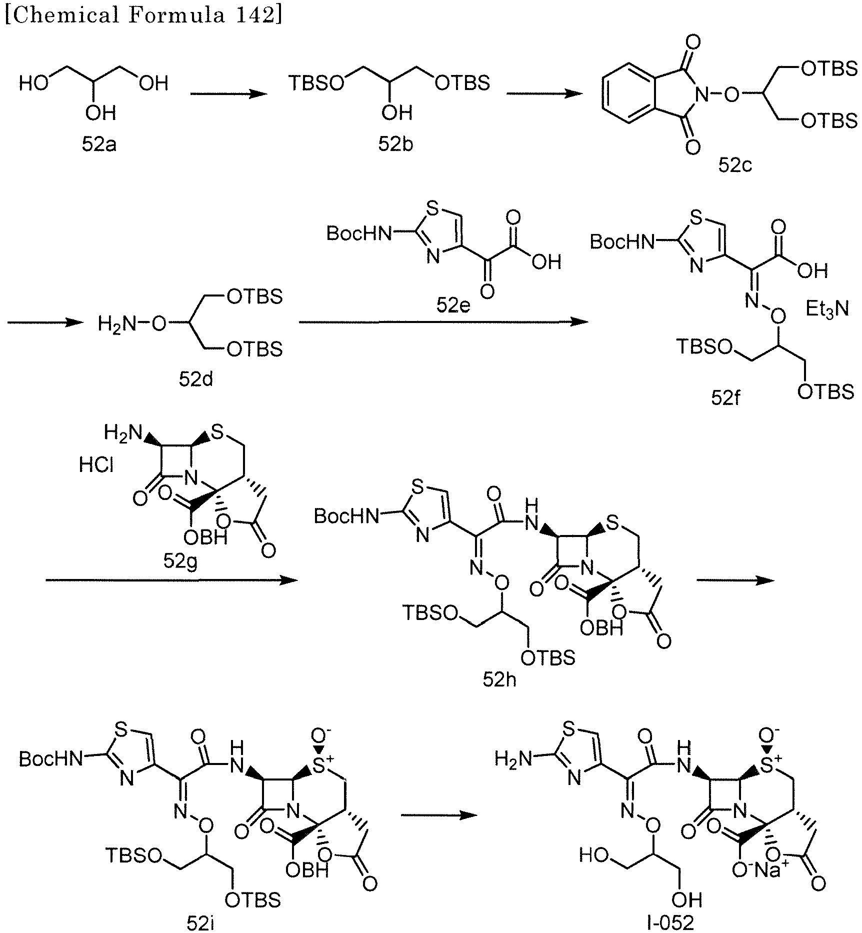

### Step 1 Synthesis of compound 52b

To a solution of glycerin (921 mg, 10 mmol) in tetrahydrofuran (9 mL), imidazole (1.70 g, 25 mmol) was added, and then, a solution of tert-butyldimethylsilyl chloride (3.17 g, 21 mmol) in tetrahydrofuran (18 mL) was added dropwise under ice cooling. The mixture was stirred overnight at room temperature. Then, water was added to the mixture, followed by extraction with ethyl acetate. The organic layer was washed by water and brine in this order and dried over anhydrous magnesium sulfate. After filtration, the filtrate was concentrated under reduced pressure. The obtained crude product was purified by silica gel column chromatography (hexane-ethyl acetate) to afford the compound 52b (3.13 g, yield 98%) as a colorless oil. ¹H-NMR (CDCl₃) δ: 0.07 (12H, s), 0.90 (18H, s), 2.45 (1H, d, J = 5.1 Hz), 3.63 (1H, s), 3.64 (4H, s).

### Step 2 Synthesis of compound 52c

To a solution of the compound 52b (3.13 g, 9.8 mmol) in tetrahydrofuran (31 mL), N-hydroxyphthalimide (1.91 g, 11.7 mmol) and triphenylphosphine (3.07 g, 11.7 mmol) were added, and then, a 1.9 mol/L solution of DIAD in toluene was added dropwise under ice cooling. The mixture was stirred at room temperature for 1 hour and then concentrated under reduced pressure. Methanol was added to the residue. The resulting solid was collected by filtration and dried under reduced pressure to afford the compound 52c (3.07 g, yield 67%) as a white solid.
¹H-NMR (CDCl₃) δ: 0.00 (6H, s), 0.02 (6H, s), 0.82 (18H, s), 3.94 (4H, ddd, J = 14.5, 9.5, 3.2 Hz), 4.33-4.38 (1H, m), 7.72 (2H, dd, J = 5.5, 3.1 Hz), 7.82 (2H, dd, J = 5.5, 3.1 Hz).

### Steps 3 and 4 Synthesis of compound 52f

To a solution of the compound 52c (3.07 g, 6.6 mmol) in dichloromethane (30 mL), methylhydrazine (0.383 mL, 7.2 mmol) was added at -30°C. Under ice cooling, the mixture was stirred for 30 minutes. Then, the resulting insoluble matter was removed by filtration. The compound 52e (1.79 g, 6.6 mmol) was added to the filtrate. The mixture was stirred at room temperature for 2 hours and then concentrated under reduced pressure. Dilute hydrochloric acid was added to the residue, followed by extraction with ethyl acetate. The organic layer was washed by water and brine in this order and dried over anhydrous magnesium sulfate. After filtration, the filtrate was concentrated under reduced pressure. The residue was dissolved in diisopropyl ether. Then, triethylamine (1.1 mL, 7.9 mmol) was added to the solution. The resulting solid was collected by filtration and dried under reduced pressure to afford the compound 52f (1.79 g, yield 39%) as a white solid.
¹H-NMR (DMSO-D₆) δ: 0.04 (6H, s), 0.04 (6H, s), 0.86 (18H, s), 1.13 (9H, t, J = 6.5 Hz), 1.47 (9H, s), 2.96 (6H, s), 3.70 (4H, d, J = 4.4 Hz), 3.96 (1H, t, J = 5.0 Hz), 7.10 (1H, s).

### Step 5 Synthesis of compound 52h

To a suspension of the compound 52f (608 mg, 0.88 mmol) in ethyl acetate (7.4 mL), the compound 52g (369 mg, 0.80 mmol) was added, and then, phenyl dichlorophosphate (0.179 mL, 1.2 mmol) and N-methylmorpholine (0.308 mL, 2.8 mmol) were added dropwise in this order at -40°C. The mixture was stirred at -40°C for 1 hour. Then, dilute hydrochloric acid was added to the mixture, followed by extraction with ethyl acetate. The organic layer was washed by water, an aqueous solution of sodium hydrogen carbonate, and brine in this order and dried over anhydrous magnesium sulfate. After filtration, the filtrate was concentrated under reduced pressure. The obtained crude product was purified by silica gel column chromatography (hexane-ethyl acetate) to afford the compound 52h (522 mg, yield 66%) as a white foam.
¹H-NMR (CDCl₃) δ: 0.04 (6H, d, J = 2.0 Hz), 0.05 (6H, d, J = 1.9 Hz), 0.86 (9H, s), 0.87 (9H, s), 1.54 (9H, s), 2.54-2.61 (2H, m), 2.67 (1H, dd, J = 14.5, 9.7 Hz), 3.04 (1H, dd, J = 14.5, 4.5 Hz), 3.17 (1H, dq, J = 12.8, 3.8 Hz), 3.82-3.91 (4H, m), 4.42-4.48 (1H, m), 5.10 (1H, d, J = 4.7 Hz), 5.59 (1H, dd, J = 8.0, 4.7 Hz), 6.97 (1H, s), 7.30-7.38 (11H, m), 7.55 (1H, d, J = 8.0 Hz), 8.09 (1H, s).

### Step 6 Synthesis of compound 52i

To a solution of the compound 52h (522 mg, 0.52 mmol) in dichloromethane (2.6 mL), 69% solution of mCPBA (157 mg, 0.63 mmol) in dichloromethane (2.6 mL) was added dropwise at -40°C. The mixture was stirred at -40°C for 40 minutes. Then, 15% aqueous solution of sodium thiosulfate was added to the mixture, followed by extraction with ethyl acetate. The organic layer was washed by an aqueous solution of sodium hydrogen carbonate and brine in this order and dried over anhydrous magnesium sulfate. After filtration, the filtrate was concentrated under reduced pressure. The obtained crude product was purified by silica gel column chromatography (hexane-ethyl acetate) to afford the compound 52i (436 mg, yield 82%) as a white foam.
¹H-NMR (CDCl₃) δ: 0.04 (6H, s), 0.05 (6H, s), 0.87 (9H, s), 0.88 (9H, s), 1.54 (9H, s), 2.32 (1H, d, J = 17.9 Hz), 2.45 (1H, t, J = 13.6 Hz), 2.64 (1H, dd, J = 17.9, 7.7 Hz), 3.41 (1H, dd, J = 14.4, 4.9 Hz), 3.70-3.90 (5H, m), 4.38-4.43 (1H, m), 4.61 (1H, d, J = 5.0 Hz), 6.07 (1H, dd, J = 10.0, 5.0 Hz), 7.00 (1H, s), 7.25-7.38 (11H, m), 7.66 (1H, d, J = 10.0 Hz), 8.22 (1H, s).

### Step 7 Synthesis of compound 1-052

A solution of the compound 52i (436 mg, 0.43 mmol) in dichloromethane (4.4 mL) was cooled to -40°C. Then, anisole (0.470 mL, 4.3 mmol) and a 2 mol/L solution of aluminum chloride in nitromethane (2.15 mL, 4.3 mmol) were added in this order to the solution. The mixture was stirred at -30°C for 30 minutes. Diisopropyl ether, ice water, and acetonitrile were added to the reaction mixture. The mixture was stirred to completely dissolve insoluble matter. Then, the aqueous layer was separated. The organic layer was subjected to extraction with water again. Then, all the aqueous layers were combined. HP20-SS resin was added to the mixture. Acetonitrile was evaporated under reduced pressure. 2 mol/L hydrochloric acid (1.0 mL) was added to the residue. The obtained mixed solution was purified by OSD column chromatography (water-acetonitrile). Fractions containing the desired compound were collected. A 0.2 mol/L aqueous solution of sodium hydroxide was added to the fractions until the pH became 6. Then, a small amount of dry ice was added to the mixture. The obtained solution was concentrated under reduced pressure and then freeze-dried to afford the compound I-052 (182 mg, yield 78%) as a white powder.
¹H-NMR (D₂O) δ: 2.57 (1H, d, J = 18.3 Hz), 2.84 (1H, dd, J = 14.8, 12.5 Hz), 3.07 (1H, dd, J = 18.3, 7.8 Hz), 3.52-3.59 (1H, m), 3.65 (1H, dd, J = 14.8, 5.3 Hz), 3.86 (4H, t, J = 5.3 Hz), 4.41-4.46 (1H, m), 5.03 (1H, d, J = 4.6 Hz), 5.90 (1H, d, J = 4.9 Hz), 7.05 (1H, s).
MS (m+1) = 518, retention time: 0.30 min, (measurement condition A)
Anal. : C17H18N5NaO10S2(H2O)3.0
Calc. : C, 34.40; H, 4.08; N, 11.80; Na, 3.87; S, 10.80 (%)
Found: C, 34.50; H, 4.20; N, 11.92; Na, 3.84; S, 10.96 (%)

### [EXAMPLE 22]

### Synthesis of compound I-053

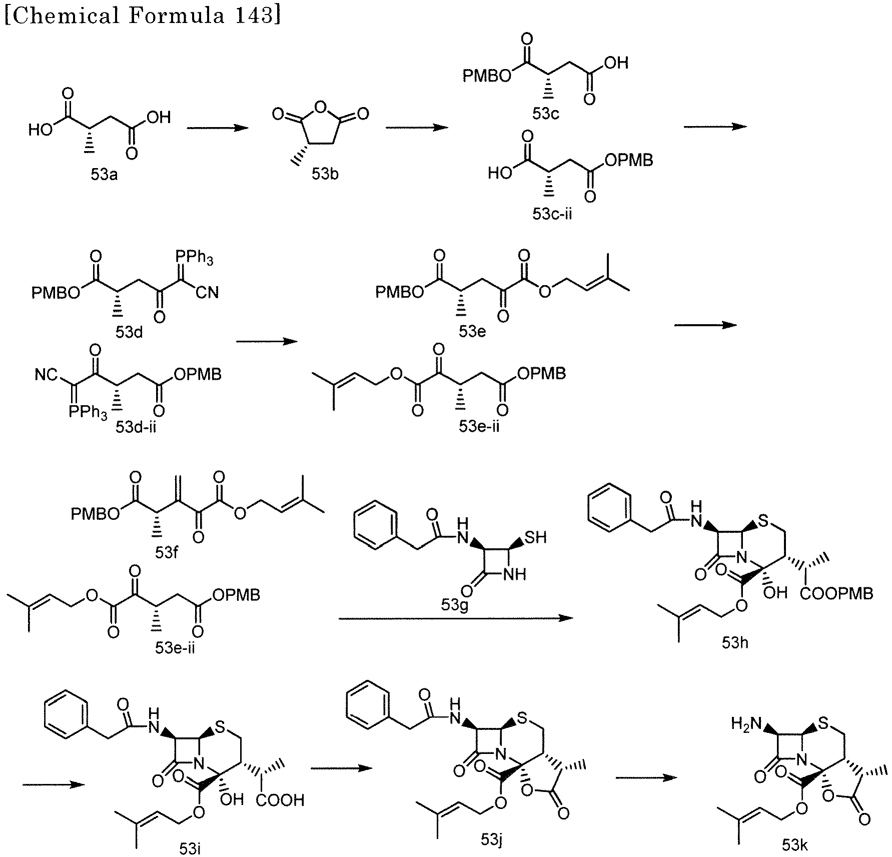

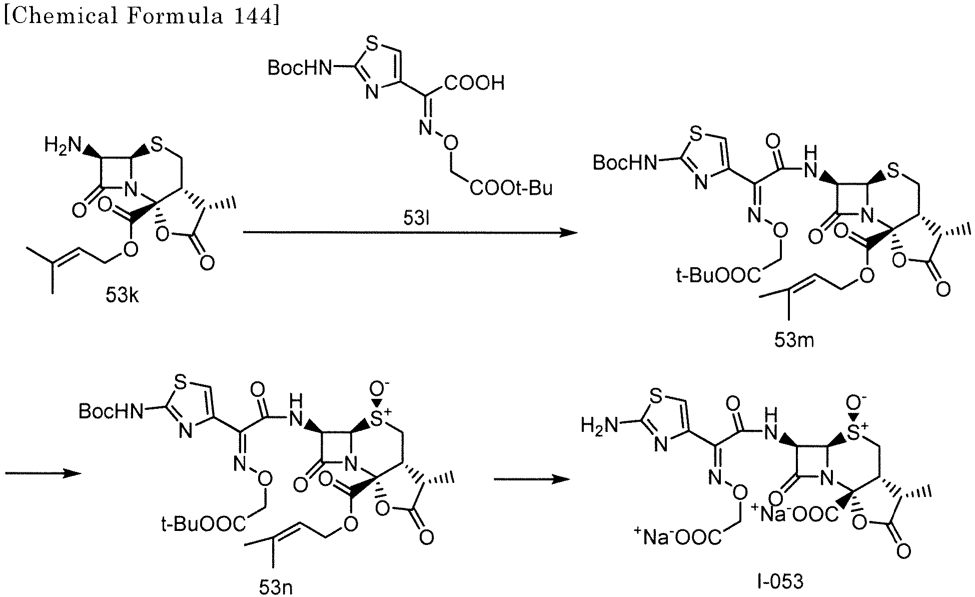

### Steps 1 and 2 Synthesis of compound 53c

To the compound 53a (13.8 g, 104 mmol), trifluoroacetic anhydride (17.7 mL, 125 mmol) was added under ice cooling. Under ice cooling, the mixture was stirred for 3 hours and then concentrated under reduced pressure. Toluene was added to the residue. The mixture was concentrated again under reduced pressure to afford the compound 53b. The obtained compound 53b was used directly in the next reaction without being purified.

To a solution of the whole amount of the obtained compound 53b in tetrahydrofuran (64 mL), p-methoxybenzyl alcohol (23.2 g, 168 mmol) and DMAP (1.37 g, 11.2 mmol) were added. The mixture was heated to reflux for 6 hours. The reaction mixture was concentrated under reduced pressure. Then, an aqueous solution of sodium hydrogen carbonate and ethyl acetate were added to the residue to separate an aqueous layer. 2 mol/L hydrochloric acid was added to the separated aqueous layer until the pH became 2, followed by extraction with ethyl acetate. The organic layer was washed by water and brine and dried over anhydrous magnesium sulfate. After filtration, the filtrate was concentrated under reduced pressure to afford a mixture of the compounds 53c and 53c-ii (21 g, yield 74%) as a colorless oil. ¹R-NMR (DMSO-D₆) δ: 1.09-1.12 (6H, m), 2.31-2.46 (2H, m), 2.53-2.63 (2H, m), 2.67-2.80 (2H, m), 3.75 (6H, s), 5.01 (4H, s), 6.92 (4H, d, J = 8.6 Hz), 7.29 (4H, d, J = 8.3 Hz).

### Step 3 Synthesis of compound 53d

To a solution of the mixture of the compounds 53c and 53c-ii (21 g, 83 mmol) in dichloromethane (300 mL), triphenylphosphoranylidene acetonitrile (26.3 g, 87 mmol), 4-dimethylaminopyridine (1.02 g, 8.3 mmol), and EDC hydrochloride (17.5 g, 92 mmol) were added in this order. The mixture was stirred at room temperature for 1.5 hours and then concentrated under reduced pressure. Water was added to the residue, followed by extraction with ethyl acetate. The organic layer was washed by water and brine in this order and dried over anhydrous magnesium sulfate. After filtration, the filtrate was concentrated under reduced pressure. The obtained crude product was purified by silica gel column chromatography (hexane-ethyl acetate) to afford a mixture of the compounds 53d and 53d-ii (25.88 g, yield 58%) as a white foam.
¹H-NMR (CDCl₃) δ: 1.20 (3H, d, J = 6.8 Hz), 1.25 (3H, d, J = 7.1 Hz), 2.30 (1H, dd, J = 16.5, 4.7 Hz), 2.77-2.85 (2H, m), 3.02 (1H, dd, J = 13.9, 7.1 Hz), 3.22 (1H, dd, J = 16.0, 7.7 Hz), 3.63-3.68 (1H, m), 3.78 (6H, s), 4.98-5.06 (4H, m), 6.84 (4H, d, J = 8.1 Hz), 7.27 (4H, d, J = 14.9 Hz), 7.49-7.61 (30H, m).

### Step 4 Synthesis of compound 53e

A solution of the mixture of the compounds 53d and 53d-ii (25.9 g, 48 mmol) in dichloromethane (520 mL) was stirred for 1 hour under bubbling with ozone gas at - 78°C. After purging of the system with nitrogen gas, dimethyl sulfide (10.7 mL, 145 mmol) was added to the solution. The mixture was stirred at -78°C for 5 minutes. Subsequently, 3-methyl-2-buten-1-ol (7.36 mL, 72.5 mmol) was added to the mixture. The mixture was stirred at -78°C for 2 hours. The reaction mixture was warmed to approximately 0°C. Then, 5% aqueous solution of sodium carbonate was added to the reaction mixture. The mixture was stirred at room temperature for 5 minutes. Dichloromethane was evaporated under reduced pressure, followed by extraction with ethyl acetate. The organic layer was washed by water and brine in this order and dried over anhydrous magnesium sulfate. After filtration, the filtrate was concentrated under reduced pressure. The obtained crude product was purified by silica gel column chromatography (hexane-ethyl acetate) to afford a mixture of the compounds 53e and 53e-ii (6.11 g, yield 36%) as a white foam.
¹H-NMR (CDCl₃) δ: 1.19 (3H, d, J = 7.1 Hz), 1.23 (3H, d, J = 7.1 Hz), 1.74 (6H, s), 1.77 (6H, s), 2.49 (1H, dd, J = 16.9, 5.3 Hz), 2.82-2.91 (2H, m), 3.02 (1H, dd, J = 13.4, 7.1 Hz), 3.31 (1H, dd, J = 18.6, 7.7 Hz), 3.63-3.70 (1H, m), 3.81 (6H, s), 4.73-4.76 (4H, m), 5.04 (4H, d, J = 8.8 Hz), 5.39 (2H, s), 6.88 (4H, d, J = 8.1 Hz), 7.26 (4H, br s).

### Steps 5 and 6 Synthesis of compound 53h

To a solution of the mixture of the compounds 53e and 53e-ii (6.11 g, 17.5 mmol) in dichloromethane (30 mL), N,N,N'N'-tetramethylmethanediamine (7.17 mL, 52.6 mmol) was added under ice cooling, and then, acetic anhydride (6.30 mL, 66.6 mmol) and acetic acid (5.32 mL, 93 mmol) were added dropwise in this order. The mixture was stirred overnight at room temperature. Then, ice water was added to the reaction mixture, followed by extraction with ethyl acetate. The organic layer was washed by water and brine in this order, dried over anhydrous magnesium sulfate, and filtered to afford a solution of a mixture of the compounds 53f and 53e-ii in ethyl acetate.

To the obtained solution of the mixture of the compounds 53f and 53e-ii in ethyl acetate, the compound 53g (2.49 g, 10.5 mmol) and hexamethylphosphoric triamide (9.16 mL, 52.6 mmol) were added. The mixture was stirred overnight at room temperature. Then, water was added to the reaction mixture, followed by extraction with ethyl acetate. The organic layer was washed by water and brine in this order and dried over anhydrous magnesium sulfate. After filtration, the filtrate was concentrated under reduced pressure. The obtained crude product was purified by silica gel column chromatography (hexane-ethyl acetate) to afford the compound 53h (2.64 g, yield 42%) as a white foam.
¹H-NMR (CDCl₃) δ: 1.13 (3H, d, J = 6.1 Hz), 1.70 (3H, s), 1.76 (3H, s), 2.50-2.54 (2H, m), 2.68 (1H, d, J = 14.7 Hz), 3.12 (1H, dd, J = 14.0, 11.5 Hz), 3.61 (2H, dd, J = 25.0, 15.9 Hz), 3.81 (3H, s), 4.04 (1H, s), 4.69 (1H, dd, J = 11.9, 7.6 Hz), 4.89 (1H, dd, J = 11.9, 7.6 Hz), 5.02 (2H, s), 5.07 (1H, d, J = 4.3 Hz), 5.37 (1H, t, J = 6.8 Hz), 5.46 (1H, dd, J = 9.1, 4.5 Hz), 6.09 (1H, d, J = 9.3 Hz), 6.88 (2H, d, J = 8.3 Hz), 7.24-7.39 (7H, m).

### Steps 7 and 8 Synthesis of compound 53j

To a solution of the compound 53h (2.64 g, 4.42 mmol) in dichloromethane (20 mL), trifluoroacetic acid (10.2 mL, 133 mmol) was added dropwise at -20°C. The mixture was stirred at -20°C for 1 hour. Then, the reaction mixture was added to an ice-cooled mixed solution of an aqueous solution of sodium hydrogen carbonate and dichloromethane. Subsequently, dilute hydrochloric acid was added to the mixture until the pH became 2, followed by extraction with dichloromethane. The organic layer was washed by brine and dried over anhydrous magnesium sulfate. After filtration, the filtrate was concentrated under reduced pressure to afford the compound 53i. The obtained compound 53i was used directly in the next reaction without being purified.

To a solution of the whole amount of the obtained compound 53i in dichloromethane (26 mL), EDC hydrochloride (933 mg, 4.87 mmol) was added under ice cooling. The mixture was stirred at room temperature for 1 hour. Then, water was added to the reaction mixture, followed by extraction with ethyl acetate. The organic layer was washed by water and brine in this order and dried over anhydrous magnesium sulfate. After filtration, the filtrate was concentrated under reduced pressure. The obtained crude product was purified by silica gel column chromatography (hexane-ethyl acetate) to afford the compound 53j (1.94 g, yield 96%) as a white foam.
¹H-NMR (CDCl₃) δ: 1.20 (3H, d, J = 7.3 Hz), 1.71 (3H, s), 1.76 (3H, s), 2.53-2.88 (4H, m), 3.64 (2H, dd, J = 25.4, 16.0 Hz), 4.72-4.82 (2H, m), 4.99 (1H, d, J = 4.8 Hz), 5.36 (1H, t, J = 6.7 Hz), 5.63 (1H, dd, J = 8.8, 4.8 Hz), 6.06 (1H, d, J = 8.8 Hz), 7.27-7.39 (5H, m).

### Steps 9 and 10 Synthesis of compound 53m

A solution of the compound 531 (963 mg, 2.4 mmol) in dimethylacetamide (4.6 mL) was cooled to -20°C. Then, triethylamine (0.416 mL, 3.0 mmol) and methanesulfonyl chloride (0.218 mL, 2.8 mmol) were added to the solution. The mixture was stirred at -20°C for 30 minutes to afford solution A.

A suspension of phosphorus pentachloride (833 mg, 4.0 mmol) in dichloromethane (4.6 mL) was cooled to -78°C. Then, pyridine (0.355 mL, 4.4 mmol) was added, and subsequently, a solution of the compound 53j (917 mg, 2.0 mmol) in dichloromethane (4.6 mL) was added dropwise to the suspension. The mixture was stirred at -10°C for 1 hour. Then, the reaction mixture was cooled to -78°C. Cooled methanol (4.6 mL) was added to the reaction mixture. After stirring at -30°C for 2 hours, an aqueous solution of sodium hydrogen carbonate was added to the reaction mixture, followed by extraction with dichloromethane. The organic layer was dried over anhydrous magnesium sulfate and then filtered. Ethyl acetate was added to the filtrate. Dichloromethane and methanol were evaporated under reduced pressure to afford a solution of the compound 53k in ethyl acetate. Under ice cooling, pyridine (0.194 mL, 2.4 mmol), and the solution A obtained above were added to the obtained solution of the compound 53k in ethyl acetate. Under ice cooling, the mixture was stirred for 30 minutes. Then, dilute hydrochloric acid was added to the mixture, followed by extraction with ethyl acetate. The organic layer was washed by water and brine in this order and dried over anhydrous magnesium sulfate. After filtration, the filtrate was concentrated under reduced pressure. The obtained crude product was purified by silica gel column chromatography (hexane-ethyl acetate) to afford the compound 53m (1.33 g, yield 92%) as a white foam.
¹H-NMR (CDCl₃) δ: 1.24 (3H, d, J = 7.1 Hz), 1.48 (9H, s), 1.54 (9H, s), 1.72 (3H, s), 1.76 (3H, s), 2.57-2.95 (4H, m), 3.70-3.85 (2H, m), 4.75 (2H, dd, J = 28.0, 16.7 Hz), 5.11 (1H, d, J = 4.7 Hz), 5.38 (1H, t, J = 7.3 Hz), 5.74 (1H, dd, J = 8.5, 4.7 Hz), 7.36 (1H, s), 8.07 (1H, s), 8.69 (1H, d, J = 8.5 Hz).

### Step 11 Synthesis of compound 53n

To a solution of the compound 53m (650 mg, 0.90 mmol) in a mixed solvent of acetonitrile (3 mL) and dimethylacetamide (3 mL), 37% peracetic acid (0.258 mL, 1.44 mmol) was added dropwise at -20°C. The mixture was stirred at -20°C for 1 hour. Then, an aqueous solution of sodium hydrogen sulfite was added to the reaction mixture, followed by extraction with ethyl acetate. The organic layer was washed by water, an aqueous solution of sodium hydrogen carbonate, and brine in this order and dried over anhydrous magnesium sulfate. After filtration, the filtrate was concentrated under reduced pressure. The obtained crude product was purified by silica gel column chromatography (hexane-ethyl acetate) to afford the compound 53n (397 mg, yield 60%) as a white foam.
¹H-NMR (CDCl₃) δ: 1.22 (3H, d, J = 7.3 Hz), 1.46 (9H, s), 1.58 (9H, s), 1.73 (3H, s), 1.77 (3H, s), 2.21-2.29 (1H, m), 3.00-3.07 (1H, m), 3.39 (1H, dd, J = 14.4, 4.8 Hz), 3.70-3.79 (3H, m), 4.59 (1H, d, J = 4.9 Hz), 4.72 (2H, dd, J = 26.7, 16.5 Hz), 5.39 (1H, t, J = 7.3 Hz), 5.95 (1H, dd, J = 9.4, 4.9 Hz), 7.34 (1H, s), 8.10 (1H, s), 8.41 (1H, d, J = 9.4 Hz).

### Step 12 Synthesis of compound I-053

The compound I-053 (139 mg, yield 46%) was obtained by the similar synthesis of the step 7 of Example 21 using the compound 53n (397 mg, 0.54 mmol).
¹H-NMR (D₂O) δ: 1.18 (3H, d, J = 7.1 Hz), 2.72 (1H, t, J = 15.2 Hz), 3.22 (1H, t, J = 7.1 Hz), 3.54-3.62 (2H, m), 4.59 (2H, s), 4.98 (1H, d, J = 4.8 Hz), 5.89 (1H, d, J = 4.8 Hz), 7.06 (1H, s).
MS (m+1) = 516, retention time: 0.49 min, (measurement condition A)
Anal. : C17H15N5Na2O10S2(H2O)3.8
Calc. : C, 32.52; H, 3.63; N, 11.15; Na, 7.32; S, 10.21 (%)
Found: C, 32.52; H, 3.57; N, 11.20; Na, 7.27; S, 10.15 (%)

### [EXAMPLE 23]

### Synthesis of compound I-054

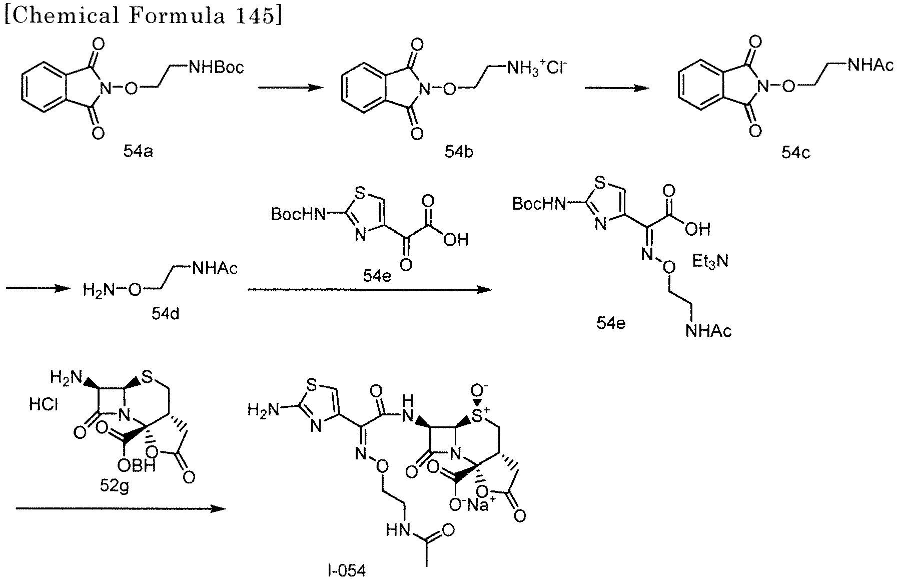

### Step 1 Synthesis of compound 54b

To a solution of the compound 54a (9.19 g, 30 mmol) in ethyl acetate (75 mL), a 4 mol/L solution of hydrochloric acid in ethyl acetate was added. The mixture was stirred at room temperature for 2 hours. Then, the resulting solid was collected by filtration and dried under reduced pressure to afford the compound 54b (7.20 g, yield 99%) as a white solid.
¹H-NMR (DMSO-D₆) δ: 3.18 (2H, s), 4.36 (2H, t, J = 5.3 Hz), 7.87-7.92 (4H, m), 8.20 (3H, s).

### Step 2 Synthesis of compound 54c

To a suspension of the compound 54b (1.21 g, 5.0 mmol) in dichloromethane (12 mL), acetyl chloride (0.428 mL, 6.0 mmol) and pyridine (1.01 mL, 12.5 mmol) were added under ice cooling. Under ice cooling, the mixture was stirred for 1 hour. Then, dilute hydrochloric acid was added to the mixture, followed by extraction with ethyl acetate. The organic layer was washed by water, an aqueous solution of sodium hydrogen carbonate, and brine in this order and dried over anhydrous magnesium sulfate. After filtration, the filtrate was concentrated under reduced pressure. Diisopropyl ether was added to the residue. The resulting solid was collected by filtration and dried under reduced pressure to afford the compound 54c (680 mg, yield 55%) as a white solid.
¹H-NMR (CDCl₃) δ: 2.09 (3H, s), 3.57 (2H, dd, J = 9.8, 5.6 Hz), 4.27 (2H, t, J = 4.8 Hz), 6.82 (1H, s), 7.79 (2H, dd, J = 5.6, 3.1 Hz), 7.87 (2H, dd, J = 5.6, 3.1 Hz).

### Step 3 Synthesis of compound 54e

The compound 54e (877 mg, yield 71%) was obtained from the compound 54c (680 mg, 2.74 mmol) by the similar synthesis of the steps 3 and 4 of Example 21.
¹H-NMR (DMSO-D₆) δ: 1.16 (9H, t, J = 7.3 Hz), 1.47 (9H, s), 1.79 (3H, s), 3.02 (6H, d, J = 5.6 Hz), 3.23 (2H, q, J = 5.8 Hz), 3.90 (2H, t, J = 5.8 Hz), 7.14 (1H, s), 8.38 (1H, s).

### Step 4 Synthesis of compound I-054

The compound 1-054 (156 mg) was obtained by the similar synthesis of the steps 5 to 7 of Example 21 using the compound 52g (369 mg, 0.80 mmol) and the compound 54e (417 mg, 0.88 mmol).
¹H-NMR (D₂O) δ: 2.00 (3H, s), 2.57 (1H, d, J = 18.3 Hz), 2.84 (1H, dd, J = 14.7, 12.5 Hz), 3.07 (1H, dd, J = 18.3, 7.8 Hz), 3.53-3.59 (3H, m), 3.64 (1H, dd, J = 14.7, 5.3 Hz), 4.28-4.37 (2H, m), 5.01 (1H, d, J = 4.8 Hz), 5.87 (1H, d, J = 4.8 Hz), 7.05 (1H, s).
MS (m+1) = 529, retention time: 0.45 min, (measurement condition A)

### [EXAMPLE 24]

### Synthesis of compound I-055

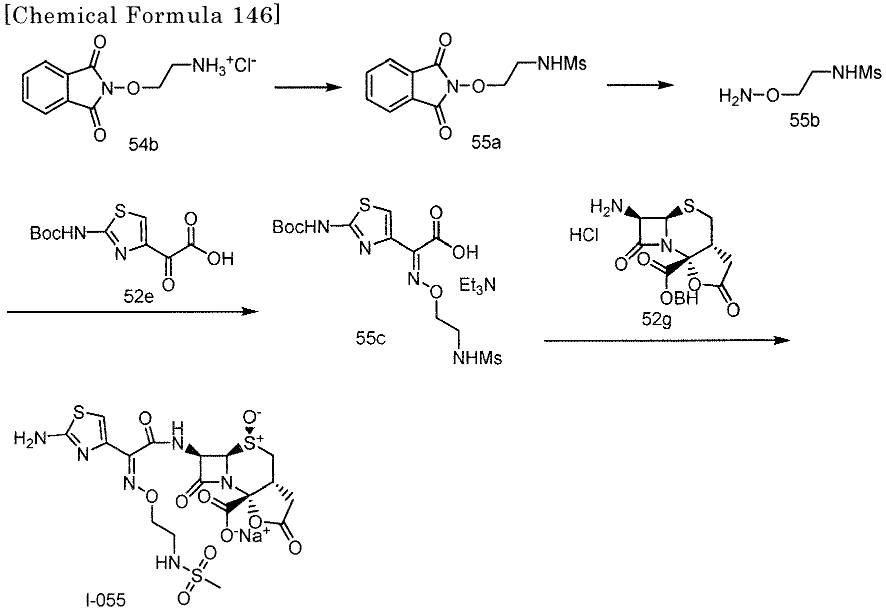

### Step 1 Synthesis of compound 55a

To a suspension of the compound 54b (1.21 g, 5.0 mmol) in dichloromethane (12 mL), methanesulfonyl chloride (0.468 mL, 6.0 mmol) and triethylamine (1.73 mL, 12.5 mmol) were added under ice cooling. Under ice cooling, the mixture was stirred for 1 hour. Then, dilute hydrochloric acid was added to the mixture, followed by extraction with ethyl acetate. The organic layer was washed by water, an aqueous solution of sodium hydrogen carbonate, and brine in this order and dried over anhydrous magnesium sulfate. After filtration, the filtrate was concentrated under reduced pressure. Isopropyl acetate was added to the residue. The resulting solid was collected by filtration and dried under reduced pressure to afford the compound 55a (1.15 g, yield 81%) as a white solid.
¹H-NMR (CDCl₃) δ: 3.05 (3H, s), 3.43-3.47 (2H, m), 4.36 (2H, t, J = 4.8 Hz), 5.67 (1H, s), 7.79-7.82 (2H, m), 7.85-7.88 (2H, m).

### Steps 2 and 3 Synthesis of compound 55c

The compound 55c (740 mg, yield 38%) was obtained by the similar synthesis of the steps 3 and 4 of Example 21 using the compound 55a (1.15 g, 4.05 mmol). ¹H-NMR (DMSO-D₆) δ: 1.16 (9H, t, J = 7.2 Hz), 1.47 (9H, s), 2.87 (3H, s), 3.03 (6H, s), 3.12 (2H, q, J = 5.6 Hz), 4.00 (2H, t, J = 5.6 Hz), 7.16 (1H, s), 7.54 (1H, t, J = 5.3 Hz). Step 3 Synthesis of compound I-055

The compound 1-055 (137 mg) was obtained by the similar synthesis of the steps 5 to 7 of Example 21 using the compound 52g (369 mg, 0.80 mmol) and the compound 55c (448 mg, 0.88 mmol).
¹H-NMR (D₂O) δ: 2.57 (1H, d, J = 18.3 Hz), 2.83 (1H, dd, J = 14.7, 12.5 Hz), 3.04-3.11 (4H, m), 3.49-3.59 (3H, m), 3.64 (1H, dd, J = 14.7, 5.2 Hz), 4.32-4.42 (2H, m), 5.02 (1H, d, J = 4.8 Hz), 5.88 (1H, d, J = 4.8 Hz), 7.06 (1H, s).
MS (m+1) = 565, retention time: 0.49 min, (measurement condition A)

### [EXAMPLE 25]

### Synthesis of compound I-056

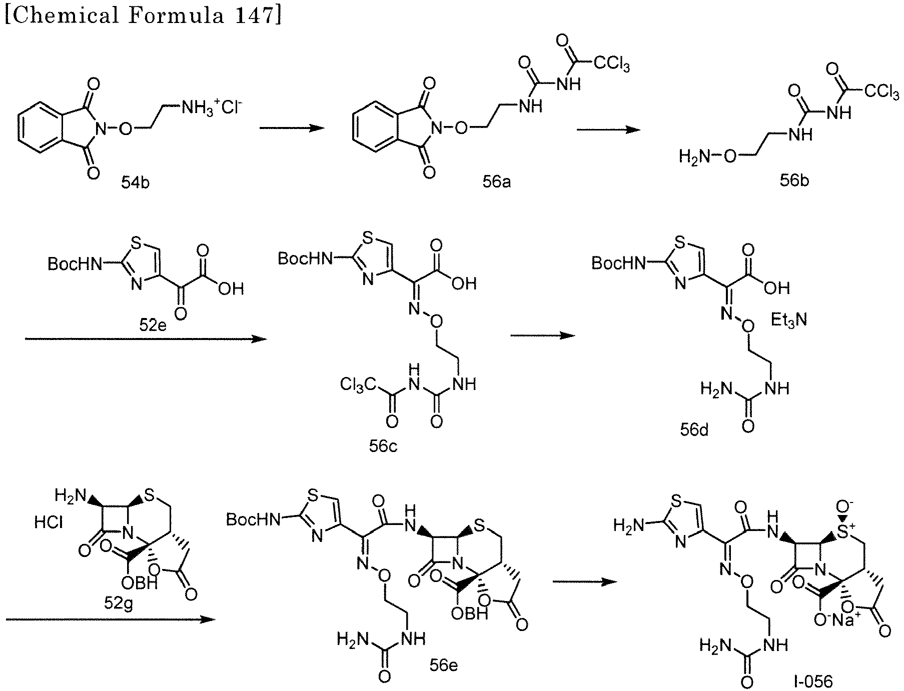

### Step 1 Synthesis of compound 56a

To a suspension of the compound 54b (2.43 g, 10 mmol) in dichloromethane (24 mL), trichloroacetyl isocyanate (1.42 mL, 12 mmol) and triethylamine (1.66 mL, 12 mmol) were added under ice cooling. Under ice cooling, the mixture was stirred for 1 hour. Then, dilute hydrochloric acid was added to the mixture, followed by extraction with ethyl acetate. The organic layer was washed by water, an aqueous solution of sodium hydrogen carbonate, and brine in this order and dried over anhydrous magnesium sulfate. After filtration, the filtrate was concentrated under reduced pressure. Isopropyl acetate was added to the residue. The resulting solid was collected by filtration and dried under reduced pressure to afford the compound 56a (2.74 g, yield 69%) as a white solid.
¹H-NMR (CDCl₃) δ: 3.69 (2H, dd, J = 10.0, 5.6 Hz), 4.33 (2H, t, J = 4.9 Hz), 7.77-7.80 (2H, m), 7.85-7.89 (2H, m), 8.41 (1H, s), 8.73 (1H, s).

### Steps 2, 3, and 4 Synthesis of compound 56d

To a solution of the compound 56a (2.74 g, 6.9 mmol) in dichloromethane (55 mL), methylhydrazine (0.385 mL, 7.3 mmol) was added at -30°C. Under ice cooling, the mixture was stirred for 30 minutes. Then, the resulting insoluble matter was removed by filtration. The compound 52e (1.80 g, 6.6 mmol) was added to the filtrate. The mixture was stirred at room temperature for 2 hours and then concentrated under reduced pressure. Dilute hydrochloric acid was added to the residue, followed by extraction with ethyl acetate. The organic layer was washed by water and brine in this order and dried over anhydrous magnesium sulfate. After filtration, the filtrate was concentrated under reduced pressure to afford the compound 56c as a yellow foam. The obtained compound 56c was used directly in the next reaction without being purified.

To a solution of the whole amount of the obtained compound 56c in methanol (34 mL), a 1 mol/L aqueous solution of sodium hydroxide (16.5 mL, 16.5 mmol) was added. The mixture was stirred at room temperature for 3 hours. Then, a 2 mol/L hydrochloric acid (10 mL) was added to the mixture, followed by extraction with ethyl acetate. The organic layer was washed by brine and dried over anhydrous magnesium sulfate. After filtration, the filtrate was concentrated under reduced pressure. The residue was dissolved in an ethyl acetate-methanol mixed solvent. Then, triethylamine (1.1 mL, 7.9 mmol) was added to the solution. The resulting solid was collected by filtration and dried under reduced pressure to afford the compound 56d (1.36 g, yield 43%) as a white solid.
¹H-NMR (DMSO-D₆) δ: 1.14 (9H, t, J = 7.2 Hz), 1.47 (9H, s), 2.97 (6H, s), 3.16 (2H, d, J = 5.3 Hz), 3.88 (2H, t, J = 5.8 Hz), 5.44 (2H, s), 6.31 (1H, s), 7.11 (1H, s).

### Step 5 Synthesis of compound 56e

A suspension of the compound 56d (418 mg, 0.88 mmol) in dimethylacetamide (3.7 mL) was cooled to -20°C. Then, triethylamine (0.022 mL, 0.16 mmol) and methanesulfonyl chloride (0.075 mL, 0.96 mmol) were added to the suspension. The mixture was stirred at -20°C for 30 minutes to afford solution B.

To a suspension of the compound 52g (369 mg, 0.80 mmol) in ethyl acetate (3.7 mL), pyridine (0.155 mL, 1.92 mmol) and the solution B obtained above were added under ice cooling. Under ice cooling, the mixture was stirred for 30 minutes. Then, dilute hydrochloric acid was added to the mixture, followed by extraction with ethyl acetate. The organic layer was washed by water and brine in this order and dried over anhydrous magnesium sulfate. After filtration, the filtrate was concentrated under reduced pressure. The obtained crude product was purified by silica gel column chromatography (hexane-ethyl acetate) to afford the compound 56e (529 mg, yield 84%) as a white foam.
¹H-NMR (CDCl₃) δ: 1.52 (9H, s), 2.46-2.66 (4H, m), 3.10-3.23 (4H, m), 3.46 (1H, br s), 4.18 (1H, s), 4.86 (2H, s), 5.09 (1H, d, J = 4.6 Hz), 5.48 (1H, s), 5.63 (1H, dd, J = 8.2, 4.6 Hz), 6.93 (1H, s), 7.22-7.36 (11H, m), 8.65 (1H, s).

### Step 6 Synthesis of compound I-056

The compound 1-056 (167 mg) was obtained by the similar synthesis of the steps 6 and 7 of Example 21 using the compound 56e (529 mg, 0.68 mmol).
¹H-NMR (D₂O) δ: 2.57 (1H, d, J = 18.3 Hz), 2.83 (1H, dd, J = 14.6, 12.5 Hz), 3.07 (1H, dd, J = 18.3, 7.7 Hz), 3.47 (2H, t, J = 5.0 Hz), 3.53-3.59 (1H, m), 3.64 (1H, dd, J = 14.6, 5.2 Hz), 4.25-4.35 (2H, m), 5.02 (1H, d, J = 4.8 Hz), 5.88 (1H, d, J = 4.8 Hz), 7.05 (1H, s).
MS (m+1) = 530, retention time: 0.40 min, (measurement condition A)

### [EXAMPLE 26]

### Synthesis of compounds I-057 and I-058

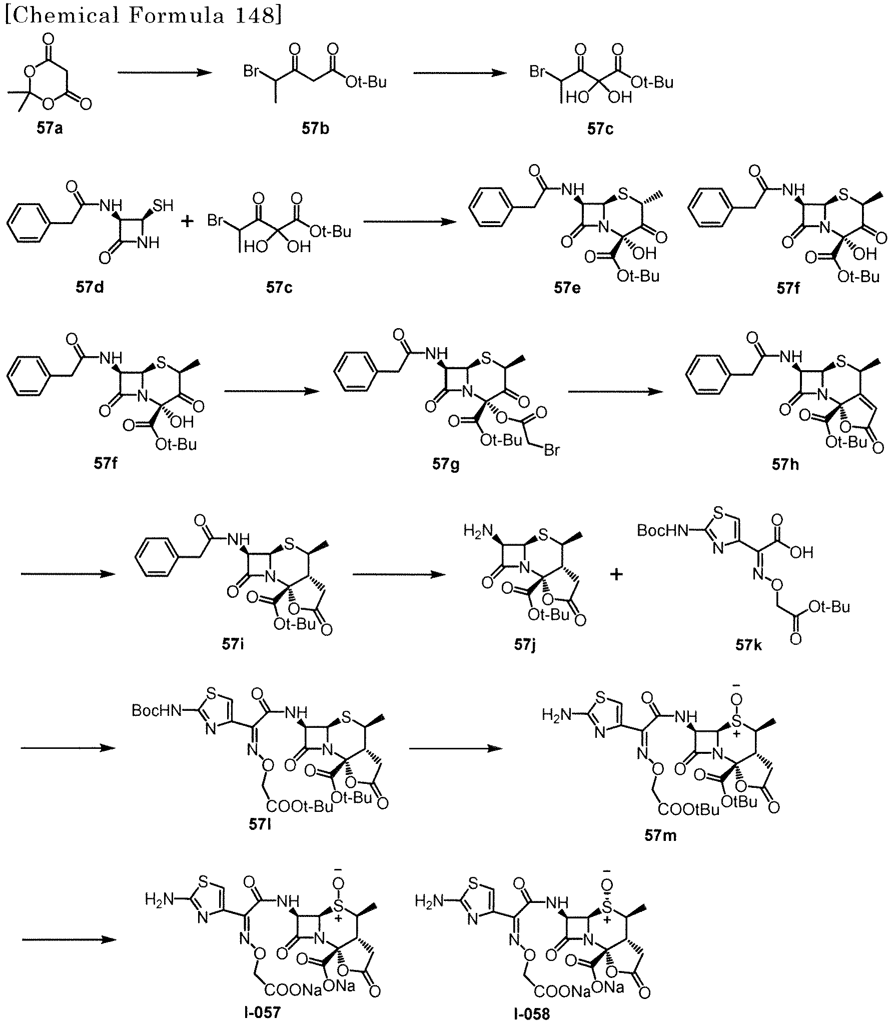

### Step 1 Synthesis of compound 57b

A solution of the Meldrum's acid 57a (30.0 g, 208 mmol) in dichloromethane (300 mL) was cooled in ice. Then, pyridine (33.6 mL, 416 mmol) was added, and subsequently, 2-bromopropionyl bromide (24.0 mL, 229 mmol) was added to the solution. Under ice cooling, the mixture was stirred for 30 minutes. Then, 1 mol/L hydrochloric acid was added to the mixture, followed by extraction with dichloromethane. The organic layer was dried over anhydrous magnesium sulfate. Magnesium sulfate was filtered off. Then, the filtrate was concentrated under reduced pressure. The obtained brown oil was dissolved in t-butyl alcohol (225 mL). The solution was stirred at 80°C for 45 minutes. Then, the solvent was evaporated under reduced pressure. The obtained residue was purified by silica gel column chromatography (hexane-ethyl acetate) to afford the compound 57b (41.5 g, 80%). The compound 57b was obtained as a mixture of keto:enol = 4.5:1.
keto: 1H-NMR (CDCl₃) δ: 1.47 (9H, s), 1.77 (3H, d, J = 6.8 Hz), 3.54 (1H, d, J = 15.9 Hz), 3.77 (1H, d, J = 15.9 Hz), 4.62 (1H, q, J = 6.8 Hz).
enol: 1H-NMR (CDCl₃) δ: 1.50 (9H, s), 1.83 (3H, d, J = 6.8 Hz), 4.43 (1H, q, J = 6.8 Hz), 5.16 (1H, s), 12.23 (1H, s).

### Step 2 Synthesis of compound 57c

To a solution of the compound 57b (41.5 g, 165 mmol) in dioxane (215 mL), selenium(IV) oxide (40.4 g, 364 mmol) was added. The mixture was stirred at 80°C for 1.5 hours. The reaction mixture was cooled to room temperature. Then, insoluble matter was filtered off through Celite. The filtrate was washed by ethyl acetate. The solvent was evaporated under reduced pressure. Then, the obtained residue was purified by silica gel column chromatography (hexane-ethyl acetate) to afford the compound 57c (21.3 g, 46%).
1H-NMR (CDCl₃) δ: 1.52 (9H, s), 1.82 (3H, d, J = 6.8 Hz), 4.77 (1H, s), 4.86 (1H, q, J = 6.8 Hz), 4.99 (1H, s).

### Step 3 Synthesis of compounds 57e and 57f

The compound 57d (3.19 g, 13.5 mmol) and the compound 57c (3.82 g, 13.5 mmol) were dissolved in acetone (38 mL). Hexamethylphosphoric triamide (15.7 mL, 90.0 mmol) was added to the solution. The mixture was stirred at room temperature for 15 minutes. The reaction mixture was cooled in ice. Then, triethylamine (2.06 mL, 14.8 mmol) was added to the reaction mixture. The mixture was stirred at 0°C for 30 minutes. A saturated aqueous solution of ammonium chloride was added to the reaction mixture, followed by extraction with ethyl acetate. The organic layer was dried over anhydrous magnesium sulfate. Magnesium sulfate was filtered off. Then, the filtrate was concentrated under reduced pressure. The obtained residue was purified by silica gel column chromatography (hexane-ethyl acetate) to afford the compounds 57e (2.18 g, 38%) and 57f (483 mg, 8.5%).
Compound 57e: 1H-NMR (CDCl₃) δ: 1.26 (3H, d, J = 6.3 Hz), 1.49 (9H, s), 3.74 (2H, s), 4.15 (1H, q, J = 6.3 Hz), 4.42 (1H, s), 4.99-5.08 (2H, m), 5.99 (1H, d, J = 7.1 Hz), 7.31-7.43 (5H, m).
Compound 57f: 1H-NMR (CDCl₃) δ: 1.36 (3H, d, J = 6.8 Hz), 1.47 (9H, s), 3.64 (1H, d, J = 16.2 Hz), 3.68 (1H, d, J = 16.2 Hz), 4.04 (1H, q, J = 6.8 Hz), 4.70 (1H, s), 5.48 (1H, dd, J = 8.6, 4.5 Hz), 5.52 (1H, d, J = 4.5 Hz), 5.88 (1H, d, J = 8.6 Hz), 7.24-7.41 (5H, m).

### Step 4 Synthesis of compound 57g

A solution of the compound 57f (505.4 mg, 1.20 mmol) in dichloromethane (5 mL) was cooled in ice. Then, bromoacetyl bromide (0.250 mL, 2.88 mmol) was added, and subsequently, triethylamine (0.400 mL, 2.88 mmol) was added to the solution. Under ice cooling, the mixture was stirred for 1 hour. Then, a saturated aqueous solution of ammonium chloride was added to the mixture, followed by extraction with ethyl acetate. The organic layer was dried over anhydrous magnesium sulfate. Magnesium sulfate was filtered off. Then, the filtrate was concentrated under reduced pressure. The obtained residue was purified by silica gel column chromatography (hexane-ethyl acetate) to afford the compound 57g (386 mg, 59%).
1H-NMR (CDCl₃) δ: 1.34 (9H, s), 1.47 (3H, d, J = 7.1 Hz), 3.67 (1H, d, J = 16.4 Hz), 3.69 (1H, d, J = 16.4 Hz), 3.95 (2H, s), 4.18 (1H, q, J = 7.1 Hz), 5.52 (1H, d, J = 4.8 Hz), 5.68 (1H, dd, J = 8.8, 4.8 Hz), 5.90 (1H, d, J = 8.8 Hz), 7.25-7.42 (5H, m).
[M+Na] = 563, retention time: 2.47 min, (measurement condition B)

### Step 5 Synthesis of compound 57h

To a solution of the compound 57g (386 mg, 0.713 mmol) in DMF (4 mL), triphenylphosphine (224 mg, 0.856 mmol) was added. The mixture was stirred at room temperature for 1 hour. Then, 8.4% aqueous solution of sodium hydrogen carbonate (1.78 mL, 1.78 mmol) was added to the mixture. The mixture was stirred at room temperature for 20 minutes. Then, water and ethyl acetate were added to the reaction mixture, followed by extraction with ethyl acetate. The organic layer was dried over anhydrous magnesium sulfate. Magnesium sulfate was filtered off. Then, the filtrate was concentrated under reduced pressure. The obtained residue was purified by silica gel column chromatography (hexane-ethyl acetate) to afford the compound 57h (375 mg, quant).
1H-NMR (CDCl₃) δ: 1.43 (9H, s), 1.50 (3H, d, J = 7.1 Hz), 3.65 (1H, d, J = 15.4 Hz), 3.66 (1H, d, J = 15.4 Hz), 4.58 (1H, q, J = 7.1 Hz), 5.04 (1H, d, J = 4.5 Hz), 5.68 (1H, dd, J = 8.8, 4.5 Hz), 6.17 (1H, s), 6.22 (1H, d, J = 8.8 Hz), 7.24-7.41 (5H, m).
[M+H] = 445, retention time: 2.17 min, (measurement condition B)

### Step 6 Synthesis of compound 57i

A solution of the compound 57h (315 mg, 0.709 mmol) in a 2-propanol (3 mL)-tetrahydrofuran (3 mL) mixed solvent was cooled to -50°C. Sodium borohydride (37.5 mg, 0.992 mmol) was added to the solution. The mixture was warmed from - 50°C to -20°C. While the temperature was kept at -20°C to -10°C, the mixture was stirred for 5.5 hours. Then, a saturated aqueous solution of ammonium chloride was added to the mixture, followed by extraction with ethyl acetate. The organic layer was dried over anhydrous magnesium sulfate. Magnesium sulfate was filtered off. Then, the filtrate was concentrated under reduced pressure. The obtained residue was purified by silica gel column chromatography (hexane-ethyl acetate) to afford the compound 57i (84.2 mg, 27%).
1H-NMR (CDCl₃) δ: 1.33 (3H, d, J = 6.8 Hz), 1.51 (9H, s), 2.52 (1H, d, J = 17.7 Hz), 2.60 (1H, dd, J = 11.1, 7.6 Hz), 2.68 (1H, dd, J = 17.7, 7.6 Hz), 2.79 (1H, dq, J = 11.1, 6.8 Hz), 3.63 (1H, d, J = 16.4 Hz), 3.65 (1H, d, J = 16.4 Hz), 5.04 (1H, d, J = 4.8 Hz), 5.60 (1H, dd, J = 8.8, 4.8 Hz), 6.02 (1H, d, J = 8.8 Hz), 7.23-7.42 (5H, m).
[M+H] = 447, retention time: 2.13 min, (measurement condition B)

### Step 7 Synthesis of compound 57j

To a suspension of phosphorus pentachloride (214 mg, 1.03 mmol) in dichloromethane (4 mL), pyridine (0.091 mL, 1.13 mmol) was added under ice cooling. The reaction mixture was cooled to -78°C. Then, a solution of the compound 57i (76.5 mg, 0.171 mmol) in dichloromethane (2 mL) was added to the reaction mixture. Under ice cooling, the mixture was stirred for 2.5 hours. Then, the reaction mixture was cooled to -50°C. Methanol (2 mL) was added to the reaction mixture. The mixture was stirred at -30°C for 1 hour. Then, water (1.23 mL) was added to the mixture. After stirring at -30°C for 30 minutes, an aqueous solution of sodium hydrogen carbonate was added to the reaction mixture, followed by extraction with dichloromethane. The organic layer was dried over anhydrous magnesium sulfate. Magnesium sulfate was filtered off. Then, dichloromethane in the filtrate was evaporated into approximately 5 mL under reduced pressure to afford a solution of the compound 57j in dichloromethane.

### Step 8 Synthesis of compound 571

A solution of the compound 57k (103 mg, 0.257 mmol) in DMA (1.5 mL) was cooled to -20°C. Triethylamine (0.045 mL, 0.325 mmol) and methanesulfonyl chloride (0.024 mL, 0.308 mmol) were added to the solution. The mixture was stirred at -20°C for 30 minutes to afford reaction mixture A. Pyridine (0.017 mL, 0.205 mmol) was added under ice cooling, and subsequently, the reaction mixture A was added dropwise to the dichloromethane solution of the compound 57j obtained in the step 7. The mixture was stirred at 0°C for 2 hours. Then, water was added to the mixture. Dichloromethane was evaporated under reduced pressure. Ethyl acetate and 2 mol/L hydrochloric acid were added to the residue, followed by extraction with ethyl acetate. The organic layer was washed by water. The organic layer was dried over anhydrous magnesium sulfate. Magnesium sulfate was filtered off. Then, the filtrate was concentrated under reduced pressure. The obtained residue was purified by silica gel column chromatography (hexane-ethyl acetate) to afford the compound 571 (99.4 mg, 82%).
1H-NMR (CDCl₃) δ: 1.47 (9H, s), 1.50 (3H, d, J = 7.0 Hz), 1.54 (9H, s), 1.55 (9H, s), 2.53 (1H, d, J = 17.9 Hz), 2.57 (1H, dd, J = 11.0, 7.8 Hz), 2.71 (1H, dd, J = 17.9, 7.8 Hz), 2.83 (1H, dq, J = 11.0, 7.0 Hz), 4.74 (1H, d, J = 16.9 Hz), 4.77 (1H, d, J = 16.9 Hz), 5.17 (1H, d, J = 4.9 Hz), 5.73 (1H, dd, J = 8.3, 4.9 Hz), 7.37 (1H, s), 8.10 (1H, brs), 8.60 (1H, d, J = 8.3 Hz).
[M+H] = 712, retention time: 2.62 min, (measurement condition B)

### Step 9 Synthesis of compound 57m

The compound 571 (99.4 mg, 0.140 mmol) was dissolved in acetonitrile (2 mL) and DMA (2 mL). The solution was cooled to -20°C. Then, 37% peracetic acid (0.075 mL, 0.419 mmol) was added to the solution. The mixture was stirred at 0°C for 40 minutes. Then, 10% aqueous solution of sodium hydrogen sulfite was added to the reaction mixture, followed by extraction with ethyl acetate. The organic layer was washed by water and then dried over anhydrous magnesium sulfate. Magnesium sulfate was filtered off. Then, the filtrate was concentrated under reduced pressure. The obtained residue was purified by silica gel column chromatography (hexane-ethyl acetate) to afford the compound 57m (98.9 mg, 97%). The compound 57m was obtained as a diastereomeric mixture of β:α = 2.5:1 of sulfoxide.
β-sulfoxide form of the compound 57m: [M+H] = 728, retention time: 2.46 min, (measurement condition B)
α-sulfoxide form of the compound 57m: [M+H] = 728, retention time: 2.42 min, (measurement condition B)

### Step 10 Synthesis of compounds I-057 and 1-058

To a solution of the compound 57m (98.9 mg, 0.136 mmol) in dichloromethane (2 mL), anisole (0.119 mL, 1.09 mmol) and a 2 mol/L solution of aluminum chloride in nitromethane (0.544 mL, 1.09 mmol) were added. The mixture was stirred at -30°C to -23°C for 30 minutes. The reaction mixture was dissolved in ice water, 1 mol/L hydrochloric acid, and acetonitrile. Then, the solution was washed by diisopropyl ether. HP20-SS resin was added to the aqueous layer. Acetonitrile was evaporated under reduced pressure. The obtained mixed solution was purified by ODS column chromatography (water-acetonitrile). Fractions containing the desired compounds were mixed. A 0.1 mol/L aqueous solution of sodium hydroxide was slowly added dropwise under ice cooling using a pH meter, and then, a small piece of dry ice was added to the mixture. This mixed solution was concentrated under reduced pressure and then freeze-dried to afford the compounds I-057 (13.5 mg, 18%) and I-058 (3.0 mg, 3.9%). The compound I-057 was obtained as a diastereomeric mixture of β:α = 2.4:1 of sulfoxide.
Compound I-057: [M+H] = 516, retention time: 0.81 min, (measurement condition B)
Compound I-058: 1H-NMR (D₂O) δ: 1.53 (3H, d, J = 6.4 Hz), 2.80 (1H, d, J = 18.6 Hz), 3.01 (1H, dd, J = 18.6, 7.9 Hz), 3.21 (1H, dq, J = 12.2, 6.4 Hz), 3.29 (1H, dd, J = 12.2, 7.9 Hz), 4.59 (2H, s), 5.16 (1H, d, J = 4.3 Hz), 5.79 (1H, d, J = 4.3 Hz), 7.07 (1H, s).
[M+H] = 516, retention time: 0.77 min, (measurement condition B)

### [EXAMPLE 27]

### Synthesis of compound I-059

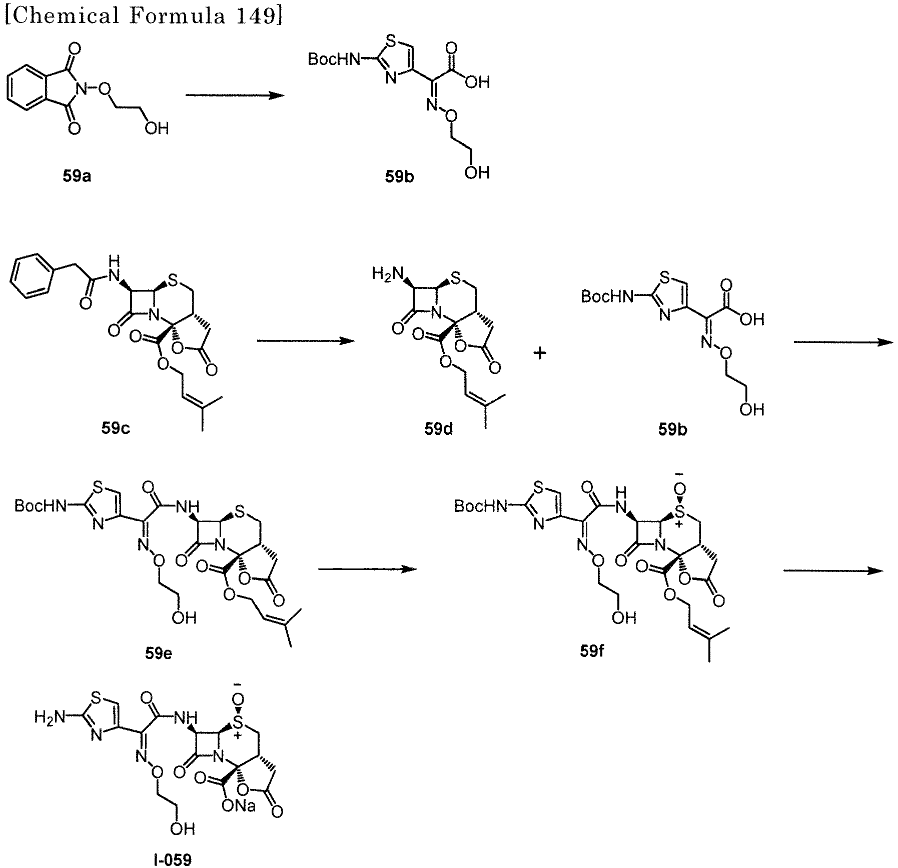

### Step 1 Synthesis of compound 59b

The compound 59b (3.13 g, 73%) was obtained by the similar synthesis of the step 2 of Example 7 using the compound 59a (2.69 g, 13.0 mmol).
1H-NMR (DMSO-d₆) δ: 1.47 (9H, s), 3.62 (2H, t, J = 5.3 Hz), 4.11 (2H, t, J = 5.3 Hz), 4.70 (1H, brs), 7.40 (1H, s), 11.76 (1H, brs).
[M+H] = 332, retention time: 1.16 min, (measurement condition B)

### Steps 2 and 3 Synthesis of compound 59e

A solution of the compound 59d in dichloromethane was obtained by the similar synthesis of the step 5 of Example 1 using the compound 59c (1.00 g, 2.25 mmol). This solution was cooled in ice. Then, the compound 59b (745 mg, 2.25 mmol) and EDC hydrochloride (948 mg, 4.95 mmol) were added to the solution. The mixture was stirred at 0°C for 2 hours. Then, water was added to the mixture, followed by extraction with ethyl acetate. The organic layer was dried over anhydrous magnesium sulfate. Magnesium sulfate was filtered off. Then, the filtrate was concentrated under reduced pressure. The obtained residue was purified by silica gel column chromatography (hexane-ethyl acetate) to afford the compound 59e (922 mg, 64%).
1H-NMR (CDCl₃) δ: 1.54 (9H, s), 1.72 (3H, s), 1.76 (3H, s), 2.61-2.74 (2H, m), 2.78 (1H, dd, J = 17.9, 8.3 Hz), 2.94 (1H, brs), 3.08 (1H, m), 3.22 (1H, m), 3.87 (2H, brs), 4.37 (2H, brs), 4.80 (2H, m), 5.12 (1H, m), 5.30 (1H, m), 5.38 (1H, m), 5.68 (1H, m), 7.31 (1H, s).
[M+H] = 640, retention time: 1.89 min, (measurement condition B)

### Step 4 Synthesis of compound 59f

The compound 59f (353.7 mg, 75%) was obtained by the similar synthesis of the step 6 of Example 1 using the compound 59e (461 mg, 0.721 mmol).
1H-NMR (CDCl₃) δ: 1.54 (9H, s), 1.74 (3H, s), 1.77 (3H, s), 2.43 (1H, d, J = 17.8 Hz), 2.50 (1H, m), 2.97 (1H, m), 3.50 (1H, dd, J = 14.6, 4.9 Hz), 3.68-3.96 (3H, m), 4.34-4.50 (2H, m), 4.71 (1H, d, J = 4.8 Hz), 4.76-4.93 (2H, m), 5.40 (1H, m), 6.04 (1H, dd, J = 9.9, 4.8 Hz), 7.35 (1H, s), 7.92 (1H, d, J = 9.9 Hz), 8.26 (1H, brs).
[M+H] = 656.00 (1.83 min) (Shimadzu)

### Step 4 Synthesis of compound I-059

The compound I-059 (215 mg, 78%) was obtained by the similar synthesis of the step 7 of Example 1 using the compound 59f (354 mg, 0.539 mmol).
1H-NMR (D₂O) δ: 2.57 (1H, d, J = 18.3 Hz), 2.83 (1H, dd, J = 14.7, 12.5 Hz), 3.07 (1H, dd, J = 18.3, 7.9 Hz), 3.55 (1H, ddd, J = 12.5, 7.9, 5.1 Hz), 3.64 (1H, dd, J = 14.7, 5.1 Hz), 3.90 (2H, m), 4.34 (2H, m), 5.01 (1H, d, J = 4.7 Hz), 5.88 (1H, d, J = 4.7 Hz), 7.04 (1H, s).
[M+H] = 488, retention time: 0.38 min, (measurement condition B) C16H16N5O9S2Na1(H2O)4.3 Calc. C : 32.74%, H : 4.23%, N : 11.93%, S : 10.93%, Na : 3.92%. Found C : 32.90 %, H : 4.16%, N : 12.09%, S : 10.75%, Na : 3.92%.

### [EXAMPLE 28]

### Synthesis of compound I-060

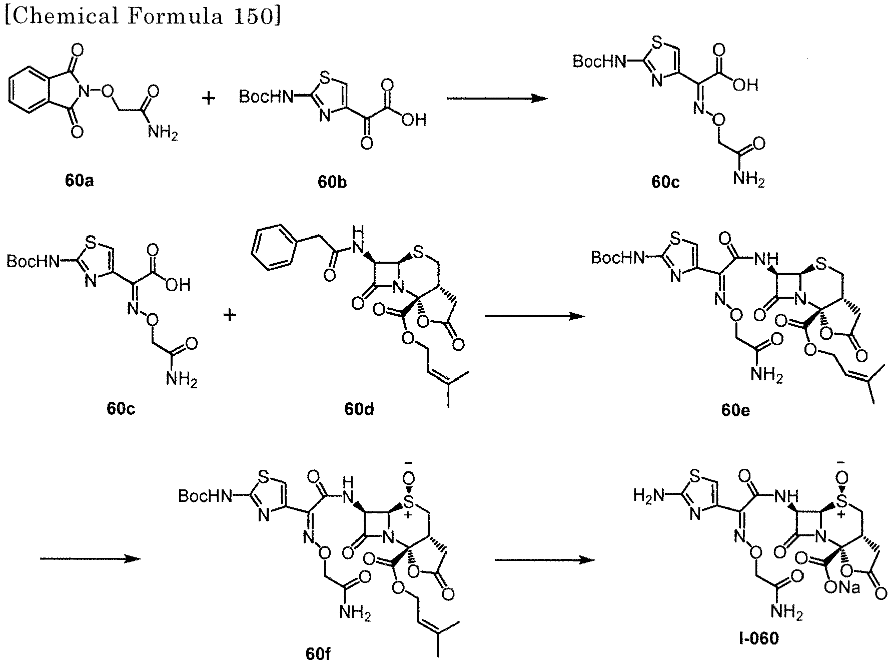

### Step 1 Synthesis of compound 60c

The compound 60a (1.29 g, 5.86 mmol) was dissolved in chloroform (200 mL). Under ice cooling, methylhydrazine (0.326 mL, 6.15 mmol) was added to the solution. The mixture was stirred at 0°C for 1.5 hours. Then, the white solid was collected by filtration and washed by dichloromethane. The filtrate was concentrated into approximately 20 mL. The white precipitates were collected by filtration and washed by dichloromethane to afford solution A. The compound 60b (1.60 g, 5.86 mmol) was dissolved in methanol (26 mL). Under ice cooling, the solution A was added to the solution. While the mixture was stirred at 0°C for 3 hours, a suspension prepared by adding dichloromethane to the white precipitates collected by filtration in obtaining the solution A was ultrasonicated and the filtrate after removal of the remaining white solid was added to the reaction mixture. This operation was repeated four times. After stirring, the solution was concentrated to precipitate a white solid. The solid was collected by filtration to obtain the compound 60c (1.56 g, 77%).
1H-NMR (DMSO-d₆) δ: 1.47 (9H, s), 4.50 (2H, s), 6.99 (1H, brs), 7.46 (1H, brs), 7.49 (1H, s), 11.82 (1H, brs).
[M+H] = 345, retention time: 1.13 min, (measurement condition B)

### Step 2 Synthesis of compound 60e

The compound 60e (718 mg, 49%) was obtained by the similar approach of the step 3 of Example 27 using the compound 60c (600 mg, 1.74 mmol) and the compound 60d (1.00 g, 2.25 mmol).
1H-NMR (CDCl₃) δ: 1.54 (9H, s), 1.69 (3H, s), 1.72 (3H, s), 2.63-2.75 (2H, m), 2.79 (1H, dd, J = 18.1, 8.3 Hz), 3.11 (1H, dd, J = 14.6, 4.4 Hz), 3.28 (1H, m), 4.65 (1H, d, J = 16.2 Hz), 4.74-4.83 (3H, m), 5.13 (1H, d, J = 4.6 Hz), 5.34 (1H, m), 5.65 (1H, dd, J = 8.0, 4.6 Hz), 6.47 (1H, brs), 6.61 (1H, brs), 7.21 (1H, s), 8.51 (1H, brs), 9.53 (1H, brs).
[M+H] = 653, retention time: 1.84 min, (measurement condition B)

### Step 3 Synthesis of compound 60f

The compound 60f (239 mg, 63%) was obtained by the similar synthesis of the step 6 of Example 1 using the compound 60e (370 mg, 0.567 mmol).
1H-NMR (CDCl3) δ: 1.54 (9H, s), 1.73 (3H, s), 1.77 (3H, s), 2.43 (1H, d, J = 18.1 Hz), 2.51 (1H, m), 2.96 (1H, m), 3.48 (1H, m), 3.70 (1H, m), 4.68 (2H, s), 4.72 (1H, m), 4.83 (2H, m), 5.39 (1H, m), 6.00 (1H, m), 6.69 (1H, brs), 6.97 (1H, brs), 7.29 (1H, s), 8.26 (1H, brs), 9.47 (1H, brs).
[M+H] = 669.05 (1.77 min) (Shimadzu)

### Step 4 Synthesis of compound I-060

The compound I-060 (132 mg, 71%) was obtained by the similar synthesis of the step 7 of Example 1 using the compound 60f (239 mg, 0.357 mmol).
1H-NMR (D₂O) δ: 2.57 (1H, d, J = 18.3 Hz), 2.83 (1H, dd, J = 14.7, 12.4 Hz), 3.07 (1H, dd, J = 18.3, 7.8 Hz), 3.55 (1H, ddd, J = 12.4, 7.8, 5.3 Hz), 3.63 (1H, dd, J = 14.7, 5.3 Hz), 4.73 (1H, d, J = 16.2 Hz), 4.76 (1H, d, J = 16.2 Hz), 5.02 (1H, d, J = 4.6 Hz), 5.88 (1H, d, J = 4.6 Hz), 7.13 (1H, s).
[M+H] = 501, retention time: 0.38 min, (measurement condition B) C16H15N6O9S2Na1(H2O)3.8 Calc. C : 32.52%, H : 3.86%, N : 14.22%, S : 10.85%, Na : 3.89%. Found C : 32.57 %, H : 3.92%, N : 14.35%, S : 10.65%, Na : 3.94%.

### [EXAMPLE 29]

### Synthesis of compound I-061

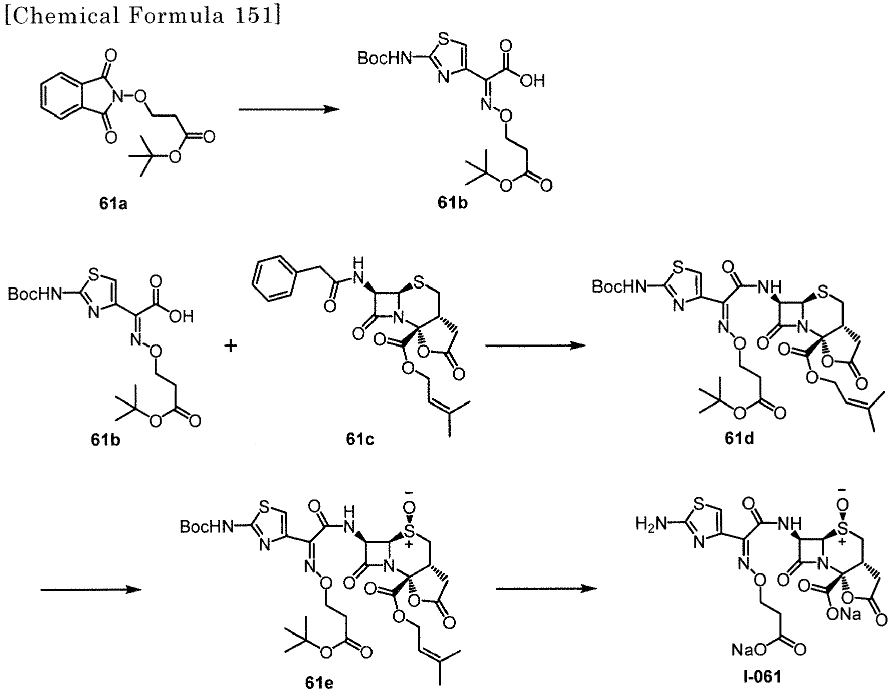

### Step 1 Synthesis of compound 61b

The compound 61b (2.37 g, 83%) was obtained by the similar synthesis of the step 1 of Example 28 using the compound 61a (2.00 g, 6.87 mmol).
1H-NMR (CDCl₃) δ: 1.43 (9H, s), 1.55 (9H, s), 2.68 (2H, t, J = 6.1 Hz), 4.52 (2H, t, J = 6.1 Hz), 7.38 (1H, s).
[M+H] = 416, retention time: 1.89 min, (measurement condition B)

### Step 2 Synthesis of compound 61d

The compound 61d (1.45 g, 89%) was obtained by the similar synthesis of the steps 2 and 3 of Example 27 using the compound 61b (1.03 g, 2.48 mmol) and the compound 61c (1.00 g, 2.25 mmol).
1H-NMR (CDCl₃) δ: 1.43 (9H, s), 1.54 (9H, s), 1.72 (3H, s), 1.76 (3H, s), 2.63-2.85 (5H, m), 3.13 (1H, dd, J = 14.4, 4.3 Hz), 3.31 (1H, m), 4.52 (2H, m), 4.81 (2H, m), 5.08 (1H, d, J = 4.6 Hz), 5.38 (1H, m), 5.62 (1H, dd, J = 8.3, 4.6 Hz), 7.32 (1H, s), 7.65 (1H, d, J = 8.3 Hz), 8.26 (1H, brs).
[M+H] = 724, retention time: 2.33 min, (measurement condition B)

### Step 3 Synthesis of compound 61e

The compound 61e (276 mg, 37%) was obtained by the similar synthesis of the step 6 of Example 1 using the compound 61d (725 mg, 1.00 mmol).
1H-NMR (CDCl₃) δ: 1.43 (9H, s), 1.54 (9H, s), 1.73 (3H, s), 1.77 (3H, s), 2.43 (1H, d, J = 17.8 Hz), 2.47 (1H, m), 2.72 (2H, t, J = 7.4 Hz), 2.98 (1H, dd, J = 17.8, 7.7 Hz), 3.48 (1H, dd, J = 14.7, 4.9 Hz), 3.76 (1H, m), 4.51 (2H, m), 4.65 (1H, d, J = 4.9 Hz), 4.81 (1H, dd, J = 12.2, 7.4 Hz), 4.89 (1H, dd, J = 12.2, 7.4 Hz), 5.40 (1H, m), 6.07 (1H, dd, J = 10.0, 4.9 Hz), 7.28 (1H, s), 7.64 (1H, d, J = 10.0 Hz), 8.28 (1H, brs).

### Step 4 Synthesis of compound I-061

The compound I-061 (133 mg, 64%) was obtained by the similar synthesis of the step 7 of Example 1 using the compound 61e (276 mg, 0.373 mmol).
1H-NMR (D₂O) δ: 2.57 (1H, d, J = 18.3 Hz), 2.68 (2H, t, J = 6.4 Hz), 2.82 (1H, dd, J = 14.8, 12.3 Hz), 3.06 (1H, dd, J = 18.3, 7.8 Hz), 3.55 (1H, ddd, J = 12.3, 7.8, 5.3 Hz), 3.64 (1H, dd, J = 14.8, 5.3 Hz), 4.45 (2H, t, J = 6.4 Hz), 5.00 (1H, d, J = 4.8 Hz), 5.86 (1H, d, J = 4.8 Hz), 7.03 (1H, s).
[M+H] = 516, retention time: 0.39 min, (measurement condition B) C17H15.2N5O10S2Na1.8(H2O)3.9 Calc. C : 32.65%, H : 3.71%, N : 11.20%, S : 10.25%, Na : 6.62%. Found C : 32.61 %, H : 3.86%, N : 11.44%, S : 10.07%, Na : 6.52%.

### [EXAMPLE 30]

### Synthesis of compounds I-062 and I-063

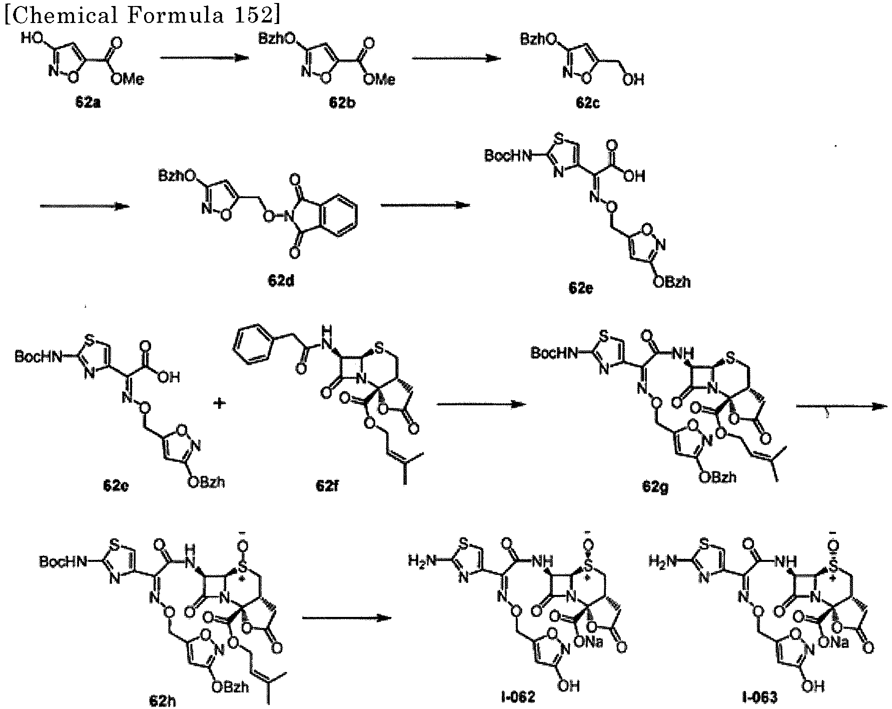

### Step 1 Synthesis of compound 62b

The compound 62a (5.0 g, 34.9 mmol) was dissolved in dichloromethane (50 ml). Diphenyldiazomethane (8.14 g, 41.9 mmol) was added to the solution. The mixture was stirred for 1 hour. The reaction mixture was concentrated under reduced pressure. Then, the residue was subjected to silica gel column chromatography, followed by elution with chloroform/ethyl acetate. Fractions containing the desired compound were concentrated under reduced pressure to afford the compound 62b (5.77 g, 53%).
¹H-NMR (CDCl₃) δ: 7.43-7.28 (10H, m), 6.74 (1H, s), 6.62 (1H, s), 3.92 (3H, s).

### Step 2 Synthesis of compound 62c

The compound 62b (5.77 g, 18.7 mmol) was dissolved in ethanol (30 ml) and tetrahydrofuran (30 ml). Under ice cooling, sodium borohydride (1.41 g, 37.3 mmol) was added to the solution. Under ice cooling, the mixture was stirred for 1 hour and then stirred at room temperature for 3 hours. Dilute hydrochloric acid was added to the mixture, followed by extraction with ethyl acetate. The organic layer was washed by water and brine in this order and dried over anhydrous magnesium sulfate. Magnesium sulfate was filtered off. Then, the filtrate was concentrated under reduced pressure to afford the compound 62c (5.50 g) as a crude product.
¹H-NMR (CDCl₃) δ: 7.43-7.26 (10H, m), 6.69 (1H, s), 5.96 (1H, s), 4.61 (2H, s), 1.96 (1H, br s).

### Step 3 Synthesis of compound 62d

The whole amount of the obtained compound 62c was dissolved in tetrahydrofuran (50 mL). Under ice cooling, N-hydroxyphthalimide (3.65 g), triphenylphosphine (5.87 g), and di-2-methoxyethyl azodicarboxylate (5.24 g) were added to the solution. Under ice cooling, the mixture was stirred for 1 hour. Then, the solvent was evaporated. The residue was purified by silica gel column chromatography (elution in the chloroform/ethyl acetate system). Fractions containing the compound of interest were collected. The solution was concentrated. The precipitates were collected by filtration using methanol and dried to synthesize the compound 62d. Yield amount: 6.27 g (79%)
¹H-NMR (CDCl₃) δ: 7.83-7.81 (2H, m), 7.76-7.74 (2H, m), 7.41-7.27 (10H, m), 6.67 (1H, s), 6.28 (1H, s), 5.16 (2H, s).

### Step 4 Synthesis of compound 62e

The compound 62e was obtained by the similar synthesis of the step 2 of Example 1 using the compound 62d (2.00 g, 4.69 mmol).
Yield amount: 1.89 g (73%)
¹H-NMR (CDCl₃) δ: 7.38-7.28 (8H, m), 7.25-7.21 (3H, m), 6.64 (1H, s), 6.02 (1H, s), 5.05 (2H, s), 1.52 (9H, s).

### Step 5 Synthesis of compound 62g

The compound 62g (1.67 g, 87%) was obtained by the similar synthesis of the steps 2 and 3 of Example 27 using the compound 62e (1.24 g, 2.25 mmol) and the compound 62f (1.00 g, 2.25 mmol).
1H-NMR (CDCl₃) δ: 1.54 (9H, s), 1.66 (3H, s), 1.70 (3H, s), 2.34 (1H, dd, J = 14.4, 9.1 Hz), 2.51 (1H, dd, J = 18.2, 3.3 Hz), 2.63 (1H, dd, J = 18.2, 8.3 Hz), 2.76 (1H, dd, J = 14.4, 4.3 Hz), 3.08 (1H, m), 4.75 (2H, m), 5.03 (1H, d, J = 4.5 Hz), 5.20 (1H, d, J = 13.9 Hz), 5.27-5.35 (2H, m), 5.70 (1H, dd, J = 8.6, 4.5 Hz), 6.07 (1H, s), 6.65 (1H, s), 7.20 (1H, s), 7.24-7.47 (10H, m), 8.65 (1H, brs).
[M+H] = 859, retention time: 2.92 min, (measurement condition B)

### Step 6 Synthesis of compound 62h

The compound 62h (782 mg, 89%) was obtained as a mixture of β and α forms of sulfoxide by the similar synthesis of the step 6 of Example 1 using the compound 62g (860 mg, 1.00 mmol).
β-sulfoxide form of the compound 62h: [M+H] = 875, retention time: 2.87 min, (measurement condition B)
α-sulfoxide form of the compound 62h: [M+H] = 875, retention time: 2.81 min, (measurement condition B)

### Step 7 Synthesis of compounds I-062 and I-063

The compounds I-062 (215 mg, 43%) and I-063 (55.6 mg, 11%) were obtained by the similar synthesis of the step 7 of Example 1 using the compound 62h (782 mg, 0.894 mmol).
Compound I-062: 1H-NMR (D₂O) δ: 2.56 (1H, d, J = 18.3 Hz), 2.79 (1H, dd, J = 13.9, 11.8 Hz), 3.06 (1H, dd, J = 18.3, 7.4 Hz), 3.54 (1H, ddd, J = 11.8, 7.4, 5.4 Hz), 3.59 (1H, dd, J = 13.9, 5.4 Hz), 4.96 (1H, d, J = 4.8 Hz), 5.22 (1H, d, J = 14.2 Hz), 5.22 (1H, d, J = 14.2 Hz), 5.86 (1H, d, J = 4.8 Hz), 6.12 (1H, s), 7.08 (1H, s).
[M+H] = 541, retention time: 0.92 min, (measurement condition B)
Compound I-063: 1H-NMR (D₂O) δ: 2.82 (1H, dd, J = 18.6, 2.9 Hz), 3.03 (1H, dd, J = 18.6, 8.3 Hz), 3.17 (1H, dd, J = 13.1, 11.0 Hz), 3.56 (1H, dddd, J = 11.0, 8.3, 4.8, 2.9 Hz), 3.65 (1H, dd, J = 13.1, 4.8 Hz), 5.08 (1H, d, J = 4.3 Hz), 5.22 (2H, s), 5.68 (1H, d, J = 4.3 Hz), 6.11 (1H, s), 7.06 (1H, s).
[M+H] = 541, retention time: 0.60 min, (measurement condition B) C18H14.7N6O10S2Na1.3(H2O)3.7 Calc. C : 34.01%, H : 3.50%, N : 13.22%, S : 10.09%, Na : 4.70%. Found C : 33.96%, H : 3.59%, N : 13.31%, S : 10.21%, Na : 4.66%.

### [EXAMPLE 31]

### Synthesis of compound I-064

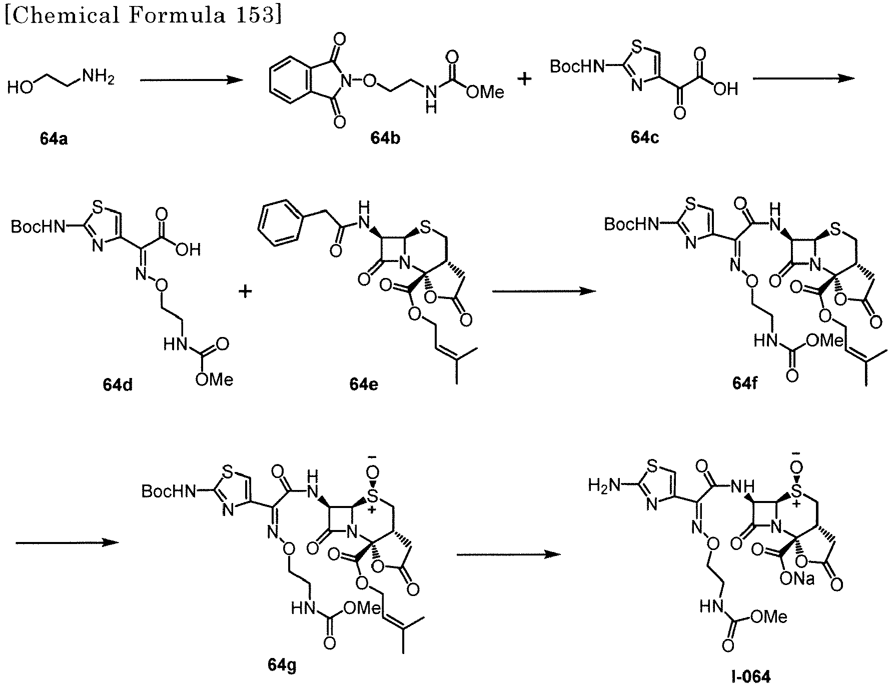

### Step 1 Synthesis of compound 64b

To a solution of the ethanolamine 64a (1.19 mL, 19.7 mmol) in dichloromethane (12 mL), dimethyl dicarbonate (2.63 g, 19.7 mmol) was added. The mixture was stirred at room temperature for 1.5 hours. Then, the reaction mixture was concentrated under reduced pressure. The obtained residue was dissolved in tetrahydrofuran (20 mL). The solution was cooled in ice. Then, N-hydroxyphthalimide (3.85 g, 23.6 mmol) and triphenylphosphine (6.18 g, 23.6 mmol) were added, and subsequently, diisopropyl azodicarboxylate (4.58 mL, 23.6 mmol) was added to the solution. The mixture was stirred at room temperature for 23 hours. Then, the reaction mixture was concentrated under reduced pressure. The residue was dissolved in methanol. Then, the solution was concentrated again under reduced pressure. The resulting white solid was collected by filtration to afford the compound 64b (1.34 g, 26%).
1H-NMR (CDCl₃) δ: 3.50 (2H, m), 3.72 (3H, s), 4.27 (2H, t, J = 4.8 Hz), 5.91 (1H, brs), 7.78 (2H, m), 7.86 (2H, m).
[M+H] = 265, retention time: 1.24 min, (measurement condition B)

### Step 2 Synthesis of compound 64d

A solution of the compound 64b (1.34 g, 5.07 mmol) in dichloromethane (20 mL) was cooled in ice. Methylhydrazine (0.282 mL, 5.32 mmol) was added to the solution. The mixture was stirred at 0°C for 50 minutes. Then, insoluble matter was removed. Dichloromethane was evaporated into approximately 10 mL under reduced pressure to afford a solution of hydroxyamine in dichloromethane. A solution of the separately prepared compound 64c (1.38 g, 5.07 mmol) in methanol (20 mL) was cooled in ice. This solution of hydroxyamine in dichloromethane was added dropwise to the solution. The mixture was stirred at 0°C for 1 hour. Then, dichloromethane was evaporated under reduced pressure. Ethyl acetate, water, and subsequently 2 mol/L hydrochloric acid were added to the residue, followed by extraction with ethyl acetate. The organic layer was dried over anhydrous magnesium sulfate. Magnesium sulfate was filtered off. Then, the filtrate was concentrated under reduced pressure to afford the compound 64d (1.80 g, 91%). 1H-NMR (CDCl₃) δ: 1.47 (9H, s), 3.25 (2H, m), 3.53 (3H, s), 4.08 (2H, t, J = 6.2 Hz), 7.15 (1H, m), 7.42 (1H, s), 11.81 (1H, s), 13.93 (1H, brs).
[M+H] = 389, retention time: 1.35 min, (measurement condition B)

### Step 3 Synthesis of compound 64f

To a suspension of phosphorus pentachloride (937 mg, 4.50 mmol) in dichloromethane (10 mL), pyridine (0.400 mL, 4.95 mmol) was added under ice cooling. The reaction mixture was cooled to -78°C. Then, the compound 64e (1.0 g, 2.25 mmol) was added to the reaction mixture. Under ice cooling, the mixture was stirred for 40 minutes. Then, the reaction mixture was cooled to -78°C. Ethanol (10 mL) was added to the reaction mixture. The mixture was stirred at -30°C for 35 minutes. Then, water (2.25 mL) was added to the mixture. After stirring at -30°C for 35 minutes, an aqueous solution of sodium hydrogen carbonate was added to the reaction mixture, followed by extraction with dichloromethane. The organic layer was dried over anhydrous magnesium sulfate. Magnesium sulfate was filtered off. Then, dichloromethane in the filtrate was evaporated into approximately 10 mL under reduced pressure to afford a dichloromethane solution (solution A). A solution of the compound 64d (961 mg, 2.48 mmol) in DMA (3.7 mL) was cooled to -20°C. Triethylamine (0.437 mL, 3.15 mmol) and methanesulfonyl chloride (0.228 mL, 2.93 mmol) were added to the solution. The mixture was stirred at -20°C for 40 minutes to afford a suspension (suspension B). Pyridine (0.218 mL, 2.70 mmol) was added under ice cooling, and subsequently, the suspension B was added dropwise to the solution A. The mixture was stirred at 0°C for 35 minutes. Then, water was added to the mixture. Dichloromethane was evaporated under reduced pressure. Ethyl acetate and 2 mol/L hydrochloric acid were added to the residue, followed by extraction with ethyl acetate. The organic layer was washed by water. The organic layer was dried over anhydrous magnesium sulfate. Magnesium sulfate was filtered off. Then, the filtrate was concentrated under reduced pressure. The obtained residue was purified by silica gel column chromatography (hexane-ethyl acetate) to afford the compound 64f (1.14 g, 72%).
1H-NMR (CDCl₃) δ: 1.54 (9H, s), 1.72 (3H, s), 1.76 (3H, s), 2.63-2.75 (2H, m), 2.80 (1H, dd, J = 18.1, 8.3 Hz), 3.13 (1H, dd, J = 14.6, 4.3 Hz), 3.31 (1H, m), 3.52 (2H, m), 3.56 (3H, s), 4.35 (2H, m), 4.81 (2H, m), 5.11 (1H, d, J = 4.6 Hz), 5.33 (1H, m), 5.38 (1H, m), 5.70 (1H, dd, J = 8.7, 4.6 Hz), 7.29 (1H, s), 7.81 (1H, d, J = 8.7 Hz), 8.25 (1H, brs).
[M+H] = 697, retention time: 2.00 min, (measurement condition B)

### Step 4 Synthesis of compound 64g

The compound 64f (563 mg, 0.808 mmol) was dissolved in acetonitrile (2.8 mL) and DMA (2.8 mL). The solution was cooled to -20°C. Then, 37% peracetic acid (0.218 mL, 1.21 mmol) was added to the solution. The mixture was stirred at 0°C for 1.5 hours. Then, 10% aqueous solution of sodium hydrogen sulfite was added to the reaction mixture, followed by extraction with ethyl acetate. The organic layer was washed by water and then dried over anhydrous magnesium sulfate. Magnesium sulfate was filtered off. Then, the filtrate was concentrated under reduced pressure. The obtained residue was purified by silica gel column chromatography (hexane-ethyl acetate, ethyl acetate-methanol) to afford the compound 64g (320 mg, 56%). 1H-NMR (CDCl₃) δ: 1.54 (9H, s), 1.74 (3H, s), 1.78 (3H, s), 2.39-2.54 (2H, m), 2.99 (1H, dd, J = 17.7, 7.6 Hz), 3.43-3.57 (3H, m), 3.65 (3H, s), 3.78 (1H, m), 4.28-4.43 (2H, m), 4.69 (1H, d, J = 4.9 Hz), 4.82 (1H, dd, J = 12.2, 7.4 Hz), 4.89 (1H, dd, J = 12.2, 7.4 Hz), 5.40 (1H, m), 5.93 (1H, m), 6.07 (1H, dd, J = 9.8, 4.9 Hz), 7.31 (1H, s), 7.74 (1H, d, J = 9.8 Hz), 8.22 (1H, brs).

### Step 5 Synthesis of compound I-064

To a solution of the compound 64g (320 mg, 0.448 mmol) in dichloromethane (6.5 mL), anisole (0.392 mL, 3.59 mmol) and a 2 mol/L solution of aluminum chloride in nitromethane (1.79 mL, 3.59 mmol) were added. The mixture was stirred at -30°C to -26°C for 1.5 hours. The reaction mixture was dissolved in ice water, 2 mol/L hydrochloric acid, and acetonitrile. Then, the solution was washed by diisopropyl ether. HP20-SS resin was added to the aqueous layer. Acetonitrile was evaporated under reduced pressure. The obtained mixed solution was purified by ODS column chromatography (water-acetonitrile). Fractions containing the desired compound were mixed. Under ice cooling, a 0.2 mol/L aqueous solution of sodium hydroxide (1.3 mL) was slowly added dropwise to the mixture. When the pH reached 4.78, a small piece of dry ice was added to the mixture so that the pH became 4.37. This mixed solution was concentrated under reduced pressure and then freeze-dried to afford the compound I-064 (185 mg, 73%).
1H-NMR (D₂O) δ: 2.57 (1H, d, J = 18.3 Hz), 2.84 (1H, dd, J = 14.3, 12.3 Hz), 3.07 (1H, dd, J = 18.3, 7.5 Hz), 3.49 (2H, m), 3.52-3.64 (2H, m), 3.65 (3H, s), 4.31 (2H, m), 5.01 (1H, d, J = 4.8 Hz), 5.88 (1H, d, J = 4.8 Hz), 7.04 (1H, s).
[M+H] = 545, retention time: 0.56 min, (measurement condition B) C18H19N6O10S2Na1(H₂O)3.5 Calc. C : 34.34%, H : 4.16%, N : 13.35%, S : 10.19%, Na : 3.65%. Found C : 34.21 %, H : 4.26%, N : 13.56%, S : 10.06%, Na : 3.50%.

### [EXAMPLE 32]

### Synthesis of compound I-065

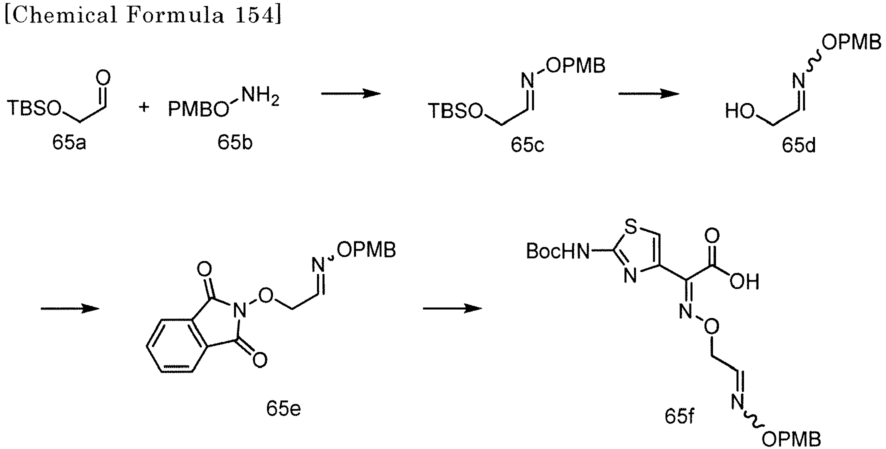

### Step 1 Synthesis of compound 65c

The compound 65a (5.00 g, 28.7 mmol) was dissolved in methanol (50 ml). Under ice cooling, a solution of the compound 65b (4.61 g, 30.1 mmol) in dichloromethane (25 ml) was added to the solution. Under ice cooling, the mixture was stirred for 1 hour and then stirred overnight at room temperature. The mixture was concentrated under reduced pressure to afford the compound 65c as a crude product. The compound 65c was used in the next reaction without being purified.

### Step 2 Synthesis of compound 65d

The obtained compound 65c (corresponding to 28.7 mmol) was dissolved in tetrahydrofuran (30 ml). Acetic acid (4.92 ml, 86 mmol) and a 1 mol/L solution of TBAF in tetrahydrofuran (86 ml, 86 mmol) were added to the solution. The mixture was stirred for 3 hours. Then, water was added to the mixture, followed by extraction with ethyl acetate. The organic layer was washed by a saturated aqueous solution of sodium bicarbonate and brine in this order and dried over anhydrous magnesium sulfate. Magnesium sulfate was filtered off. Then, the filtrate was concentrated under reduced pressure. The residue was subjected to silica gel column chromatography, followed by elution with hexane/ethyl acetate. Fractions containing the desired compound were concentrated under reduced pressure to afford the compound 65d (4.36 g, 78%) as isomeric mixture of approximately 2:1 of oxime.
¹H-NMR (CDCl₃) δ: 7.55 (1H, t, J = 4.0 Hz), 7.29 (3H, t, J = 7.8 Hz), 6.89 (4H, d, J = 8.1 Hz), 5.04 (1H, s), 5.02 (2H, s), 4.41 (1H, d, J = 3.5 Hz), 4.26 (2H, d, J = 4.0 Hz), 3.81 (5H, s).

### Step 3 Synthesis of compound 65e

The compound 65d (4.36 g, 22.33 mmol), N-hydroxyphthalimide (4.37 g, 26.8 mmol), and triphenylphosphine (7.03 g, 26.8 mmol) were dissolved in tetrahydrofuran (45 ml). Under ice cooling, bis(2-methoxyethyl)azodicarboxylate (6.28 g, 26.8 mmol) was added to the solution. Under ice cooling, the mixture was stirred for 1 hour and then concentrated under reduced pressure. Methanol was added to the residue.
The precipitates were collected by filtration to afford the compound 65e (5.06 g, 67%) as an isomeric mixture of approximately 2:1 of oxime.
¹H-NMR (CDCl₃) δ: 7.85-7.81 (3.0H, m), 7.77-7.75 (3.0H, m), 7.68 (1.0H, t, J = 6.2 Hz), 7.23-7.17 (3.0H, m), 6.84 (1.0H, d, J = 7.8 Hz), 6.76 (2.0H, d, J = 8.3 Hz), 5.03-5.01 (1.0H, m), 5.00 (1.0H, br s), 4.94 (2.0H, br s), 4.75 (2.0H, d, J = 6.3 Hz), 3.79 (1.5H, s), 3.77 (3.0H, s).

### Step 4 Synthesis of compound 65f

The crude product of the compound 65f (1.20 g, 88%) was obtained as an isomeric mixture of approximately 2:1 of oxime by the similar synthesis of the step 2 of Example 1 using the compound 65e (1.00 g, 2.94 mmol).
[M+H] = 465, retention time: 1.98 min, (measurement condition A)

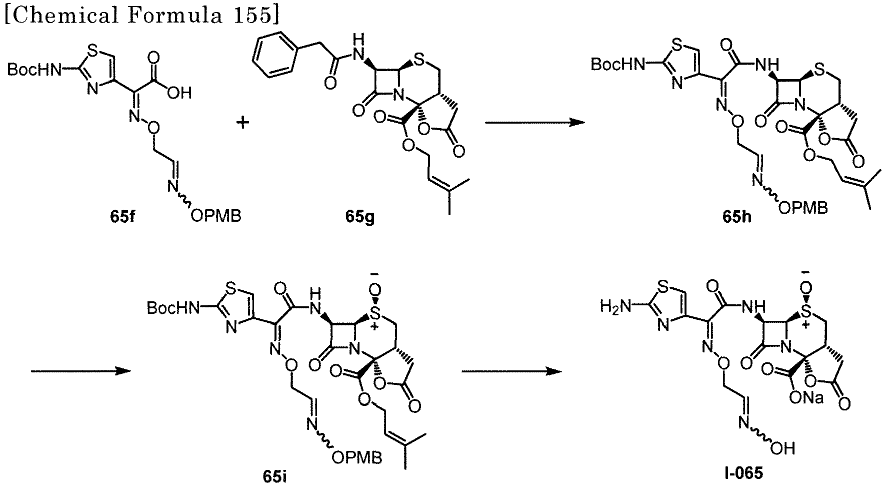

### Step 5 Synthesis of compound 65h

The crude product of the compound 65h (1.60 g, 92%) was obtained as an isomeric mixture of approximately 1.2:1 of oxime by the similar synthesis of the steps 2 and 3 of Example 27 using the compound 65f (1.04 g, 2.25 mmol) and the compound 65g (1.00 g, 2.25 mmol).
[M+H] = 773, retention time: 2.67 min, (measurement condition B)

### Step 6 Synthesis of compound 65i

The compound 65i (613 mg, 70%) was obtained as an isomeric mixture of approximately 1.1:1 of oxime by the similar synthesis of the step 4 of Example 31 using the compound 65h (860 mg, 1.11 mmol).
[M+H] = 789, retention time: 2.60 min, 2.63 min, (measurement condition B)

### Step 7 Synthesis of compound I-065

The compound I-065 (87.1 mg, 21%) was obtained as a 1.7:1 isomeric mixture of oxime by the similar synthesis of the step 6 of Example 1 using the compound 65i (613 mg, 0.777 mmol).
major: 1H-NMR (D2O) δ: 2.57 (1H, d, J = 18.3 Hz), 2.82 (1H, dd, J = 14.6, 12.4 Hz), 3.07 (1H, dd, J = 18.3, 7.8 Hz), 3.55 (1H, ddd, J = 12.4, 7.8, 5.3 Hz), 3.62 (1H, dd, J = 14.6, 5.3 Hz), 4.84 (2H, d, J = 5.6 Hz), 5.00 (1H, d, J = 4.8 Hz), 5.87 (1H, d, J = 4.8 Hz), 7.08 (1H, s), 7.71 (1H, t, J = 5.6 Hz).
minor: 1H-NMR (D2O) δ: 2.57 (1H, d, J = 18.3 Hz), 2.82 (1H, dd, J = 14.6, 12.4 Hz), 3.07 (1H, dd, J = 18.3, 7.8 Hz), 3.50-3.67 (2H, m), 5.01 (1H, d, J = 4.6 Hz), 5.07 (1H, dd, J = 16.4, 3.9 Hz), 5.09 (1H, dd, J = 16.4, 3.9 Hz), 5.88 (1H, d, J = 4.6 Hz), 7.09 (1H, s), 7.15 (1H, t, J = 3.9 Hz).
[M+H] = 501, retention time: 0.57 min, (measurement condition B) C16H15N6O9S2Na1(H2O)3.3 Calc. C : 33.03%, H : 3.74%, N : 14.44%, S : 11.02%, Na : 3.95%. Found C : 32.99%, H : 3.74%, N : 14.68%, S : 10.79%, Na : 3.83%.

### [EXAMPLE 33]

### Synthesis of compounds I-066 and I-067

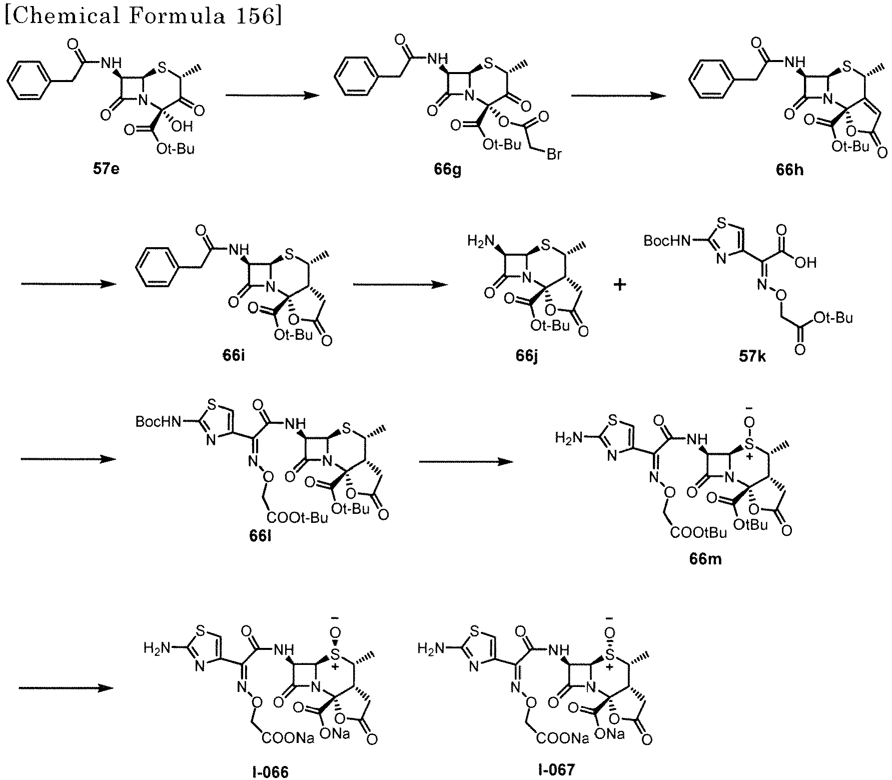

### Step 1 Synthesis of compound 66g

A solution of the compound 57e (5.54 g, 13.2 mmol) in dichloromethane (55 mL) was cooled in ice. Then, bromoacetyl bromide (2.06 mL, 23.7 mmol) was added, and subsequently, triethylamine (3.29 mL, 23.7 mmol) was added to the solution. Under ice cooling, the mixture was stirred for 2 hours. Then, a saturated aqueous solution of ammonium chloride was added to the mixture, followed by extraction with ethyl acetate. The organic layer was dried over anhydrous magnesium sulfate. Magnesium sulfate was filtered off. Then, the filtrate was concentrated under reduced pressure. The obtained residue was purified by silica gel column chromatography (hexane-ethyl acetate) to afford the compound 66g (4.21 g, 59%). 1H-NMR (CDCl₃) δ: 1.32 (3H, d, J = 6.7 Hz), 1.48 (9H, s), 3.73 (2H, s), 4.01 (1H, d, J = 13.6 Hz), 4.02 (1H, d, J = 13.6 Hz), 4.26 (1H, q, J = 6.7 Hz), 5.07-5.13 (2H, m), 6.03 (1H, m), 7.29-7.43 (5H, m).

### Step 2 Synthesis of compound 66h

To a solution of the compound 66g (4.21 g, 7.78 mmol) in DMF (42 mL), triphenylphosphine (2.45 g, 9.33 mmol) was added. The mixture was stirred at room temperature for 1.5 hours. Then, 8.4% aqueous solution of sodium hydrogen carbonate (20.0 mL, 20.0 mmol) was added to the mixture. The mixture was stirred at room temperature for 20 minutes. Then, water and ethyl acetate were added to the reaction mixture, followed by extraction with ethyl acetate. The organic layer was dried over anhydrous magnesium sulfate. Magnesium sulfate was filtered off. Then, the filtrate was concentrated under reduced pressure. The obtained residue was purified by silica gel column chromatography (hexane-ethyl acetate) to afford the compound 66h (1.76 g, 51%).
1H-NMR (CDCl₃) δ: 1.44 (9H, s), 1.65 (3H, d, J = 6.6 Hz), 3.65 (1H, d, J = 16.7 Hz), 3.66 (1H, d, J = 16.7 Hz), 4.13 (1H, m), 5.04 (1H, d, J = 4.3 Hz), 5.60 (1H, dd, J = 9.1, 4.3 Hz), 6.10 (1H, m), 6.19 (1H, d, J = 9.1 Hz), 7.24-7.42 (5H, m).
[M+Na] = 467, retention time: 2.19 min, (measurement condition B)

### Step 3 Synthesis of compound 66i

A solution of the compound 66h (360 mg, 0.810 mmol) in methanol (10.5 mL) was cooled to -50°C. Sodium borohydride (92.0 mg, 2.43 mmol) was added to the solution. The mixture was stirred at -50°C for 4.5 hours. Then, a saturated aqueous solution of ammonium chloride was added to the mixture, followed by extraction with ethyl acetate. The organic layer was dried over anhydrous magnesium sulfate. Magnesium sulfate was filtered off. Then, the filtrate was concentrated under reduced pressure. The obtained residue was purified by silica gel column chromatography (hexane-ethyl acetate) to afford the compound 66i (216 mg, 60%).
1H-NMR (CDCl₃) δ: 1.12 (3H, d, J = 6.9 Hz), 1.53 (9H, s), 2.60 (1H, dd, J = 18.6, 10.2 Hz), 3.12 (1H, dd, J = 18.6, 8.5 Hz), 3.21 (1H, qd, J = 6.9, 2.6 Hz), 3.34 (1H, ddd, J = 10.2, 8.5, 2.6 Hz), 3.69 (2H, s), 4.84 (1H, d, J = 4.4 Hz), 5.35 (1H, dd, J = 8.4, 4.4 Hz), 6.11 (1H, d, J = 8.4 Hz), 7.28-7.41 (5H, m).
[M+Na] = 469, retention time: 2.13 min, (measurement condition B)

### Step 4 Synthesis of compound 66j

To a suspension of phosphorus pentachloride (218 mg, 1.05 mmol) in dichloromethane (2.5 mL), pyridine (0.093 mL, 1.15 mmol) was added under ice cooling. The reaction mixture was cooled to -78°C. Then, a solution of the compound 66i (234 mg, 0.524 mmol) in dichloromethane (2.5 mL) was added to the reaction mixture. Under ice cooling, the mixture was stirred for 30 minutes. Then, the reaction mixture was cooled to -78°C. Ethanol (2.5 mL) was added to the reaction mixture. The mixture was stirred for at -30°C for 45 minutes. Then, water (0.944 mL) was added to the reaction mixture. After stirring at -30°C for 25 minutes, an aqueous solution of sodium hydrogen carbonate was added to the reaction mixture, followed by extraction with dichloromethane. The organic layer was dried over anhydrous magnesium sulfate. Magnesium sulfate was filtered off. Then, dichloromethane in the filtrate was evaporated into approximately 10 mL under reduced pressure to afford a solution of the compound 66j in dichloromethane.

### Step 5 Synthesis of compound 661

A solution of the compound 57k (210 mg, 0.524 mmol) in DMA (1.5 mL) was cooled to -20°C. Triethylamine (0.102 mL, 0.734 mmol) and methanesulfonyl chloride (0.053 mL, 0.681 mmol) were added to the solution. The mixture was stirred at -20°C for 30 minutes to afford reaction mixture A. Pyridine (0.051 mL, 0.629 mmol) was added under ice cooling, and subsequently, the reaction mixture A was added dropwise to the dichloromethane solution of the compound 66j obtained in the step 4. The mixture was stirred at 0°C for 40 minutes. Then, water was added to the mixture. Dichloromethane was evaporated under reduced pressure. Ethyl acetate and 2 mol/L hydrochloric acid were added to the residue, followed by extraction with ethyl acetate. The organic layer was washed by water. The organic layer was dried over anhydrous magnesium sulfate. Magnesium sulfate was filtered off. Then, the filtrate was concentrated under reduced pressure. The obtained residue was purified by silica gel column chromatography (hexane-ethyl acetate) to afford the compound 661 (334 mg, 90%).
1H-NMR (CDCl₃) δ: 1.19 (3H, d, J = 6.8 Hz), 1.46 (9H, s), 1.54 (9H, s), 1.55 (9H, s), 2.64 (1H, dd, J = 18.4, 10.1 Hz), 3.22 (1H, dd, J = 18.4, 8.3 Hz), 3.38 (1H, m), 3.56 (1H, m), 4.76 (1H, d, J = 16.9 Hz), 4.78 (1H, d, J = 16.9 Hz), 4.97 (1H, d, J = 4.3 Hz), 5.52 (1H, dd, J = 8.1, 4.3 Hz), 7.44 (1H, s), 8.07 (1H, s), 8.69 (1H, d, J = 8.1 Hz).
[M+H] = 712, retention time: 2.63 min, (measurement condition B)

### Step 6 Synthesis of compound 66m

The compound 661 (431 mg, 0.605 mmol) was dissolved in acetonitrile (2 mL) and DMA (2 mL). The solution was cooled to -20°C. Then, 37% peracetic acid (0.380 mL, 2.12 mmol) was added to the solution. The mixture was stirred at 0°C for 50 minutes. Then, 10% aqueous solution of sodium hydrogen sulfite was added to the reaction mixture, followed by extraction with ethyl acetate. The organic layer was washed by water and then dried over anhydrous magnesium sulfate. Magnesium sulfate was filtered off. Then, the filtrate was concentrated under reduced pressure. The obtained residue was purified by silica gel column chromatography (hexane-ethyl acetate) to afford the compound 66m (283 mg, 64%). The compound 66m was obtained as a 4:1 diastereomeric mixture of sulfoxide.
[M+H] = 728, retention time: 2.45 min, (measurement condition B)

### Step 7 Synthesis of compounds I-066 and I-067

To a solution of the compound 66m (283 mg, 0.388 mmol) in dichloromethane (6 mL), anisole (0.339 mL, 3.11 mmol) and a 2 mol/L solution of aluminum chloride in nitromethane (1.55 mL, 3.11 mmol) were added. The mixture was stirred at -30°C to -23°C for 20 minutes. The reaction mixture was dissolved in ice water, 2 mol/L hydrochloric acid, and acetonitrile. Then, the solution was washed by diisopropyl ether. HP20-SS resin was added to the aqueous layer. Acetonitrile was evaporated under reduced pressure. The obtained mixed solution was purified by ODS column chromatography (water-acetonitrile). Fractions containing the desired compounds were mixed. A 0.1 mol/L aqueous solution of sodium hydroxide was slowly added dropwise under ice cooling, and then, a small piece of dry ice was added to the mixture. This mixed solution was concentrated under reduced pressure and then freeze-dried to afford the compound I-066 (28.8 mg, 13%) and the compound I-067 (138 mg, 63%).
Compound I-066: 1H-NMR (D₂O) δ: 1.29 (3H, d, J = 7.6 Hz), 2.74 (1H, d, J = 18.4 Hz), 3.01 (1H, dd, J = 18.4, 8.3 Hz), 3.68 (1H, m), 3.82 (1H, m), 4.59 (2H, s), 5.17 (1H, d, J = 4.8 Hz), 5.95 (1H, d, J = 4.8 Hz), 7.07 (1H, s).
[M+H] = 516, retention time: 0.82 min, (measurement condition B)
Compound I-067: 1H-NMR (D₂O) δ: 1.56 (3H, d, J = 7.3 Hz), 2.99 (1H, dd, J = 22.5, 13.9 Hz), 3.39-3.51 (2H, m), 3.64 (1H, m), 4.59 (2H, s), 5.13 (1H, d, J = 4.8 Hz), 5.54 (1H, d, J = 4.8 Hz), 7.00 (1H, s).
[M+H] = 516, retention time: 0.47 min, (measurement condition B) C17H15.1N5O10S2Na1.9(H₂O)3.8 Calc. C : 32.63%, H : 3.66%, N : 11.19%, S : 10.25%,
Na : 6.98%. Found C : 32.65%, H : 3.78%, N : 11.39%, S : 10.05%, Na : 7.00%.

The following compounds were synthesized by the similar procedures.

**[Table 1]**

| **Compound No.** | **Structure** | **[M+H]** | **Retention time (min.)** | **LCMS measurement condition** |
|---|---|---|---|---|
| I-004 | 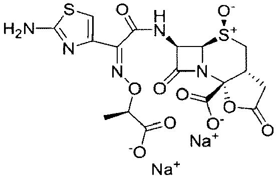 | 516 | 0.47 | B |
| I-005 | 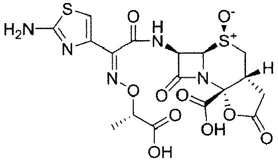 | 516 | 0.41 | A |
| I-006 | 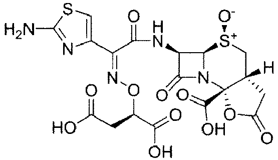 | 560 | 0.30 | A |
| I-007 | 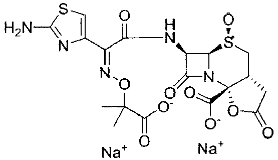 | 530 | 0.61 | A |

**[Table 2]**

| | | | | |
|---|---|---|---|---|
| I-008 | 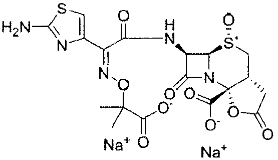 | 530 | 0.51 | A |
| I-009 | 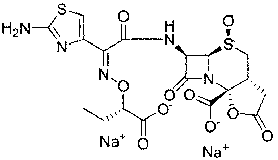 | 530 | 0.59 | A |
| I-010 | 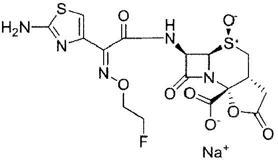 | 490 | 0.49 | A |
| I-011 | 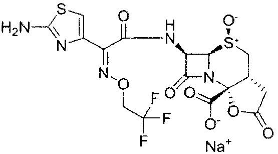 | 526 | 0.73 | A |

**[Table 3]**

| | | | | |
|---|---|---|---|---|
| I-012 | 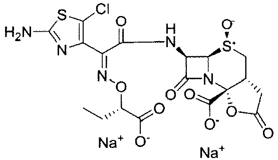 | 564 | 0.85 | A |
| I-013 | 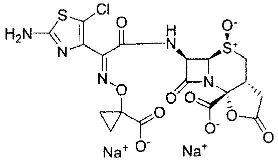 | 562 | 0.82 | A |
| I-014 | 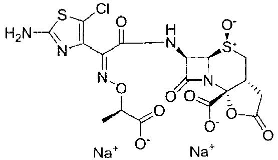 | 550 | 0.77 | A |
| I-015 | 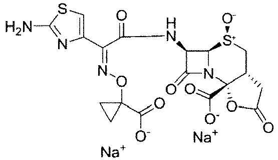 | 528 | 0.58 | A |

**[Table 4]**

| | | | | |
|---|---|---|---|---|
| I-016 | 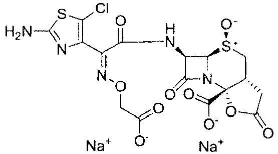 | 536 | 0.62 | A |
| I-017 | 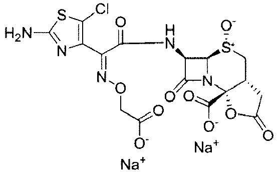 | 536 | 0.55 | A |
| I-018 | 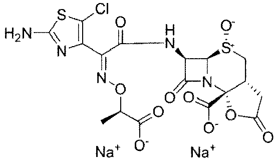 | 550 | 0.65 | A |
| I-019 | 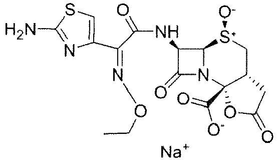 | 472 | 0.58 | B |

**[Table 5]**

| | | | | |
|---|---|---|---|---|
| I-020 | 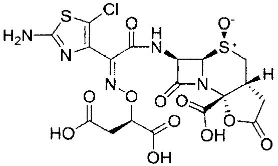 | 594 | 0.60 | A |
| I-021 | 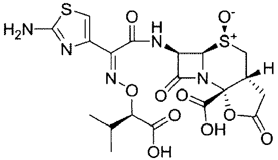 | 544 | 0.74 | A |
| I-022 | 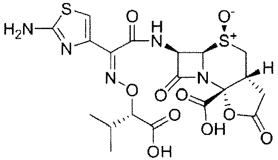 | 544 | 0.72 | A |
| I-023 | 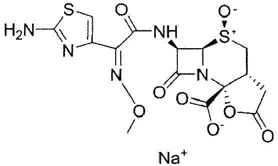 | 458 | 0.44 | A |

**[Table 6]**

| | | | | |
|---|---|---|---|---|
| I-024 | 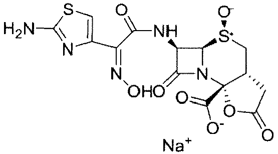 | 444 | 0.37 | B |
| I-025 | 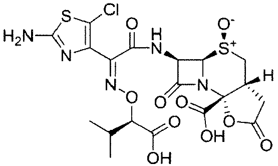 | 578 | 1.00 | A |
| I-026 | 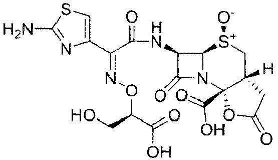 | 532 | 0.25 | A |

**[Table 7]**

| | | | | |
|---|---|---|---|---|
| I-027 | 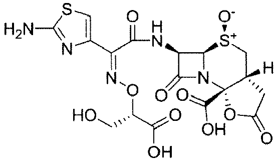 | 532 | 0.25 | A |
| I-028 | 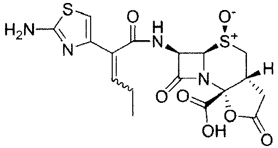 | 455 | 0.25 | A |
| I-029 | 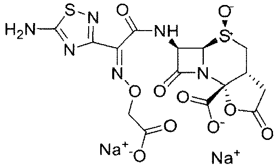 | 503 | 0.45 | B |

**[Table 8]**

| | | | | |
|---|---|---|---|---|
| I-030 | 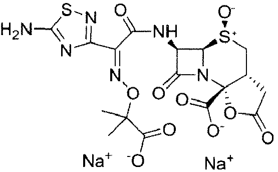 | 531 | 0.67 | B |
| I-031 | 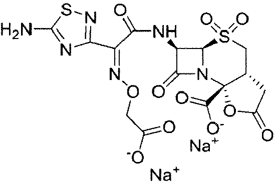 | 519 | 0.45 | B |
| I-032 | 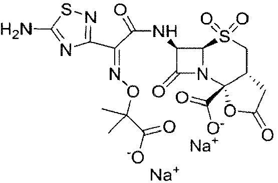 | 547 | 0.72 | B |
| I-033 | 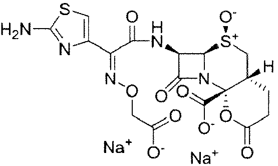 | 516 | 0.41 | A |

**[Table 9]**

| | | | | |
|---|---|---|---|---|
| I-034 | 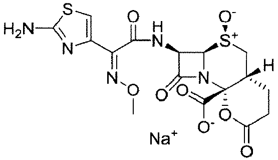 | 472 | 0.48 | A |
| I-035 | 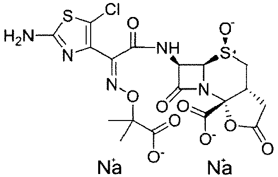 | 564 | 0.88 | A |

**[Table 10]**

| **Compound No.** | **NMR** | **Anal.** |
|---|---|---|
| I-004 | 1H-NMR (D2O) *δ* : 1.47 (3H, d, J = 6.9 Hz), 2.57 (1H, d, J = 18.2 Hz), 2.82 (1H, dd, J = 14.7, 12.1 Hz), 3.07 (1H, dd, J = 18.2, 7.6 Hz), 3.56 (1H, ddd, J = 12.1, 7.6, 5.1 Hz), 3.63 (1H, dd, J = 14.7, 5.1 Hz), 4.62 (1H, q, J = 6.9 Hz), 5.01 (1H, d, J = 4.8 Hz), 5.88 (1H, d, J = 4.8 Hz), 7.05 (1H, s). | C17H15N5O10S2Na1.8(H2O)4.9 |
| | | Calc. C : 31.75% H : 3.89% N : 10.89% S : 9.97% Na : 6.43% |
| | | Found C : 31.73% H : 3.84% N : 11.09% S : 9.94% Na : 6.26% |
| I-005 | ¹H-NMR (D₂O) *δ* : 1.58 (3H, d, *J* = 7.2 Hz), 2.56 (1H, d, *J* = 18.3 Hz), 2.83 (1H, dd, *J* = 14.6, 12.5 Hz), 3.07 (1H, dd, *J* = 18.3, 7.8 Hz), 3.51-3.57 (1H, m), 3.61-3.69 (1H, m), 4.96 (1H, q, *J* = 7.1 Hz), 5.02 (1H, d, *J* = 4.8 Hz), 5.91 (1H, t, *J* = 7.0 Hz), 7.24 (1H, s). | C17H17N5O10S2(H2O)2.5 |
| | | Calc. C: 36.43% H: 3.96% N: 12.49% S: 11.44% |
| | | Found C: 36.28% H: 3.93% N: 12.63% S: 11.64% |
| I-006 | ¹H-NMR (D₂O) *δ* : 2.56 (1H, d, *J* = 18.3 Hz), 2.82 (1H. dd, *J* = 14.6, 12.5 Hz), 3.05 (3H, ddd, *J* = 26.7, 14.6, 6.5 Hz), 3.54 (1H. dt, *J* = 14.3, 5.0 Hz), 3.63 (1H, dd, *J* = 14.7, 5.2 Hz), 4.99 (1H, d, *J* = 4.8 Hz), 5.11 (1H, dd, *J* = 8.4, 4.8 Hz), 5.85 (1H, d, *J* = 4.8 Hz), 7.24 (1H, s). | C18H17N5O12S2(H2O)2.6 |
| | | Calc. C: 35.66% H: 3.69% N: 11.55% S: 10.58% |
| | | Found C: 35.58% H: 3.74% N: 11.84% S: 10.59% |
| I-007 | 1H-NMR (D2O) *δ* : 1.49 (3H, s), 1.52 (3H, s), 2.57 (1H, d, J = 18.2 Hz), 2.79-2.86 (1H. m), 3.07 (1H, dd, J = 18.2, 7.6 Hz), 3.53-3.59 (1H, m), 3.63 (1H, dd, J = 14.5, 5.2 Hz), 5.02 (1H, d, J = 4.8 Hz), 5.90 (1H, d, J = 4.8 Hz), 7.02 (1H, s). | C18H17N5Na2O10S2(H2O)3.6 |
| | | Calc. C : 33.87% H : 3.82% N : 10.97% Na : 7.20% S : 10.05% |
| | | Found C : 33.65% H : 3.87% N : 11.25% Na : 6.96% S : 9.84% |

**[Table 11]**

| | | |
|---|---|---|
| I-008 | 1H-NMR (D2O) *δ* : 1.50 (3H, s), 1.52 (3H, s), 2.93 (1H, dd, J = 18.4, 3.8 Hz), 3.04 (1H, dd, J = 18.4, 8.1 Hz), 3.23 (1H, dd, J = 13.5, 10.4 Hz), 3.57-3.64 (1H, m), 3.72 (1H, dd, J = 13.5, 4.4 Hz), 5.11 (1H, d, J = 4.5 Hz), 5.67 (1H, d, J = 4.5 Hz), 6.98 (1H, s). | C18H17N5Na2O10S2(H2O)4.6 |
| | | Calc. C : 32.94% H : 4.02% N : 10.67% Na : 7.01% S : 9.77% |
| | | Found C : 32.89% H : 3.99% N : 10.94% Na : 76.97% S : 9.75% |
| I-009 | 1H-NMR (D2O) *δ* : 0.98 (3H, t, J = 7.5 Hz), 1.85-1.92 (2H, m), 2.57 (1H, d, J = 18.2 Hz), 2.79-2.86 (1H. m), 3.07 (1H, dd, J = 18.2, 7.6 Hz), 3.53-3.59 (1H, m), 3.63 (1H, dd, J = 14.7, 5.1 Hz), 4.55 (1H, t, J = 6.1 Hz), 5.02 (1H, d, J = 4.5 Hz), 5.92 (1H, d, J = 4.5 Hz), 7.04 (1H, s). | C18H17N5Na2O10S2(H2O)3.5(NaH CO3)0.2 |
| | | Calc. C : 33.46% H : 3.73% N : 10.72% Na : 7.74% S : 9.81 % |
| | | Found C : 33.31% H : 3.75% N : 10.92% Na : 7.87% S : 9.83% |
| I-010 | 1 H-NMR (D2O) *δ* : 2.57 (1H, d, J = 18.3 Hz), 2.83 (1H, dd, J = 14.6, 12.4 Hz), 3.07 (1H, dd, J = 18.3, 7.7 Hz), 3.52-3.59 (1H, m), 3.64 (1H, dd, J = 14.6, 5.2 Hz), 4.45-4.47 (1H, m), 4.53-4.55 (1H,m), 5.01 (1H, d, J = 4.8 Hz), 5.89 (1H, d, J = 4.8 Hz), 7.07 (1H, s). | C16H15FN5NaO8S2 (NaHCO3)0.5(H2O)3.8 |
| | | Calc. C : 31.87% H : 3.74% F : 3.05% N : 11.26% Na : 5.55% S : 10.31% |
| | | Found C : 31.74% H : 3.79% F : 3.07% N : 11.44% Na : 5.48% S : 10.54% |
| I-011 | 1H-NMR (D2O) *δ* : 2.57 (1H, d, J = 18.3 Hz), 2.83 (1H, dd, J = 14.6, 12.4 Hz), 3.07 (1H, dd, J = 18.3, 7.8 Hz), 3.52-3.58 (1H, m), 3.63 (1H, dd, J = 14.6, 5.3 Hz), 4.74 (2H, q, J = 8.7 Hz), 5.01 (1H, d, J = 4.8 Hz), 5.88 (1H, d, J = 4.8 Hz), 7.14 (1H, s). | C16H13F3N5NaO8S2(NaHCO3)0.2( H2O)4.4 |
| | | Calc. C : 30.24% H : 3.45% F : 8.86% N : 10.88% Na : 4.29% S : 9.96% |
| | | Found C : 30.13% H : 3.34% F : 8.87% N : 11.06% Na : 4.18% S : 10.12% |

**[Table 12]**

| | | |
|---|---|---|
| I-012 | 1H-NMR (D2O) *δ* : 0.99 (3H, t, J = 7.5 Hz), 1.86-1.93 (2H, m), 2.56 (1H, d, J = 18.2 Hz), 2.77-2.84 (1H, m), 3.07 (1H, dd, J = 18.2, 7.5 Hz), 3.53-3.65 (2H, m), 4.57 (1H, t, J = 6.1 Hz), 5.01 (1H, d, J = 4.8 Hz), 5.94 (1H. d, J = 4.8 Hz). | C18H16ClN5Na2O10S2(H2O)4.2(N aHCO3)0.1 |
| | | Calc. C : 31.42% H : 3.57% Cl : 5.12% N : 10.12% Na : 6.98% S : 9.27% |
| | | Found C : 31.25% H : 3.50% Cl : 5.40% N : 10.28% Na : 6.89% S : 9.37% |
| I-013 | 1H-NMR (D2O) *δ* : 1.28-1.41 (4H, m), 2.56 (1H, d, J = 18.2 Hz), 2.77-2.84 (1H, m), 3.07 (1H, dd, J = 18.2, 7.6 Hz), 3.53-3.65 (2H, m), 4.99 (1H, d, J = 4.8 Hz), 5.88 (1H, d, J = 4.8 Hz). | C18H14ClN5Na2O10S2(H2O)5.6 |
| | | Measured: C : 30.59% H : 3.59% Cl : 5.02% N : 9.91% Na : 6.51% S : 9.07% |
| | | Found : C : 30.37% H : 3.43% Cl : 5.95% N : 9.99% Na : 6.53% S : 8.98% |
| I-014 | 1H-NMR (D2O) *δ* : 1.49 (3H, d, J = 6.8 Hz), 2.57 (1H, d, J = 18.2 Hz), 2.77-2.84 (1H, m), 3.07 (1H, dd, J = 18.2, 7.7 Hz), 3.53-3.66 (2H, m), 4.64 (1H, q, J = 6.8 Hz), 5.01 (1H, d, J = 4.8 Hz), 5.90 (1H, d, J = 4.8 Hz). | C17H14ClN5Na2O10S2 (H2O)3.2(NaHCO3)0.1 |
| | | Calc. : C : 31.12% H : 3.13% Cl : 5.37% N : 10.61% Na : 7.32% S : 9.72% |
| | | Measured: C : 30.96% H : 3.28% Cl : 5.41% N : 10.98% Na : 7.25% S : 9.79% |
| I-015 | 1H-NMR (D2O) *δ* : 1.26-1.42 (4H, m), 2.57 (1H, d, J = 18.4 Hz), 2.79-2.85 (1H, m), 3.07 (1H, dd, J = 18.4, 7.6 Hz), 3.66-3.52 (2H, m), 5.00 (1H, d, J = 4.8 Hz), 5.86 (1H, d, J = 4.8 Hz), 7.08 (1H, s). | C18H15N5Na2O10S2(H2O)5.1 |
| | | Calc. : C : 32.59% H : 3.83% N : 10.56% Na : 6.93% S : 9.67% |
| | | Found : C : 32.49% H : 3.80% N : 10.74% Na : 7.08% S : 9.58% |

**[Table 13]**

| | | |
|---|---|---|
| I-016 | 1 H-NMR (D2O) *δ* : 2.57 (1H, d, J = 18.2 Hz), 2.77-2.84 (1H, m), 3.07 (1H, dd, J = 18.2, 7.6 Hz), 3.59 (2H, d, J = 50.5 Hz), 4.60 (2H, s), 5.00 (1H, d, J = 4.5 Hz), 5.91 (1H, d, J = 4.5 Hz). | C16H12ClN5Na2O10S2(H2O)3.2(N aHCO3)0.1 |
| | | Calc. : C : 29.94% H : 2.89%Cl : 5.49% N : 10.84% Na : 7.47% S : 9.93% |
| | | Measured: C : 29.92% H : 2.95% Cl : 5.50% N : 10.94% Na : 7.53% S : 9.92% |
| I-017 | 1 H-NMR (D2O) *δ* : 2.79 (1H, d, J = 18.7 Hz), 3.03 (1H, dd, J = 18.7, 8.3 Hz), 3.13-3.19 (1H, m), 3.56-3.62 (1H, m), 3.71 (1H, dd, J = 13.0, 4.7 Hz), 4.62 (2H, s), 5.09 (1H, d, J = 4.3 Hz), 5.74 (1H, d, J = 4.3 Hz). | |
| I-018 | 1 H-NMR (D2O) *δ* : 1.51 (3H, d, J = 6.8 Hz), 2.82 (1H, dd, J = 18.4, 2.3 Hz), 3.04 (1H, dd, J = 18.4, 8.2 Hz), 3.14-3.20 (1H, m), 3.56-3.64 (1H, m), 3.71 (1H, dd, J = 13.1, 4.5 Hz), 4.69 (1H, q, J = 6.8 Hz), 5.10 (1H, d, J = 4.3 Hz), 5.69 (1H. d, J = 4.3 Hz). | |
| I-019 | 1H-NMR (D2O) *δ* : 1.33 (3H, t, J = 7.1 Hz), 2.57 (1H, d, J = 18.3 Hz), 2.83 (1H, dd, J = 14.7, 12.4 Hz), 3.07 (1H, dd, J = 18.3, 7.8 Hz), 3.55 (1H, ddd, J = 12.4, 7.8, 5.3 Hz), 3.64 (1H, dd, J = 14.7, 5.3 Hz), 4.29 (2H, m), 5.01 (1H, d, J = 4.8 Hz), 5.88 (1H, d, J = 4.8 Hz), 7.03 (1H, s). | C16H16N5O8S2Na1(H2O)4.0 |
| | | Calc. C : 33.98% H : 4.28% N : 12.38% S : 11.34% Na : 4.07% |
| | | Found C : 34.03% H : 4.20% N : 12.51% S : 11.52% Na : 4.23% |

**[Table 14]**

| | | |
|---|---|---|
| I-020 | ¹H-NMR (D₂O) *δ* : 2.55 (1H, d, *J* = 18.4. Hz), 2.79 (1H, t, *J* = 13.4 Hz), 3.03 (3H, ddd, *J* = 26.7, 14.3, 6.7 Hz), 3.51-3.57 (1H, m), 3.62 (1H, dd, *J* = 14.8, 5.2 Hz), 4.98 (1H, d, *J* = 4.8 Hz), 5.09 (1H, dd, *J* = 8.2, 4.4 Hz), 5.87 (1H, d, *J* = 4.5 Hz). | C18H16ClN5O12S2(H2O)3.2 |
| | | Calc. C: 33.18% H: 3.47% Cl: 5.44% N: 10.75% S: 9.84% |
| | | Found C: 33.13% H: 3.48% Cl: 5.49% N: 10.98% S: 9.77% |
| I-021 | ¹H-NMR (D₂O) *δ* : 1.01 (6H, dd, *J* = 6.6, 3.5 Hz). 2.19-2.26 (1H, m), 2.55 (1H, d, *J* = 18.2 Hz), 2.82 (1H, t, *J* = 13.5 Hz), 3.06 (1H, dd, *J* = 18.2, 7.8 Hz), 3.50-3.57 (1H, m), 3.63 (1H, dd, *J* = 14.9, 5.1 Hz), 4.51 (1H, d, *J* = 6.1 Hz), 5.01 (1H, d, *J* = 4.5 Hz), 5.87 (1H, d, *J* = 4.8 Hz), 7.21 (1H, s). | C19H21N5O10S2(H2O)3.1 |
| | | Calc. C: 38.07% H: 4.57% N: 11.68% S: 10.70% |
| | | Found C: 38.01% H: 4.48% N: 11.92% S: 10.70% |
| I-022 | ¹H-NMR (D₂O) *δ* : 1.01 (6H, t, *J* = 6.2 Hz), 2.19 (1H, dd, *J* = 13.0, 6.9 Hz), 2.55 (1H, d, *J* = 18.4 Hz), 2.81 (1H, t, *J* = 13.6 Hz), 3.06 (1H, dd, *J* = 18.3, 7.7 Hz), 3.50-3.57 (1H, m), 3.62 (1H, dd, *J* = 14.7, 5.1 Hz), 4.46 (1H, d, *J* = 5.6 Hz), 5.01 (1H, d, *J* = 4.5 Hz), 5.90 (1H, d, *J* = 5.1 Hz), 7.13 (1H, s). | C19H21N5O10S2(H2O)3.2 |
| | | Calc. C: 37.96% H: 4.59% N: 11.65% S: 10.67% |
| | | Found C: 37.90% H: 4.44% N: 11.87% S: 10.67% |
| I-023 | 1H-NMR (D2O) *δ* : 2.57 (1H, d, J = 18.2 Hz), 2.83 (1H, dd, J = 14.7, 12.6 Hz), 3.07 (1H, dd, J = 18.2, 7.6 Hz), 3.55 (1H, m), 3.64 (1H, m), 4.02 (3H, s), 5.00 (1H, d, J = 4.5 Hz), 5.86 (1H, d, J = 4.5 Hz), 7.04 (1H, s). | |

**[Table 15]**

| | | |
|---|---|---|
| I-024 | 1H-NMR (D2O) *δ* : 2.57 (1H, d, J = 18.2 Hz), 2.83 (1H, dd, J = 14.9, 12.4 Hz), 3.07 (1H, dd, J = 18.2, 7.6 Hz), 3.55 (1H, ddd, J = 12.4, 7.6, 4.8 Hz), 3.64 (1H, dd, J = 14.9, 4.8 Hz), 5.01 (1H, d, J = 4.5 Hz), 5.90 (1H, d, J = 4.5 Hz), 7.02 (1H, s). | C14H12N5O8S2Na1(H2O)3.7 |
| | | Calc. C : 31.61% H : 3.68% N : 13.16% S : 12.05% Na : 4.32% |
| | | Found C : 31.77% H : 3.52% N : 13.38% S : 11.86% Na : 4.39% |
| I-025 | ¹H-NMR (D₂O) *δ* : 1.02 (6H, t, *J* = 7.3 Hz), 2.23 (1H, td, *J* = 13.2, 6.6 Hz), 2.55 (1H, d, *J* = 18.3 Hz), 2.80 (1H, dd, *J* = 14.4, 12.5 Hz), 3.06 (1H, dd, *J* = 18.4, 7.7 Hz), 3.51-3.57 (1H, m), 3.62 (1H, dd, *J* = 14.6, 5.2 Hz), 4.53 (1H, d, *J* = 6.0 Hz), 5.00 (1H, d, *J* = 4.8 Hz), 5.91 (1H, d, *J* = 4.8 Hz). | C19H20ClN5O10S2(H2O)2.7 |
| | | Calc. C: 36.42% H: 4.09% Cl: 5.66% N: 11.18% S: 10.23% |
| | | Found C: 36.35% H: 4.04% Cl: 5.62% N: 11.40% S: 10.22% |
| I-026 | ¹H-NMR (D₂O) *δ* : 2.55 (1H, d, *J* = 18.3 Hz), 2.82 (1H, dd, *J* = 14.7, 12.5 Hz), 3.06 (2H, dd, *J* = 18.3, 7.7 Hz), 3.51-3.57 (1H, m), 3.64 (1H, dd, *J* = 14.7, 5.3 Hz), 4.01 (2H, ddd, *J* = 21.7, 12.8, 5.0 Hz), 4.81-4.82 (1H, m), 5.02 (1H, d, *J* = 4.8 Hz), 5.88 (1H, d, *J* = 4.9 Hz), 7.17 (1H, d, *J* = 5.9 Hz). | C17H17N5O11S2(H2O)3.3 |
| | | Calc. C: 34.55% H: 4.03% N: 11.85% S: 10.85% |
| | | Found C: 34.40% H: 3.95% N: 12.14% S: 10.84% |

**[Table 16]**

| | | |
|---|---|---|
| I-027 | ¹ H-NMR (D₂O) *δ* : 2.56 (1.0H, d, *J* = 18.2 Hz), 2.81-2.84 (1.2H, m), 3.00-3.09 (1.2H, m), 3.22 (0.2H, dd, *J* = 12.0, 6.0 Hz), 3.51-3.57 (1.2H, m), 3.65 (1.0H, dd, *J* = 14.9, 5.1 Hz), 3.72 (0.2H, dd, *J* = 7.1, 3.5 Hz), 4.04-4.08 (2.4H, m), 4.94-4.95 (1.2H, m), 5.02 (1.0H, d, *J* = 4.5 Hz), 5.13 (0.2H, d, *J* = 4.3 Hz), 5.71 (0.2H, d, *J* = 4.3 Hz), 5.91 (1.0H, d, *J* = 4.8 Hz), 6.80 (0.2H, s), 7.19 (0.2H, s), 7.23 (1.0H, s). | C17H17N5O11S2(H2O)2.6 |
| | | Calc. C: 35.31% H: 3.87% N: 12.11% S: 11.09% |
| | | Found C: 35.46% H: 3.94% N: 12.09% S: 10.68% |
| I-028 | ¹H-NMR (D₂O) *δ* : 1.01-1.09 (4.05H, m), 2.24-2.31 (2.70H, m), 2.53-2.58 (1.35H, m), 2.81-2.84 (1.35H, m), 3.06 (1.35H, dd, *J* = 18.1, 7.5 Hz), 3.49-3.56 (1.35H, m), 3.63 (1.35H, dd, *J* = 14.8, 4.7 Hz), 4.94 (0.35H, d, *J* = 5.1 Hz), 4.98 (1.00H, d, *J* = 4.5 Hz), 5.83 (1.35H, d, *J* = 4.0 Hz), 6.39 (1.00H, t, *J* = 7.7 Hz), 6.60 (1.00H, s), 6.76 (0.35H, s), 7.07 (0.35H, t, *J* = 7.8 Hz). | C17H18N4O7S2(H2O)2.4 |
| | | Calc. C: 41.03% H: 4.62% N: 11.26% S: 12.88% |
| | | Found C: 41.28% H: 4.53% N: 11.48% S: 12.62% |
| I-029 | 1H-NMR (D2O) *δ* : 2.57 (1H, d, J = 18.2 Hz), 2.81 (1H, dd, J = 14.4, 12.4 Hz), 3.06 (1H, dd, J = 18.2, 7.6 Hz), 3.55 (1H, ddd, J = 12.4, 7.6, 5.1 Hz), 3.62 (1H, dd, J = 14.4, 5.1 Hz), 4.67 (2H, s), 5.01 (1H, d, J = 4.5 Hz), 5.93 (1H, d, J = 4.5 Hz). | C15H12N6O10S2Na2(H2O)3.1 |
| | | Calc. C : 29.92% H : 3.05% N : 13.95% S : 10.65% Na : 7.63% |
| | | Found C : 29.90% H : 3.00% N : 14.10% S : 10.46% Na : 7.64% |

**[Table 17]**

| | | |
|---|---|---|
| I-030 | 1H-NMR (D2O) *δ* : 1.53 (3H, s), 1.56 (3H, s), 2.56 (1H, d, J = 18.4 Hz), 2.81 (1H, dd, J = 14.7, 12.1 Hz), 3.06 (1H, dd, J = 18.4, 7.6 Hz), 3.55 (1H, ddd, J = 12.1, 7.6, 5.1 Hz), 3.62 (1H, dd, J = 14.7, 5.1 Hz), 5.01 (1H, d, J = 4.7 Hz), 5.92 (1H, d, J = 4.7 Hz). | C17H16N6O10S2Na1.8(H2O)4.7 |
| | | Calc. C : 31.20% H : 3.91% N : 12.84% S : 9.80% Na : 6.32% |
| | | Found C : 31.28% H : 4.00% N : 12.87% S : 9.64% Na : 6.29% |
| I-031 | 1 H-NMR (D2O) *δ* : 2.54 (1H, d, J = 18.4 Hz), 2.99 (1H, dd, J = 18.4, 7.6 Hz), 3.50 (1H, dd, J = 15.4, 12.1 Hz), 3.60 (1H, dd, J = 15.4, 5.6 Hz), 3.86 (1H, ddd, J = 12.1, 7.6, 5.6 Hz), 4.66 (2H, s), 5.37 (1H, d, J = 4.8 Hz), 5.98 (1H, d, J = 4.8 Hz). | |
| I-032 | 1H-NMR (D2O) *δ* : 1.50 (3H, s), 1.51 (3H, s), 2.56 (1H, d, J = 18.4 Hz), 3.01 (1H, dd, J = 18.4, 7.6 Hz), 3.52 (1H, dd, J = 15.2, 12.4 Hz), 3.62 (1H, dd, J = 15.2, 5.6 Hz), 3.87 (1H, ddd, J = 12.4, 7.6, 5.6 Hz), 5.37 (1H, d, J = 4.8 Hz), 5.98 (1H, d, J = 4.8 Hz). | C17H16N6O11S2Na1.7(H2O)4.1 |
| | | Calc. C : 31.06% H : 3.71% N : 12.78% S : 9.75% Na : 5.94% |
| | | Found C : 31.10% H : 3.76% N : 12.87% S : 9.51% Na : 5.83% |
| I-033 | ¹H-NMR (D₂O) *δ* : 1.79 (1H, tt, *J* = 8.5, 3.8 Hz), 2.18 (1H tt, *J* = 13.9, 4.7 Hz), 2.64 (2H, dd, *J* = 9.5, 5.5 Hz), 3.12 (1H, dd, *J* = 26.2, 11.9 Hz), 3.30-3.41 (2H, m), 4.59 (2H, dt, *J* = 17.8, 8.1 Hz), 5.01 (1H, d, *J* = 4.8 Hz), 5.83 (1H, d, *J* = 4.8 Hz), 7.07 (1H, t, *J* = 5.3 Hz). | C17H15N5Na2O10S2(NaHCO3)0.1( H2O)3.1 |
| | | Calc. C: 32.93% H: 3.44% N: 11.23% S: 10.28% Na: 7.74% |
| | | Found C: 33.05% H: 3.47% N: 11.43% S: 9.99% Na: 7.70% |

**[Table 18]**

| | | |
|---|---|---|
| I-034 | ¹H-NMR (D₂O) *δ* : 1.79 (1H, tt, *J* = 8.5, 3.8 Hz), 2.18 (1H, tt, *J* = 13.9, 4.7 Hz), 2.64 (2H, dd, *J* = 9.5, 5.5 Hz), 3.12 (1H, dd, *J* = 26.2. 11.9 Hz), 3.30-3.41 (2H, m), 4.59 (2H, dt, *J* = 17.8, 8.1 Hz), 5.01 (1H. d, *J* = 4.8 Hz), 5.83 (1H, d, *J* = 4.8 Hz), 7.07 (1H, t, *J* = 5.3 Hz). | C16H16N5NaO8S2(NaHCO3)0.2(H2 O)4 |
| | | Calc. C: 33.42% H: 4.19% N: 12.03% S: 11.01% Na: 4.74% |
| | | Found C: 33.36% H: 4.14% N: 12.12% S: 10.93% Na: 4.72% |
| I-035 | 1H-NMR (D2O) *δ* : 1.50 (3H, s), 1.53 (3H, s), 2.57 (1H, d, J = 18.2 Hz), 2.82 (1H, dd, J = 14.4, 12.4 Hz), 3.07 (1H, dd, J = 18.2, 7.6 Hz), 3.53-3.66 (2H, m), 5.01 (1H, d, J = 4.8 Hz), 5.91 (1H, d, J = 4.8 Hz). | C18H16ClN5Na2O10S2(H2O)4.4 |
| | | Calc.: C : 31.46% H : 3.64% Cl : 5.16% N : 10.19% Na : 6.69% S : 9.33% |
| | | Found : C : 31.40% H : 3.67% Cl : 5.16% N : 10.26% Na : 7.04% S : 9.03% |

**[Table 19]**

| Compound No. | Structure | [M+H] | Retention time (min.) | LCMS measurement condition |
|---|---|---|---|---|
| I-068 | 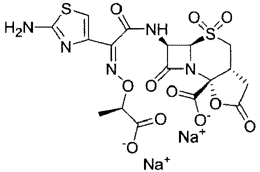 | 532 | 0.55 | B |
| I-069 | 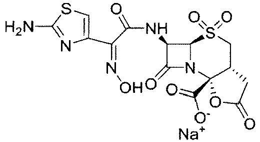 | 460 | 0.37 | B |
| I-070 | 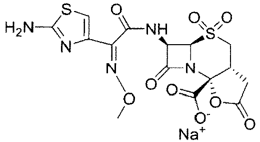 | 474 | 0.47 | B |
| I-071 | 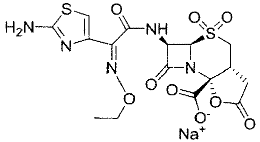 | 488 | 0.60 | B |

**[Table 20]**

| | | | | |
|---|---|---|---|---|
| I-072 | 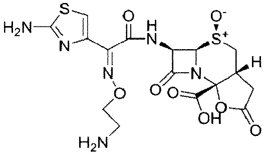 | 487 | 0.33 | A |
| I-073 | 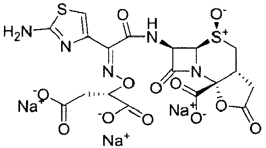 | 560 | 0.32 | A |

**[Table 21]**

| Compound No. | NMR | Anal. |
|---|---|---|
| I-068 | 1 H-NMR (D₂O) δ: 1.44 (3H, d, J = 6.8 Hz), 2.57 (1H, d, J = 18.4 Hz), 3.01 (1H, dd, J = 18.4, 7.6 Hz), 3.53 (1H, dd, J = 14.9, 12.6 Hz), 3.64 (1H, dd, J = 14.9, 5.3 Hz), 3.88 (1H, ddd, J = 12.6, 7.6, 5.3 Hz), 4.62 (1H, q, J = 6.8 Hz), 5.38 (1H, d, J = 4.4 Hz), 5.90 (1H, d, J = 4.4 Hz), 7.01 (1H, s). | |
| I-069 | 1H-NMR (D2O) δ: 2.57 (1H, d, J = 18.4 Hz), 3.01 (1H, dd, J = 18.4, 7.8 Hz), 3.54 (1H, dd, J = 15.2, 12.4 Hz), 3.64 (1H, dd, J = 15.2, 5.3 Hz), 3.87 (1H, ddd, J = 12.4, 7.8, 5.3 Hz), 5.40 (1H, d, J = 4.8 Hz), 5.94 (1H, d, J = 4.8 Hz), 6.98 (1H, s). | C14H12.1N5O9S2Na0.9(H2O)3.3 |
| | | Calc. C : 31.22%, H : 3.50%, N : 13.00%, S : 11.90%, Na : 3.84%. Found C : 31.37 %, H : 3.62%, N : 12.96%, S : 11.72%, Na : 3.82%. |
| I-070 | 1H-NMR (D2O) δ: 2.57 (1H, d, J = 18.4 Hz), 3.01 (1H, dd, J = 18.4, 7.8 Hz), 3.54 (1H, dd, J = 15.4, 12.1 Hz), 3.64 (1H, dd, J = 15.4, 5.6 Hz), 3.88 (1H, ddd, J = 12.1, 7.8, 5.6 Hz), 3.97 (3H, s), 5.39 (1H, d, J = 4.8 Hz), 5.91 (1H, d, J = 4.8 Hz), 7.01 (1H, s). | |
| I-071 | 1H-NMR (D2O) δ: 1.30 (3H, t, J = 7.1 Hz), 2.57 (1H, d, J = 18.4 Hz), 3.01 (1H, dd, J = 18.4, 7.8 Hz), 3.54 (1H, dd, J = 15.2, 12.4 Hz), 3.64 (1H, dd, J = 15.2, 5.6 Hz), 3.87 (1H, ddd, J = 12.4, 7.8, 5.6 Hz), 4.25 (2H, q, J = 7.1 Hz), 5.38 (1H, d, J = 4.8 Hz), 5.92 (1H, d, J = 4.8 Hz), 6.99 (1H, s). | C16H16.2N5O9S2Na0.8(H2O)3.5( NaHCO3)0.2 |
| | | Calc. C : 33.27%, H : 4.03%, N : 11.97%, S : 10.96%, Na : 3.93%. Found C : 33.52 %, H : 4.14%, N : 12.20%, S : 10.71%, Na : 3.77%. |

**[Table 22]**

| | | |
|---|---|---|
| I-072 | 1H-NMR (D2O) δ: 7.09 (s, 1H), 5.88 (d, J = 4.8 Hz, 1H), 5.02 (d, J = 4.5 Hz, 1H), 4.56-4.43 (m, 2H), 3.65 (dd, J = 14.8, 5.2 Hz, 1H), 3.58-3.52 (m, 1H), 3.48-3.37 (m, 2H), 3.08 (dd, J = 18.2, 7.8 Hz, 1H), 2.84 (dd, J = 14.5, 12.8 Hz, 1H), 2.57 (d, J = 18.2 Hz, 1H). | C16H18N6O8S2(H2O)3.4 |
| | | Calc. : C : 35.09% ,H : 4.56%,N : 15.34%, S : 11.71% |
| | | Found : C : 35.06% ,H : 4.51%,N : 15.46% , S : 11.79% |
| I-073 | 1H-NMR (D2O) δ: 2.57 (1H, d, J = 18.3 Hz), 2.66 (1H, dd, J = 16.1, 9.9 Hz), 2.74-2.84 (2H, m), 3.06 (1H, dd, J = 18.3, 7.7 Hz), 3.52-3.58 (1H, m), 3.63 (1H, dd, J = 14.6, 5.3 Hz), 4.96 (1H, dd, J = 10.0, 3.6 Hz), 5.00 (1H, d, J = 4.8 Hz), 5.89 (1H, d, J = 4.8 Hz), 7.05 (1H, s). | C18H 14N5Na2.7O12S2(H2O)4.6 |
| | | Calc. : C,30.82; H,3.33; N,9.99; Na,8.85; S,9.14 (%) |
| | | Found : C,30.77; H,3.34; N,10.20; Na,8.99; S,8.98 (%) |

### Test Example 1

The *in vitro* antibacterial activity of the compounds of the present invention was confirmed.

### (Methodology)

The minimum inhibitory concentration (MIC) was measured by the microbroth dilution method in accordance with the procedures recommended by CLSI (Clinical and Laboratory Standards Institute) using 1 × 105 CFU/mL as an inoculum dose and a cation-adjusted Mueller-Hinton broth as a test medium.

The strains used are as described in the table below.

**[Table 23]**

| No. | Strain name | Name | Produced enzyme | Property |
|---|---|---|---|---|
| 1 | E.cloacae | ATCC13047 | AmpC | Ceftazidime-resistant, cefepime-sensitive |
| 2 | K.pneumoniae | SR01358 | KPC-2 | KPC-producing strain |
| 3 | K.pneumoniae | SR01453 | NDM-1 | MBL-producing strain |
| | | | | Car bapenem-resistant |
| 4 | K.pneumoniae | NCTC13443 | NDM-1 | MBL-producing strain |
| | | | | Carbapenem-resistant |
| 5 | E.cloacae | SR36276 | AmpC | Highly AmpC-producing strain |
| | | | | Ceftazidime-resistant, cefepime-sensitive |
| 6 | E.coli | SR09613 | CMY-2 type | Plasmid AmpC-producing strain |
| | | | | Ceftazidime-resistant, cefepime-sensitive |
| 7 | E.coli | SR09616 | CMY-2 type | Plasmid AmpC-producing strain |
| | | | | Ceftazidime-resistant, cefepime-sensitive |

### (Results)

The test results are described below. In the tables, the unit for the numerical value of the inhibitory activity is µg/mL.

**[Table 24]**

| Compound No. | Strain No. | | | | |
|---|---|---|---|---|---|
| | No.1 | No.2 | No.3 | No.4 | No.5 |
| I-001 | 0.125 | 0.25 | 0.063 | 1 | 0.063 |
| I-003 | 0.25 | 0.5 | 0.25 | 4 | 1 |
| I-004 | 0.063 | 0.25 | 0.25 | 1 | 0.125 |
| I-005 | 0.5 | 0.5 | 0.125 | 1 | 0.125 |
| I-007 | 0.25 | 0.5 | 0.5 | 2 | 0.25 |
| I-009 | 0.5 | | 0.5 | 4 | 0.25 |
| I-010 | 0.25 | | 0.25 | 8 | 1 |
| I-015 | 0.125 | 0.5 | 0.125 | 1 | 0.125 |
| I-019 | 0.5 | | 0.5 | 8 | 1 |
| I-022 | | | 1 | | 0.5 |
| I-023 | 0.25 | 1 | 0.25 | 4 | 1 |
| I-026 | 0.125 | 0.5 | 0.125 | 1 | 0.125 |
| I-027 | 0.063 | 0.25 | 0.063 | 1 | ≤0.031 |
| I-029 | 1 | | | | 1 |
| I-031 | 0.5 | 1 | | | |

**[Table 25]**

| Compound No. | Strain No. | | | | | | |
|---|---|---|---|---|---|---|---|
| | No.1 | No.2 | No.3 | No.4 | No.5 | No.6 | No.7 |
| I-024 | | | 2 | | 4 | | |
| I-036 | 0.5 | | 1 | 8 | 1 | | |
| I-037 | 0.5 | 1 | 0.25 | 8 | 0.5 | | |
| I-039 | 0.5 | | 0.125 | 4 | 2 | 0.25 | 1 |
| I-040 | | | | | | 4 | |
| I-041 | 1 | | 0.5 | | | 0.5 | 2 |
| I-042 | | | 2 | | | 4 | 4 |
| I-043 | | | 2 | | | 2 | |
| I-044 | 0.5 | | 1 | 2 | 1 | 0.25 | 1 |
| I-045 | 0.5 | | 0.25 | 8 | 2 | 0.25 | |
| I-046 | 1 | | 0.5 | | 0.5 | | |
| I-047 | 0.25 | | 0.25 | 2 | 0.25 | 0.063 | 0.25 |
| I-048 | 0.25 | | 0.125 | 4 | 1 | 0.125 | 0.5 |
| I-049 | 0.125 | | 0.063 | 2 | 0.5 | 0.063 | 0.5 |
| I-050 | 0.5 | | 0.5 | 4 | 1 | 0.5 | 4 |
| I-052 | 0.063 | | ≦0.031 | 0.5 | 0.125 | ≦0.031 | 0.063 |
| I-053 | 0.125 | 0.5 | 0.125 | 2 | 0.125 | | |
| I-054 | 1 | | 0.5 | | 4 | 0.5 | |
| I-055 | 2 | | 1 | | | 1 | 2 |
| I-056 | 0.5 | | 0.5 | | 2 | 0.5 | 2 |
| I-057 | 1 | | 1 | | 1 | 1 | 2 |
| I-059 | 0.063 | 0.5 | 0.063 | 1 | 0.25 | | |
| I-060 | 0.125 | 0.5 | 0.125 | 4 | 0.5 | | |
| I-061 | 0.125 | 0.5 | 0.25 | 4 | 0.25 | | |
| I-062 | 0.25 | | 0.5 | | 0.5 | 0.125 | 0.25 |
| I-063 | | | | | | 4 | |
| I-064 | | | 2 | | | | |
| I-065 | 0.5 | | 0.25 | 8 | 4 | 0.5 | 0.5 |
| I-066 | | | | | | | |
| I-067 | 1 | | 2 | | 2 | | |
| I-068 | 1 | | 1 | 8 | 1 | | |
| I-070 | 1 | 1 | 0.25 | 4 | 4 | | |
| I-071 | 2 | | 1 | 8 | 4 | | |
| I-072 | | | | | | 4 | 4 |
| I-073 | 1 | | 2 | | 1 | 2 | 4 |

As seen from these results, the compounds represented by formula (I) have a wide antibacterial spectrum and exhibit a strong antibacterial spectrum against, particularly, gram-negative bacteria, and/or exhibit a strong antibacterial activity against multidrug-resistant bacteria, particularly, class B metallo-β-lactamase-producing gram-negative bacteria, and/or exhibit a strong antibacterial activity against gram-negative bacteria producing class A β-lactamases such as KPC. Furthermore, the compounds also have a strong antibacterial activity against class C β-lactamase-producing gram-negative bacteria. Moreover, the compounds are found to be effective even for multidrug-resistant bacteria including carbapenemase-resistant bacteria and to have high stability even against β-lactamase-producing gram-negative bacteria.

### Test Example 2: CYP inhibition test

Using commercially available pooled human hepatic microsome, and employing, as markers, 7-ethoxyresorufin O-deethylation (CYP1A2), tolbutamide methyl-hydroxylation (CYP2C9), mephenytoin 4'-hydroxylation (CYP2C19), dextromethorphan O-demethylation (CYP2D6), and terfenedine hydroxylation as typical substrate metabolism reactions of human main five CYP enzyme forms (CYP1A2, 2C9, 2C19, 2D6, 3A4), an inhibitory degree of each metabolite production amount by the compound of the present invention was assessed.

The reaction conditions are as follows: substrate, 0.5 µmol/L ethoxyresorufin (CYP1A2), 100 µmol/L tolbutamide (CYP2C9), 50 µmol/L S-mephenitoin (CYP2C19), 5 µmol/L dextromethorphan (CYP2D6), 1 µmοl/L terfenedine (CYP3A4); reaction time, 15 minutes; reaction temperature, 37°C; enzyme, pooled human hepatic microsome 0.2 mg protein/mL; concentration of the compound of the present invention, 1.0, 5.0, 10, 20 µmol/L (four points).

Each five kinds of substrates, human hepatic microsome, or the compound of the present invention in 50 mmol/L Hepes buffer as a reaction solution was added to a 96-well plate at the composition as described above, NADPH, as a cofactor was added to initiate metabolism reactions as markers and, after the incubation at 37°C for 15 minutes, a methanol/acetonitrile = 1/1 (V/V) solution was added to stop the reaction. After the centrifugation at 3000 rpm for 15 minutes, resorufin (CYP1A2 metabolite) in the supernatant was quantified by a fluorescent multilabel counter and tributamide hydroxide (CYP2CP metabolite), mephenytoin 4' hydroxide (CYP2C19 metabolite), dextromethorphan (CYP2D6 metabolite), and terfenadine alcohol (CYP3A4 metabolite) were quantified by LC/MS/MS.

Addition of only DMSO being a solvent dissolving the compound of the present invention to a reaction system was adopted as a control (100%). Remaining activity (%) at each concentration of the compound of the present invention added to the solvent was calculated. IC₅₀ was calculated by reverse presumption by a logistic model using the concentration and an inhibition rate.

### Test Example 3: BA test

Materials and Methods for experiments to evaluate oral absorption
(1) Animals: the mice or SD rats were used.
(2) Breeding conditions: the mice or SD rats were allowed to freely take solid food and sterilized tap water.
(3) Dose and grouping: orally or intravenously administered at a predetermined dose; grouping was as follows (Dose depended on the compound)
   Oral administration: 1 to 30 mg/kg (n = 2 to 3)
   Intravenous administration: 0.5 to 10 mg/kg (n = 2 to 3)
(4) Preparation of dosing solution: for oral administration, in a solution or a suspension state; for intravenous administration, in a solubilized state
(5) Administration method: in oral administration, forcedly administer into ventriculus with oral probe; in intravenous administration, administer from caudal vein with a needle-equipped syringe
(6) Evaluation items: blood was collected over time, and the plasma concentration of the compound of the present invention was measured by LC/MS/MS.
(7) Statistical analysis: regarding the transition of the plasma concentration of the compound of the present invention, the area under the plasma concentration-time curve (AUC) was calculated by non-linear least squares program WinNonlin (Registered trade name), and the bioavailability (BA) of the compound of the present invention was calculated from the AUCs of the oral administration group and intravenous administration group.

### Test Example 4: Metabolism Stability Test

Using commercially available pooled human hepatic microsomes, the compound of the present invention was reacted for a constant time, a remaining rate was calculated by comparing a reacted sample and an unreacted sample, thereby, a degree of metabolism of the compound of the present invention in liver was assessed.

A reaction was performed (oxidative reaction) at 37°C for 0 minute or 30 minutes in the presence of 1 mmol/L NADPH in 0.2 mL of a buffer (50 mmol/L Tris-HCl pH 7.4, 150 mmol/L potassium chloride, 10 mmol/L magnesium chloride) containing 0.5 mg protein/mL of human liver microsomes. After the reaction, 50 µL of the reaction solution was added to 100 µL of a methanol/acetonitrile = 1/1 (v/v), mixed and centrifuged at 3000 rpm for 15 minutes. The compound of the present invention in the supernatant was quantified by LC/MS/MS, and a remaining amount of the compound of the present invention after the reaction was calculated, letting a compound amount at 0 minute reaction time to be 100%. Hydrolysis reaction is performed in the absence of NADPH, and glucuronidation reaction is performed in the presence of 5 mmol/L UDP-glucuronic acid instead of NADPH. Then, the same operation can be carried out.

### Test Example 5: CYP3A4 fluorescent MBI test

The CYP3A4 fluorescent MBI test is a test of investigating enhancement of CYP3A4 inhibition of the compound of the present invention by a metabolism reaction. The test was performed using, as an index, a reaction in which 7-benzyloxytrifluoromethylcoumarin (7-BFC) was debenzylated by CYP3A4 enzyme expressed in Escherichia coli and employing to produce a metabolite, 7-hydroxytrifluoromethylcoumarin (HFC) emitting fluorescent light.

The reaction conditions are as follows: substrate, 5.6 µmol/L 7-BFC; pre-reaction time, 0 or 30 minutes; reaction time, 15 minutes; reaction temperature, 25°C (room temperature); CYP3A4 content (expressed in *Escherichia coli*), at pre-reaction 62.5 pmol/mL, at reaction 6.25 pmol/mL (at 10-fold dilution); concentration of the compound of the present invention, 0.625, 1.25, 2.5, 5, 10, 20 µmol/L (six points).

An enzyme in K-Pi buffer (pH 7.4) and a solution of the compound of the present invention as a pre-reaction solution were added to a 96-well plate at the above composition of the pre-reaction. A part of pre-reaction solution was transferred to another 96-well plate so that it was 1/10 diluted with a substrate and K-Pi buffer. NADPH as a co-factor was added in order to initiate a reaction as an index (without preincubation). After a predetermined time of a reaction, acetonitrile/0.5 mol/L Tris (trishydroxyaminomethane) = 4/1 (V/V) was added to stop the reaction. On the other hand, NADPH was also added to a remaining preincubation solution in order to initiate a preincubation (with preincubation). After a predetermined time of a preincubation, a part was transferred to another plate so that it was 1/10 diluted with a substrate and K-Pi buffer to initiate a reaction as an index. After a predetermined time of a reaction, acetonitrile/0.5 mol/L Tris (trishydroxyaminomethane) = 4/1 (V/V) was added in order to stop the reaction. For the plate on which each index reaction had been performed, a fluorescent value of 7-HFC which was a metabolite was measured with a fluorescent plate reader. (Ex = 420 nm, Em = 535 nm).

Addition of only DMSO which was a solvent dissolving the compound of the present invention to a reaction system was adopted as a control (100 %), remaining activity (%) was calculated at each concentration of the compound of the present invention added as the solution. IC₅₀ was calculated by reverse-presumption by a logistic model using a concentration and an inhibition rate. When a difference between IC₅₀ values was 5 µmol/L or more, this was defined as (+). When the difference is 3 µmol/L or less, this was defined as (-).

### Test Example 6: Fluctuation Ames Test

Mutagenicity of the compound of the present invention was evaluated.

20 µL of freezing-stored rat typhoid bacillus (Salmonella typhimurium TA98 strain, TA100 strain) was inoculated on 10 mL of a liquid nutrient medium (2.5% Oxoid nutrient broth No.2), and this was cultured before shaking at 37°C for 10 hours. 9 mL of a bacterial solution of the TA98 strain was centrifuged (2000 × g, 10 minutes) to remove a culturing solution. The bacteria was suspended in 9 mL of a Micro F buffer (K₂HPO₄: 3.5 g/L, KH₂PO₄: 1 g/L, (NH4)₂SO₄: 1 g/L, trisodium citrate dehydrate: 0.25 g/L, MgSO₄ • 7H₂O: 0.1 g/L), the suspension was added to 110 mL of an Exposure medium (Micro F buffer containing Biotin: 8 µg/mL, histidine: 0.2 µg/mL, glucose: 8 mg/mL). The TA100 strain was added to 120 mL of the Exposure medium relative to 3.16 mL of the bacterial solution to prepare a test bacterial solution. Each 12 µL of DMSO solution of the compound of the present invention (several stage dilution from maximum dose 50 mg/mL at 2 to 3 fold ratio), DMSO as a negative control, and 50 µg/mL of 4-nitroquinoline-1-oxide DMSO solution for the TA98 strain, 0.25 µg/mL of 2-(2-furyl)-3-(5-nitro-2-furyl)acrylamide DMSO solution for the TA100 strain under the non-metabolism activating condition, 40 µg/mL of 2-aminoanthracene DMSO solution for the TA98 strain, 20 µg/mL of 2-aminoanthracene DMSO solution for the TA100 strain under the metabolism activating condition as a positive control, and 588 µL of the test bacterial solution (a mixed solution of 498 µl of the test bacterial solution and 90 µL of S9 mix under the metabolism activating condition) were mixed, and this was shaking-cultured at 37°C for 90 minutes. 460 µL of the bacterial solution exposed to the compound of the present invention was mixed with 2300 µL of an Indicator medium (Micro F buffer containing biotin: 8 µg/mL, histidine: 0.2 µg/mL, glucose: 8 mg/mL, Bromo Cresol Purple: 37.5 µg/mL), each 50 µL was dispensed into microplate 48 wells/dose, and this was subjected to stationary culturing at 37°C for 3 days. Since a well containing a bacterium which had obtained the proliferation ability by mutation of an amino acid (histidine) synthesizing enzyme gene turns from purple to yellow due to a pH change, the bacterium proliferation well which had turned to yellow in 48 wells per dose was counted, and was assessed by comparing with a negative control group. (-) means that mutagenicity is negative and (+) is positive.

### Test Example 7: hERG Test

For the purpose of assessing risk of an electrocardiogram QT interval prolongation of the compound of the present invention, effects of the compound of the present invention on delayed rectifier K+ current (I_{Kr}), which plays an important role in the ventricular repolarization process, was studied using HEK293 cells expressing human ether-a-go-go related gene (hERG) channel.

After a cell was retained at a membrane potential of -80 mV by whole cell patch clamp method using an automated patch clamp system (PatchXpress 7000A, Axon Instruments Inc.), I_{Kr} induced by depolarization pulse stimulation at +40 mV for 2 seconds and, further, repolarization pulse stimulation at -50 mV for 2 seconds, was recorded. After the generated current was stabilized, extracellular solution (NaCl: 135 mmol/L, KCl: 5.4 mmol/L, NaH₂PO₄: 0.3 mmol/L, CaCl₂·2H₂O: 1.8 mmol/L, MgCl₂·6H₂O: 1 mmol/L, glucose: 10 mmol/L, HEPES (4-(2-hydroxyethyl)-1-piperazineethanesulfonic acid): 10 mmol/L, pH = 7.4), in which the compound of the present invention had been dissolved at an objective concentration, was applied to the cell at room temperature for 10 minutes. From the recording I_{Kr}, an absolute value of the tail peak current was measured based on the current value at the resting membrane potential using analysis software (DataXpress ver. 1, Molecular Devices Corporation). Further, the % inhibition relative to the tail peak current before application of the compound of the present invention was calculated, and compared with the vehicle-applied group (0.1% dimethyl sulfoxide solution) to assess influence of the compound of the present invention on I_{Kr}.

### Test Example 8: Solubility test

The solubility of the compound of the present invention was determined under 1% DMSO addition conditions. 10 mmol/L solution of the compound was prepared with DMSO, and 6 µL of the solution of the compound of the present invention was added to 594 µL of pH 6.8 artificial intestinal juice (118 mL of 0.2 mol/L NaOH test solution and water were added to 250 mL of 0.2 mol/L potassium dihydrogen phosphate test solution to reach 1000 mL). The mixture was left standing for 16 hours at 25°C, and the mixture was vacuum-filtered. The filtrate was two-fold diluted with methanol/water = 1/1 (v/v), and the compound concentration in the filtrate was measured with HPLC or LC/MS/MS by the absolute calibration method.

### Test Example 9: Powder solubility test

Appropriate quantity of the compound of the present invention is put in suitable containers. 200 µL of JP-1 solution (water is added to 2.0 g of sodium chloride and 7.0 mL of hydrochloric acid to reach 1000 mL), 200 µL of JP-2 solution (500 mL of water is added to 500 mL of phosphate buffer (pH 6.8)) or 20 mmol/L sodium taurocholate (TCA)/JP-2 solution (JP-2 solution is added to 1.08 g of TCA to reach 100 mL) is independently added to each container. When total amount is dissolved after adding the test reagent, the compound of the present invention is added appropriately. After sealing and shaking at 37°C for 1 hour, solution is filtrated and 100µL of methanol is added to 100 µL of each filtrate to dilute two-fold. The dilution rate is changed as necessary. After checking that there is no bubble and deposit, the container is sealed and shaken. The compound of the present invention is measured using HPLC by absolute calibration curve method.

### Test Example 10: Visual solubility test

Approximately 5 mg of the compound is weighed into three microtubes, and each vehicle (injectable water, saline, 0.5% glucose solution) is added to attain a compound concentration of 20%. After stirring by vortex, the presence or absence of dissolution is visually confirmed. When the dissolution is present, the solubility in the vehicle is defined as >20%. Each vehicle (injectable water, saline, 0.5% glucose solution) is further added to these test reagents to prepare test reagents having a compound concentration of 10%. After stirring by vortex, the presence or absence of dissolution is visually confirmed. When the dissolution is present, the solubility in the vehicle is defined as 20% to 10%. Likewise, the test is conducted for 5% concentration, 2.5% concentration, and 1% concentration. When the dissolution is absent at the 1% concentration, the solubility in the vehicle is defined as <1%. The pH in the test reagents having the 1% concentration is measured and recorded.

### Test Example 11: pKa measurement (measurement method of capillary electrophoresis (CE))

This approach employs the capillary zone electrophoresis technique to separate each sample component on the basis of its free migration in a buffer containing an electrolyte.

After a compound solution is injected to fused silica capillaries packed with buffers adjusted to pH 2.5 to 11.5, high voltage (inlet side: +, outlet side: -) is applied to the capillaries so that the compound migrates at a speed reflecting an ionized state at the pH of the buffer (i.e., a positively charged compound migrates fast, while a negatively charged compound migrates slowly). The difference between the migration time of this compound and the migration time of a neutral molecule (DMSO) was plotted against pH, and pKa was calculated by fitting. The measurement conditions are described below.
Apparatus used: Beckman P/ACE system MDQ PDA
Carrier solution: pH 2.5 to 11.5 buffers (containing 10 vol% MeOH)
Sample solution: Blank DMSO (10 µL) + injectable water (90 µL) mixture
   Sample 10 mM DMSO stock solution (4 µL) + DMSO (6 µL) + injectable water (90 µL)

### (Method)

Capillary: Fused silica capillary (Beckman Coulter, Inc., inside diameter: 50 µm, full length: 30.2 cm, effective length: 20.0 cm)
Applied voltage: 10 kV (331 V/cm)
Applied pressure of air: 0.7 psi
Capillary temperature: 25°C
Electroosmotic flow marker: DMSO
Detection: Detection based on ultraviolet light absorption at multiple wavelengths
(measurement wavelength; 215 nm, 238 nm)
Sample injection: Pressurization method (0.5 psi, 5 sec)

### Formulation Example

The following Formulation Example s are only exemplified and not intended to limit the scope of the invention.

### Formulation Example 1: Tablets

The compounds of the present invention, lactose and calcium stearate are mixed. The mixture is crushed, granulated and dried to give a suitable size of granules. Next, calcium stearate is added to the granules, and the mixture is compressed and molded to give tablets.

### Formulation Example 2: Capsules

The compounds of the present invention, lactose and calcium stearate are mixed uniformly to obtain powder medicines in the form of powders or fine granules. The powder medicines are filled into capsule containers to give capsules.

### Formulation Example 3: Granules

The compounds of the present invention, lactose and calcium stearate are mixed uniformly and the mixture is compressed and molded. Then, it is crushed, granulated and sieved to give suitable sizes of granules.

### Formulation Example 4: Orally dispersing tablets

The compounds of the present invention and crystalline cellulose are mixed, granulated and tablets are made to give orally dispersing tablets.

### Formulation Example 5: Dry syrups

The compounds of the present invention and lactose are mixed, crushed, granulated and sieved to give suitable sizes of dry syrups.

### Formulation Example 6: Injections

The compounds of the present invention and phosphate buffer are mixed to give injections.

### Formulation Example 7: Infusions

The compounds of the present invention and phosphate buffer are mixed to give infusions.

### Formulation Example 8: Inhalations

The compound of the present invention and lactose are mixed and crushed finely to give inhalations.

### Formulation Example 9: Ointments

The compounds of the present invention and petrolatum are mixed to give ointments.

### Formulation Example 10: Patches

The compounds of the present invention and base such as adhesive plaster or the like are mixed to give patches.

### [INDUSTRIAL APPLICABILITY]

The compounds of the present invention have a wide antibacterial spectrum against, particularly, gram-negative bacteria, and are effective as antibacterial agents having high stability against β-lactamase-producing gram-negative bacteria. The compounds of the present invention also have good *in vivo* kinetics and high water solubility and are therefore effective, particularly, as injections or oral drugs.

## Claims

1. A compound represented by formula (I): 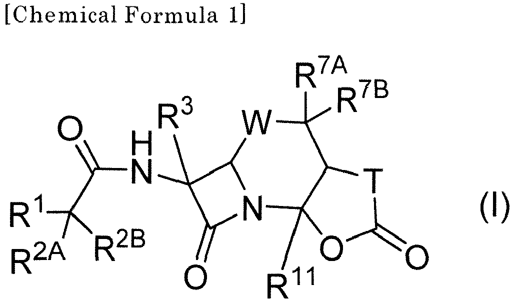 wherein
-W- is -S(=O)- or -S(=O)₂-;
-T- is -CR^{4A}R^{4B}- or -CR^{5A}R^{5B}-CR^{6A}R^{6B}-;
R^{4A}, R^{4B}, R^{5A}, R^{5B}, R^{6A} and R^{6B} are each independently a hydrogen atom, halogen, hydroxy, carboxy, acyl, acyloxy, sulfanyl, sulfo, phospho, cyano, nitro, ureido, amidino, guanidino, substituted or unsubstituted amino, substituted or unsubstituted carbamoyl, substituted or unsubstituted sulfamoyl, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted alkylsulfonyl, substituted or unsubstituted alkenylsulfonyl, substituted or unsubstituted alkynylsulfonyl, substituted or unsubstituted alkyloxycarbonyl, substituted or unsubstituted alkenyloxycarbonyl, substituted or unsubstituted alkynyloxycarbonyl, substituted or unsubstituted alkylsulfanyl, substituted or unsubstituted alkenylsulfanyl, substituted or unsubstituted alkynylsulfanyl, substituted or unsubstituted alkylsulfinyl, substituted or unsubstituted alkenylsulfinyl, substituted or unsubstituted alkynylsulfinyl, substituted or unsubstituted non-aromatic carbocyclyl, substituted or unsubstituted non-aromatic heterocyclyl, substituted or unsubstituted aromatic carbocyclyl, substituted or unsubstituted aromatic heterocyclyl, substituted or unsubstituted non-aromatic carbocyclyloxy, substituted or unsubstituted non-aromatic heterocyclyloxy, substituted or unsubstituted aromatic carbocyclyloxy, substituted or unsubstituted aromatic heterocyclyloxy, substituted or unsubstituted non-aromatic carbocyclyloxycarbonyl, substituted or unsubstituted non-aromatic heterocyclyloxycarbonyl, substituted or unsubstituted aromatic carbocyclyloxycarbonyl, substituted or unsubstituted aromatic heterocyclyloxycarbonyl, substituted or unsubstituted non-aromatic carbocyclylsulfanyl, substituted or unsubstituted non-aromatic heterocyclylsulfanyl, substituted or unsubstituted aromatic carbocyclylsulfanyl, substituted or unsubstituted aromatic heterocyclylsulfanyl, substituted or unsubstituted non-aromatic carbocyclylsulfinyl, substituted or unsubstituted non-aromatic heterocyclylsulfinyl, substituted or unsubstituted aromatic carbocyclylsulfinyl, substituted or unsubstituted aromatic heterocyclylsulfinyl, substituted or unsubstituted non-aromatic carbocyclylsulfonyl, substituted or unsubstituted non-aromatic heterocyclylsulfonyl, substituted or unsubstituted aromatic carbocyclylsulfonyl, or substituted or unsubstituted aromatic heterocyclylsulfonyl;
R¹ is substituted or unsubstituted carbocyclyl or substituted or unsubstituted heterocyclyl;
as for R^{2A} and R^{2B},
a) R^{2A} and R^{2B} are each independently a hydrogen atom, substituted or unsubstituted amino, sulfo, substituted or unsubstituted sulfamoyl, carboxy, substituted or unsubstituted alkyloxycarbonyl, substituted or unsubstituted carbamoyl, hydroxy, or carbonyloxy having a substituent, or
b) R^{2A} and R^{2B} are taken together to form substituted or unsubstituted methylidene, or substituted or unsubstituted hydroxyimino;
R³ is a hydrogen atom, -OCH₃ or -NH-CH(=O);
R¹¹ is carboxy or tetrazolyl; and
R^{7A} and R^{7B} are each independently a hydrogen atom or substituted or unsubstituted alkyl,
its ester form or a pharmaceutically acceptable salt thereof, or a hydrate thereof.

2. The compound according to claim 1, wherein R¹ is substituted or unsubstituted aromatic heterocyclyl, its ester form or a pharmaceutically acceptable salt thereof, or a hydrate thereof.

3. The compound according to claim 1, wherein R¹ is a group represented by the following formula: 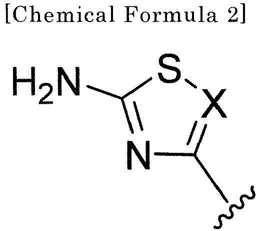 wherein X is CH, CCl, CF, or N,
its ester form or a pharmaceutically acceptable salt thereof, or a hydrate thereof.

4. The compound according to any one of claims 1 to 3, wherein R^{2A} and R^{2B} are taken together to be methylidene having a substituent of the formulas of: 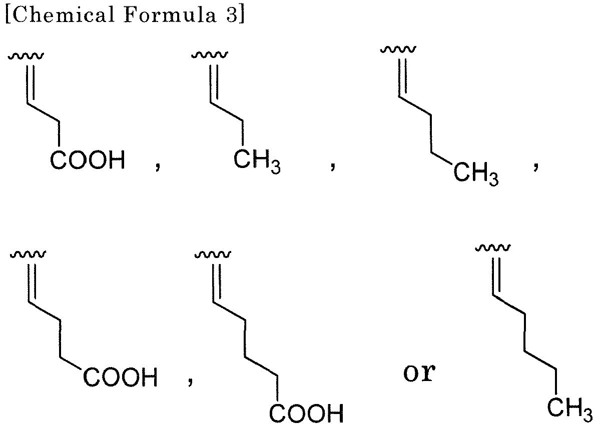 or substituted or unsubstituted hydroxyimino of the formula of: 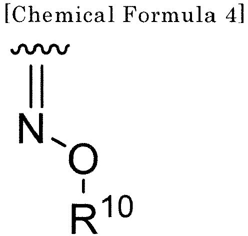 wherein R¹⁰ is a hydrogen atom, substituted or unsubstituted carbocyclyl, substituted or unsubstituted heterocyclyl, or substituted or unsubstituted alkyl,
its ester form or a pharmaceutically acceptable salt thereof, or a hydrate thereof.

5. The compound according to any one of claims 1 to 3, wherein R^{2A} and R^{2B} are taken together to be a group represented by the following formula: 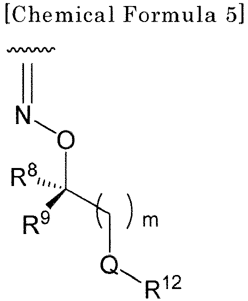 wherein R⁸ and R⁹ are each independently a hydrogen atom, halogen, hydroxy, carboxy, substituted or unsubstituted alkyl, substituted or unsubstituted carbocyclyl, or substituted or unsubstituted heterocyclyl, R⁸ and R⁹ are taken together to form substituted or unsubstituted methylidene, or R⁸ and R⁹ are taken together with the adjacent atoms to form substituted or unsubstituted non-aromatic carbocycle or substituted or unsubstituted non-aromatic heterocycle; Q is a single bond, substituted or unsubstituted carbocyclediyl or substituted or unsubstituted heterocyclediyl; m is an integer from 0 to 3; R¹² is a hydrogen atom, halogen, alkyl, haloalkyl, alkyloxy, carboxy, hydroxy, amino, sulfo, phospho, cyano, hydroxyiminomethyl, carbamoyl, alkyloxycarbonylamino, alkyloxycarbamoyl, hydroxycarbamoyl, ureido, alkylsulfonylamino, sulfamoyl, sulfamoylamino, alkylsulfonylcarbamoylamino, alkylsulfamoylcarbamoyl, substituted or unsubstituted carbocyclyl, substituted or unsubstituted heterocyclyl, or a substituted or unsubstituted quaternary ammonium group,
its ester form or a pharmaceutically acceptable salt thereof, or a hydrate thereof.

6. The compound according to any one of claims 1 to 5, wherein R³ is a hydrogen atom, its ester form or a pharmaceutically acceptable salt thereof, or a hydrate thereof.

7. The compound according to any one of claims 1 to 6, wherein -T- is -CR^{4A}R^{4B}-, and R^{4A} and R^{4B}, are each independently a hydrogen atom or substituted or unsubstituted alkyl, its ester form or a pharmaceutically acceptable salt thereof, or a hydrate thereof.

8. The compound according to any one of claims 1 to 7, wherein -W- is -S(=O)-, its ester form or a pharmaceutically acceptable salt thereof, or a hydrate thereof.

9. The compound according to any one of claims 1 to 7, wherein -W- is represented by the following formula: 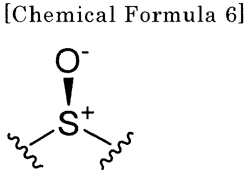 its ester form or a pharmaceutically acceptable salt thereof, or a hydrate thereof.

10. The compound according to any one of claims 1 to 7, wherein -W- is represented by the following formula: 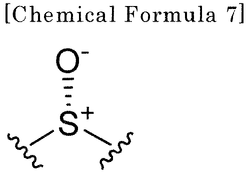 its ester form or a pharmaceutically acceptable salt thereof, or a hydrate thereof.

11. The compound according to any one of claims 1 to 7, wherein -W- is -S(=O)₂-, its ester form or a pharmaceutically acceptable salt thereof, or a hydrate thereof.

12. The compound according to any one of claims 1 to 11, wherein R¹¹ is carboxy, its ester form or a pharmaceutically acceptable salt thereof, or a hydrate thereof.

13. The compound according to any one of claims 1 to 12, wherein both of R^{7A} is a hydrogen atom and R^{7B} is a hydrogen atom, its ester form or a pharmaceutically acceptable salt thereof, or a hydrate thereof.

14. The compound according to claim 1, wherein the formula (I) is the following formula: 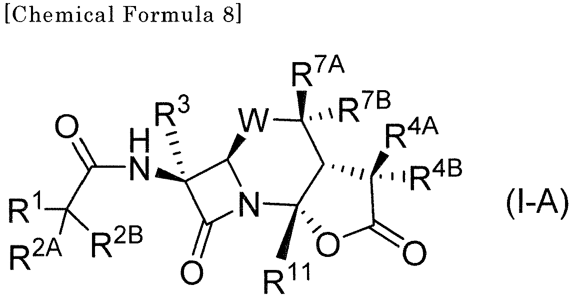 wherein -W- is represented by the following formula: 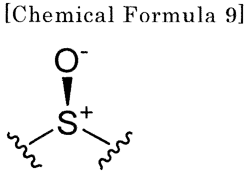 R^{4A} is a hydrogen atom; R^{4B}, is a hydrogen atom or methyl;
R¹ is represented by the following formula: 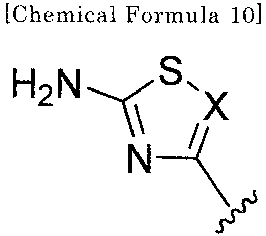 wherein X is CH;
each of R^{7A} is a hydrogen atom or alkyl; R^{7B} is a hydrogen atom;
R^{2A} and R^{2B} are taken together to be hydroxyimino or a group of the following formula: 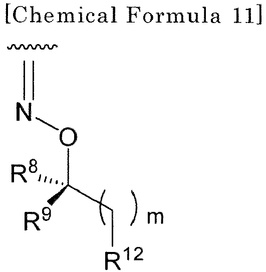 wherein R⁸ and R⁹ are each independently a hydrogen atom, halogen, hydroxy, carboxy, alkyl, or hydroxyalkyl, or R⁸ and R⁹ are taken together with the adjacent carbon atom to form substituted or unsubstituted non-aromatic carbocycle or substituted or unsubstituted non-aromatic heterocycle; m is an integer from 0 to 3; and R¹² is a hydrogen atom, carboxy, sulfo, carbamoyl, hydroxycarbamoyl, alkyloxycarbamoyl, or hydroxy;
R³ is a hydrogen atom; and
R¹¹ is carboxy,
its ester form or a pharmaceutically acceptable salt thereof, or a hydrate thereof.

15. The compound according to claim 1, which is any of the compounds I-001, I-005, I-007, I-009, I-015, I-023, I-024, I-026, I-027, I-047, I-048, I-049, I-052, 1-053, I-059, I-060, and I-061, its ester form or a pharmaceutically acceptable salt thereof, or a hydrate thereof.

16. A pharmaceutical composition comprising the compound according to any one of claims 1 to 15, its ester form or a pharmaceutically acceptable salt thereof, or a hydrate thereof.

17. The pharmaceutical composition according to claim 16, which has an antibacterial activity.

18. A method for treating or preventing diseases related to bacterial infections, which comprises administering the compound according to any one of claims 1 to 15, its ester form or a pharmaceutically acceptable salt thereof, or a hydrate thereof.

19. The compound according to any one of claims 1 to 15, its ester form or a pharmaceutically acceptable salt thereof, or a hydrate thereof for treating or preventing diseases related to bacterial infections.
